# EUROPEAN PATENT APPLICATION

(11) **EP 1 696 035 A2**
(43) Date of publication of application: **30.08.2006**
(21) Application number: 06115212.0
(22) Date of filing: 25.03.2003
(51) Int. Cl.: C12N 15/75, C12N 1/21, C07K 14/32, C12N 9/52

(54) **Improved bacillus host cell**

(30) Priority: 10.04.2002 DK 200200531
(62) Divisional of application: 03709681.5
(71) Applicant: Novozymes A/S, 2880 Bagsvaerd (DK)
(72) Inventor: Andersen, Jens, Toenne, 2850 Naerum (DK); Joergsen, Steen, Troels, 3450 Alleroed (DK); Rasmussen, Michael, Dolbjerg, 2625 Vallensbaek (DK); Olsen, Peter, Bjarke, 2100 Copenhagen (DK); Clausen, Ib, Groth, 3400 Hilleroed (DK)

(57) **Abstract**

A *Bacillus licheniformis* mutant host cell derived from a parent *B. licheniformis* host cell, which mutant host cell is mutated in one or more gene(s) encoding one or more polypeptide(s) having proteolytic activity which is at least 80% identical to one or more of the polypeptides shown in SEO ID NO's: 2 to 84, wherein the mutant host cell expresses at least 5% less of the one or more polypeptide(s) having proteolytic activity than the parent host cell, when they are cultivated under comparable conditions.

## Description

### TECHNICAL FIELD

*Bacillus sp.* are attractive hosts for the production of heterologous proteins due their ability to secrete proteins directly into the culture medium. They have a high capacity for protein secretion, are genetically highly amenable, nonpathogenic and free of endotoxins, and consequently a large variety of proteins from different organisms have been efficiently produced and secreted in *Bacillus sp. i.e.* in *Bacillus licheniformis.*
In the highly competitive biotech industry, even slightly improved *Bacillus* host cells are in demand, which may provide more attractive production systems e.g. in terms of product stability, product purity etc., or may even just be alternative production systems.

### BACKGROUND

Many industrial products of commercial interest can be produced biologically in *Bacillus sp.* host cells e.g. heterologous polypeptides, amino acids, carbohydrates etc. Contaminant polypeptides native to the *Bacillus* production host cell however are also produced along with the product, some of which may be secreted into the culture medium, and some of which remain within the cell or remain associated with the cell membrane. Such contaminants often have to be removed or inhibited in order to ensure e.g. the stability or shelf-life of the product, or to obtain a sufficient purity of the product.
Production of heterologous polypeptides in *Bacillus licheniformis* in particular is often impaired by the presence of contaminant native polypeptides having proteolytic activity. Different approaches have been exploited in order to by-pass the problem of extracellular degradation including deletion of the genes encoding known proteases (Sloma *et al.,* 1989; Wu *et al.,* 1991; Sloma *et al.,* 1991) or addition of protease inhibitors during the fermentation (Simonen & Palva, 1993).

### SUMMARY

A problem to be solved by the present invention is how to provide an improved *Bacillus licheniformis* host cell having a reduced expression of one or more native polypeptide(s) having proteolytic activity. The present invention provides a solution to this problem by identifying a number of hitherto unknown polypeptides from *Bacillus licheniformis* having proteolytic activity, and polynucleotides encoding these, thereby allowing the construction of a mutant host cell which has a reduced expression of one or more of these polypeptide(s) and/or its closely related homologues.

Accordingly, in a first aspect the invention relates to a *Bacillus licheniformis* mutant host cell derived from a parent *B. licheniformis* host cell, which mutant host cell is mutated in one or more gene(s) encoding one or more polypeptide(s) having proteolytic activity which is at least 80% identical to one or more of the polypeptides shown in SEQ ID NO's: 2 to 64 (both included), or in SEQ ID NO's: 2 to 84 (both included), preferably at least 85% identical, more preferably at least 90% identical, still more preferably at least 95% identical, and most preferably at least 97% identical to one or more of the polypeptides shown in ID NO's: 2 to 64 (both included), or in SEQ ID NO's: 2 to 84 (both included), wherein the mutant host cell expresses at least 5% less of the one or more polypeptide(s) having proteolytic activity than the parent host cell, when they are cultivated under comparable conditions. Preferably the mutant host cell expresses at least 10% less, more preferably at least 20% less, still more preferably at least 30% less, even more preferably at least 40% less, yet more preferably at least 50% less, or at least 60% less, or at least 70% less, or at least 80%, or most preferably at least 90% less of the one or more polypeptide(s) having proteolytic activity than the parent host cell, when they are cultivated under comparable conditions. Most preferably the mutant host cell expresses absolutely nothing of the one or more polypeptide(s) having proteolytic activity.

Comparable conditions of cultivation must be used in order to compare the expression level of the one or more polypeptide(s) having proteolytic activity in a mutant host cell of the invention with that in a parent host cell. They are cultivated separately under identical conditions in identical setups, of course allowing for the usual standard deviations of the operating parameters normally associated with growth experiments, such as temperature control etc. The quantification of the expression level of the one or more polypeptide(s) is done by standard text-book assay techniques as known in the art e.g. mRNA quantification or immuno-based assays.

In a second aspect the invention relates to a process for producing at least one product of interest in a *Bacillus licheniformis* mutant host cell, comprising cultivating a *B.licheniformis* mutant host cell as defined in the previous aspect in a suitable medium, whereby the said product is produced.

Finally, an aspect of the invention relates to a use of a *Bacillus licheniformis* mutant host cell as defined in the first aspect for producing at least one product of interest comprising cultivating the mutant host cell in a suitable medium whereby the said product is produced.

### DEFINITIONS

Nucleic acid construct: When used herein, the term "nucleic acid construct" means a nucleic acid molecule, either single- or double-stranded, which is isolated from a naturally occurring gene or which has been modified to contain segments of nucleic acids in a manner that would not otherwise exist in nature. The term nucleic acid construct is synonymous with the term "expression cassette" when the nucleic acid construct contains the control sequences required for expression of a coding sequence of the present invention.

Control sequence: The term "control sequences" is defined herein to include all components, which are necessary or advantageous for the expression of a polypeptide of the present invention. Each control sequence may be native or foreign to the nucleotide sequence encoding the polypeptide. Such control sequences include, but are not limited to, a leader, polyadenylation sequence, propeptide sequence, promoter, signal peptide sequence, and transcription terminator. At a minimum, the control sequences include a promoter, and transcriptional and translational stop signals. The control sequences may be provided with linkers for the purpose of introducing specific restriction sites facilitating ligation of the control sequences with the coding region of the nucleotide sequence encoding a polypeptide.

Operably linked: The term "operably linked" is defined herein as a configuration in which a control sequence is appropriately placed at a position relative to the coding sequence of the DNA sequence such that the control sequence directs the expression of a polypeptide.

Coding sequence: When used herein the term "coding sequence" is intended to cover a nucleotide sequence, which directly specifies the amino acid sequence of its protein product. The boundaries of the coding sequence are generally determined by an open reading frame, which usually begins with the ATG start codon. The coding sequence typically include DNA, cDNA, and recombinant nucleotide sequences.

Expression: In the present context, the term "expression" includes any step involved in the production of the polypeptide including, but not limited to, transcription, post-transcriptional modification, translation, post-translational modification, and secretion.

Expression vector: In the present context, the term "expression vector" covers a DNA molecule, linear or circular, that comprises a segment encoding a polypeptide of the invention, and which is operably linked to additional segments that provide for its transcription.

### DETAILED DISCLOSURE

A *Bacillus licheniformis* mutant host cell derived from a parent *B. licheniformis* host cell, which mutant host cell is mutated in one or more gene(s) encoding one or more polypeptide(s) having proteolytic activity which is at least 80% identical to one or more of the polypeptides shown in SEQ ID NO's: 2 to 64 (both included), or in SEQ ID NO's: 2 to 84 (both included), wherein the mutant host cell expresses at least 5% less of the one or more polypeptide(s) having proteolytic activity than the parent host cell, when they are cultivated under comparable conditions.

The term "parent host cell" in the context of the present invention means a cell which is genetically identical, or isogenic, to the progeny mutant or mutant cell of the present invention, except for the mutated one or more gene(s) encoding one or more polypeptide(s) having proteolytic activity in said mutant.

The degree of identity, or %-identity of polypeptide sequences can suitably be investigated by aligning the sequences using a computer program known in the art, such as "GAP" provided in the GCG program package (Program Manual for the Wisconsin Package, Version 8, August 1994, Genetics Computer Group, 575 Science Drive, Madison, Wisconsin, USA 53711)(Needleman, S.B. and Wunsch, C.D., (1970), Journal of Molecular Biology, 48, 443-453). Using GAP with the following settings for DNA sequence comparison: GAP creation penalty of 5.0 and GAP extension penalty of 0.3".

An object of the present invention is to provide a bacterial culture free of native bacterial proteases so as to reduce the product purification to a minimum, and to achieve better product stability and higher product yield. This may be done according to the invention by reducing or even completely abolishing the expression of one or more gene(s) encoding a native polypeptide(s) having proteolytic activity via mutagenisation of that (those) gene(s). One of the very well-known method of ensuring that a gene is not expressed into an active polypeptide within a cell is simply to delete or partially delete the encoding gene. Many techniques have been described in the art on how to specifically delete or partially delete one or more gene(s) in the genome of a cell, and certainly from the genome of a *Bacillus licheniformis* cell (see e.g. Novozymes A/S WO 01/90393, Novozymes A/S WO 02/00907). Accordingly, a preferred embodiment of the present invention relates to a host cell of the first aspect, which is mutated by a partial or complete deletion of the one or more gene(s) encoding the one or more polypeptide(s) having proteolytic activity.

A preferred embodiment of the present invention relates to a host cell of the first aspect, which is mutated in two or more genes encoding two or more polypeptides having proteolytic activity.

The product of interest to be produced by the mutant host cell of the first aspect may be one or more polypeptide(s) encoded by one or more heterologous gene(s). Consequently, a preferred embodiment of the present invention relates to a host cell of the first aspect, which comprises one or more heterologous gene(s) encoding one or more heterologous polypeptide(s).

In the industrial production of polypeptides it is of interest to achieve a product yield as high as possible. One way to increase the yield is to increase the copy number of a gene encoding a polypeptide of interest. This can be done by placing the gene on a high copy number plasmid. However, plasmids are unstable and are often lost from the host cells if there is no selective pressure during the cultivation of the host cells. Another way to increase the copy number of the gene of interest is to integrate it into the host cell chromosome in multiple copies. Integration of two genes has been described in WO 91/09129 and WO 94/14968 (Novozymes A/S) the content of which is hereby incorporated by reference. A preferred embodiment of the present invention relates to a host cell of the first aspect, wherein the heterologous gene(s) is present in at least two copies, preferably at least 4 copies, and most preferably at least 6 copies. In another embodiment the heterologous gene(s) is present in at least ten copies. If carried on a plasmid the gene(s) may be present in several hundred copies per cell, so in a still further embodiment of the present invention the heterologous gene(s) is present in at least 100 copies.

Integration of two genes closely spaced in anti-parallel tandem to achieve better stability has been described in WO 99/41358 (Novozymes A/S) the content of which is hereby incorporated by reference, as well as the stable chromosomal multi-copy integration of genes described in WO 02/00907 (Novozymes A/S) the content of which is incorporated herein by reference. A preferred embodiment of the present invention relates to a host cell of the first aspect, wherein the heterologous gene(s) are stably integrated into the genome of the cell.

Selection of chromosomal integrant has for convenience resulted in the use of selectable markers such as antibiotic resistance markers. However it is desirable if possible to avoid the use of antibiotic marker genes. WO 01 /90393 discloses a method for the integration of a gene in the chromosome of a host cell without leaving antibiotic resistance markers behind in the strain, the content of which is hereby incorporated by reference A preferred embodiment of the present invention relates to a host cell of the first aspect wherein the heterologous gene(s) is integrated into the genome of the cell without leaving any antibiotic resistance marker gene(s) at the site of integration.

The present invention also relates to nucleic acid constructs comprising a nucleotide sequence encoding a product of interest, which may be operably linked to one or more control sequences that direct the expression of the coding sequence in a suitable host cell under conditions compatible with the control sequences.

A nucleotide sequence encoding a polypeptide ofinterest may be manipulated in a variety of ways to provide for expression of the polypeptide. Manipulation of the nucleotide sequence prior to its insertion into a vector may be desirable or necessary depending on the expression vector. The techniques for modifying nucleotide sequences utilizing recombinant DNA methods are well known in the art.

Other ways of increasing the product yield would be to increase promoter activity of the specific promoter regulating the expression of a specific gene of interest. Also a more general increase in the activity of several promoters at the same time could lead to an improved product yield. The control sequence may be an appropriate promoter sequence, a nucleotide sequence which is recognized by a host cell for expression of the nucleotide sequence. The promoter sequence contains transcriptional control sequences, which mediate the expression of the polypeptide. The promoter may be any nucleotide sequence which shows transcriptional activity in the host cell of choice including mutant, truncated, and hybrid promoters, and may be obtained from genes encoding extracellular or intracellular polypeptides either homologous or heterologous to the host cell.

Examples of suitable promoters for directing the transcription of the nucleic acid constructs of the present invention, especially in a bacterial host cell, are the promoters obtained from the E. coli lac operon, Streptomyces coelicolor agarase gene (dagA), Bacillus subtilis levansucrase gene (sacB), Bacillus licheniformis alpha-amylase gene (amyL), Bacillus stearothermophilus maltogenic amylase gene (amyM), Bacillus amyloliquefaciens alpha-amylase gene (amyQ), Bacillus licheniformis penicillinase gene (penP), Bacillus subtilis xylA and xylB genes, and prokaryotic beta-lactamase gene (Villa-Kamaroff et al., 1978, Proceedings of the National Academy of Sciences USA 75: 3727-3731), as well as the tac promoter (DeBoer et al., 1983, Proceedings of the National Academy of Sciences USA 80: 21-25). Further promoters are described in "Useful proteins from recombinant bacteria" in Scientific American, 1980, 242: 74-94; and in Sambrook et al., 1989, supra.

Other useful promoters are described in WO 93/10249, WO 98/07846, and WO 99/43835 (Novozymes A/S) the contents of which are incorporated fully herein by reference. A preferred embodiment of the present invention relates to a host cell of the first aspect, wherein the heterologous gene(s) are transcribed from a heterologous promoter or from an artificial promoter.

The control sequence may also be a suitable transcription terminator sequence, a sequence recognized by a host cell to terminate transcription. The terminator sequence is operably linked to the 3' terminus of the nucleotide sequence encoding the polypeptide. Any terminator which is functional in the host cell of choice may be used in the present invention.

The control sequence may also be a suitable leader sequence, a nontranslated region of an mRNA which is important for translation by the host cell. The leader sequence is operably linked to the 5' terminus of the nucleotide sequence encoding the polypeptide. Any leader sequence that is functional in the host cell of choice may be used in the present invention.

The control sequence may also be a polyadenylation sequence, a sequence operably linked to the 3' terminus of the nucleotide sequence and which, when transcribed, is recognized by the host cell as a signal to add polyadenosine residues to transcribed mRNA. Any polyadenylation sequence which is functional in the host cell of choice may be used in the present invention.

The control sequence may also be a signal peptide coding region that codes for an amino acid sequence linked to the amino terminus of a polypeptide and directs the encoded polypeptide into the cell's secretory pathway. The 5' end of the coding sequence of the nucleotide sequence may inherently contain a signal peptide coding region naturally linked in translation reading frame with the segment of the coding region which encodes the secreted polypeptide. Alternatively, the 5' end of the coding sequence may contain a signal peptide coding region which is foreign to the coding sequence. The foreign signal peptide coding region may be required where the coding sequence does not naturally contain a signal peptide coding region. Alternatively, the foreign signal peptide coding region may simply replace the natural signal peptide coding region in order to enhance secretion of the polypeptide. However, any signal peptide coding region which directs the expressed polypeptide into the secretory pathway of a host cell of choice may be used in the present invention.

Effective signal peptide coding regions for bacterial host cells are the signal peptide coding regions obtained from the genes for Bacillus NCIB 11837 maltogenic amylase, Bacillus stearothermophilus alpha-amylase, Bacillus licheniformis subtilisin, Bacillus licheniformis beta-lactamase, Bacillus stearothermophilus neutral proteases (nprT, nprS, nprM), and Bacillus subtilis prsA. Further signal peptides are described by Simonen and Palva, 1993, Microbiological Reviews 57: 109-137.

The control sequence may also be a propeptide coding region that codes for an amino acid sequence positioned at the amino terminus of a polypeptide. The resultant polypeptide is known as a proenzyme or propolypeptide (or a zymogen in some cases). A propolypeptide is generally inactive and can be converted to a mature active polypeptide by catalytic or autocatalytic cleavage of the propeptide from the propolypeptide. The propeptide coding region may be obtained from the genes for Bacillus subtilis alkaline protease (aprE), Bacillus subtilis neutral protease (nprT), Saccharomyces cerevisiae alpha-factor, Rhizomucor miehei aspartic proteinase, and Myceliophthora thermophila laccase (WO 95/33836).

Where both signal peptide and propeptide regions are present at the amino terminus of a polypeptide, the propeptide region is positioned next to the amino terminus of a polypeptide and the signal peptide region is positioned next to the amino terminus of the propeptide region.

It may also be desirable to add regulatory sequences which allow the regulation of the expression of the polypeptide relative to the growth of the host cell. Examples of regulatory systems are those which cause the expression of the gene to be turned on or off in response to a chemical or physical stimulus, including the presence of a regulatory compound. Regulatory systems in prokaryotic systems include the lac, tac, and trp operator systems. In yeast, the ADH2 system or GAL1 system may be used. In eukaryotic systems, these include the dihydrofolate reductase gene which is amplified in the presence of methotrexate, and the metallothionein genes which are amplified with heavy metals. In these cases, the nucleotide sequence encoding the polypeptide would be operably linked with the regulatory sequence.

The present invention also relates to recombinant expression vectors comprising the nucleic acid construct of the invention. The various nucleotide and control sequences described above may be joined together to produce a recombinant expression vector which may include one or more convenient restriction sites to allow for insertion or substitution of the nucleotide sequence encoding the polypeptide at such sites. Alternatively, the nucleotide sequence of the present invention may be expressed by inserting the nucleotide sequence or a nucleic acid construct comprising the sequence into an appropriate vector for expression. In creating the expression vector, the coding sequence is located in the vector so that the coding sequence is operably linked with the appropriate control sequences for expression.

The recombinant expression vector may be any vector (e.g., a plasmid or virus) which can be conveniently subjected to recombinant DNA procedures and can bring about the expression of the nucleotide sequence. The choice of the vector will typically depend on the compatibility of the vector with the host cell into which the vector is to be introduced. The vectors may be linear or closed circular plasmids.

The vector may be an autonomously replicating vector, i.e., a vector which exists as an extrachromosomal entity, the replication of which is independent of chromosomal replication, e.g., a plasmid, an extrachromosomal element, a minichromosome, or an artificial chromosome.

The vector may contain any means for assuring self-replication. Alternatively, the vector may be one which, when introduced into the host cell, is integrated into the genome and replicated together with the chromosome(s) into which it has been integrated. Furthermore, a single vector or plasmid or two or more vectors or plasmids which together contain the total DNA to be introduced into the genome of the host cell, or a transposon may be used.

The vectors of the present invention preferably contain one or more selectable markers which permit easy selection of transformed cells. A selectable marker is a gene the product of which provides for biocide or viral resistance, resistance to heavy metals, prototrophy to auxotrophs, and the like.

Examples of bacterial selectable markers are the dal genes from Bacillus subtilis or Bacillus licheniformis, or markers which confer antibiotic resistance such as ampicillin, kanamycin, chloramphenicol or tetracycline resistance.

The vectors of the present invention preferably contain an element(s) that permits stable integration of the vector into the host cell's genome or autonomous replication of the vector in the cell independent of the genome.

For integration into the host cell genome, the vector may rely on the nucleotide sequence encoding the polypeptide or any other element of the vector for stable integration of the vector into the genome by homologous or nonhomologous recombination. Alternatively, the vector may contain additional nucleotide sequences for directing integration by homologous recombination into the genome of the host cell. The additional nucleotide sequences enable the vector to be integrated into the host cell genome at a precise location(s) in the chromosome(s). To increase the likelihood of integration at a precise location, the integrational elements should preferably contain a sufficient number of nucleotides, such as 100 to 1,500 base pairs, preferably 400 to 1,500 base pairs, and most preferably 800 to 1,500 base pairs, which are highly homologous with the corresponding target sequence to enhance the probability of homologous recombination. The integrational elements may be any sequence that is homologous with the target sequence in the genome of the host cell. Furthermore, the integrational elements may be non-encoding or encoding nucleotide sequences. On the other hand, the vector may be integrated into the genome of the host cell by non-homologous recombination.

For autonomous replication, the vector may further comprise an origin of replication enabling the vector to replicate autonomously in the host cell in question. Examples of bacterial origins of replication are the origins of replication of plasmids pBR322, pUC19, pACYC177, and pACYC184 permitting replication in E. coli, and pUB110, pE194, pTA1060, and pAMβ1 permitting replication in Bacillus. The origin of replication may be one having a mutation which makes its functioning temperature-sensitive in the host cell (see, e.g., Ehrlich, 1978, Proceedings of the National Academy of Sciences USA 75: 1433).

More than one copy of a nucleotide sequence of the present invention may be inserted into the host cell to increase production of the gene product. An increase in the copy number of the nucleotide sequence can be obtained by integrating at least one additional copy of the sequence into the host cell genome or by including an amplifiable selectable marker gene with the nucleotide sequence where cells containing amplified copies of the selectable marker gene, and thereby additional copies of the nucleotide sequence, can be selected for by cultivating the cells in the presence of the appropriate selectable agent.

The procedures used to ligate the elements described above to construct the recombinant expression vectors of the present invention are well known to one skilled in the art (see, e.g., Sambrook et al., 1989, supra).

The introduction of a vector into a bacterial host cell may, for instance, be effected by protoplast transformation (see, e.g., Chang and Cohen, 1979, Molecular General Genetics 168: 111-115), using competent cells (see, e.g., Young and Spizizin, 1961, Journal of Bacteriology 81: 823-829, or Dubnau and Davidoff-Abelson, 1971, Journal of Molecular Biology 56: 209-221), electroporation (see, e.g., Shigekawa and Dower, 1988, Biotechniques 6: 742-751), or conjugation (see, e.g., Koehler and Thorne, 1987, Journal of Bacteriology 169: 5771-5278).

A preferred embodiment of the present invention relates to a host cell of the first aspect, wherein the heterologous gene(s) are comprised in an operon, preferably a polycistronic operon. The term "operon" in the context of the present invention means a polynucleotide comprising several genes that are clustered and perhaps even transcribed together into a polycistronic mRNA, e.g. genes coding for the enzymes of a metabolic pathway. The transcription of an operon may be initiated at a promoter region and controlled by a neighboring regulatory gene, which encodes a regulatory protein, which in turn binds to the operator sequence in the operon to respectively inhibit or enhance the transcription. The gene or the operon can be carried on a suitable plasmid that can be stably maintained, e.g. capable of stable autonomous replication in the host cell (the choice of plasmid will typically depend on the compatibility of the plasmid with the host cell into which the plasmid is to be introduced) or it can be carried on the chromosome of the host. The said gene may be endogenous to the host cell in which case the product of interest is a protein naturally produced by the host cell and in most cases the gene will be in it normal position on the chromosome. If the gene encoding the product of interest is an exogenous gene, the gene could either be carried on a suitable plasmid or it could be integrated on the host chromosome. In one embodiment of the invention the eubacterium is a recombinant eubacterium. Also the product of interest may in another embodiment be a recombinant protein.

The product of interest is any gene product or product of a metabolic pathway which is industrially useful and which can be produced in a bacterial cell such as a *B. licheniformis.*

In one preferred embodiment, the heterologous polypeptide(s) is an antimicrobial peptide, or a fusion peptide comprising a peptide part which in its native form has antimicrobial activity.

In another preferred embodiment, the heterologous polypeptide(s) has biosynthetic activity and produces a compound or an intermediate of interest.

Yet another embodiment relates to a host cell of the first aspect, wherein the compound or intermediate of interest comprises vitamins, amino acids, antibiotics, carbohydrates, or surfactants, and preferably the carbohydrates comprise hyaluronic acid.

In one embodiment the heterologous polypeptide(s) is an enzyme, particularly the enzyme is an enzyme of a class selected from the group of enzyme classes consisting of oxidoreductases (EC 1), transferases (EC 2), hydrolases (EC 3), lyases (EC 4), isomerases (EC 5), and ligases (EC 6). Preferably the enzyme is an enzyme with an activity selected from the group consisting of aminopeptidase, amylase, amyloglucosidase, mannanase, carbohydrase, carboxypeptidase, catalase, cellulase, chitinase, cutinase, cyclodextrin glycosyltransferase, deoxyribonuclease, esterase, galactosidase, beta-galactosidase, glucoamylase, glucose oxidase, glucosidase, haloperoxidase, hemicellulase, invertase, isomerase, laccase, ligase, lipase, lyase, mannosidase, oxidase, pectinase, peroxidase, phytase, phenoloxidase, polyphenoloxidase, protease, ribonuclease, transferase, transglutaminase, or xylanase. Preferably the enzyme is an amylase or a mannanase.

A second aspect of the invention relates to a process for producing at least one product of interest in a *Bacillus licheniformis* mutant host cell, comprising cultivating a *B.licheniformis* mutant host cell as defined in the first aspect of the invention in a suitable medium, whereby the said product is produced. One embodiment relates to a process of the second aspect, further comprising isolating or purifying the product of interest. Suitable media for the cultivation is described below as well as methods for the purification or isolation of the produced product which is an optional additional step to the process of the present invention.

In the production methods of the present invention, the cells are cultivated in a nutrient medium suitable for production of the polypeptide using methods known in the art. For example, the cell may be cultivated by shake flask cultivation, small-scale or large-scale fermentation (including continuous, batch, fed-batch, or solid state fermentations) in laboratory or industrial fermentors performed in a suitable medium and under conditions allowing the polypeptide to be expressed and/or isolated. The cultivation takes place in a suitable nutrient medium comprising carbon and nitrogen sources and inorganic salts, using procedures known in the art. Suitable media are available from commercial suppliers or may be prepared according to published compositions (e.g., in catalogues of the American Type Culture Collection). If the polypeptide is secreted into the nutrient medium, the polypeptide can be recovered directly from the medium. If the polypeptide is not secreted, it can be recovered from cell lysates.

The medium used to culture the cells may be any conventional medium suitable for growing the host cells, such as minimal or complex media containing appropriate supplements. Suitable media are available from commercial suppliers or may be prepared according to published recipes (e.g. in catalogues of the American Type Culture Collection). The media are prepared using procedures known in the art (see, e.g., references for bacteria and yeast; Bennett, J.W. and LaSure, L., editors, More Gene Manipulations in Fungi, Academic Press, CA, 1991).

The polypeptides may be detected using methods known in the art that are specific for the polypeptides. These detection methods may include use of specific antibodies, formation of an enzyme product, or disappearance of an enzyme substrate. For example, an enzyme assay may be used to determine the activity of the polypeptide as described herein.

The resulting polypeptide may be recovered by methods known in the art. For example, the polypeptide may be recovered from the nutrient medium by conventional procedures including, but not limited to, centrifugation, filtration, extraction, spray-drying, evaporation, or precipitation.

The polypeptides of the present invention may be purified by a variety of procedures known in the art including, but not limited to, chromatography (e.g., ion exchange, affinity, hydrophobic, chromatofocusing, and size exclusion), electrophoretic procedures (e.g., preparative isoelectric focusing), differential solubility (e.g., ammonium sulfate precipitation), SDS-PAGE, or extraction (see, *e.g., Protein Purification,* J.-C. Janson and Lars Ryden, editors, VCH Publishers, New York, 1989).

A third aspect of the present invention relates to the use of a *Bacillus licheniformis* mutant host cell as defined in the first aspect for producing at least one product of interest comprising cultivating the mutant host cell in a suitable medium whereby the said product is produced, and optionally isolating or purifying the produced product.

## Claims

1. A *Bacillus licheniformis* mutant host cell derived from a parent *B*. *licheniformis* host cell, which mutant host cell is mutated in one or more gene(s) encoding one or more polypeptide(s) having proteolytic activity which is at least 80% identical to one or more of the polypeptides shown in SEQ ID NO's: 2 to 84, wherein the mutant host cell expresses at least 5% less of the one or more polypeptide(s) having proteolytic activity than the parent host cell, when they are cultivated under comparable conditions.

2. The host cell according to claim 1, which is mutated by a partial or complete deletion of the one or more gene(s) encoding the one or more polypeptide(s) having proteolytic activity.

3. The host cell according to any of claims 1 - 2, which is mutated in two or more genes encoding two or more polypeptides having proteolytic activity.

4. The host cell according to any of claims 1 - 3, which comprises one or more heterologous gene(s) encoding one or more heterologous polypeptide(s).

5. The host cell according to claim 4, wherein the heterologous gene(s) is present in at least two copies.

6. The host cell according to claim 4 or 5, wherein the heterologous gene(s) are stably integrated into the genome of the cell.

7. The host cell according to any of claims 4 - 6, wherein the heterologous gene(s) is integrated into the genome of the cell without leaving any antibiotic resistance marker genes at the site of integration.

8. The host cell according to any of claims 4 - 7, wherein the heterologous gene(s) are transcribed from a heterologous promoter or from an artificial promoter.

9. The host cell according to any of claims 4-8, wherein the heterologous gene(s) are comprised in an operon, preferably a polycistronic operon.

10. The host cell according to any of claims 4 - 9, wherein the heterologous polypeptide(s) is an antimicrobial peptide, or a fusion peptide comprising a peptide part which in its native form has antimicrobial activity.

11. The host cell according to any of claims 4 - 9, wherein the heterologous polypeptide(s) has biosynthetic activity and produces a compound or an intermediate of interest.

12. The host cell according to claim 11, wherein the compound or intermediate of interest comprises vitamins, amino acids, antibiotics, carbohydrates, or surfactants.

13. The host cell according to claim 12, wherein the carbohydrates comprise hyaluronic acid.

14. The host cell according to any of claims 4 - 9, wherein the heterologous polypeptide(s) is an enzyme, preferably a secreted enzyme.

15. The host cell according to claim 14, wherein the enzyme is an enzyme of a class selected from the group of enzyme classes consisting of oxidoreductases (EC 1), transferases (EC 2), hydrolases (EC 3), lyases (EC 4), isomerases (EC 5), and ligases (EC 6).

16. The host cell according to claim 15, wherein the enzyme is an enzyme with an activity selected from the group of enzyme activities consisting of aminopeptidase, amylase, amyloglucosidase, mannanase, carbohydrase, carboxypeptidase, catalase, cellulase, chitinase, cutinase, cyclodextrin glycosyltransferase, deoxyribonuclease, esterase, galactosidase, beta-galactosidase, glucoamylase, glucose oxidase, glucosidase, haloperoxidase, hemicellulase, invertase, isomerase, laccase, ligase, lipase, lyase, mannosidase, oxidase, pectinase, peroxidase, phytase, phenoloxidase, polyphenoloxidase, protease, ribonuclease, transferase, transglutaminase, and xylanase.

17. The host cell according to claim 16, wherein the enzyme is an amylase or a mannanase.

18. A process for producing at least one product of interest in a *Bacillus licheniformis* mutant host cell, comprising cultivating a *B.licheniformis* mutant host cell as defined in any of the claims 1 - 17 in a suitable medium, whereby the said product is produced.

19. The process according to claim 18, further comprising isolating or purifying the product of interest.

20. A use of a *Bacillus licheniformis* mutant host cell as defined in any of the claims 1 - 17 for producing at least one product of interest comprising cultivating the mutant host cell in a suitable medium whereby the said product is produced.

21. The use according to claim 20 further comprising isolating or purifying the product of interest.


SEQUENCE LISTING

<110>  Novozymes A/S
       Rasmussen, Michael Dolberg
       Jørgensen, Steen Troels
       Olesen, Peter Bjarke
       Andersen, Jens Tønne

<120>  Improved Bacillus host cell

<130>  10196.204

<160>  84

<170>  PatentIn version 3.2

<210>  1
<211>  4325
<212>  DNA
<213>  Bacillus licheniformis

<220>
<221>  CDS
<222>  (3)..(3836)

<400>  1
   tg gaa caa gta gcg gct ttt ccg gaa gta gaa aaa gtg ctt ccg aac      47
      Glu Gln Val Ala Ala Phe Pro Glu Val Glu Lys Val Leu Pro Asn
      1               5                   10                  15
   gaa aaa agg cag ctg atc aaa ccg act aaa cag tct gtg aaa aaa tca      95
   Glu Lys Arg Gln Leu Ile Lys Pro Thr Lys Gln Ser Val Lys Lys Ser
                   20                  25                  30
   gca gcc aag gat gaa aaa gaa atc gaa tgg aat atc aat cga gtc gac      143
   Ala Ala Lys Asp Glu Lys Glu Ile Glu Trp Asn Ile Asn Arg Val Asp
               35                  40                  45
   gca cca aaa gcc tgg aaa ttg gga tat gac gga tcg ggc acg gtt gtc      191
   Ala Pro Lys Ala Trp Lys Leu Gly Tyr Asp Gly Ser Gly Thr Val Val
           50                  55                  60
   gcg tcg att gat acg ggc gtg cag tgg gat cat ccg gcc ttg aaa gaa      239
   Ala Ser Ile Asp Thr Gly Val Gln Trp Asp His Pro Ala Leu Lys Glu
       65                  70                  75
   aaa tac cgc gga tat gac ccg cag cat ccc gat cag cct aac cac gaa      287
   Lys Tyr Arg Gly Tyr Asp Pro Gln His Pro Asp Gln Pro Asn His Glu
   80                  85                  90                  95
   ttc agt tgg tat gat gcg gtt tca ggc gct tct gaa cca ttt gac gat      335
   Phe Ser Trp Tyr Asp Ala Val Ser Gly Ala Ser Glu Pro Phe Asp Asp
                   100                 105                 110
   ctg gag cac ggc acg cat gta acc ggg acg atg gtc ggc tca gag cct      383
   Leu Glu His Gly Thr His Val Thr Gly Thr Met Val Gly Ser Glu Pro
               115                 120                 125
   gac ggc caa aac caa atc ggc gtg gcg ccg ggt gcg aag tgg att gct      431
   Asp Gly Gln Asn Gln Ile Gly Val Ala Pro Gly Ala Lys Trp Ile Ala
           130                 135                 140
   gtg aaa gct ttt tca gat gac ggc ggt acg gac gaa gac ctt att gca      479
   Val Lys Ala Phe Ser Asp Asp Gly Gly Thr Asp Glu Asp Leu Ile Ala
       145                 150                 155
   gca ggt gaa tgg att ttg gcg cca aaa gat aaa gac ggc aac ccg cac      527
   Ala Gly Glu Trp Ile Leu Ala Pro Lys Asp Lys Asp Gly Asn Pro His
   160                 165                 170                 175
   cct gaa atg gcg ccg gat gtc gtc aat aac tca tgg tca ggg gga gcc     575
   Pro Glu Met Ala Pro Asp Val Val Asn Asn Ser Trp Ser Gly Gly Ala
                   180                 185                 190
   ggg att gat gag ttt tac agg gat atc gta aaa gcg tgg cga gct gcc     623
   Gly Ile Asp Glu Phe Tyr Arg Asp Ile Val Lys Ala Trp Arg Ala Ala
               195                 200                 205
   ggc atc ttt cct gag ttc tct gcg ggg aac gtc gat ttc gcc aat ccg     671
   Gly Ile Phe Pro Glu Phe Ser Ala Gly Asn Val Asp Phe Ala Asn Pro
           210                 215                 220
   gga gga cct gct tcg att gcc aat ccg gca aac tac cct gaa gcg ttc     719
   Gly Gly Pro Ala Ser Ile Ala Asn Pro Ala Asn Tyr Pro Glu Ala Phe
       225                 230                 235
   gca acc gga gcg aca gac atc gat aat cgc tta gcc gat ttt tca ctt     767
   Ala Thr Gly Ala Thr Asp Ile Asp Asn Arg Leu Ala Asp Phe Ser Leu
   240                 245                 250                 255
   caa ggt cct tcc ccg tat gat gag act aag cct gaa ata tcg gcg cct     815
   Gln Gly Pro Ser Pro Tyr Asp Glu Thr Lys Pro Glu Ile Ser Ala Pro
                   260                 265                 270
   ggt gtc aac att cgc tca tct gtg cct gga agc ggc tat caa gac ggc     863
   Gly Val Asn Ile Arg Ser Ser Val Pro Gly Ser Gly Tyr Gln Asp Gly
               275                 280                 285
   tgg gac ggc act tca atg gct ggc ccg cat gtc gca gcc gcc gcc gct     911
   Trp Asp Gly Thr Ser Met Ala Gly Pro His Val Ala Ala Ala Ala Ala
           290                 295                 300
   ctt atc aag caa gcc gat tcg tcg atc act gtt gat gag acc gaa aag     959
   Leu Ile Lys Gln Ala Asp Ser Ser Ile Thr Val Asp Glu Thr Glu Lys
       305                 310                 315
   att ttg atg gaa act gca aca ccg ctc aca gac agc aaa ttt acc gaa     1007
   Ile Leu Met Glu Thr Ala Thr Pro Leu Thr Asp Ser Lys Phe Thr Glu
   320                 325                 330                 335
   tcg ccg aac aac gga tac ggc cac gga ctc gtg aac gtg ttt gac gca     1055
   Ser Pro Asn Asn Gly Tyr Gly His Gly Leu Val Asn Val Phe Asp Ala
                   340                 345                 350
   gtg tcc gct gtg aca gac ggg ttg ggc aga gcg gaa gga caa gtc gga     1103
   Val Ser Ala Val Thr Asp Gly Leu Gly Arg Ala Glu Gly Gln Val Gly
               355                 360                 365
   aaa gaa ggc gaa gac aaa agt ccg ccg gcc tta aac cat caa gag atc     1151
   Lys Glu Gly Glu Asp Lys Ser Pro Pro Ala Leu Asn His Gln Glu Ile
           370                 375                 380
   acc gaa att tac tca ggc tcg gaa acg cct ctt aaa gct gat gta gaa     1199
   Thr Glu Ile Tyr Ser Gly Ser Glu Thr Pro Leu Lys Ala Asp Val Glu
       385                 390                 395
   gac gat gtc agc atc ata tcg gtc aaa ttg agc tat aaa aca gat gac     1247
   Asp Asp Val Ser Ile Ile Ser Val Lys Leu Ser Tyr Lys Thr Asp Asp
   400                 405                 410                 415
   gcc gat tgg gag acg atc gct gcc aaa caa act tcc ggc gac tat aaa     1295
   Ala Asp Trp Glu Thr Ile Ala Ala Lys Gln Thr Ser Gly Asp Tyr Lys
                   420                 425                 430
   aaa gga acg tac gag gcg gct gta act gtt tct gaa ggg aaa acc ctt     1343
   Lys Gly Thr Tyr Glu Ala Ala Val Thr Val Ser Glu Gly Lys Thr Leu
   435 440 445
   tcc tac aag tgg att gtc acc gat ttc gga ggc aac aaa acg gaa tcc 1391
   Ser Tyr Lys Trp Ile Val Thr Asp Phe Gly Gly Asn Lys Thr Glu Ser
   450 455 460
   aaa gtg tac gaa gtc ccg att tcc ccg gct gtg aca aca ggg tat aaa 1439
   Lys Val Tyr Glu Val Pro Ile Ser Pro Ala Val Thr Thr Gly Tyr Lys
   465 470 475
   caa gat ttc gag aat tcc gcc agc ggc tgg ctc aca tca ggc gtg aag 1487
   Gln Asp Phe Glu Asn Ser Ala Ser Gly Trp Leu Thr Ser Gly Val Lys
   480 485 490 495
   aat tca tgg gag cgc gga att cct cag tca ggc ccg aac gcc gca gca 1535
   Asn Ser Trp Glu Arg Gly Ile Pro Gln Ser Gly Pro Asn Ala Ala Ala
   500 505 510
   tcg gga aaa aac gta ttc gca aca aat ctg aca ggg ccg tat gaa agt 1583
   Ser Gly Lys Asn Val Phe Ala Thr Asn Leu Thr Gly Pro Tyr Glu Ser
   515 520 525
   tcg gct aat atg aat ctt ctc atg cct cct gtc agc gtt cca aaa aat 1631
   Ser Ala Asn Met Asn Leu Leu Met Pro Pro Val Ser Val Pro Lys Asn
   530 535 540
   caa aag ctt tat ctc aca tac aaa tat tgg cgc gat att gaa gag gac 1679
   Gln Lys Leu Tyr Leu Thr Tyr Lys Tyr Trp Arg Asp Ile Glu Glu Asp
   545 550 555
   ttt gat tac ggc ttc gtt tac gta cag cct gaa gga aaa ggt gaa tgg 1727
   Phe Asp Tyr Gly Phe Val Tyr Val Gln Pro Glu Gly Lys Gly Glu Trp
   560 565 570 575
   atc ccg gct gcc gag tac agc ggc aag aca tca gaa tgg aaa gac gga 1775
   Ile Pro Ala Ala Glu Tyr Ser Gly Lys Thr Ser Glu Trp Lys Asp Gly
   580 585 590
   cag atc gat ttg tcg gaa tac ggg gga cag acg att aag gtc atg ttc 1823
   Gln Ile Asp Leu Ser Glu Tyr Gly Gly Gln Thr Ile Lys Val Met Phe
   595 600 605
   aac ctt caa tct gac gac agc att gaa ggt gac ggg ctg tac atc gat 1871
   Asn Leu Gln Ser Asp Asp Ser Ile Glu Gly Asp Gly Leu Tyr Ile Asp
   610 615 620
   gat gta gcg ctt gtc aag gaa gtg aag agc gcc ggt acg aaa aaa cga 1919
   Asp Val Ala Leu Val Lys Glu Val Lys Ser Ala Gly Thr Lys Lys Arg
   625 630 635
   ttg ggc gtt gaa aaa cag ccg gcc aaa atg aag gat aag aaa aca aag 1967
   Leu Gly Val Glu Lys Gln Pro Ala Lys Met Lys Asp Lys Lys Thr Lys
   640 645 650 655
   aaa cga atg att gat ccg aaa aaa gca aaa ccg gca gaa gcc ctt cag 2015
   Lys Arg Met Ile Asp Pro Lys Lys Ala Lys Pro Ala Glu Ala Leu Gln
   660 665 670
   gaa aaa acc gaa acg aaa aaa gca gct cct gcg gtt ctt ccg gtg cgc 2063
   Glu Lys Thr Glu Thr Lys Lys Ala Ala Pro Ala Val Leu Pro Val Arg
   675 680 685
   gct cag gtc agc gtg ctg gaa acg ggc aaa tcg aca tat tcc aac cag 2111
   Ala Gln Val Ser Val Leu Glu Thr Gly Lys Ser Thr Tyr Ser Asn Gln
   690 695 700
   gca aca ggc gca tac agc ttg gct cat gca cct gga acc tat acg ctg 2159
   Ala Thr Gly Ala Tyr Ser Leu Ala His Ala Pro Gly Thr Tyr Thr Leu
   705 710 715
   aaa gcg gaa gcg tac gga tat gaa tca acc gcc caa acc gta aaa atc 2207
   Lys Ala Glu Ala Tyr Gly Tyr Glu Ser Thr Ala Gln Thr Val Lys Ile
   720 725 730 735
   gag tcc gac aag aca aca acg gca gac ttt gtt tta aaa gaa cta aaa 2255
   Glu Ser Asp Lys Thr Thr Thr Ala Asp Phe Val Leu Lys Glu Leu Lys
   740 745 750
   aaa ggg acg ctc acc gga acg ata aaa aat aaa aaa acg ggc gaa ccc 2303
   Lys Gly Thr Leu Thr Gly Thr Ile Lys Asn Lys Lys Thr Gly Glu Pro
   755 760 765
   gtc cgg cat gca aag ttg tat atc gtt gaa gat gca gct gta aaa cca 2351
   Val Arg His Ala Lys Leu Tyr Ile Val Glu Asp Ala Ala Val Lys Pro
   770 775 780
   gtc cag acg gat gat gac gga agc tat tca tta acg gcc tat gaa ggc 2399
   Val Gln Thr Asp Asp Asp Gly Ser Tyr Ser Leu Thr Ala Tyr Glu Gly
   785 790 795
   tcg tat acg gta aag gtc tct gca aac gga tat tac agc agc gag ttt 2447
   Ser Tyr Thr Val Lys Val Ser Ala Asn Gly Tyr Tyr Ser Ser Glu Phe
   800 805 810 815
   tcc gtt gat tta aaa ggc gat gtc tcg aaa gat atc gat ctc gat cct 2495
   Ser Val Asp Leu Lys Gly Asp Val Ser Lys Asp Ile Asp Leu Asp Pro
   820 825 830
   ttc atc ggc tat ccg ggg gaa ata ggc tat gat gac gga acc ggg gaa 2543
   Phe Ile Gly Tyr Pro Gly Glu Ile Gly Tyr Asp Asp Gly Thr Gly Glu
   835 840 845
   aac gcc tgg gct ttc tat gaa tcc ggc aac ggg ctg gcg gtt aaa atg 2591
   Asn Ala Trp Ala Phe Tyr Glu Ser Gly Asn Gly Leu Ala Val Lys Met
   850 855 860
   acg ctt gaa aac ggg cag gaa aaa gcc atg ctg aaa ggc ggt ttg ttt 2639
   Thr Leu Glu Asn Gly Gln Glu Lys Ala Met Leu Lys Gly Gly Leu Phe
   865 870 875
   aaa ttc tgg gat aca gaa ttc cct gat ccg ggc ggc act gat ttt gca 2687
   Lys Phe Trp Asp Thr Glu Phe Pro Asp Pro Gly Gly Thr Asp Phe Ala
   880 885 890 895
   gta gag gtc tat gat gct tcg ggt gaa aaa ggc tct ccg ggc aaa aaa 2735
   Val Glu Val Tyr Asp Ala Ser Gly Glu Lys Gly Ser Pro Gly Lys Lys
   900 905 910
   atc gcc ggc ccg ttc aag gct gaa gcg ctc cgc aca ggc gaa tgg acg 2783
   Ile Ala Gly Pro Phe Lys Ala Glu Ala Leu Arg Thr Gly Glu Trp Thr
   915 920 925
   acc gtt gat tta ggt gac gaa ggc atc atc gtc gga aaa gac ttt tac 2831
   Thr Val Asp Leu Gly Asp Glu Gly Ile Ile Val Gly Lys Asp Phe Tyr
   930 935 940
   ctc gta tac gtc cag aaa gag gat ctg gcc aac tca cct gga ctt gct 2879
   Leu Val Tyr Val Gln Lys Glu Asp Leu Ala Asn Ser Pro Gly Leu Ala
   945 950 955
   aca gac gaa gac gga gaa tat tcc ggc cgc aac tgg cag tat acg gac 2927
   Thr Asp Glu Asp Gly Glu Tyr Ser Gly Arg Asn Trp Gln Tyr Thr Asp
   960 965 970 975
   ggc tca tgg tca aaa gcg cca tca gac caa ggc aac ttt atg atc cgc 2975
   Gly Ser Trp Ser Lys Ala Pro Ser Asp Gln Gly Asn Phe Met Ile Arg
   980 985 990
   gct ttg gtc gac tat gaa ttg tcg gtg cct gtg att acg tct cct aaa 3023
   Ala Leu Val Asp Tyr Glu Leu Ser Val Pro Val Ile Thr Ser Pro Lys
   995 1000 1005
   gac gga ttc atc acc aac caa aag aac gct gtc att gaa ggg aca 3068
   Asp Gly Phe Ile Thr Asn Gln Lys Asn Ala Val Ile Glu Gly Thr
   1010 1015 1020
   tcc tcg ccg aat acg acc gtt cat ctc ttt aac ggg gat gaa gaa 3113
   Ser Ser Pro Asn Thr Thr Val His Leu Phe Asn Gly Asp Glu Glu
   1025 1030 1035
   gcc gga aca gcg gaa acg gcg gca gac ggc act ttc tca aaa gaa 3158
   Ala Gly Thr Ala Glu Thr Ala Ala Asp Gly Thr Phe Ser Lys Glu
   1040 1045 1050
   ata ccg ttg aat aaa ggg gaa aat gtc atc acc gcg aaa tcg tca 3203
   Ile Pro Leu Asn Lys Gly Glu Asn Val Ile Thr Ala Lys Ser Ser
   1055 1060 1065
   tcg gct tcc gga acg act gat gct tca gaa ccg gtc cgg atc gtc 3248
   Ser Ala Ser Gly Thr Thr Asp Ala Ser Glu Pro Val Arg Ile Val
   1070 1075 1080
   ctt gac cag aaa aag ccg aag ctt aca atc gac acg ccg gaa agc 3293
   Leu Asp Gln Lys Lys Pro Lys Leu Thr Ile Asp Thr Pro Glu Ser
   1085 1090 1095
   ggc tca aaa ctc aat aaa gag acc gtc acc gtg aaa gga aca gta 3338
   Gly Ser Lys Leu Asn Lys Glu Thr Val Thr Val Lys Gly Thr Val
   1100 1105 1110
   tca gac gat cac tta gaa tct gtt cat gta aac ggc aaa aaa gca 3383
   Ser Asp Asp His Leu Glu Ser Val His Val Asn Gly Lys Lys Ala
   1115 1120 1125
   gct gtt gac aat ggc gaa tat tct gca agg att atg ctt gac aac 3428
   Ala Val Asp Asn Gly Glu Tyr Ser Ala Arg Ile Met Leu Asp Asn
   1130 1135 1140
   ggc aaa aat gaa atc aaa gta acg gca tcg gat gcg gca ggc aac 3473
   Gly Lys Asn Glu Ile Lys Val Thr Ala Ser Asp Ala Ala Gly Asn
   1145 1150 1155
   aaa acg acc aaa aag gtc acg gta gat gtc aac ttt gaa gcg ccg 3518
   Lys Thr Thr Lys Lys Val Thr Val Asp Val Asn Phe Glu Ala Pro
   1160 1165 1170
   caa atc acc ggc ttg aaa ccg gcc gaa gat ctc gag ctc aaa acg 3563
   Gln Ile Thr Gly Leu Lys Pro Ala Glu Asp Leu Glu Leu Lys Thr
   1175 1180 1185
   gga gaa acc gtg aaa atc gag ttt gaa agc gcc gct gat ttg gat 3608
   Gly Glu Thr Val Lys Ile Glu Phe Glu Ser Ala Ala Asp Leu Asp
   1190 1195 1200
   gct gtc ttt gtg atc aga atg ccg ctg acc aat ttc aaa acc gct 3653
   Ala Val Phe Val Ile Arg Met Pro Leu Thr Asn Phe Lys Thr Ala
   1205 1210 1215
   gcc caa aac gta acg gag ctg ccg atc aga gaa gtc tca aaa gga 3698
   Ala Gln Asn Val Thr Glu Leu Pro Ile Arg Glu Val Ser Lys Gly
   1220 1225 1230
   aaa tat gaa gga tat tgg acc gct act tca act gca aaa gca aaa 3743
   Lys Tyr Glu Gly Tyr Trp Thr Ala Thr Ser Thr Ala Lys Ala Lys
           1235                1240                1245
   gga gcg gaa atc gag gtc atc gtc aga gat gat tac ggc aat gaa      3788
   Gly Ala Glu Ile Glu Val Ile Val Arg Asp Asp Tyr Gly Asn Glu
           1250                1255                1260
   acg aga caa acg gca aaa ggc aag ctg tat atc aat gaa aag ctg      3833
   Thr Arg Gln Thr Ala Lys Gly Lys Leu Tyr Ile Asn Glu Lys Leu
           1265                1270                1275
   aaa taaaggtgaa aagacgctgt ctttaatggc agcgtttttt tcgttttacg      3886
   Lys
   atcgacaaat tcagtacgaa aacttcaaaa aatgtacgat ttacgcaaca ttaattgaca   3946
   gactttacct ttgggcttga tttatactta ggaaaacaaa cactaaggtc accgagccgc   4006
   agaaagggga aggatgtgga aatctattta gatgcgatat ggctgttaaa cttttgtttt   4066
   gacttgctgc ttttaatgat gaccgcattt attttaaagc gaagggttaa aaagcggagg   4126
   ctgatcctag gggcatttgt cgcgtcaagc atcgttctgt ttatgtttac acctttttca   4186
   ccgtacgtcc ttcatcctgc cggcaaactg tcgttttcgg ttgtgatcgt tcttgtggca   4246
   tttggtttta agcggttccg gttttttttg cagaatttgt tttcttttta ttttgccact   4306
   tttttaatgg gaggaggga                                                4325

<210>  2
<211>  1278
<212>  PRT
<213>  Bacillus licheniformis

<400>  2

Glu Gln Val Ala Ala Phe Pro Glu Val Glu Lys Val Leu Pro Asn Glu
1               5                   10                  15

Lys Arg Gln Leu Ile Lys Pro Thr Lys Gln Ser Val Lys Lys Ser Ala
            20                  25                  30

Ala Lys Asp Glu Lys Glu Ile Glu Trp Asn Ile Asn Arg Val Asp Ala
        35                  40                  45

Pro Lys Ala Trp Lys Leu Gly Tyr Asp Gly Ser Gly Thr Val Val Ala
    50                  55                  60

Ser Ile Asp Thr Gly Val Gln Trp Asp His Pro Ala Leu Lys Glu Lys
65                  70                  75                  80

Tyr Arg Gly Tyr Asp Pro Gln His Pro Asp Gln Pro Asn His Glu Phe
                85                  90                  95

Ser Trp Tyr Asp Ala Val Ser Gly Ala Ser Glu Pro Phe Asp Asp Leu
            100                 105                 110

Glu His Gly Thr His Val Thr Gly Thr Met Val Gly Ser Glu Pro Asp

        115                 120                 125

Gly Gln Asn Gln Ile Gly Val Ala Pro Gly Ala Lys Trp Ile Ala Val
    130                 135                 140

Lys Ala Phe Ser Asp Asp Gly Gly Thr Asp Glu Asp Leu Ile Ala Ala
145                 150                 155                 160

Gly Glu Trp Ile Leu Ala Pro Lys Asp Lys Asp Gly Asn Pro His Pro
                165                 170                 175

Glu Met Ala Pro Asp Val Val Asn Asn Ser Trp Ser Gly Gly Ala Gly
            180                 185                 190

Ile Asp Glu Phe Tyr Arg Asp Ile Val Lys Ala Trp Arg Ala Ala Gly
        195                 200                 205

Ile Phe Pro Glu Phe Ser Ala Gly Asn Val Asp Phe Ala Asn Pro Gly
    210                 215                 220

Gly Pro Ala Ser Ile Ala Asn Pro Ala Asn Tyr Pro Glu Ala Phe Ala
225                 230                 235                 240

Thr Gly Ala Thr Asp Ile Asp Asn Arg Leu Ala Asp Phe Ser Leu Gln
                245                 250                 255

Gly Pro Ser Pro Tyr Asp Glu Thr Lys Pro Glu Ile Ser Ala Pro Gly
            260                 265                 270

Val Asn Ile Arg Ser Ser Val Pro Gly Ser Gly Tyr Gln Asp Gly Trp
        275                 280                 285

Asp Gly Thr Ser Met Ala Gly Pro His Val Ala Ala Ala Ala Ala Leu
    290                 295                 300

Ile Lys Gln Ala Asp Ser Ser Ile Thr Val Asp Glu Thr Glu Lys Ile
305                 310                 315                 320

Leu Met Glu Thr Ala Thr Pro Leu Thr Asp Ser Lys Phe Thr Glu Ser
                325                 330                 335

Pro Asn Asn Gly Tyr Gly His Gly Leu Val Asn Val Phe Asp Ala Val
            340                 345                 350

Ser Ala Val Thr Asp Gly Leu Gly Arg Ala Glu Gly Gln Val Gly Lys
        355                 360                 365

Glu Gly Glu Asp Lys Ser Pro Pro Ala Leu Asn His Gln Glu Ile Thr
    370                 375                 380

Glu Ile Tyr Ser Gly Ser Glu Thr Pro Leu Lys Ala Asp Val Glu Asp

385                 390                 395                 400

Asp Val Ser Ile Ile Ser Val Lys Leu Ser Tyr Lys Thr Asp Asp Ala
                405                 410                 415

Asp Trp Glu Thr Ile Ala Ala Lys Gln Thr Ser Gly Asp Tyr Lys Lys
            420                 425                 430

Gly Thr Tyr Glu Ala Ala Val Thr Val Ser Glu Gly Lys Thr Leu Ser
        435                 440                 445

Tyr Lys Trp Ile Val Thr Asp Phe Gly Gly Asn Lys Thr Glu Ser Lys
    450                 455                 460

Val Tyr Glu Val Pro Ile Ser Pro Ala Val Thr Thr Gly Tyr Lys Gln
465                 470                 475                 480

Asp Phe Glu Asn Ser Ala Ser Gly Trp Leu Thr Ser Gly Val Lys Asn
                485                 490                 495

Ser Trp Glu Arg Gly Ile Pro Gln Ser Gly Pro Asn Ala Ala Ala Ser
            500                 505                 510

Gly Lys Asn Val Phe Ala Thr Asn Leu Thr Gly Pro Tyr Glu Ser Ser
        515                 520                 525

Ala Asn Met Asn Leu Leu Met Pro Pro Val Ser Val Pro Lys Asn Gln
    530                 535                 540

Lys Leu Tyr Leu Thr Tyr Lys Tyr Trp Arg Asp Ile Glu Glu Asp Phe
545                 550                 555                 560

Asp Tyr Gly Phe Val Tyr Val Gln Pro Glu Gly Lys Gly Glu Trp Ile
                565                 570                 575

Pro Ala Ala Glu Tyr Ser Gly Lys Thr Ser Glu Trp Lys Asp Gly Gln
            580                 585                 590

Ile Asp Leu Ser Glu Tyr Gly Gly Gln Thr Ile Lys Val Met Phe Asn
        595                 600                 605

Leu Gln Ser Asp Asp Ser Ile Glu Gly Asp Gly Leu Tyr Ile Asp Asp
    610                 615                 620

Val Ala Leu Val Lys Glu Val Lys Ser Ala Gly Thr Lys Lys Arg Leu
625                 630                 635                 640

Gly Val Glu Lys Gln Pro Ala Lys Met Lys Asp Lys Lys Thr Lys Lys
                645                 650                 655

Arg Met Ile Asp Pro Lys Lys Ala Lys Pro Ala Glu Ala Leu Gln Glu

            660                 665                 670

Lys Thr Glu Thr Lys Lys Ala Ala Pro Ala Val Leu Pro Val Arg Ala
        675                 680                 685

Gln Val Ser Val Leu Glu Thr Gly Lys Ser Thr Tyr Ser Asn Gln Ala
    690                 695                 700

Thr Gly Ala Tyr Ser Leu Ala His Ala Pro Gly Thr Tyr Thr Leu Lys
705                 710                 715                 720

Ala Glu Ala Tyr Gly Tyr Glu Ser Thr Ala Gln Thr Val Lys Ile Glu
                725                 730                 735

Ser Asp Lys Thr Thr Thr Ala Asp Phe Val Leu Lys Glu Leu Lys Lys
            740                 745                 750

Gly Thr Leu Thr Gly Thr Ile Lys Asn Lys Lys Thr Gly Glu Pro Val
        755                 760                 765

Arg His Ala Lys Leu Tyr Ile Val Glu Asp Ala Ala Val Lys Pro Val
    770                 775                 780

Gln Thr Asp Asp Asp Gly Ser Tyr Ser Leu Thr Ala Tyr Glu Gly Ser
785                 790                 795                 800

Tyr Thr Val Lys Val Ser Ala Asn Gly Tyr Tyr Ser Ser Glu Phe Ser
                805                 810                 815

Val Asp Leu Lys Gly Asp Val Ser Lys Asp Ile Asp Leu Asp Pro Phe
            820                 825                 830

Ile Gly Tyr Pro Gly Glu Ile Gly Tyr Asp Asp Gly Thr Gly Glu Asn
        835                 840                 845

Ala Trp Ala Phe Tyr Glu Ser Gly Asn Gly Leu Ala Val Lys Met Thr
    850                 855                 860

Leu Glu Asn Gly Gln Glu Lys Ala Met Leu Lys Gly Gly Leu Phe Lys
865                 870                 875                 880

Phe Trp Asp Thr Glu Phe Pro Asp Pro Gly Gly Thr Asp Phe Ala Val
                885                 890                 895

Glu Val Tyr Asp Ala Ser Gly Glu Lys Gly Ser Pro Gly Lys Lys Ile
            900                 905                 910

Ala Gly Pro Phe Lys Ala Glu Ala Leu Arg Thr Gly Glu Trp Thr Thr
        915                 920                 925

Val Asp Leu Gly Asp Glu Gly Ile Ile Val Gly Lys Asp Phe Tyr Leu

930                    935                    940

Val Tyr Val Gln Lys Glu Asp Leu Ala Asn Ser Pro Gly Leu Ala Thr
945                 950                 955                 960

Asp Glu Asp Gly Glu Tyr Ser Gly Arg Asn Trp Gln Tyr Thr Asp Gly
                965                 970                 975

Ser Trp Ser Lys Ala Pro Ser Asp Gln Gly Asn Phe Met Ile Arg Ala
            980                 985                 990

Leu Val Asp Tyr Glu Leu Ser Val  Pro Val Ile Thr Ser  Pro Lys Asp
        995                 1000                 1005

Gly Phe  Ile Thr Asn Gln Lys  Asn Ala Val Ile Glu  Gly Thr Ser
    1010                 1015                 1020

Ser Pro  Asn Thr Thr Val His  Leu Phe Asn Gly Asp  Glu Glu Ala
    1025                 1030                 1035

Gly Thr  Ala Glu Thr Ala Ala  Asp Gly Thr Phe Ser  Lys Glu Ile
    1040                 1045                 1050

Pro Leu  Asn Lys Gly Glu Asn  Val Ile Thr Ala Lys  Ser Ser Ser
    1055                 1060                 1065

Ala Ser  Gly Thr Thr Asp Ala  Ser Glu Pro Val Arg  Ile Val Leu
    1070                 1075                 1080

Asp Gln  Lys Lys Pro Lys Leu  Thr Ile Asp Thr Pro  Glu Ser Gly
    1085                 1090                 1095

Ser Lys  Leu Asn Lys Glu Thr  Val Thr Val Lys Gly  Thr Val Ser
    1100                 1105                 1110

Asp Asp  His Leu Glu Ser Val  His Val Asn Gly Lys  Lys Ala Ala
    1115                 1120                 1125

Val Asp  Asn Gly Glu Tyr Ser  Ala Arg Ile Met Leu  Asp Asn Gly
    1130                 1135                 1140

Lys Asn  Glu Ile Lys Val Thr  Ala Ser Asp Ala Ala  Gly Asn Lys
    1145                 1150                 1155

Thr Thr  Lys Lys Val Thr Val  Asp Val Asn Phe Glu  Ala Pro Gln
    1160                 1165                 1170

Ile Thr  Gly Leu Lys Pro Ala  Glu Asp Leu Glu Leu  Lys Thr Gly
    1175                 1180                 1185

Glu Thr  Val Lys Ile Glu Phe  Glu Ser Ala Ala Asp  Leu Asp Ala

1190                1195                1200

Val Phe Val Ile Arg Met Pro Leu Thr Asn Phe Lys Thr Ala Ala
    1205                1210                1215

Gln Asn Val Thr Glu Leu Pro Ile Arg Glu Val Ser Lys Gly Lys
    1220                1225                1230

Tyr Glu Gly Tyr Trp Thr Ala Thr Ser Thr Ala Lys Ala Lys Gly
    1235                1240                1245

Ala Glu Ile Glu Val Ile Val Arg Asp Asp Tyr Gly Asn Glu Thr
    1250                1255                1260

Arg Gln Thr Ala Lys Gly Lys Leu Tyr Ile Asn Glu Lys Leu Lys
    1265                1270                1275

<210>  3
<211>  3405
<212>  DNA
<213>  Bacillus licheniformis

<220>
<221>  CDS
<222>  (501)..(2906)

<400>  3
aatgatatca aacctttcat accgatccct ccagtttcgt tttgataaaa ctagcaactc      60

tattaaactt tcttgctcta tcttatccca gcaaaatgaa aatgtttgtc acaatgtgtg     120

tgcaaaatga ttctagtttt tagaagtttt gttgaaaact gaaggaatcg catgattcag     180

cggatacaaa ccatgaatgt aacttactca cagcttatcc taaggataaa cacatattac     240

ccacaggata tatccacata tccacatact tattcaatat ttagtataag aacgtatatt     300

ccctacaata tctatacaca agtttattca cttatacaca gtaaattgtg cataaatcta     360

gagaaattca ctccaataca ttgaatcttt gaaaattatt tctatatata gaaggatttt     420

tttgaaactg agagaatatt ttaaaagttc gaaactctaa taattacaaa gaaacattta     480

tccagaaggg ggaaaacaga ttg aga aaa agt atc gtg cgc tat ttt gtt atg     533
                     Leu Arg Lys Ser Ile Val Arg Tyr Phe Val Met
                     1               5                   10

gct ttt att cta tta ttt gcg tta tcc aca ttc ctc acc gga gtg cag       581
Ala Phe Ile Leu Leu Phe Ala Leu Ser Thr Phe Leu Thr Gly Val Gln
            15                  20                  25

gca act tcc gtt ccc gat aaa aag tcg cct gag ctt gag aaa gct gaa       629
Ala Thr Ser Val Pro Asp Lys Lys Ser Pro Glu Leu Glu Lys Ala Glu
        30                  35                  40

atc tac ggt gat att gat gtg acg tct gat aaa cag acg acg gtt atc       677
Ile Tyr Gly Asp Ile Asp Val Thr Ser Asp Lys Gln Thr Thr Val Ile
    45                  50                  55

gtg gaa ctg aaa gaa aag tcg ctt gcc gaa gca aaa gcg gac gga gaa       725
Val Glu Leu Lys Glu Lys Ser Leu Ala Glu Ala Lys Ala Asp Gly Glu

60                  65                  70                  75

aaa caa acg aag gct tct cta aaa aca gct cga agc aaa gcg ctg aaa      773
Lys Gln Thr Lys Ala Ser Leu Lys Thr Ala Arg Ser Lys Ala Leu Lys
                80                  85                  90

aca ctt aaa aaa gcg aaa gta aac cgc gaa tac gac cgt gta ttt tcg      821
Thr Leu Lys Lys Ala Lys Val Asn Arg Glu Tyr Asp Arg Val Phe Ser
            95                  100                 105

ggc ttt tct atg aaa ctg ccg gcc agt gaa att cca aag ctg ctc gcc      869
Gly Phe Ser Met Lys Leu Pro Ala Ser Glu Ile Pro Lys Leu Leu Ala
        110                 115                 120

gtc aag gaa gtt aaa gcg gtt tat cca aac gca act tac aaa cct gac      917
Val Lys Glu Val Lys Ala Val Tyr Pro Asn Ala Thr Tyr Lys Pro Asp
    125                 130                 135

agc gta aaa gga aaa gac gtg aca ctt gca gca gac gcc att tat cca      965
Ser Val Lys Gly Lys Asp Val Thr Leu Ala Ala Asp Ala Ile Tyr Pro
140                 145                 150                 155

cag atg gat aaa agt gcc ccg ttc atc gga gcg gat cag gca tgg aaa      1013
Gln Met Asp Lys Ser Ala Pro Phe Ile Gly Ala Asp Gln Ala Trp Lys
                160                 165                 170

tcg ggc tat acc ggt aaa ggc att aag gta gcc gtt att gat acg ggc      1061
Ser Gly Tyr Thr Gly Lys Gly Ile Lys Val Ala Val Ile Asp Thr Gly
            175                 180                 185

gtg gat tac acc cat ccc gac tta aag aat aat ttc ggc cca tat aag      1109
Val Asp Tyr Thr His Pro Asp Leu Lys Asn Asn Phe Gly Pro Tyr Lys
        190                 195                 200

ggc tat gac ttt gtg gat aat gat tat gat cca cag gaa aca ccg aca      1157
Gly Tyr Asp Phe Val Asp Asn Asp Tyr Asp Pro Gln Glu Thr Pro Thr
    205                 210                 215

ggc gac ccg cgc ggc gga gcg act gat cac ggc act cat gtt gca gga      1205
Gly Asp Pro Arg Gly Gly Ala Thr Asp His Gly Thr His Val Ala Gly
220                 225                 230                 235

aca atc gcc gcg aac gga cag att aaa ggc gtg gcg cct gaa gca acg      1253
Thr Ile Ala Ala Asn Gly Gln Ile Lys Gly Val Ala Pro Glu Ala Thr
                240                 245                 250

ctt ctt gct tac cgc gtg ctc ggc ccc ggc ggc tca ggc aca acc gag      1301
Leu Leu Ala Tyr Arg Val Leu Gly Pro Gly Gly Ser Gly Thr Thr Glu
            255                 260                 265

aat gtg att gcg ggc att gaa aaa gcc gtt gca gac gga gca aaa gtg      1349
Asn Val Ile Ala Gly Ile Glu Lys Ala Val Ala Asp Gly Ala Lys Val
        270                 275                 280

atg aac ctc tca ttg gga aat tcg ctg aac agc cct gat tat gca aca      1397
Met Asn Leu Ser Leu Gly Asn Ser Leu Asn Ser Pro Asp Tyr Ala Thr
    285                 290                 295

agc atc gca ctg gac tgg gcg atg gct gaa ggg gtt gtc gcc gtt acg      1445
Ser Ile Ala Leu Asp Trp Ala Met Ala Glu Gly Val Val Ala Val Thr
300                 305                 310                 315

tca aac ggt aac agc gga ccg gaa aac tgg acg gtc ggc tcg ccg gga      1493
Ser Asn Gly Asn Ser Gly Pro Glu Asn Trp Thr Val Gly Ser Pro Gly
                320                 325                 330

aca tca agg gtt gcg att tcc gtc ggc gca tca cag ctt ccg tat aat      1541
Thr Ser Arg Val Ala Ile Ser Val Gly Ala Ser Gln Leu Pro Tyr Asn

            335                 340                 345

gag tat tcg gtg acg ctt cct tcg tat tca tca gca aag gtg atg gga 1589
Glu Tyr Ser Val Thr Leu Pro Ser Tyr Ser Ser Ala Lys Val Met Gly
        350                 355                 360

tac caa gag gaa aag gat ctt gaa gca ttg aac ggt caa gag gtg gaa 1637
Tyr Gln Glu Glu Lys Asp Leu Glu Ala Leu Asn Gly Gln Glu Val Glu
    365                 370                 375

ctc gtt gaa gcc ggc ctc gga caa gct gac gat ttc tca ggc aaa gat 1685
Leu Val Glu Ala Gly Leu Gly Gln Ala Asp Asp Phe Ser Gly Lys Asp
380                 385                 390                 395

gtg aaa gga aaa gtt gcg gtt atc cag aga ggc gtc ata cca ttt gtt 1733
Val Lys Gly Lys Val Ala Val Ile Gln Arg Gly Val Ile Pro Phe Val
                400                 405                 410

gat aag gct gaa aat gcc aaa aac gcc ggc gct atc gga gcc gtg att 1781
Asp Lys Ala Glu Asn Ala Lys Asn Ala Gly Ala Ile Gly Ala Val Ile
            415                 420                 425

tac aat aat gca aca ggg gaa atc gag gca aac gtc atg ggg atg gcc 1829
Tyr Asn Asn Ala Thr Gly Glu Ile Glu Ala Asn Val Met Gly Met Ala
        430                 435                 440

gta ccg acc gtc aaa ttg tca aaa gaa gag ggc gaa aag ctt gtt caa 1877
Val Pro Thr Val Lys Leu Ser Lys Glu Glu Gly Glu Lys Leu Val Gln
    445                 450                 455

cag atc aaa gaa ggg aaa cac tcc gtt gtc ttt tct ttc aaa ttg gac 1925
Gln Ile Lys Glu Gly Lys His Ser Val Val Phe Ser Phe Lys Leu Asp
460                 465                 470                 475

aaa aag ctg ggg gaa aca att gcc tca ttc tcg tcc cgc ggg cct gtc 1973
Lys Lys Leu Gly Glu Thr Ile Ala Ser Phe Ser Ser Arg Gly Pro Val
                480                 485                 490

atg gat aca tgg atg att aaa cct gac gtt tca gcg cca ggc gtc aac 2021
Met Asp Thr Trp Met Ile Lys Pro Asp Val Ser Ala Pro Gly Val Asn
            495                 500                 505

atc gtc agc acc att ccg acc cac gat ccg aaa aac ccg tac ggt tac 2069
Ile Val Ser Thr Ile Pro Thr His Asp Pro Lys Asn Pro Tyr Gly Tyr
        510                 515                 520

ggt tca aaa cag gga aca agc atg gct tcc ccg cat gtc gcg gga acg 2117
Gly Ser Lys Gln Gly Thr Ser Met Ala Ser Pro His Val Ala Gly Thr
    525                 530                 535

gca gcc ata tta aag cag gcc aaa ccg gat tgg acg cct gag caa atc 2165
Ala Ala Ile Leu Lys Gln Ala Lys Pro Asp Trp Thr Pro Glu Gln Ile
540                 545                 550                 555

aaa ggc gta ctg atg aat acg gcg gaa aaa ctg acg gat gaa aat gga 2213
Lys Gly Val Leu Met Asn Thr Ala Glu Lys Leu Thr Asp Glu Asn Gly
                560                 565                 570

aag cct ctc cct cac aat acg caa gga gcg ggc agc atc cgc atc atg 2261
Lys Pro Leu Pro His Asn Thr Gln Gly Ala Gly Ser Ile Arg Ile Met
            575                 580                 585

gag gcc ctt aaa gct tca tcc att gta acg ccg ggc agc cat tca tac 2309
Glu Ala Leu Lys Ala Ser Ser Ile Val Thr Pro Gly Ser His Ser Tyr
        590                 595                 600

gga aca ttc ctg aaa gac aaa ggg aaa cag acg aaa aaa caa gcg ttc 2357
Gly Thr Phe Leu Lys Asp Lys Gly Lys Gln Thr Lys Lys Gln Ala Phe

605                 610                 615

acg att gaa aac ctt tct tca cac aga aaa gcc tat cag ctc gaa tac      2405
Thr Ile Glu Asn Leu Ser Ser His Arg Lys Ala Tyr Gln Leu Glu Tyr
620                 625                 630                 635

tcc ttt aaa gga acg ggc atc acg gta tca gga acg gaa cga gtc gtg      2453
Ser Phe Lys Gly Thr Gly Ile Thr Val Ser Gly Thr Glu Arg Val Val
                640                 645                 650

gta ccg gcc aat caa aca ggt aaa gca gcg gca aaa gta acc gtc aat      2501
Val Pro Ala Asn Gln Thr Gly Lys Ala Ala Ala Lys Val Thr Val Asn
            655                 660                 665

tcc gcg aaa acg aaa gca ggc aca tat gaa ggc acg gtt tac atc cgt      2549
Ser Ala Lys Thr Lys Ala Gly Thr Tyr Glu Gly Thr Val Tyr Ile Arg
        670                 675                 680

gaa gac gga aga aaa gtc gcc gaa atc ccg ctc cta ttg atc gtc aaa      2597
Glu Asp Gly Arg Lys Val Ala Glu Ile Pro Leu Leu Leu Ile Val Lys
    685                 690                 695

gag cca gac tac ccg cgc gtc aca tcc gta aca gtt gaa ccg gga gca      2645
Glu Pro Asp Tyr Pro Arg Val Thr Ser Val Thr Val Glu Pro Gly Ala
700                 705                 710                 715

aag cag ggc gct tac acg atc gaa gcc tac ctg ccg ggc ggg gct gaa      2693
Lys Gln Gly Ala Tyr Thr Ile Glu Ala Tyr Leu Pro Gly Gly Ala Glu
                720                 725                 730

gag ctc gca ttt ctc gtc tat gat gaa aac ctg aac ctt ctc ggc cag      2741
Glu Leu Ala Phe Leu Val Tyr Asp Glu Asn Leu Asn Leu Leu Gly Gln
            735                 740                 745

gcc ggc gtt tac aaa aac cag ggc aaa ggc tat caa tct tat caa tgg      2789
Ala Gly Val Tyr Lys Asn Gln Gly Lys Gly Tyr Gln Ser Tyr Gln Trp
        750                 755                 760

aac ggc aaa atc aat gac gcc gca tcc ctt aag tcc gga aaa tac tat      2837
Asn Gly Lys Ile Asn Asp Ala Ala Ser Leu Lys Ser Gly Lys Tyr Tyr
    765                 770                 775

atg ctt gcc tat gca tcc gcc aaa ggg aaa tca agc tac gta ttg acg      2885
Met Leu Ala Tyr Ala Ser Ala Lys Gly Lys Ser Ser Tyr Val Leu Thr
780                 785                 790                 795

gaa gac cct ttt atc gtc gaa taatgacaag ccttgttgag aaccactcaa      2936
Glu Asp Pro Phe Ile Val Glu
                800

caaggctttt ttatgttaaa atacggataa tgcgttcagg agaagctccc ccttctcttc      2996

aaaacgtgaa aaaagcaatc ggaggacatc gtgtatatgc tttcttttat cgtattattc      3056

ggcttatcct tcattattgt ctgctttata tttttcacga ttttgtactt cgccgtcaac      3116

ctgcagaagc gcgagcccaa gccttttcaa aaagctgcgg agcaaaccgt cgataccatc      3176

atcctcattc cgatcagctg gctgtttacc gctttataca tatgcattct gtttattctt      3236

ttcccaatcc gccattttct cgattttttt cagcaaaaac gctaaattga ctgatgaaac      3296

gcttcggcca gcagccggta tgaatccaat ctgtcttgaa aatcgtgggt gatcgtcacc      3356

gccatgattt cgtccgttcc gtaagcgccg gccagttcaa gcagctgtt      3405

<210>  4

<211>  802
<212>  PRT
<213>  Bacillus licheniformis

<400>  4

Leu Arg Lys Ser Ile Val Arg Tyr Phe Val Met Ala Phe Ile Leu Leu
1               5                   10                  15

Phe Ala Leu Ser Thr Phe Leu Thr Gly Val Gln Ala Thr Ser Val Pro
            20                  25                  30

Asp Lys Lys Ser Pro Glu Leu Glu Lys Ala Glu Ile Tyr Gly Asp Ile
        35                  40                  45

Asp Val Thr Ser Asp Lys Gln Thr Thr Val Ile Val Glu Leu Lys Glu
    50                  55                  60

Lys Ser Leu Ala Glu Ala Lys Ala Asp Gly Glu Lys Gln Thr Lys Ala
65                  70                  75                  80

Ser Leu Lys Thr Ala Arg Ser Lys Ala Leu Lys Thr Leu Lys Lys Ala
                85                  90                  95

Lys Val Asn Arg Glu Tyr Asp Arg Val Phe Ser Gly Phe Ser Met Lys
            100                 105                 110

Leu Pro Ala Ser Glu Ile Pro Lys Leu Leu Ala Val Lys Glu Val Lys
        115                 120                 125

Ala Val Tyr Pro Asn Ala Thr Tyr Lys Pro Asp Ser Val Lys Gly Lys
    130                 135                 140

Asp Val Thr Leu Ala Ala Asp Ala Ile Tyr Pro Gln Met Asp Lys Ser
145                 150                 155                 160

Ala Pro Phe Ile Gly Ala Asp Gln Ala Trp Lys Ser Gly Tyr Thr Gly
                165                 170                 175

Lys Gly Ile Lys Val Ala Val Ile Asp Thr Gly Val Asp Tyr Thr His
            180                 185                 190

Pro Asp Leu Lys Asn Asn Phe Gly Pro Tyr Lys Gly Tyr Asp Phe Val
        195                 200                 205

Asp Asn Asp Tyr Asp Pro Gln Glu Thr Pro Thr Gly Asp Pro Arg Gly
    210                 215                 220

Gly Ala Thr Asp His Gly Thr His Val Ala Gly Thr Ile Ala Ala Asn
225                 230                 235                 240

Gly Gln Ile Lys Gly Val Ala Pro Glu Ala Thr Leu Leu Ala Tyr Arg
                245                 250                 255

Val Leu Gly Pro Gly Gly Ser Gly Thr Thr Glu Asn Val Ile Ala Gly
            260                 265                 270

Ile Glu Lys Ala Val Ala Asp Gly Ala Lys Val Met Asn Leu Ser Leu
        275                 280                 285

Gly Asn Ser Leu Asn Ser Pro Asp Tyr Ala Thr Ser Ile Ala Leu Asp
    290                 295                 300

Trp Ala Met Ala Glu Gly Val Val Ala Val Thr Ser Asn Gly Asn Ser
305                 310                 315                 320

Gly Pro Glu Asn Trp Thr Val Gly Ser Pro Gly Thr Ser Arg Val Ala
                325                 330                 335

Ile Ser Val Gly Ala Ser Gln Leu Pro Tyr Asn Glu Tyr Ser Val Thr
            340                 345                 350

Leu Pro Ser Tyr Ser Ser Ala Lys Val Met Gly Tyr Gln Glu Glu Lys
        355                 360                 365

Asp Leu Glu Ala Leu Asn Gly Gln Glu Val Glu Leu Val Glu Ala Gly
    370                 375                 380

Leu Gly Gln Ala Asp Asp Phe Ser Gly Lys Asp Val Lys Gly Lys Val
385                 390                 395                 400

Ala Val Ile Gln Arg Gly Val Ile Pro Phe Val Asp Lys Ala Glu Asn
                405                 410                 415

Ala Lys Asn Ala Gly Ala Ile Gly Ala Val Ile Tyr Asn Asn Ala Thr
            420                 425                 430

Gly Glu Ile Glu Ala Asn Val Met Gly Met Ala Val Pro Thr Val Lys
        435                 440                 445

Leu Ser Lys Glu Glu Gly Glu Lys Leu Val Gln Gln Ile Lys Glu Gly
    450                 455                 460

Lys His Ser Val Val Phe Ser Phe Lys Leu Asp Lys Lys Leu Gly Glu
465                 470                 475                 480

Thr Ile Ala Ser Phe Ser Ser Arg Gly Pro Val Met Asp Thr Trp Met
                485                 490                 495

Ile Lys Pro Asp Val Ser Ala Pro Gly Val Asn Ile Val Ser Thr Ile
            500                 505                 510

Pro Thr His Asp Pro Lys Asn Pro Tyr Gly Tyr Gly Ser Lys Gln Gly
        515                 520                 525

Thr Ser Met Ala Ser Pro His Val Ala Gly Thr Ala Ala Ile Leu Lys
    530                 535                 540

Gln Ala Lys Pro Asp Trp Thr Pro Glu Gln Ile Lys Gly Val Leu Met
545                 550                 555                 560

Asn Thr Ala Glu Lys Leu Thr Asp Glu Asn Gly Lys Pro Leu Pro His
                565                 570                 575

Asn Thr Gln Gly Ala Gly Ser Ile Arg Ile Met Glu Ala Leu Lys Ala
            580                 585                 590

Ser Ser Ile Val Thr Pro Gly Ser His Ser Tyr Gly Thr Phe Leu Lys
        595                 600                 605

Asp Lys Gly Lys Gln Thr Lys Lys Gln Ala Phe Thr Ile Glu Asn Leu
    610                 615                 620

Ser Ser His Arg Lys Ala Tyr Gln Leu Glu Tyr Ser Phe Lys Gly Thr
625                 630                 635                 640

Gly Ile Thr Val Ser Gly Thr Glu Arg Val Val Val Pro Ala Asn Gln
                645                 650                 655

Thr Gly Lys Ala Ala Ala Lys Val Thr Val Asn Ser Ala Lys Thr Lys
            660                 665                 670

Ala Gly Thr Tyr Glu Gly Thr Val Tyr Ile Arg Glu Asp Gly Arg Lys
        675                 680                 685

Val Ala Glu Ile Pro Leu Leu Leu Ile Val Lys Glu Pro Asp Tyr Pro
    690                 695                 700

Arg Val Thr Ser Val Thr Val Glu Pro Gly Ala Lys Gln Gly Ala Tyr
705                 710                 715                 720

Thr Ile Glu Ala Tyr Leu Pro Gly Gly Ala Glu Glu Leu Ala Phe Leu
                725                 730                 735

Val Tyr Asp Glu Asn Leu Asn Leu Leu Gly Gln Ala Gly Val Tyr Lys
            740                 745                 750

Asn Gln Gly Lys Gly Tyr Gln Ser Tyr Gln Trp Asn Gly Lys Ile Asn
        755                 760                 765

Asp Ala Ala Ser Leu Lys Ser Gly Lys Tyr Tyr Met Leu Ala Tyr Ala
    770                 775                 780

Ser Ala Lys Gly Lys Ser Ser Tyr Val Leu Thr Glu Asp Pro Phe Ile
785                 790                 795                 800

Val Glu

<210> 5
<211> 2771
<212> DNA
<213> Bacillus licheniformis

<220>
<221> CDS
<222> (175)..(2268)

<400> 5
gggcctactg tccttaaaaa attacacttc tatccagaac aaaagaaaaa gctagaactt 60

gaaagtcaaa gaaggtcaat gtatactaat agtcagaaat ggtcaaactg cagaccattt 120

tttctaagtc aattggtcaa aaaaggtcaa attcaataaa gggggttttg gcaa atg 177
Met
1

cgt tgt caa cat tgt caa aaa aat gag gcg acg att cgc cta aac atg 225
Arg Cys Gln His Cys Gln Lys Asn Glu Ala Thr Ile Arg Leu Asn Met
            5                   10                  15

caa atc aac ttt aca aaa aaa caa atg gat tta tgt gaa gac tgc tat 273
Gln Ile Asn Phe Thr Lys Lys Gln Met Asp Leu Cys Glu Asp Cys Tyr
        20                  25                  30

aaa gag ctg act cag cct tct atg ttt tca gga aat cac ttt ttt ggc 321
Lys Glu Leu Thr Gln Pro Ser Met Phe Ser Gly Asn His Phe Phe Gly
    35                  40                  45

ggc gga ggt tct ttc ttc agc cag cca tca caa gcg cag acg caa acc 369
Gly Gly Gly Ser Phe Phe Ser Gln Pro Ser Gln Ala Gln Thr Gln Thr
50                  55                  60                  65

gct gcc aaa aaa ggc ttg ctc gat gaa ctt gcc gtc aat tta aca aat 417
Ala Ala Lys Lys Gly Leu Leu Asp Glu Leu Ala Val Asn Leu Thr Asn
                70                  75                  80

gca gca aag acc ggc ttg atc gat ccg gtg atc ggc cgc gat gat gaa 465
Ala Ala Lys Thr Gly Leu Ile Asp Pro Val Ile Gly Arg Asp Asp Glu
            85                  90                  95

gta gcc cgt gtg atc gag atc tta aac cgc aga aat aaa aac aat ccg 513
Val Ala Arg Val Ile Glu Ile Leu Asn Arg Arg Asn Lys Asn Asn Pro
        100                 105                 110

gtc tta atc ggt gaa ccg ggc gtc ggt aaa aca gcc gtc gct gaa gga 561
Val Leu Ile Gly Glu Pro Gly Val Gly Lys Thr Ala Val Ala Glu Gly
    115                 120                 125

ctt gct ctc aaa atc gcc gaa ggc gat gtt cca aat aaa ttg aaa aac 609
Leu Ala Leu Lys Ile Ala Glu Gly Asp Val Pro Asn Lys Leu Lys Asn
130                 135                 140                 145

aaa gag ctc tac ctg ctt gat gtc gct tcc ctc gtg gca aac acc ggc 657
Lys Glu Leu Tyr Leu Leu Asp Val Ala Ser Leu Val Ala Asn Thr Gly
                150                 155                 160

atc aga ggg caa ttt gaa gaa aga atg aaa cag ctg atg aaa gag ctg 705
Ile Arg Gly Gln Phe Glu Glu Arg Met Lys Gln Leu Met Lys Glu Leu
            165                 170                 175

aaa gaa cgc aaa aac gca atc ttg ttt att gac gaa att cac ctt ctt 753
Lys Glu Arg Lys Asn Ala Ile Leu Phe Ile Asp Glu Ile His Leu Leu
        180                 185                 190

gtc ggg gcc ggc tct gca gaa ggc tcc atg gat gcg ggc aac att tta 801
Val Gly Ala Gly Ser Ala Glu Gly Ser Met Asp Ala Gly Asn Ile Leu
    195                 200                 205

aaa cct gca ctt gcc aga ggc gag ctg caa gtt atc ggc gct acg acg 849
Lys Pro Ala Leu Ala Arg Gly Glu Leu Gln Val Ile Gly Ala Thr Thr
210                 215                 220                 225

ttg aag gaa tat cgc caa att gaa aaa gat gcg gcg tta gag cgg cgc 897
Leu Lys Glu Tyr Arg Gln Ile Glu Lys Asp Ala Ala Leu Glu Arg Arg
                230                 235                 240

ttt cag ccg atc atg gtg cac gaa ccg aca att gag cag gcc gca gcg 945
Phe Gln Pro Ile Met Val His Glu Pro Thr Ile Glu Gln Ala Ala Ala
            245                 250                 255

att tta aac ggg ctc aag gaa aaa tac gaa gca tat cac gat gtg act 993
Ile Leu Asn Gly Leu Lys Glu Lys Tyr Glu Ala Tyr His Asp Val Thr
        260                 265                 270

tac acg gat gaa gcg atc aaa gcc tgc gtc att ctg tca agc cgc tat 1041
Tyr Thr Asp Glu Ala Ile Lys Ala Cys Val Ile Leu Ser Ser Arg Tyr
    275                 280                 285

att cag gac cgc cat ctt ccc gat aag gcg atc gac ctt ttg gat gaa 1089
Ile Gln Asp Arg His Leu Pro Asp Lys Ala Ile Asp Leu Leu Asp Glu
290                 295                 300                 305

gcc ggt tca aaa gcg aac ttg aaa atc gac acg gtc agc ggt gaa caa 1137
Ala Gly Ser Lys Ala Asn Leu Lys Ile Asp Thr Val Ser Gly Glu Gln
                310                 315                 320

gct tcc caa cgc ctg caa gaa atc gaa gcg gaa aaa gcc aaa gcg ctt 1185
Ala Ser Gln Arg Leu Gln Glu Ile Glu Ala Glu Lys Ala Lys Ala Leu
            325                 330                 335

caa gaa gaa aac tac gag ctt gca gca aaa ctc cgc gac gaa gaa gaa 1233
Gln Glu Glu Asn Tyr Glu Leu Ala Ala Lys Leu Arg Asp Glu Glu Glu
        340                 345                 350

gcg ctg cat aaa aaa atg gaa agc cat gag gaa gaa aaa cgg gca aca 1281
Ala Leu His Lys Lys Met Glu Ser His Glu Glu Glu Lys Arg Ala Thr
    355                 360                 365

gtt gaa gcg gaa gac att caa gcg atc atc gaa caa aaa acc ggc att 1329
Val Glu Ala Glu Asp Ile Gln Ala Ile Ile Glu Gln Lys Thr Gly Ile
370                 375                 380                 385

ccg gtc ggc aag ctt cag cac gac gat cag aag aaa atg aaa gag ctt 1377
Pro Val Gly Lys Leu Gln His Asp Asp Gln Lys Lys Met Lys Glu Leu
                390                 395                 400

gaa gca cac ctt tca agc cgc gtg atc ggt caa aaa gaa gcg gtg aaa 1425
Glu Ala His Leu Ser Ser Arg Val Ile Gly Gln Lys Glu Ala Val Lys
            405                 410                 415

aaa gtg gca aaa gcc gtc aga aga agc cgc gcc ggc ctg aaa tca aaa 1473
Lys Val Ala Lys Ala Val Arg Arg Ser Arg Ala Gly Leu Lys Ser Lys
        420                 425                 430

aac agg cct gtc ggt tca ttc ctg ttc gtc ggc ccg aca ggt gtc ggt 1521
Asn Arg Pro Val Gly Ser Phe Leu Phe Val Gly Pro Thr Gly Val Gly
    435                 440                 445

aaa aca gaa ctt tct aaa agg ctt gcg gaa gaa ctg ttc ggc acg aag 1569
Lys Thr Glu Leu Ser Lys Arg Leu Ala Glu Glu Leu Phe Gly Thr Lys
450                 455                 460                 465

gat gcc atc atc cgc ctc gat atg agc gaa tac atg gaa aaa cac gcc 1617
Asp Ala Ile Ile Arg Leu Asp Met Ser Glu Tyr Met Glu Lys His Ala
                470                 475                 480

gta tca aag ctg atc ggt tcg cct cct gga tat gtc ggc cac gaa gaa 1665
Val Ser Lys Leu Ile Gly Ser Pro Pro Gly Tyr Val Gly His Glu Glu
            485                 490                 495

gcc gga cag ctt acc gaa aaa gtc cgg agg aac ccg tac agc atc gtt 1713
Ala Gly Gln Leu Thr Glu Lys Val Arg Arg Asn Pro Tyr Ser Ile Val
        500                 505                 510

ttg ctc gat gaa atc gaa aaa gcc cat ccc gat gtc cag cac atg ttc 1761
Leu Leu Asp Glu Ile Glu Lys Ala His Pro Asp Val Gln His Met Phe
    515                 520                 525

ctg caa atc atg gaa gac gga cgt ctg aca gac agc caa ggg cgg acg 1809
Leu Gln Ile Met Glu Asp Gly Arg Leu Thr Asp Ser Gln Gly Arg Thr
530                 535                 540                 545

gtc agc ttt aaa gat acc gtc att att atg aca agc aat gcc ggt gta 1857
Val Ser Phe Lys Asp Thr Val Ile Ile Met Thr Ser Asn Ala Gly Val
                550                 555                 560

tct gaa aag cgg aca acc gtc ggt ttt aac cgg gaa gag caa gta atg 1905
Ser Glu Lys Arg Thr Thr Val Gly Phe Asn Arg Glu Glu Gln Val Met
            565                 570                 575

ggc gaa caa tca atg atc gat tca tta agc ggc tac ttt aag ccg gaa 1953
Gly Glu Gln Ser Met Ile Asp Ser Leu Ser Gly Tyr Phe Lys Pro Glu
        580                 585                 590

ttc ctg aac aga ttt gac agc atc atc gaa ttc cag ccg ctc gaa aaa 2001
Phe Leu Asn Arg Phe Asp Ser Ile Ile Glu Phe Gln Pro Leu Glu Lys
    595                 600                 605

gaa gat ctg gtc aag atc gcc gcc ctc ttg ctg aaa gag ctt gaa gaa 2049
Glu Asp Leu Val Lys Ile Ala Ala Leu Leu Leu Lys Glu Leu Glu Glu
610                 615                 620                 625

acg tta aaa gag caa aac atg acg ctc gaa gtt tca gat gag gca aaa 2097
Thr Leu Lys Glu Gln Asn Met Thr Leu Glu Val Ser Asp Glu Ala Lys
                630                 635                 640

gaa aaa atc gct gaa atc gga tac cat ccg gcc ttt ggc gcc cgc cca 2145
Glu Lys Ile Ala Glu Ile Gly Tyr His Pro Ala Phe Gly Ala Arg Pro
            645                 650                 655

tta aga aga acc att caa aca gcg gtc gaa gat caa atg acc gat ctg 2193
Leu Arg Arg Thr Ile Gln Thr Ala Val Glu Asp Gln Met Thr Asp Leu
        660                 665                 670

ctg ctt gaa gaa gaa cac atc aaa ggt ttt gcg gtt atc gtt gaa aac 2241
Leu Leu Glu Glu Glu His Ile Lys Gly Phe Ala Val Ile Val Glu Asn
    675                 680                 685

gat gaa atc cgg gtg aaa aca acc aga taacacacac aacggcctcc 2288
Asp Glu Ile Arg Val Lys Thr Thr Arg
690                 695

gatcgcggag gtcgttttac atacaacata agaatcacaa aagttctatt ttttaacatt 2348

aaataaccct aaagtccctt ttcaccatga ttttgcaaca atttaagaaa cagcctaaag 2408

ttcctatcac gcaacaccga tattaacgat agaaaagtta gttaaaggaa ttggtgaaaa 2468

aatggacaaa acatcgttaa ttggaatcat cttagctatc atcggagtag gtgtcggcat 2528

ggtcatgaaa ggtgtcagcc cttccgttct cgtcaatccc gcggcatggc tgatcatcct 2588

tgtcgggaca atcgcagcag ttgtgatcgc ttttccaatc cgcgagctca aaaaggttcc 2648

caaactcttc ggcatcttgt tcaaagagaa gaagatgatc gaaatcaacg aactgatccc 2708

gatgttttcc gagtgggcgc aaatcgcaag acgcgaagga cttctcgctt tggaagcaaa 2768

ttt 2771

<210> 6
<211> 698
<212> PRT
<213> Bacillus licheniformis

<400> 6

Met Arg Cys Gln His Cys Gln Lys Asn Glu Ala Thr Ile Arg Leu Asn
1               5                   10                  15

Met Gln Ile Asn Phe Thr Lys Lys Gln Met Asp Leu Cys Glu Asp Cys
            20                  25                  30

Tyr Lys Glu Leu Thr Gln Pro Ser Met Phe Ser Gly Asn His Phe Phe
        35                  40                  45

Gly Gly Gly Gly Ser Phe Phe Ser Gln Pro Ser Gln Ala Gln Thr Gln
    50                  55                  60

Thr Ala Ala Lys Lys Gly Leu Leu Asp Glu Leu Ala Val Asn Leu Thr
65                  70                  75                  80

Asn Ala Ala Lys Thr Gly Leu Ile Asp Pro Val Ile Gly Arg Asp Asp
                85                  90                  95

Glu Val Ala Arg Val Ile Glu Ile Leu Asn Arg Arg Asn Lys Asn Asn
            100                 105                 110

Pro Val Leu Ile Gly Glu Pro Gly Val Gly Lys Thr Ala Val Ala Glu
        115                 120                 125

Gly Leu Ala Leu Lys Ile Ala Glu Gly Asp Val Pro Asn Lys Leu Lys
    130                 135                 140

Asn Lys Glu Leu Tyr Leu Leu Asp Val Ala Ser Leu Val Ala Asn Thr
145                 150                 155                 160

Gly Ile Arg Gly Gln Phe Glu Glu Arg Met Lys Gln Leu Met Lys Glu
                165                 170                 175

Leu Lys Glu Arg Lys Asn Ala Ile Leu Phe Ile Asp Glu Ile His Leu

            180                 185                 190

Leu Val Gly Ala Gly Ser Ala Glu Gly Ser Met Asp Ala Gly Asn Ile
        195                 200                 205

Leu Lys Pro Ala Leu Ala Arg Gly Glu Leu Gln Val Ile Gly Ala Thr
    210                 215                 220

Thr Leu Lys Glu Tyr Arg Gln Ile Glu Lys Asp Ala Ala Leu Glu Arg
225                 230                 235                 240

Arg Phe Gln Pro Ile Met Val His Glu Pro Thr Ile Glu Gln Ala Ala
                245                 250                 255

Ala Ile Leu Asn Gly Leu Lys Glu Lys Tyr Glu Ala Tyr His Asp Val
            260                 265                 270

Thr Tyr Thr Asp Glu Ala Ile Lys Ala Cys Val Ile Leu Ser Ser Arg
        275                 280                 285

Tyr Ile Gln Asp Arg His Leu Pro Asp Lys Ala Ile Asp Leu Leu Asp
    290                 295                 300

Glu Ala Gly Ser Lys Ala Asn Leu Lys Ile Asp Thr Val Ser Gly Glu
305                 310                 315                 320

Gln Ala Ser Gln Arg Leu Gln Glu Ile Glu Ala Glu Lys Ala Lys Ala
                325                 330                 335

Leu Gln Glu Glu Asn Tyr Glu Leu Ala Ala Lys Leu Arg Asp Glu Glu
            340                 345                 350

Glu Ala Leu His Lys Lys Met Glu Ser His Glu Glu Glu Lys Arg Ala
        355                 360                 365

Thr Val Glu Ala Glu Asp Ile Gln Ala Ile Ile Glu Gln Lys Thr Gly
    370                 375                 380

Ile Pro Val Gly Lys Leu Gln His Asp Asp Gln Lys Lys Met Lys Glu
385                 390                 395                 400

Leu Glu Ala His Leu Ser Ser Arg Val Ile Gly Gln Lys Glu Ala Val
                405                 410                 415

Lys Lys Val Ala Lys Ala Val Arg Arg Ser Arg Ala Gly Leu Lys Ser
            420                 425                 430

Lys Asn Arg Pro Val Gly Ser Phe Leu Phe Val Gly Pro Thr Gly Val
        435                 440                 445

Gly Lys Thr Glu Leu Ser Lys Arg Leu Ala Glu Glu Leu Phe Gly Thr

450                 455                 460

Lys Asp Ala Ile Ile Arg Leu Asp Met Ser Glu Tyr Met Glu Lys His
465                 470                 475                 480

Ala Val Ser Lys Leu Ile Gly Ser Pro Pro Gly Tyr Val Gly His Glu
                485                 490                 495

Glu Ala Gly Gln Leu Thr Glu Lys Val Arg Arg Asn Pro Tyr Ser Ile
            500                 505                 510

Val Leu Leu Asp Glu Ile Glu Lys Ala His Pro Asp Val Gln His Met
        515                 520                 525

Phe Leu Gln Ile Met Glu Asp Gly Arg Leu Thr Asp Ser Gln Gly Arg
    530                 535                 540

Thr Val Ser Phe Lys Asp Thr Val Ile Ile Met Thr Ser Asn Ala Gly
545                 550                 555                 560

Val Ser Glu Lys Arg Thr Thr Val Gly Phe Asn Arg Glu Glu Gln Val
                565                 570                 575

Met Gly Glu Gln Ser Met Ile Asp Ser Leu Ser Gly Tyr Phe Lys Pro
            580                 585                 590

Glu Phe Leu Asn Arg Phe Asp Ser Ile Ile Glu Phe Gln Pro Leu Glu
        595                 600                 605

Lys Glu Asp Leu Val Lys Ile Ala Ala Leu Leu Leu Lys Glu Leu Glu
    610                 615                 620

Glu Thr Leu Lys Glu Gln Asn Met Thr Leu Glu Val Ser Asp Glu Ala
625                 630                 635                 640

Lys Glu Lys Ile Ala Glu Ile Gly Tyr His Pro Ala Phe Gly Ala Arg
                645                 650                 655

Pro Leu Arg Arg Thr Ile Gln Thr Ala Val Glu Asp Gln Met Thr Asp
            660                 665                 670

Leu Leu Leu Glu Glu Glu His Ile Lys Gly Phe Ala Val Ile Val Glu
        675                 680                 685

Asn Asp Glu Ile Arg Val Lys Thr Thr Arg
    690                 695

<210>  7
<211>  2958
<212>  DNA
<213>  Bacillus licheniformis

<220>
<221>  CDS
<222>  (501)..(2459)

<400>  7
gctgacaaaa gatgcgcggg atgttcttcc cgaaagcctc tcttatccga aagccgtgga      60

agcaagcgcg gtcagcaata tgctcgtcgc cggactgatg gcgaaagatt ggaagcttgt     120

cggccgcatg atgcaaaaag acctctttca ccagccatac cgaagggcgc tcgtccctga     180

gctttcgaaa gtggagcatg aagcaggcca aaacggcgcg ttcgggacgg ctttaagcgg     240

agccggcccg acgatccttt cctttattga aaaaggaaaa ggagaggcgc tgagaaacca     300

gcttgcgtcc aagtttccgc actgtgaggt cgactgcctt tatgtcccgg atacaggcat     360

tatcgttgaa agaaaatcag taaacagtgt ttaacgaaaa aagagctttt tcggctcttt     420

ttttgtttgc cttcaaaaca aggattttta tttaggaagt cgaagtagta tggcaaacag     480

acatgaggag gagaaacgac atg aag cag ctg ata acc gaa aaa gac ctc atc     533
                    Met Lys Gln Leu Ile Thr Glu Lys Asp Leu Ile
                    1               5                   10

aag ctt gta tcc att acc gat ccc cag tat tcg ccc gat ggt gca aaa       581
Lys Leu Val Ser Ile Thr Asp Pro Gln Tyr Ser Pro Asp Gly Ala Lys
            15                  20                  25

atc gcc tac gtc caa aca aaa gta aac gaa aaa cag gac tca tat gat       629
Ile Ala Tyr Val Gln Thr Lys Val Asn Glu Lys Gln Asp Ser Tyr Asp
        30                  35                  40

tcg cat atc atg atc tac gac cgc gaa aaa caa gct tcg gtg caa tgg       677
Ser His Ile Met Ile Tyr Asp Arg Glu Lys Gln Ala Ser Val Gln Trp
    45                  50                  55

acg ttt ggg aaa ggc aga aac cag cat ccc cgc tgg tcg ccg gac ggc       725
Thr Phe Gly Lys Gly Arg Asn Gln His Pro Arg Trp Ser Pro Asp Gly
60                  65                  70                  75

aaa tac ctt gca ttt acg tcc aat cgc gag gaa acc gca caa att tac       773
Lys Tyr Leu Ala Phe Thr Ser Asn Arg Glu Glu Thr Ala Gln Ile Tyr
                80                  85                  90

gtg ata agc gcc gcc ggg gga gaa gcg aga aaa gtg acc gat att ccc       821
Val Ile Ser Ala Ala Gly Gly Glu Ala Arg Lys Val Thr Asp Ile Pro
            95                  100                 105

tat gat gta tca cag cct gaa tgg tcg ccg gac ggc aag tca ttg ctt       869
Tyr Asp Val Ser Gln Pro Glu Trp Ser Pro Asp Gly Lys Ser Leu Leu
        110                 115                 120

tgt tca gtg aag ctg aca aaa gag gaa agc gtc gac gat gag aag aaa       917
Cys Ser Val Lys Leu Thr Lys Glu Glu Ser Val Asp Asp Glu Lys Lys
    125                 130                 135

acc gag att gaa gac cac gaa ccg ctg gaa gtc gat tct ttg tca tat       965
Thr Glu Ile Glu Asp His Glu Pro Leu Glu Val Asp Ser Leu Ser Tyr
140                 145                 150                 155

aaa gca gac ggc caa ggg ttt aaa agg gga aag tat acg cag ctt gtc      1013
Lys Ala Asp Gly Gln Gly Phe Lys Arg Gly Lys Tyr Thr Gln Leu Val
                160                 165                 170

ctc gtt caa gtg gaa acc ggg gaa atg aaa cag ctg acc gat ctt gaa      1061
Leu Val Gln Val Glu Thr Gly Glu Met Lys Gln Leu Thr Asp Leu Glu

            175                 180                 185

cgc gac cat ttc agc cat gca ttt tca ccg tgc gga gat cag att gca 1109
Arg Asp His Phe Ser His Ala Phe Ser Pro Cys Gly Asp Gln Ile Ala
        190                 195                 200

ttt tgc gcc aat caa acg gat atg cgt gtg aac gat gtc tac ttg atg 1157
Phe Cys Ala Asn Gln Thr Asp Met Arg Val Asn Asp Val Tyr Leu Met
    205                 210                 215

aac ctt tcg aca ggt gat ttg aag cgc ctc aca ggt caa aac ggc att 1205
Asn Leu Ser Thr Gly Asp Leu Lys Arg Leu Thr Gly Gln Asn Gly Ile
220                 225                 230                 235

ttt tct tca cta tcg ttt tct cct gac ggc aaa cac ctt gca ttt atc 1253
Phe Ser Ser Leu Ser Phe Ser Pro Asp Gly Lys His Leu Ala Phe Ile
                240                 245                 250

gga aac gag aaa gaa tat caa aac gcc acg ctc gat aaa gca tgg ctg 1301
Gly Asn Glu Lys Glu Tyr Gln Asn Ala Thr Leu Asp Lys Ala Trp Leu
            255                 260                 265

tat gat att gaa aca gga aaa cta acg tgt ctg acg gaa atg ctt gac 1349
Tyr Asp Ile Glu Thr Gly Lys Leu Thr Cys Leu Thr Glu Met Leu Asp
        270                 275                 280

gtc cat ctc agc gat gcg gtg gca gga gac agc ctt gtc gga ggc gtt 1397
Val His Leu Ser Asp Ala Val Ala Gly Asp Ser Leu Val Gly Gly Val
    285                 290                 295

ctt ccg aaa ccg gcc tgg acc aaa gac gga aac ggc ttt tat gtc atc 1445
Leu Pro Lys Pro Ala Trp Thr Lys Asp Gly Asn Gly Phe Tyr Val Ile
300                 305                 310                 315

gga tct gac cag ggt tct aca ggc att tac tac ata tca atc gaa ggt 1493
Gly Ser Asp Gln Gly Ser Thr Gly Ile Tyr Tyr Ile Ser Ile Glu Gly
                320                 325                 330

ctt gct tat ccg gtc cgg ttg gaa aaa gag cat gtg aac gga ttc agc 1541
Leu Ala Tyr Pro Val Arg Leu Glu Lys Glu His Val Asn Gly Phe Ser
            335                 340                 345

ctg cat ccg gac gaa agt ggc ttt gcg gca tcc ata gcc ctg ccc gtc 1589
Leu His Pro Asp Glu Ser Gly Phe Ala Ala Ser Ile Ala Leu Pro Val
        350                 355                 360

cgg ccg agc gaa ctt tac cat ctc caa ttg ggt gaa gaa aaa gcg gag 1637
Arg Pro Ser Glu Leu Tyr His Leu Gln Leu Gly Glu Glu Lys Ala Glu
    365                 370                 375

cga cta aca gat gcc aac caa gcg ttt acg gaa gaa cat atc ata tcg 1685
Arg Leu Thr Asp Ala Asn Gln Ala Phe Thr Glu Glu His Ile Ile Ser
380                 385                 390                 395

gaa ccg gag gag ctt caa ttt cag gca gcg gac ggt ctg aca att cac 1733
Glu Pro Glu Glu Leu Gln Phe Gln Ala Ala Asp Gly Leu Thr Ile His
                400                 405                 410

gga tgg ctg ata aag ccc gcc caa tat gaa aaa gga aac acg tat ccg 1781
Gly Trp Leu Ile Lys Pro Ala Gln Tyr Glu Lys Gly Asn Thr Tyr Pro
            415                 420                 425

ctc att ctg gaa gtg cac ggc gga cct cac gcc atg tat gcg aat gct 1829
Leu Ile Leu Glu Val His Gly Gly Pro His Ala Met Tyr Ala Asn Ala
        430                 435                 440

tat ttt cat gaa ttt caa gtg ctg gcc gca aag gga agc gcc gtc gta 1877
Tyr Phe His Glu Phe Gln Val Leu Ala Ala Lys Gly Ser Ala Val Val

445                 450                 455

tat gtt aac ccg cgg gga agc cac ggc tac gga cag gat ttt gtc aac      1925
Tyr Val Asn Pro Arg Gly Ser His Gly Tyr Gly Gln Asp Phe Val Asn
460                 465                 470                 475

cgg gtg agg ggc gac tac ggc ggt gga gat ttt aag gat gta atg gcg      1973
Arg Val Arg Gly Asp Tyr Gly Gly Gly Asp Phe Lys Asp Val Met Ala
                480                 485                 490

gct gtt gac cac gtg ctt gaa cat tat gat ttc gtc gat caa gaa agg      2021
Ala Val Asp His Val Leu Glu His Tyr Asp Phe Val Asp Gln Glu Arg
            495                 500                 505

ctc ggc atc acc gga gga agc tac ggg ggc ttt atg aca aac tgg gct      2069
Leu Gly Ile Thr Gly Gly Ser Tyr Gly Gly Phe Met Thr Asn Trp Ala
        510                 515                 520

gtc ggc cac acg aag cgc ttc aag gcc gca gtg aca cag agg tcc att      2117
Val Gly His Thr Lys Arg Phe Lys Ala Ala Val Thr Gln Arg Ser Ile
    525                 530                 535

tcc aac tgg atc agt ttt tac ggg gta agc gac atc ggc tat ttt ttc      2165
Ser Asn Trp Ile Ser Phe Tyr Gly Val Ser Asp Ile Gly Tyr Phe Phe
540                 545                 550                 555

aca gac tgg cag ctc ggt gca gac ctt ttt gaa gat cca ggc aaa ctg      2213
Thr Asp Trp Gln Leu Gly Ala Asp Leu Phe Glu Asp Pro Gly Lys Leu
                560                 565                 570

tgg gag cat tcg ccg ctc aaa tac gcg gac aag gtg gag acc cct ctt      2261
Trp Glu His Ser Pro Leu Lys Tyr Ala Asp Lys Val Glu Thr Pro Leu
            575                 580                 585

ctc att ctg cat ggc gaa cgg gac gac agg tgc ccg att gaa cag gcg      2309
Leu Ile Leu His Gly Glu Arg Asp Asp Arg Cys Pro Ile Glu Gln Ala
        590                 595                 600

gaa cag ctg ttc acg gcg ctg aaa aaa atg ggg aaa gaa gta aaa ctc      2357
Glu Gln Leu Phe Thr Ala Leu Lys Lys Met Gly Lys Glu Val Lys Leu
    605                 610                 615

gtc aga ttt ccg aac gcg tca cac gat tta tca aga agc ggg cat ccg      2405
Val Arg Phe Pro Asn Ala Ser His Asp Leu Ser Arg Ser Gly His Pro
620                 625                 630                 635

aag cag cgg atc agg cgg ctt gag tat att gca ggc tgg ttt gaa gct      2453
Lys Gln Arg Ile Arg Arg Leu Glu Tyr Ile Ala Gly Trp Phe Glu Ala
                640                 645                 650

tat cta taaaaaagaa aaggctgccg tacactacgg cagcctcagc gtgtcgacaa      2509
Tyr Leu

accctcgcat tcgttgtcag ggctgcgcgt cggtgctcac gaattccaac attcgctccg      2569

ctccgatgct cgaccttcct agactgcaag ggttttcaat cacgctgaga ggatgacaaa      2629

atcctaaact gaaaaccgtt ttaggatttt gtcaacaatt taggctgccg ttacggcagc      2689

cttctcaaat taaaatactt gctctacttc aatgacgccc ggaacttctt ctaaaagggc      2749

gcgctcaatt ccagctttaa gggtgatggt ggaacttggg cagcttccgc atgcgccgag      2809

caggcgaagc ttgacgatgc cgtcttcaac atcgacgagt tcacagtcac cgccgtcacg      2869

gagtaaaaac ggacgaagtt tgtccagcac ttcctgtact tggtccttca tttcgacctc      2929

tgtcgccatt cgtatcgctc cttttaaat                                    2958

<210>  8
<211>  653
<212>  PRT
<213>  Bacillus licheniformis

<400>  8

Met Lys Gln Leu Ile Thr Glu Lys Asp Leu Ile Lys Leu Val Ser Ile
1               5                   10                  15

Thr Asp Pro Gln Tyr Ser Pro Asp Gly Ala Lys Ile Ala Tyr Val Gln
            20                  25                  30

Thr Lys Val Asn Glu Lys Gln Asp Ser Tyr Asp Ser His Ile Met Ile
        35                  40                  45

Tyr Asp Arg Glu Lys Gln Ala Ser Val Gln Trp Thr Phe Gly Lys Gly
    50                  55                  60

Arg Asn Gln His Pro Arg Trp Ser Pro Asp Gly Lys Tyr Leu Ala Phe
65                  70                  75                  80

Thr Ser Asn Arg Glu Glu Thr Ala Gln Ile Tyr Val Ile Ser Ala Ala
                85                  90                  95

Gly Gly Glu Ala Arg Lys Val Thr Asp Ile Pro Tyr Asp Val Ser Gln
            100                 105                 110

Pro Glu Trp Ser Pro Asp Gly Lys Ser Leu Leu Cys Ser Val Lys Leu
        115                 120                 125

Thr Lys Glu Glu Ser Val Asp Asp Glu Lys Lys Thr Glu Ile Glu Asp
    130                 135                 140

His Glu Pro Leu Glu Val Asp Ser Leu Ser Tyr Lys Ala Asp Gly Gln
145                 150                 155                 160

Gly Phe Lys Arg Gly Lys Tyr Thr Gln Leu Val Leu Val Gln Val Glu
                165                 170                 175

Thr Gly Glu Met Lys Gln Leu Thr Asp Leu Glu Arg Asp His Phe Ser
            180                 185                 190

His Ala Phe Ser Pro Cys Gly Asp Gln Ile Ala Phe Cys Ala Asn Gln
        195                 200                 205

Thr Asp Met Arg Val Asn Asp Val Tyr Leu Met Asn Leu Ser Thr Gly
    210                 215                 220

Asp Leu Lys Arg Leu Thr Gly Gln Asn Gly Ile Phe Ser Ser Leu Ser
225                 230                 235                 240

Phe Ser Pro Asp Gly Lys His Leu Ala Phe Ile Gly Asn Glu Lys Glu
                245                 250                 255

Tyr Gln Asn Ala Thr Leu Asp Lys Ala Trp Leu Tyr Asp Ile Glu Thr
            260                 265                 270

Gly Lys Leu Thr Cys Leu Thr Glu Met Leu Asp Val His Leu Ser Asp
        275                 280                 285

Ala Val Ala Gly Asp Ser Leu Val Gly Gly Val Leu Pro Lys Pro Ala
    290                 295                 300

Trp Thr Lys Asp Gly Asn Gly Phe Tyr Val Ile Gly Ser Asp Gln Gly
305                 310                 315                 320

Ser Thr Gly Ile Tyr Tyr Ile Ser Ile Glu Gly Leu Ala Tyr Pro Val
                325                 330                 335

Arg Leu Glu Lys Glu His Val Asn Gly Phe Ser Leu His Pro Asp Glu
            340                 345                 350

Ser Gly Phe Ala Ala Ser Ile Ala Leu Pro Val Arg Pro Ser Glu Leu
        355                 360                 365

Tyr His Leu Gln Leu Gly Glu Glu Lys Ala Glu Arg Leu Thr Asp Ala
    370                 375                 380

Asn Gln Ala Phe Thr Glu Glu His Ile Ile Ser Glu Pro Glu Glu Leu
385                 390                 395                 400

Gln Phe Gln Ala Ala Asp Gly Leu Thr Ile His Gly Trp Leu Ile Lys
                405                 410                 415

Pro Ala Gln Tyr Glu Lys Gly Asn Thr Tyr Pro Leu Ile Leu Glu Val
            420                 425                 430

His Gly Gly Pro His Ala Met Tyr Ala Asn Ala Tyr Phe His Glu Phe
        435                 440                 445

Gln Val Leu Ala Ala Lys Gly Ser Ala Val Val Tyr Val Asn Pro Arg
    450                 455                 460

Gly Ser His Gly Tyr Gly Gln Asp Phe Val Asn Arg Val Arg Gly Asp
465                 470                 475                 480

Tyr Gly Gly Gly Asp Phe Lys Asp Val Met Ala Ala Val Asp His Val
                485                 490                 495

Leu Glu His Tyr Asp Phe Val Asp Gln Glu Arg Leu Gly Ile Thr Gly
            500                 505                 510

Gly Ser Tyr Gly Gly Phe Met Thr Asn Trp Ala Val Gly His Thr Lys
        515                 520                 525

Arg Phe Lys Ala Ala Val Thr Gln Arg Ser Ile Ser Asn Trp Ile Ser
    530                 535                 540

Phe Tyr Gly Val Ser Asp Ile Gly Tyr Phe Phe Thr Asp Trp Gln Leu
545                 550                 555                 560

Gly Ala Asp Leu Phe Glu Asp Pro Gly Lys Leu Trp Glu His Ser Pro
                565                 570                 575

Leu Lys Tyr Ala Asp Lys Val Glu Thr Pro Leu Leu Ile Leu His Gly
            580                 585                 590

Glu Arg Asp Asp Arg Cys Pro Ile Glu Gln Ala Glu Gln Leu Phe Thr
        595                 600                 605

Ala Leu Lys Lys Met Gly Lys Glu Val Lys Leu Val Arg Phe Pro Asn
    610                 615                 620

Ala Ser His Asp Leu Ser Arg Ser Gly His Pro Lys Gln Arg Ile Arg
625                 630                 635                 640

Arg Leu Glu Tyr Ile Ala Gly Trp Phe Glu Ala Tyr Leu
                645                 650

<210>  9
<211>  2473
<212>  DNA
<213>  Bacillus licheniformis

<220>
<221>  CDS
<222>  (148)..(1974)

<400>  9
tatgtatttt atttttaac ggcgggattt tgtgtctttt ttttcgcata tgatttgaca      60

aaagacggtg aaaaaacaga agatgaacac aatacaagtt tattggataa caatggactt     120

ggagtaaaga aagcgagggg attgaca gtg gct gaa gaa aaa aaa tcg aag aaa     174
                              Val Ala Glu Glu Lys Lys Ser Lys Lys
                              1               5

ctg cct tca aga gaa gag gtg aag caa gaa gac acg tgg aga ctg gag       222
Leu Pro Ser Arg Glu Glu Val Lys Gln Glu Asp Thr Trp Arg Leu Glu
10                  15                  20                  25

gat atc ttc ccg tca gat gac gct tgg aac gag gaa ttt caa gcc gtt       270
Asp Ile Phe Pro Ser Asp Asp Ala Trp Asn Glu Glu Phe Gln Ala Val
                30                  35                  40

aaa gaa ctg ctg ccc aag ctt tcc gaa ttc aag ggc aag ctc ggc cat       318
Lys Glu Leu Leu Pro Lys Leu Ser Glu Phe Lys Gly Lys Leu Gly His
            45                  50                  55

tcc gcc gat gat tta tat gaa gcg ctg act tat cag gat aag gtg atg 366
Ser Ala Asp Asp Leu Tyr Glu Ala Leu Thr Tyr Gln Asp Lys Val Met
        60                  65                  70

gag cgt ctc ggg aag ctg tat act tac gct cat atg cgc tat gat cag 414
Glu Arg Leu Gly Lys Leu Tyr Thr Tyr Ala His Met Arg Tyr Asp Gln
    75                  80                  85

gat acg gga aat tct ttt tac caa ggt ctc aat gat aaa gcg gcg aac 462
Asp Thr Gly Asn Ser Phe Tyr Gln Gly Leu Asn Asp Lys Ala Ala Asn
90                  95                  100                 105

ctc tat act cag gct gcg agc gcc acg gcc tac atg gtg ccg gaa ata 510
Leu Tyr Thr Gln Ala Ala Ser Ala Thr Ala Tyr Met Val Pro Glu Ile
                110                 115                 120

tta tcg att cag gaa gaa aag ctt cag cag ttc ctg ttg gaa aaa gag 558
Leu Ser Ile Gln Glu Glu Lys Leu Gln Gln Phe Leu Leu Glu Lys Glu
            125                 130                 135

gaa tta aag ctt tac tca cac gct ctc gaa gaa atc aat aaa gag cgc 606
Glu Leu Lys Leu Tyr Ser His Ala Leu Glu Glu Ile Asn Lys Glu Arg
        140                 145                 150

cct cat gtg ctt agc gag gaa gag gaa ggg att ttg gcc gaa gcg tct 654
Pro His Val Leu Ser Glu Glu Glu Glu Gly Ile Leu Ala Glu Ala Ser
    155                 160                 165

gat gtt ctt tca tct cct tcc aat aca ttc ggc atg ctg aat aac gcc 702
Asp Val Leu Ser Ser Pro Ser Asn Thr Phe Gly Met Leu Asn Asn Ala
170                 175                 180                 185

gat atg gaa ttt ccg gag atc acc gat gaa aac gga gaa aag gtg cag 750
Asp Met Glu Phe Pro Glu Ile Thr Asp Glu Asn Gly Glu Lys Val Gln
                190                 195                 200

ctc aca cac ggc aat tat atc acc ttt ttg gaa agc gag aac cgc gat 798
Leu Thr His Gly Asn Tyr Ile Thr Phe Leu Glu Ser Glu Asn Arg Asp
            205                 210                 215

gtc cgc cgc gcg gca ttc aag gcc gtt tat gaa aca tac ggc cgc ttt 846
Val Arg Arg Ala Ala Phe Lys Ala Val Tyr Glu Thr Tyr Gly Arg Phe
        220                 225                 230

aaa aac acg cta gct tcc acg ctc agc gga gct gtg aaa aaa gac aac 894
Lys Asn Thr Leu Ala Ser Thr Leu Ser Gly Ala Val Lys Lys Asp Asn
    235                 240                 245

ttt tat gcg aag gtt aaa cat tat aaa tcg gcg cgg gaa gct gcc ttg 942
Phe Tyr Ala Lys Val Lys His Tyr Lys Ser Ala Arg Glu Ala Ala Leu
250                 255                 260                 265

tcg cgc aac agc att cct gaa gaa gtc tac gac aat ctt gtc agc acg 990
Ser Arg Asn Ser Ile Pro Glu Glu Val Tyr Asp Asn Leu Val Ser Thr
                270                 275                 280

atc aac aag cat ctc ccg ctt ctt cac cgc tat gtt gag att aga aaa 1038
Ile Asn Lys His Leu Pro Leu Leu His Arg Tyr Val Glu Ile Arg Lys
            285                 290                 295

aaa gtg ctt gag ctt gat gaa gtg cac atg tat gac ctg tat acc cct 1086
Lys Val Leu Glu Leu Asp Glu Val His Met Tyr Asp Leu Tyr Thr Pro
        300                 305                 310

ctg gtc aag gat tct gga atg aaa gtc act tac gag cag gca aag gac 1134
Leu Val Lys Asp Ser Gly Met Lys Val Thr Tyr Glu Gln Ala Lys Asp
    315                 320                 325

tat atg ctg aag ggt ctg gcg cct ttg ggg gaa gaa tat tca tcc atc 1182
Tyr Met Leu Lys Gly Leu Ala Pro Leu Gly Glu Glu Tyr Ser Ser Ile
330                 335                 340                 345

ctg aaa gaa gga ttg aac aac cgc tgg gtc gat gtg tat gaa aac aaa 1230
Leu Lys Glu Gly Leu Asn Asn Arg Trp Val Asp Val Tyr Glu Asn Lys
                350                 355                 360

gga aaa cga agc ggc gcc tat tct tca gga act tac gga acc aat ccg 1278
Gly Lys Arg Ser Gly Ala Tyr Ser Ser Gly Thr Tyr Gly Thr Asn Pro
            365                 370                 375

tat att ttg atg aac tgg cag gac aac gtc aac aat ctg ttt acg ctc 1326
Tyr Ile Leu Met Asn Trp Gln Asp Asn Val Asn Asn Leu Phe Thr Leu
        380                 385                 390

gtc cat gaa ttc ggc cat tcc gtg cac agc tac tat acg aga aaa tat 1374
Val His Glu Phe Gly His Ser Val His Ser Tyr Tyr Thr Arg Lys Tyr
    395                 400                 405

cag cct tac ccg tac ggt aac tac agc att ttc gta gcc gaa gtt gct 1422
Gln Pro Tyr Pro Tyr Gly Asn Tyr Ser Ile Phe Val Ala Glu Val Ala
410                 415                 420                 425

tct acg acg aat gaa gcg ctg ttg gga gaa tac ctg ctg aat aca atc 1470
Ser Thr Thr Asn Glu Ala Leu Leu Gly Glu Tyr Leu Leu Asn Thr Ile
                430                 435                 440

gaa gat gaa aaa cag cgg ctc tat att ttg aat cat atg ctc gaa ggc 1518
Glu Asp Glu Lys Gln Arg Leu Tyr Ile Leu Asn His Met Leu Glu Gly
            445                 450                 455

ttt aaa gga acg gtt ttc aga cag acg atg ttt gcc gaa ttt gaa cat 1566
Phe Lys Gly Thr Val Phe Arg Gln Thr Met Phe Ala Glu Phe Glu His
        460                 465                 470

gag atc cat gtc aaa gcc cag gaa ggg gaa ccg ctg aca cct gag ctg 1614
Glu Ile His Val Lys Ala Gln Glu Gly Glu Pro Leu Thr Pro Glu Leu
    475                 480                 485

tta acg agc ata tac tat gat ttg aat aag aaa tat ttc gga gac aat 1662
Leu Thr Ser Ile Tyr Tyr Asp Leu Asn Lys Lys Tyr Phe Gly Asp Asn
490                 495                 500                 505

att gag att gat aag gaa atc ggt ctt gag tgg tca aga att ccg cat 1710
Ile Glu Ile Asp Lys Glu Ile Gly Leu Glu Trp Ser Arg Ile Pro His
                510                 515                 520

ttc tat tac aat tat tat gta tat cag tat gcg aca ggc ttc agt gca 1758
Phe Tyr Tyr Asn Tyr Tyr Val Tyr Gln Tyr Ala Thr Gly Phe Ser Ala
            525                 530                 535

gct cag gcg ctc agc cag cag att tta aaa gaa ggc aaa tcg gct gtt 1806
Ala Gln Ala Leu Ser Gln Gln Ile Leu Lys Glu Gly Lys Ser Ala Val
        540                 545                 550

gac cgc tat att gag ttc ttg aaa gct gga agc tcg gat tat ccg att 1854
Asp Arg Tyr Ile Glu Phe Leu Lys Ala Gly Ser Ser Asp Tyr Pro Ile
    555                 560                 565

gaa gtg ctg aaa aaa gcc ggc gtc gat atg acg tct tca gag ccg atc 1902
Glu Val Leu Lys Lys Ala Gly Val Asp Met Thr Ser Ser Glu Pro Ile
570                 575                 580                 585

gaa gct gcg tgc aaa aag ttt gaa gag cag ctg aat gaa atg gaa gaa 1950
Glu Ala Ala Cys Lys Lys Phe Glu Glu Gln Leu Asn Glu Met Glu Glu
                590                 595                 600

ctc ctg caa aaa gtc aat cat tca taaaaggcgt atgcatgaaa gaggctgccg 2004
Leu Leu Gln Lys Val Asn His Ser
            605

gtctaccggc agccttgttc gcgatgatgt caaaaacctt taaacgtttt gcggtggttg 2064

aggcggctga tcgtaaaaag gattgatata aataataggg gagacgtaat ataaaggggt 2124

agcatattca tcaaacctaa aatattcaag acgaaaaaaa ataaaattga tgcaagtccc 2184

aaaatgatct gaagcataaa aaccctccta aggactgata aaatattaag ggggcgacga 2244

agcgcaataa gaccggcaaa accgggcgcc agtccgcata attcggggtt tggcagcgtg 2304

cgtcgggctc gttttctttg ttactttgat catatgatgg acatctatta tttatgtttt 2364

tgtgacaaaa atgtgaaaac taatgaaatc agttgtcaaa actcgacatg atttgatatt 2424

ataaagatgt gaaattgatc acaaacaaac atttacccct ttgtttgac 2473

<210> 10
<211> 609
<212> PRT
<213> Bacillus licheniformis

<400> 10

Val Ala Glu Glu Lys Lys Ser Lys Lys Leu Pro Ser Arg Glu Glu Val
1               5                   10                  15

Lys Gln Glu Asp Thr Trp Arg Leu Glu Asp Ile Phe Pro Ser Asp Asp
            20                  25                  30

Ala Trp Asn Glu Glu Phe Gln Ala Val Lys Glu Leu Leu Pro Lys Leu
        35                  40                  45

Ser Glu Phe Lys Gly Lys Leu Gly His Ser Ala Asp Asp Leu Tyr Glu
    50                  55                  60

Ala Leu Thr Tyr Gln Asp Lys Val Met Glu Arg Leu Gly Lys Leu Tyr
65                  70                  75                  80

Thr Tyr Ala His Met Arg Tyr Asp Gln Asp Thr Gly Asn Ser Phe Tyr
                85                  90                  95

Gln Gly Leu Asn Asp Lys Ala Ala Asn Leu Tyr Thr Gln Ala Ala Ser
            100                 105                 110

Ala Thr Ala Tyr Met Val Pro Glu Ile Leu Ser Ile Gln Glu Glu Lys
        115                 120                 125

Leu Gln Gln Phe Leu Leu Glu Lys Glu Glu Leu Lys Leu Tyr Ser His
    130                 135                 140

Ala Leu Glu Glu Ile Asn Lys Glu Arg Pro His Val Leu Ser Glu Glu
145                 150                 155                 160

Glu Glu Gly Ile Leu Ala Glu Ala Ser Asp Val Leu Ser Ser Pro Ser
                165                 170                 175

Asn Thr Phe Gly Met Leu Asn Asn Ala Asp Met Glu Phe Pro Glu Ile
            180                 185                 190

Thr Asp Glu Asn Gly Glu Lys Val Gln Leu Thr His Gly Asn Tyr Ile
        195                 200                 205

Thr Phe Leu Glu Ser Glu Asn Arg Asp Val Arg Arg Ala Ala Phe Lys
    210                 215                 220

Ala Val Tyr Glu Thr Tyr Gly Arg Phe Lys Asn Thr Leu Ala Ser Thr
225                 230                 235                 240

Leu Ser Gly Ala Val Lys Lys Asp Asn Phe Tyr Ala Lys Val Lys His
                245                 250                 255

Tyr Lys Ser Ala Arg Glu Ala Ala Leu Ser Arg Asn Ser Ile Pro Glu
            260                 265                 270

Glu Val Tyr Asp Asn Leu Val Ser Thr Ile Asn Lys His Leu Pro Leu
        275                 280                 285

Leu His Arg Tyr Val Glu Ile Arg Lys Lys Val Leu Glu Leu Asp Glu
    290                 295                 300

Val His Met Tyr Asp Leu Tyr Thr Pro Leu Val Lys Asp Ser Gly Met
305                 310                 315                 320

Lys Val Thr Tyr Glu Gln Ala Lys Asp Tyr Met Leu Lys Gly Leu Ala
                325                 330                 335

Pro Leu Gly Glu Glu Tyr Ser Ser Ile Leu Lys Glu Gly Leu Asn Asn
            340                 345                 350

Arg Trp Val Asp Val Tyr Glu Asn Lys Gly Lys Arg Ser Gly Ala Tyr
        355                 360                 365

Ser Ser Gly Thr Tyr Gly Thr Asn Pro Tyr Ile Leu Met Asn Trp Gln
    370                 375                 380

Asp Asn Val Asn Asn Leu Phe Thr Leu Val His Glu Phe Gly His Ser
385                 390                 395                 400

Val His Ser Tyr Tyr Thr Arg Lys Tyr Gln Pro Tyr Pro Tyr Gly Asn
                405                 410                 415

Tyr Ser Ile Phe Val Ala Glu Val Ala Ser Thr Thr Asn Glu Ala Leu
            420                 425                 430

Leu Gly Glu Tyr Leu Leu Asn Thr Ile Glu Asp Glu Lys Gln Arg Leu
        435                 440                 445

Tyr Ile Leu Asn His Met Leu Glu Gly Phe Lys Gly Thr Val Phe Arg
    450                 455                 460

Gln Thr Met Phe Ala Glu Phe Glu His Glu Ile His Val Lys Ala Gln
465                 470                 475                 480

Glu Gly Glu Pro Leu Thr Pro Glu Leu Leu Thr Ser Ile Tyr Tyr Asp
                485                 490                 495

Leu Asn Lys Lys Tyr Phe Gly Asp Asn Ile Glu Ile Asp Lys Glu Ile
            500                 505                 510

Gly Leu Glu Trp Ser Arg Ile Pro His Phe Tyr Tyr Asn Tyr Tyr Val
        515                 520                 525

Tyr Gln Tyr Ala Thr Gly Phe Ser Ala Ala Gln Ala Leu Ser Gln Gln
    530                 535                 540

Ile Leu Lys Glu Gly Lys Ser Ala Val Asp Arg Tyr Ile Glu Phe Leu
545                 550                 555                 560

Lys Ala Gly Ser Ser Asp Tyr Pro Ile Glu Val Leu Lys Lys Ala Gly
                565                 570                 575

Val Asp Met Thr Ser Ser Glu Pro Ile Glu Ala Ala Cys Lys Lys Phe
            580                 585                 590

Glu Glu Gln Leu Asn Glu Met Glu Glu Leu Leu Gln Lys Val Asn His
        595                 600                 605

Ser

<210>  11
<211>  2746
<212>  DNA
<213>  Bacillus licheniformis

<220>
<221>  CDS
<222>  (491)..(2245)

<400>  11
atctcctttt gtctcatact cttttaaggt atccgcgaat tctttaaggg ttgtctgatt      60

cgtcacttta tagttaacaa aatactcagc tgtttcatct aaattttttg caatttccgt     120

caaaagcacg gaaaacttgt cttttttttct tttcaacatt tgtaaccctc cattgtttaa     180

atgagacaat gcacacatca aaatattata taactttttg tcgaaaaaaa acaggtgcag     240

tcgaaaaaaa caattaattt tcaaatcgtc ataccttttta gataggcgca agcaccagct 300

ggcatccggg atttaagat gacaataggc gatttttcct ttgatatcat cgttccaaaa 360

gaacggatct caggatcgtt tcgagaaatt tgggagttta ggttcaaaaa aatagaaaat 420

tgggagtttt tcttattttc agtccgcttt cctgttatag tagtaataaa ctggaaagga 480

aggtgatctg atg aga cgg tta gct ttc tta gtt gtt gcg ttt tgc ttg 529

1               5                   10

gcg gtt ggt tgc ttt ttc agt ccg gtg tcg aaa gca gaa gga gtc atg 577

    15                  20                  25

agc ggg gga gat ggt gat aaa gtc gcc gtc ggg aaa gac ggt atg gtg 625

30                  35                  40                  45

gct acg gcc cat ccg ctt gca tca aaa atc gga gcc gaa gtt ttg aaa 673

                50                  55                  60

aag ggc ggg aat gcc att gat gca gct att gcg atc cag tac gca ctg 721

            65                  70                  75

aat gta aca gag ccg atg atg tct gga atc ggc ggc ggt ttc atg 769

        80                  85                  90

atg gtc tac gac ggc gag acg aag gaa acg tcg atc atc aac agc agg 817

    95                  100                 105

gag cgt gct ccg gag gga gcc aag cct gac atg ttt ttg gat gaa gac 865

110                 115                 120                 125

gga aag gtg att ccg ttt tca gaa agg tcg aga cac gga aat gcg gtc 913

                130                 135                 140

ggt gtg ccg gga acg ctg aaa ggg ctt gaa gca gcg cat aag aag tgg 961

            145                 150                 155

gga aca aag aaa atg gaa gac ttg att tcg cct tct ata aag ctt gca 1009

        160                 165                 170

gag gaa ggg ttc cca atc gac tcg gtt ttg gct gac gcg ata aag gat 1057

    175                 180                 185

cat cag gac aag ctg agc aaa acg gcc gca aag gat att ttc ctt ccg 1105

190                 195                 200                 205

gac ggt gaa cct ttg aaa gaa ggc gat atc ctt gta caa aag gat ttg 1153

                210                 215                 220

gcc aaa acg ttt aaa ttg ata cgg aaa gaa ggg tca aaa gcc ttt tat 1201

            225                 230                 235

gat ggg gaa atc ggc cgg gcg att gca gat gtc gtg cag gat ttc ggg 1249

        240                 245                 250

ggt tca atg aca cct gat gac ctg tcc cgc tac gag gtc aca aca gat 1297

    255                 260                 265

aag ccc atc tgg ggc gag tac cac ggc tat gat att gca agc atg cct 1345

270                 275                 280                 285

cct cca agc tca ggc gga gtg ttc atg ctg cag atg ctg aaa ttg att 1393

                290                 295                 300

gat gat ttt cat tta tcg caa tac gat cca aag tcg ttt gaa aaa tat 1441

            305                 310                 315

cat ctg ctc gcg gaa acg atg cat ctc tcc tat gcg gac agg gct gca 1489

        320                 325                 330

tat gcg ggc gat ccg gaa ttt gtc gat gtt ccg ctc aga ggg ctg ctc 1537

    335                 340                 345

gat ccc gat tat att aag gag cgg caa aag ctg atc agc ctt gat tcg 1585

350                 355                 360                 365

atg aac cgc gat gtg aaa gag ggc gat ccg tgg aaa tat gag gaa ggc 1633

                370                 375                 380

gag ccg aat tat gag atc gtg cct cag cct gaa gat aaa acg atc ggg 1681

            385                 390                 395

gag acg acg cat ttt acc gtc acg gat cag tgg ggc aat gtt gtc tct 1729

        400                 405                 410

tat acg aca acg att gaa cag ctt ttt gga acg ggg att tta gtg ccc 1777

    415                 420                 425

ggg tac gga ctg ttt ttg aac aat gaa ctg acc gat ttt gat gcg att 1825

430                 435                 440                 445

cca ggc ggc gcc aac gaa gtg cag ccg aat aaa agg ccg ctt tca agc 1873

                450                 455                 460

atg acg ccg acg atc gta ttc aaa gat gaa aaa ccg gtg ctg act gtc 1921

            465                 470                 475

ggt tcc cct ggc gga acg acg atc att gct tcc gtt ttc cag acg atc 1969

        480                 485                 490

ttg aat tat ttt gag tac ggc atg agc ctt cag gat gcg att gaa gag 2017

    495                 500                 505

ccg agg atc tat acg aac agc ctg act tcg tac cgc tat gaa tcc gga     2065

510                 515                 520                 525

atg ccg gag gat gtc aga cgg aag ctc aat gac ttc ggc cac aaa ttc     2113

                530                 535                 540

ggc tcg aat ccg gtt gat atc ggc aat gtc caa agc att ttc att gac     2161

            545                 550                 555

cgg gag aac aaa acg ttt atg ggg gtt gcc gat tca agc aga aac gga     2209

        560                 565                 570

acg gcg gtt ggg gtc aac att aag aca tcg gct aaa tagcggagca          2255

    575                 580                 585

aggcttggtc taaagtattt agatcaagcc ttcttccgtc agcggggaaa agaggatttt   2315

tttcgaaaga atagaaatat atttttgaa agcgtttacc aatttataaa ggaggggtac    2375

catgattcac acgttggcaa atggatttta ccgcttttgc gagtgggtga tgcggcttgc   2435

ctatttgaat ctgctgtgga tcggctttac gctggcggga gcggtcatct tcggtttagc   2495

gccggcgacc gccgcgatgt tcgctgtgac tagacagtgg acgctgggaa aaacggatgt   2555

cccggtgttt cagacgtttt ttcgggcgtt taaaaagaaa tgggccaaat caagcgttct   2615

cggtttgata ttgggtctgg tcggcttatt gctgtttgtt gattttcaga ttgcggccgt   2675

ctattttcac gatcagccag tgtttttaag cctgttcatc agtttgttta tcatttatgc   2735

catcatcctc t                                                        2746

<210>  12
<211>  585
<212>  PRT
<213>  Bacillus licheniformis

<400>  12

<210>  13
<211>  2314
<212>  DNA
<213>  Bacillus licheniformis

<220>
<221>  CDS
<222>  (493)..(1818)

<400> 13
ctgaaatcta tcaaggcaga cgctttaaag tttatcgcgg catgggttct gtagctgcga 60

tggaaaaagg aagcaaagac cgctacttcc aagaggaaaa caaaaaattc gttcctgaag 120

gcattgaagg ccgtacgcct tataaaggcc ctgttgctga taccgtttat cagcttgtcg 180

gcggcttgaa atccggcatg ggctactgcg gaacgaaaga tttgcacgca ttgagggaag 240

atgcgcagtt catccgcatg acaggagcag gcctgcggga aagccatcct cacgatgtgc 300

aaattaccaa agaatcgcct aactatacca tttcctaatc aaatatgaca aaaattaaac 360

cgtagacaga gtgatactct gtctattttt ttttgcttca ttatgataaa atttataatg 420

ttgtgtttga aaaatgtctt aaaaggcttt gatatagatt attagtctga tgaaaagcgg 480

aggtatcacg at ttg aaa agc aag aga tta aag cag ctg atc atg tta atc 531
Leu Lys Ser Lys Arg Leu Lys Gln Leu Ile Met Leu Ile
1               5                   10

gtt gct ttt gcc gtg act gta ggt gca ttt tct cct atg tca act gca 579
Val Ala Phe Ala Val Thr Val Gly Ala Phe Ser Pro Met Ser Thr Ala
    15                  20                  25

aaa gct gca aat gat cca atc aat gta gac gcg aaa gca gca atc ctg 627
Lys Ala Ala Asn Asp Pro Ile Asn Val Asp Ala Lys Ala Ala Ile Leu
30                  35                  40                  45

gtg gaa gct tca tcg ggg aaa att ttg tac agt aaa aat gcc gac cag 675
Val Glu Ala Ser Ser Gly Lys Ile Leu Tyr Ser Lys Asn Ala Asp Gln
                50                  55                  60

cgt ctt cct gtt gcc agc atg gca aaa atg atg acg gag tat ctt ctg 723
Arg Leu Pro Val Ala Ser Met Ala Lys Met Met Thr Glu Tyr Leu Leu
            65                  70                  75

ttg gaa gcg att cac gaa ggc aaa gtg aaa tgg gat cag aag tac acg 771
Leu Glu Ala Ile His Glu Gly Lys Val Lys Trp Asp Gln Lys Tyr Thr
        80                  85                  90

cct gac gat tat gta tac gag att tcc caa gat cgt tca cta tcc aat 819
Pro Asp Asp Tyr Val Tyr Glu Ile Ser Gln Asp Arg Ser Leu Ser Asn
    95                  100                 105

gtt cct ctt cgt aaa gac gga tca tac act gta aaa gag ctt tat cag 867
Val Pro Leu Arg Lys Asp Gly Ser Tyr Thr Val Lys Glu Leu Tyr Gln
110                 115                 120                 125

gcg act gca att tac tct gca aac gcg gcg gct att gca ctt tct gaa 915
Ala Thr Ala Ile Tyr Ser Ala Asn Ala Ala Ala Ile Ala Leu Ser Glu
                130                 135                 140

att gtt gcc ggt tct gaa tcg aag ttt gta gag tta atg aac aaa aaa 963
Ile Val Ala Gly Ser Glu Ser Lys Phe Val Glu Leu Met Asn Lys Lys
            145                 150                 155

gcc aaa gag ctt gga atg aaa gat ttc aaa ttt gtg aat gcc aca ggt 1011
Ala Lys Glu Leu Gly Met Lys Asp Phe Lys Phe Val Asn Ala Thr Gly
        160                 165                 170

ttg gaa aac aag gat ctt cac ggc aaa cat ccg agc gga aca aat gcc 1059
Leu Glu Asn Lys Asp Leu His Gly Lys His Pro Ser Gly Thr Asn Ala
    175                 180                 185

aat gaa gaa aat gaa gtg tct gcg aga gac atg gcg ctc ctt gcc gat 1107
Asn Glu Glu Asn Glu Val Ser Ala Arg Asp Met Ala Leu Leu Ala Asp
190                 195                 200                 205

cat tta gtg agc gat tat cca gag atc ctc gat acg gcg agc att gcc     1155
His Leu Val Ser Asp Tyr Pro Glu Ile Leu Asp Thr Ala Ser Ile Ala
                210                 215                 220

aaa acg aag ttc aga aaa ggc aca gac gat gaa atg gat atg ccg aac     1203
Lys Thr Lys Phe Arg Lys Gly Thr Asp Asp Glu Met Asp Met Pro Asn
            225                 230                 235

tgg aac ttt atg ctg aaa ggc ctt gtt cag caa tat gaa ggt gtg gac     1251
Trp Asn Phe Met Leu Lys Gly Leu Val Gln Gln Tyr Glu Gly Val Asp
        240                 245                 250

ggg ctg aaa acc ggg tca acc gac tca gcc gga tca tgc ttt acg gct     1299
Gly Leu Lys Thr Gly Ser Thr Asp Ser Ala Gly Ser Cys Phe Thr Ala
    255                 260                 265

act gct gaa aga aac ggc atg aga gtg att tcc gtc gtt ctg aat gcg     1347
Thr Ala Glu Arg Asn Gly Met Arg Val Ile Ser Val Val Leu Asn Ala
270                 275                 280                 285

aaa ggc gac ctt cat acg gcg cga ttt aaa gaa acg aaa aaa atg ctc     1395
Lys Gly Asp Leu His Thr Ala Arg Phe Lys Glu Thr Lys Lys Met Leu
                290                 295                 300

gat tat gca ttt gaa aac ttc act atg aaa gag ttt tac gca aaa ggc     1443
Asp Tyr Ala Phe Glu Asn Phe Thr Met Lys Glu Phe Tyr Ala Lys Gly
            305                 310                 315

gca caa gtg aaa ggc aac gaa aca gtc aca gtc gac aaa ggg aaa gaa     1491
Ala Gln Val Lys Gly Asn Glu Thr Val Thr Val Asp Lys Gly Lys Glu
        320                 325                 330

act gaa gtc ggc atc gtc aca gaa aaa gcc ttt tca atg cct gtg aag     1539
Thr Glu Val Gly Ile Val Thr Glu Lys Ala Phe Ser Met Pro Val Lys
    335                 340                 345

aac ggt gaa gaa aag aat tac aaa gcg aaa gtg acg ctg aac aaa gac     1587
Asn Gly Glu Glu Lys Asn Tyr Lys Ala Lys Val Thr Leu Asn Lys Asp
350                 355                 360                 365

gag ctg acc gca cct gtt aaa aaa ggt gag aaa gtc ggt acc ttg acg     1635
Glu Leu Thr Ala Pro Val Lys Lys Gly Glu Lys Val Gly Thr Leu Thr
                370                 375                 380

gcc tcc tat aag gga gaa gaa aag gac tac gga ttt ctc ggc agc gat     1683
Ala Ser Tyr Lys Gly Glu Glu Lys Asp Tyr Gly Phe Leu Gly Ser Asp
            385                 390                 395

gta tca ggt gta aac ctg gtg aca aaa gaa gat gat gag aaa gca aac     1731
Val Ser Gly Val Asn Leu Val Thr Lys Glu Asp Asp Glu Lys Ala Asn
        400                 405                 410

tgg ttt att tta acg atg aga agc atc ggc ggc ttt ttt gcc ggt ata     1779
Trp Phe Ile Leu Thr Met Arg Ser Ile Gly Gly Phe Phe Ala Gly Ile
    415                 420                 425

tgg aac agc att gtt gaa atg gta acc ggc tgg ttt taa aaataaaga     1828
Trp Asn Ser Ile Val Glu Met Val Thr Gly Trp Phe
430                 435                 440

gctctatttg atagggctct ttattgtcat attcagatag aattaccatt tacaatccta     1888

acgatttgtt attaaaatag aaataatctt cgtgtttttc atatgatttt gtggaattat     1948

aggatacaat aggattaaat ctgacaattc aatctttgat tagggggatc cttgaaatgg     2008

ctcaaacagg tactgatcgc gtaaaacgcg gtatggcaga aatgcagaag ggcggcgtca     2068

ttatggacgt tgtcaatgct gaacaagcaa aaatcgcaga agaagcagga gccgtcgcag 2128

tcatggcgct ggagcgcgtt ccggctgata tccgtgcggc aggcggagtt gcccgcatgg 2188

cagatccgac tattgtcgaa gaagtgatga acgctgtttc aatcccggtt atggcaaaag 2248

cacgcatcgg gcatatcgtt gaagcgcgtg ttcttgaagc gcttggcgtt gactatattg 2308

atgaaa 2314

<210> 14
<211> 441
<212> PRT
<213> Bacillus licheniformis

<400> 14

Leu Lys Ser Lys Arg Leu Lys Gln Leu Ile Met Leu Ile Val Ala Phe
1               5                   10                  15

Ala Val Thr Val Gly Ala Phe Ser Pro Met Ser Thr Ala Lys Ala Ala
            20                  25                  30

Asn Asp Pro Ile Asn Val Asp Ala Lys Ala Ala Ile Leu Val Glu Ala
        35                  40                  45

Ser Ser Gly Lys Ile Leu Tyr Ser Lys Asn Ala Asp Gln Arg Leu Pro
    50                  55                  60

Val Ala Ser Met Ala Lys Met Met Thr Glu Tyr Leu Leu Leu Glu Ala
65                  70                  75                  80

Ile His Glu Gly Lys Val Lys Trp Asp Gln Lys Tyr Thr Pro Asp Asp
                85                  90                  95

Tyr Val Tyr Glu Ile Ser Gln Asp Arg Ser Leu Ser Asn Val Pro Leu
            100                 105                 110

Arg Lys Asp Gly Ser Tyr Thr Val Lys Glu Leu Tyr Gln Ala Thr Ala
        115                 120                 125

Ile Tyr Ser Ala Asn Ala Ala Ala Ile Ala Leu Ser Glu Ile Val Ala
    130                 135                 140

Gly Ser Glu Ser Lys Phe Val Glu Leu Met Asn Lys Lys Ala Lys Glu
145                 150                 155                 160

Leu Gly Met Lys Asp Phe Lys Phe Val Asn Ala Thr Gly Leu Glu Asn
                165                 170                 175

Lys Asp Leu His Gly Lys His Pro Ser Gly Thr Asn Ala Asn Glu Glu
            180                 185                 190

Asn Glu Val Ser Ala Arg Asp Met Ala Leu Leu Ala Asp His Leu Val

        195                 200                 205

Ser Asp Tyr Pro Glu Ile Leu Asp Thr Ala Ser Ile Ala Lys Thr Lys
    210                 215                 220

Phe Arg Lys Gly Thr Asp Asp Glu Met Asp Met Pro Asn Trp Asn Phe
225                 230                 235                 240

Met Leu Lys Gly Leu Val Gln Gln Tyr Glu Gly Val Asp Gly Leu Lys
                245                 250                 255

Thr Gly Ser Thr Asp Ser Ala Gly Ser Cys Phe Thr Ala Thr Ala Glu
            260                 265                 270

Arg Asn Gly Met Arg Val Ile Ser Val Val Leu Asn Ala Lys Gly Asp
        275                 280                 285

Leu His Thr Ala Arg Phe Lys Glu Thr Lys Lys Met Leu Asp Tyr Ala
    290                 295                 300

Phe Glu Asn Phe Thr Met Lys Glu Phe Tyr Ala Lys Gly Ala Gln Val
305                 310                 315                 320

Lys Gly Asn Glu Thr Val Thr Val Asp Lys Gly Lys Glu Thr Glu Val
                325                 330                 335

Gly Ile Val Thr Glu Lys Ala Phe Ser Met Pro Val Lys Asn Gly Glu
            340                 345                 350

Glu Lys Asn Tyr Lys Ala Lys Val Thr Leu Asn Lys Asp Glu Leu Thr
        355                 360                 365

Ala Pro Val Lys Lys Gly Glu Lys Val Gly Thr Leu Thr Ala Ser Tyr
    370                 375                 380

Lys Gly Glu Glu Lys Asp Tyr Gly Phe Leu Gly Ser Asp Val Ser Gly
385                 390                 395                 400

Val Asn Leu Val Thr Lys Glu Asp Asp Glu Lys Ala Asn Trp Phe Ile
                405                 410                 415

Leu Thr Met Arg Ser Ile Gly Gly Phe Phe Ala Gly Ile Trp Asn Ser
            420                 425                 430

Ile Val Glu Met Val Thr Gly Trp Phe
        435                 440

<210> 15
<211> 2431
<212> DNA
<213> Bacillus licheniformis

<220>
<221> CDS
<222> (519)..(1958)

<400> 15
agaagcttga cgtcattgtt gccagcaatt tattcggcga tattttgacc gatatcgggg 60

cggcgattat gggaagcata ggtgtggcgc cggccgccaa cattaatgta aacggcaaat 120

atccatctat gtttgagcct gtgcacggct ctgctccgga catcatctga tagcgagtca 180

tcatcggaaa aggaatcgcc aatccgatcg gccaaatttg gacggcaaaa atgatgctcg 240

atcatttcgg ggaagaagaa ctcggtgcaa gattgcttga cgtgattgaa gacgttctaa 300

agagcggcta cttaacgcct gatatcggcg gttcaagtac gacggatgat gtgacagaag 360

aaatcatcaa gcgcgtgagg accatataag cgaaagctgc cagcactccg gcagcttttt 420

ttgctgtgga atggaaaatc gattacatga tacgggcggt tttggcgcat actacatcaa 480

gcaacaaaac atagtgcagg cagaagagga ggtatagg atg aat cag aag att gtg 536
Met Asn Gln Lys Ile Val
1               5

tct gtg ctc atc att gcg ctc agc ctg tta tgc ggc ttg gta aca ggc 584
Ser Val Leu Ile Ile Ala Leu Ser Leu Leu Cys Gly Leu Val Thr Gly
            10                  15                  20

gcc ggc ccg att ccg tta cat gat gtc gtt tca aaa gca tca gcg gct 632
Ala Gly Pro Ile Pro Leu His Asp Val Val Ser Lys Ala Ser Ala Ala
        25                  30                  35

gaa gaa gct gca gcc aaa agc acc gct aga aac gaa ttg gag aaa att 680
Glu Glu Ala Ala Ala Lys Ser Thr Ala Arg Asn Glu Leu Glu Lys Ile
    40                  45                  50

gag aaa gca tat gac ctg att tca aat gaa tat gtg gag cag gtc gac 728
Glu Lys Ala Tyr Asp Leu Ile Ser Asn Glu Tyr Val Glu Gln Val Asp
55                  60                  65                  70

agg gaa aaa ctg ttg gaa gga gcc ata cag ggc atg ctt tca aca ttg 776
Arg Glu Lys Leu Leu Glu Gly Ala Ile Gln Gly Met Leu Ser Thr Leu
                75                  80                  85

aat gac cca tac tcg gtc tat atg gat aaa cag acg gca aag cgg ttt 824
Asn Asp Pro Tyr Ser Val Tyr Met Asp Lys Gln Thr Ala Lys Arg Phe
            90                  95                  100

tca gat tcc ctt gat tcc tct ttt gaa gga atc ggt gca gaa atc gga 872
Ser Asp Ser Leu Asp Ser Ser Phe Glu Gly Ile Gly Ala Glu Ile Gly
        105                 110                 115

atg gaa gac aga aaa atc att atc gtt tct cct ttt aaa cag tct ccg 920
Met Glu Asp Arg Lys Ile Ile Ile Val Ser Pro Phe Lys Gln Ser Pro
    120                 125                 130

gca gag aaa gcc gga tta aag ccg aac gat gaa atc att agc atc gac 968
Ala Glu Lys Ala Gly Leu Lys Pro Asn Asp Glu Ile Ile Ser Ile Asp
135                 140                 145                 150

ggc gat tcc atg agc gga atg gat ttg aat gac gcc gta tta aag atc 1016
Gly Asp Ser Met Ser Gly Met Asp Leu Asn Asp Ala Val Leu Lys Ile
                155                 160                 165

aga ggc aaa aag gga tcg acg gtt aca ctg aaa gtt cac cga ccc gga 1064
Arg Gly Lys Lys Gly Ser Thr Val Thr Leu Lys Val His Arg Pro Gly

                170                 175                 180

atg aaa gac cag ctg aca ttt acg att aag cgc gat gaa atc ccg ctg        1112
Met Lys Asp Gln Leu Thr Phe Thr Ile Lys Arg Asp Glu Ile Pro Leu
        185                 190                 195

gaa acg gtg ttt gct tcc ata aag aaa gta cag gat aaa cct gtc ggc        1160
Glu Thr Val Phe Ala Ser Ile Lys Lys Val Gln Asp Lys Pro Val Gly
    200                 205                 210

tat atc gca ata tct tcc ttt tcc gaa cat acg gca aaa gat ttt act        1208
Tyr Ile Ala Ile Ser Ser Phe Ser Glu His Thr Ala Lys Asp Phe Thr
215                 220                 225                 230

gct gaa ctg aaa aag ctt gag aaa aag gga ata aaa ggg ctc gtt ttg        1256
Ala Glu Leu Lys Lys Leu Glu Lys Lys Gly Ile Lys Gly Leu Val Leu
                235                 240                 245

gat gta aga gga aat ccg ggc ggg tac ttg caa agc gtg gaa gac att        1304
Asp Val Arg Gly Asn Pro Gly Gly Tyr Leu Gln Ser Val Glu Asp Ile
            250                 255                 260

tta aaa cat ttt gtg aca aaa gat cat ccg tat ata caa atc gcc gaa        1352
Leu Lys His Phe Val Thr Lys Asp His Pro Tyr Ile Gln Ile Ala Glu
        265                 270                 275

aga aac gga aat aaa aaa caa tac ttc tca aaa tta aaa gag aaa aaa        1400
Arg Asn Gly Asn Lys Lys Gln Tyr Phe Ser Lys Leu Lys Glu Lys Lys
    280                 285                 290

cca tac cct gtg agc gtg atc acg gac aag ggc agc gct tcg gct tcg        1448
Pro Tyr Pro Val Ser Val Ile Thr Asp Lys Gly Ser Ala Ser Ala Ser
295                 300                 305                 310

gaa att ctt gca ggc gca tta aaa gaa gcc gag ggc tac gac gtg gtt        1496
Glu Ile Leu Ala Gly Ala Leu Lys Glu Ala Glu Gly Tyr Asp Val Val
                315                 320                 325

ggc gat cct tcc ttt gga aag gga acc gtc cag cag gcg gtg ccg atg        1544
Gly Asp Pro Ser Phe Gly Lys Gly Thr Val Gln Gln Ala Val Pro Met
            330                 335                 340

gga gac ggt agc aat att aag ctg acg ctg tac aaa tgg ctg aca cct        1592
Gly Asp Gly Ser Asn Ile Lys Leu Thr Leu Tyr Lys Trp Leu Thr Pro
        345                 350                 355

aaa gga aat tgg atc cat aag cag ggc att cag ccg acc gtt cca gtc        1640
Lys Gly Asn Trp Ile His Lys Gln Gly Ile Gln Pro Thr Val Pro Val
    360                 365                 370

acg cag cct gca tat ttc tcg gcg ggg cct tta cag ctg aaa gaa ccc        1688
Thr Gln Pro Ala Tyr Phe Ser Ala Gly Pro Leu Gln Leu Lys Glu Pro
375                 380                 385                 390

ctc aag ccg gat atg aac aac aat gaa att aag cgg gcg cag ttc ctg        1736
Leu Lys Pro Asp Met Asn Asn Asn Glu Ile Lys Arg Ala Gln Phe Leu
                395                 400                 405

ctg aaa gga ctc ggg ttt gtc ccc ggc cgg gaa gac ggc tat tac aat        1784
Leu Lys Gly Leu Gly Phe Val Pro Gly Arg Glu Asp Gly Tyr Tyr Asn
            410                 415                 420

gaa agc aca aaa aaa gcc gtc atg gcg ttt cag gca gca aac aag ctg        1832
Glu Ser Thr Lys Lys Ala Val Met Ala Phe Gln Ala Ala Asn Lys Leu
        425                 430                 435

aag caa aca ggg atc att gac caa aaa acg gca aat aca atg aac ctg        1880
Lys Gln Thr Gly Ile Ile Asp Gln Lys Thr Ala Asn Thr Met Asn Leu

440                 445                 450

cgg att gag gag aaa aag atg gat gaa aag aac gat ctt cag ctg caa      1928
Arg Ile Glu Glu Lys Lys Met Asp Glu Lys Asn Asp Leu Gln Leu Gln
455                 460                 465                 470

gcc gca tta aaa gtg ctg ttt aac aaa aag tgagggcacg ggatgtcctc        1978
Ala Ala Leu Lys Val Leu Phe Asn Lys Lys
                475                 480

acttttttta ttctcagaaa gtaaattctc aatcttttta cctaaaattt ttgctttttc    2038

ataaaaaaaa cattcataat gaatagaatt gacccgataa cataagtata tgggagactt    2098

tataaggatg taaagatgca gtaaaaatgt aacttttgca agttgtataa cataggaaaa    2158

ctttttaaaa tagaaccaaa gctttttcaa gaattataat gtgtgcattt tttttgcgga    2218

gaatgttagc gctgtttgcg ttcttcgtct ctgttggtgg agtcgtctga cgcttgcgct    2278

tttcttatat ataaataata aatgggggta cacgattgaa aagggcaagt attgtgagag    2338

agaaaaaata ctatgaatta gtggagcagt taaaagttcg atcacaagac gttacgtttt    2398

ccgctacaaa ggcagtagga ttgcttatgc tgt                                 2431

<210>  16
<211>  480
<212>  PRT
<213>  Bacillus licheniformis

<400>  16

Met Asn Gln Lys Ile Val Ser Val Leu Ile Ile Ala Leu Ser Leu Leu
1               5                   10                  15

Cys Gly Leu Val Thr Gly Ala Gly Pro Ile Pro Leu His Asp Val Val
            20                  25                  30

Ser Lys Ala Ser Ala Ala Glu Glu Ala Ala Ala Lys Ser Thr Ala Arg
        35                  40                  45

Asn Glu Leu Glu Lys Ile Glu Lys Ala Tyr Asp Leu Ile Ser Asn Glu
    50                  55                  60

Tyr Val Glu Gln Val Asp Arg Glu Lys Leu Leu Glu Gly Ala Ile Gln
65                  70                  75                  80

Gly Met Leu Ser Thr Leu Asn Asp Pro Tyr Ser Val Tyr Met Asp Lys
                85                  90                  95

Gln Thr Ala Lys Arg Phe Ser Asp Ser Leu Asp Ser Ser Phe Glu Gly
            100                 105                 110

Ile Gly Ala Glu Ile Gly Met Glu Asp Arg Lys Ile Ile Ile Val Ser
        115                 120                 125

Pro Phe Lys Gln Ser Pro Ala Glu Lys Ala Gly Leu Lys Pro Asn Asp
    130                 135                 140

Glu Ile Ile Ser Ile Asp Gly Asp Ser Met Ser Gly Met Asp Leu Asn
145                 150                 155                 160

Asp Ala Val Leu Lys Ile Arg Gly Lys Lys Gly Ser Thr Val Thr Leu
                165                 170                 175

Lys Val His Arg Pro Gly Met Lys Asp Gln Leu Thr Phe Thr Ile Lys
            180                 185                 190

Arg Asp Glu Ile Pro Leu Glu Thr Val Phe Ala Ser Ile Lys Lys Val
        195                 200                 205

Gln Asp Lys Pro Val Gly Tyr Ile Ala Ile Ser Ser Phe Ser Glu His
    210                 215                 220

Thr Ala Lys Asp Phe Thr Ala Glu Leu Lys Lys Leu Glu Lys Lys Gly
225                 230                 235                 240

Ile Lys Gly Leu Val Leu Asp Val Arg Gly Asn Pro Gly Gly Tyr Leu
                245                 250                 255

Gln Ser Val Glu Asp Ile Leu Lys His Phe Val Thr Lys Asp His Pro
            260                 265                 270

Tyr Ile Gln Ile Ala Glu Arg Asn Gly Asn Lys Lys Gln Tyr Phe Ser
        275                 280                 285

Lys Leu Lys Glu Lys Lys Pro Tyr Pro Val Ser Val Ile Thr Asp Lys
    290                 295                 300

Gly Ser Ala Ser Ala Ser Glu Ile Leu Ala Gly Ala Leu Lys Glu Ala
305                 310                 315                 320

Glu Gly Tyr Asp Val Val Gly Asp Pro Ser Phe Gly Lys Gly Thr Val
                325                 330                 335

Gln Gln Ala Val Pro Met Gly Asp Gly Ser Asn Ile Lys Leu Thr Leu
            340                 345                 350

Tyr Lys Trp Leu Thr Pro Lys Gly Asn Trp Ile His Lys Gln Gly Ile
        355                 360                 365

Gln Pro Thr Val Pro Val Thr Gln Pro Ala Tyr Phe Ser Ala Gly Pro
    370                 375                 380

Leu Gln Leu Lys Glu Pro Leu Lys Pro Asp Met Asn Asn Asn Glu Ile
385                 390                 395                 400

Lys Arg Ala Gln Phe Leu Leu Lys Gly Leu Gly Phe Val Pro Gly Arg
                405                 410                 415

Glu Asp Gly Tyr Tyr Asn Glu Ser Thr Lys Lys Ala Val Met Ala Phe
            420                 425                 430

Gln Ala Ala Asn Lys Leu Lys Gln Thr Gly Ile Ile Asp Gln Lys Thr
        435                 440                 445

Ala Asn Thr Met Asn Leu Arg Ile Glu Glu Lys Lys Met Asp Glu Lys
    450                 455                 460

Asn Asp Leu Gln Leu Gln Ala Ala Leu Lys Val Leu Phe Asn Lys Lys
465                 470                 475                 480

<210>  17
<211>  2230
<212>  DNA
<213>  Bacillus licheniformis

<220>
<221>  CDS
<222>  (501)..(1730)

<400>  17
   ctgtcctccg gagctgagcg gagacattgt cgaccggggc gtgattttga ccggaggagg      60
   cgcgcttttg aacggcatca aagaatggct gtcacaggaa atcgttgtgc ccgtgcatgt     120
   tgctgcaaat ccgcttgaat ctgtcgctat cggaacgggt cgttctctcg aagtgatcga     180
   taaattgcaa aaagcggcta aataattttt gttttcgcgt ctttttcccc ggcttctttc     240
   atatcgtgag gaatggagaa acgataagga ggaagaccat gcttagagat atgggaagac     300
   gcctggccat caccgttatt ttaagcggta ttattctcgg ggggatgagc atctcccttg     360
   caaacatgcc aagctcgccc cccggccaaa cggccaaaat caaccgttaa aaaagaagca     420
   gcctgaaccg ggctgcttct ttttttcttt tcaatcaaat atttgatatg atgttatgga     480
   caataaagga ggttttgcca atg aat tcg ttt act gat aaa ctg agc aaa tat     533
                         Met Asn Ser Phe Thr Asp Lys Leu Ser Lys Tyr
                         1               5                   10
   gca aag ctt gtg gcc gaa gtc ggc gta aac gtt caa aaa gga cag caa       581
   Ala Lys Leu Val Ala Glu Val Gly Val Asn Val Gln Lys Gly Gln Gln
               15                  20                  25
   gtc gtc atc aac gcg tca aca gaa gta cgc gat ttt gca cgg ctc ctc       629
   Val Val Ile Asn Ala Ser Thr Glu Val Arg Asp Phe Ala Arg Leu Leu
           30                  35                  40
   gtc aaa agc gcc tat caa aga ggg gcg aaa aac gtg acg gtc cgc tgg       677
   Val Lys Ser Ala Tyr Gln Arg Gly Ala Lys Asn Val Thr Val Arg Trp
       45                  50                  55
   cag gat gat gag gtc aca agg ctg aaa tac gag ctg gct cct gcg gaa       725
   Gln Asp Asp Glu Val Thr Arg Leu Lys Tyr Glu Leu Ala Pro Ala Glu
   60                  65                  70                  75
   gcc ttt gaa gag ttc cct gaa tgg gaa gca aag ggg ctt gag acg ctc       773
   Ala Phe Glu Glu Phe Pro Glu Trp Glu Ala Lys Gly Leu Glu Thr Leu
                   80                  85                  90
   gcc aaa gaa ggc gct gct ttt att tcc att gtc tct tca agt cct gat     821
   Ala Lys Glu Gly Ala Ala Phe Ile Ser Ile Val Ser Ser Ser Pro Asp
               95                  100                 105
   ctt ttg aaa ggc atc gat tcc aaa aaa atc tcc gca cag cag aaa gcc     869
   Leu Leu Lys Gly Ile Asp Ser Lys Lys Ile Ser Ala Gln Gln Lys Ala
           110                 115                 120
   gca gga aaa gcg atg cat aca tac agg caa atg att caa tcg gat aaa     917
   Ala Gly Lys Ala Met His Thr Tyr Arg Gln Met Ile Gln Ser Asp Lys
       125                 130                 135
   gtc agc tgg act gtc gtc gca gcg cct tcg ccg gcc tgg gcg aaa aaa     965
   Val Ser Trp Thr Val Val Ala Ala Pro Ser Pro Ala Trp Ala Lys Lys
   140                 145                 150                 155
   gta ttc cct gag gca gat gaa gag aca gcc gtg caa aag cta tgg aat     1013
   Val Phe Pro Glu Ala Asp Glu Glu Thr Ala Val Gln Lys Leu Trp Asn
                   160                 165                 170
   gag att ttc aaa acg acc cgc gtt gac cgt gaa gat ccc gtt caa gcg     1061
   Glu Ile Phe Lys Thr Thr Arg Val Asp Arg Glu Asp Pro Val Gln Ala
               175                 180                 185
   tgg aaa gat cac gat caa acc ttg cgc gat aaa gta agc gtc tta aac     1109
   Trp Lys Asp His Asp Gln Thr Leu Arg Asp Lys Val Ser Val Leu Asn
           190                 195                 200
   gac aag cat tat cac gcc ctt cat tac caa gca aag gga aca gat ttg     1157
   Asp Lys His Tyr His Ala Leu His Tyr Gln Ala Lys Gly Thr Asp Leu
       205                 210                 215
   acg atc gag ctt ccc gag aag cat ctg tgg gtc ggc gcc gga agc aca     1205
   Thr Ile Glu Leu Pro Glu Lys His Leu Trp Val Gly Ala Gly Ser Thr
   220                 225                 230                 235
   aac gaa aac ggc gtc gag ttc atg gcc aat atg ccg aca gag gaa gta     1253
   Asn Glu Asn Gly Val Glu Phe Met Ala Asn Met Pro Thr Glu Glu Val
                   240                 245                 250
   ttc act gct ccg aaa aaa gac ggg gtg aac ggc acc gtt tca agc acg     1301
   Phe Thr Ala Pro Lys Lys Asp Gly Val Asn Gly Thr Val Ser Ser Thr
               255                 260                 265
   aag ccg tta agc tat ggc ggc aat tta atc gac ggc ttt aca ctg aca     1349
   Lys Pro Leu Ser Tyr Gly Gly Asn Leu Ile Asp Gly Phe Thr Leu Thr
           270                 275                 280
   ttt aaa aac gga cgg att acg gat gtc aaa gct gga caa ggc gaa gaa     1397
   Phe Lys Asn Gly Arg Ile Thr Asp Val Lys Ala Gly Gln Gly Glu Glu
       285                 290                 295
   att tta aaa gag ctg atc gaa acg gat gag ggt tca cac tac tta ggc     1445
   Ile Leu Lys Glu Leu Ile Glu Thr Asp Glu Gly Ser His Tyr Leu Gly
   300                 305                 310                 315
   gaa att gcg ctt gtt gcc cac gat tcg ccg att tcc aag tcc aat att     1493
   Glu Ile Ala Leu Val Ala His Asp Ser Pro Ile Ser Lys Ser Asn Ile
                   320                 325                 330
   ctg ttt tac aac aca ctg ttt gat gaa aat gcc tca aac cac ctc gcg     1541
   Leu Phe Tyr Asn Thr Leu Phe Asp Glu Asn Ala Ser Asn His Leu Ala
               335                 340                 345
   atc ggc agt gca tac gca ttc aac atc gaa ggc gga aaa cag atg tcg     1589
   Ile Gly Ser Ala Tyr Ala Phe Asn Ile Glu Gly Gly Lys Gln Met Ser
           350                 355                 360
   cgc gag gag ctg gcg aaa gaa gga tta aac gaa agc atc aca cat gtt     1637
   Arg Glu Glu Leu Ala Lys Glu Gly Leu Asn Glu Ser Ile Thr His Val
       365                 370                 375
   gac ttt atg atc ggt tca gga gaa atg aat ata gac gga atc act gct     1685
   Asp Phe Met Ile Gly Ser Gly Glu Met Asn Ile Asp Gly Ile Thr Ala
   380                 385                 390                 395
   gac gga aaa aga gag ccg ata ttc aga aac ggc aac tgg gcg ata         1730
   Asp Gly Lys Arg Glu Pro Ile Phe Arg Asn Gly Asn Trp Ala Ile
                   400                 405                 410
   taaagccttc cgcaaaaaaa gacctcagaa aacttctgag gtccttttat atcgtgtccg    1790
   catgccgccc gcaatcctaa acttccctgt acatgcatgg cggctgtaac agctgaaaat    1850
   caggtccatg ctccgccgcc tcatcgcttg ggcaaactgc ttaaatgttc atgcttcagt    1910
   ttccagcgtc ctgactcgtc ctttacaaat acgtttgtcg cacggcctgc cccttgaacc    1970
   ggttcgcccc tatataaccc tttccattcg tatgtataaa gacagacggc ctgttcgtcc    2030
   gtttccgtga tccagctgac atgaccggcg ctgtatactt cattttcgat ttgcttccag    2090
   gcgttttcaa aataacgctg aatctcctca aagcccgagc acgtttgatc cgaaaaccag    2150
   tacacagcgc cggggtcaag gagggctttg acattctcaa actcatgtgt gtttgttgct    2210
   tgaatatatt gctcaagagc                                                2230

<210>  18
<211>  410
<212>  PRT
<213>  Bacillus licheniformis

<400>  18

Met Asn Ser Phe Thr Asp Lys Leu Ser Lys Tyr Ala Lys Leu Val Ala
1               5                   10                  15

Glu Val Gly Val Asn Val Gln Lys Gly Gln Gln Val Val Ile Asn Ala
            20                  25                  30

Ser Thr Glu Val Arg Asp Phe Ala Arg Leu Leu Val Lys Ser Ala Tyr
        35                  40                  45

Gln Arg Gly Ala Lys Asn Val Thr Val Arg Trp Gln Asp Asp Glu Val
    50                  55                  60

Thr Arg Leu Lys Tyr Glu Leu Ala Pro Ala Glu Ala Phe Glu Glu Phe
65                  70                  75                  80

Pro Glu Trp Glu Ala Lys Gly Leu Glu Thr Leu Ala Lys Glu Gly Ala
                85                  90                  95

Ala Phe Ile Ser Ile Val Ser Ser Ser Pro Asp Leu Leu Lys Gly Ile
            100                 105                 110

Asp Ser Lys Lys Ile Ser Ala Gln Gln Lys Ala Ala Gly Lys Ala Met

115                 120                 125

His Thr Tyr Arg Gln Met Ile Gln Ser Asp Lys Val Ser Trp Thr Val
    130                 135                 140

Val Ala Ala Pro Ser Pro Ala Trp Ala Lys Lys Val Phe Pro Glu Ala
145                 150                 155                 160

Asp Glu Glu Thr Ala Val Gln Lys Leu Trp Asn Glu Ile Phe Lys Thr
                165                 170                 175

Thr Arg Val Asp Arg Glu Asp Pro Val Gln Ala Trp Lys Asp His Asp
            180                 185                 190

Gln Thr Leu Arg Asp Lys Val Ser Val Leu Asn Asp Lys His Tyr His
        195                 200                 205

Ala Leu His Tyr Gln Ala Lys Gly Thr Asp Leu Thr Ile Glu Leu Pro
    210                 215                 220

Glu Lys His Leu Trp Val Gly Ala Gly Ser Thr Asn Glu Asn Gly Val
225                 230                 235                 240

Glu Phe Met Ala Asn Met Pro Thr Glu Glu Val Phe Thr Ala Pro Lys
                245                 250                 255

Lys Asp Gly Val Asn Gly Thr Val Ser Ser Thr Lys Pro Leu Ser Tyr
            260                 265                 270

Gly Gly Asn Leu Ile Asp Gly Phe Thr Leu Thr Phe Lys Asn Gly Arg
        275                 280                 285

Ile Thr Asp Val Lys Ala Gly Gln Gly Glu Glu Ile Leu Lys Glu Leu
    290                 295                 300

Ile Glu Thr Asp Glu Gly Ser His Tyr Leu Gly Glu Ile Ala Leu Val
305                 310                 315                 320

Ala His Asp Ser Pro Ile Ser Lys Ser Asn Ile Leu Phe Tyr Asn Thr
                325                 330                 335

Leu Phe Asp Glu Asn Ala Ser Asn His Leu Ala Ile Gly Ser Ala Tyr
            340                 345                 350

Ala Phe Asn Ile Glu Gly Gly Lys Gln Met Ser Arg Glu Glu Leu Ala
        355                 360                 365

Lys Glu Gly Leu Asn Glu Ser Ile Thr His Val Asp Phe Met Ile Gly
    370                 375                 380

Ser Gly Glu Met Asn Ile Asp Gly Ile Thr Ala Asp Gly Lys Arg Glu

385                 390                 395                 400

Pro Ile Phe Arg Asn Gly Asn Trp Ala Ile
                405                 410

<210>  19
<211>  2390
<212>  DNA
<213>  Bacillus licheniformis

<220>
<221>  CDS
<222>  (1273)..(2388)

<400>  19
caggtgaaaa agatataaaa atgaaagggc cgatatcaat cggccttttc atattaccaa      60

aaaaacaagc ctgcaagagc cacaccggca attagaccga ggccaagtcc gtatacaggg     120

ccccaacccc accagccgcc gtaatatccg taggcgaacc cgcggcctcc ccgtctgatc     180

atcggggcaa tatatacttt gcttggtgaa acacggacga ttcttccgac atggacgcgg     240

cctcctttgt cggtgattct cgcaactctg ccaatatttt ggtggcaaaa cctataatat     300

cggtccactt gaatgctcct tccatcatct aatctattcc tagtcagctt atgacggatt     360

taatagaaaa ggttggacga aagaaggagg acaaatgatg atttatagat gtgatataaa     420

aataatatga tcgagagagt cccggtattt ttccaggtaa tgcaggtgtt tttccatcgt     480

ttcatagtct ttgtcggaaa ttgcaggcga cagatccatt tcaacggcgt catccgggtc     540

tcctgcatct tccaatacga caggaatgat gtcaatcttt gaaaatcctt cttctcgaag     600

gacattgacc cactcatcct cgtcatataa tgcggtgaat ccgtaaaatt ccgtgatttc     660

tttttttgct tcgtctgtca gtgcgtttcc ttccttaagg cagacttcga tcgcgaccag     720

catccctccg ggttttaaaa tccgccggat ttcctgtaaa gcagaccgga tatttgtgaa     780

gctcagcaca gattcagtta atacacaggc aaagctttca gactgaaacg gcatttcctc     840

aagtggagag cggacagccg gaatggaaag ctgttctttt ttaaaacgct gtcgggcttt     900

atcgatcatg accgggtcgg catcaagggc agtgacagga aacagaagct gacccaaata     960

agcggcggtc tggccggttc cgcagcctgc atcaagaatc ggctgctcag ggtcgatgcc    1020

ggctttggcg agcactgcct ttgacaggga catcccgccg ggatgagcgg atgagacgcc    1080

gaagctggcg agcatgtcta aataatcgct caaattgaga tccctccttt tttgtatgca    1140

tatgcaaaag cggaataatg gttgaaaacg atcgaatgcc agcatcccgg aaaaatcggt    1200

atttaagtga ttcgagttga cgaataaaga ttcgtccatg ataatatggt aagaagaaaa    1260

ggtggtgttc gt ttg aaa cag cag ttg aaa ctc ttt ttg gct gta ttg att    1311
              Leu Lys Gln Gln Leu Lys Leu Phe Leu Ala Val Leu Ile
              1               5                   10

acg gcc gtc gcc gcc tcc gca ctg acg ctt tat ctg gtg ggc aaa ggc      1359
Thr Ala Val Ala Ala Ser Ala Leu Thr Leu Tyr Leu Val Gly Lys Gly
    15                  20                  25

agc ggc ggt tca gta tat tcc gct tca gat tcc gag aaa ttc gaa aag     1407
Ser Gly Gly Ser Val Tyr Ser Ala Ser Asp Ser Glu Lys Phe Glu Lys
30                  35                  40                  45

ctg atg gcg gcg tac gac aaa atc aag agc gac tat tat caa aat gca     1455
Leu Met Ala Ala Tyr Asp Lys Ile Lys Ser Asp Tyr Tyr Gln Asn Ala
                50                  55                  60

gac gat gaa aag ctg act gac ggc gcg att aaa ggg atg ctt gga gct     1503
Asp Asp Glu Lys Leu Thr Asp Gly Ala Ile Lys Gly Met Leu Gly Ala
            65                  70                  75

ttg gat gat cca tat tct aca tac atg gat aag aaa gaa gct aaa agc     1551
Leu Asp Asp Pro Tyr Ser Thr Tyr Met Asp Lys Lys Glu Ala Lys Ser
        80                  85                  90

ttt gaa gaa agc att aca tct tct ttt gaa gga atc ggc gcc caa gta     1599
Phe Glu Glu Ser Ile Thr Ser Ser Phe Glu Gly Ile Gly Ala Gln Val
    95                  100                 105

gaa gaa aaa aat gga caa att cta atc gtc gcg cct att aaa gga tct     1647
Glu Glu Lys Asn Gly Gln Ile Leu Ile Val Ala Pro Ile Lys Gly Ser
110                 115                 120                 125

ccc gca gaa aaa gcc ggc tta aag ccg cat gat gaa att cag aaa gtg     1695
Pro Ala Glu Lys Ala Gly Leu Lys Pro His Asp Glu Ile Gln Lys Val
                130                 135                 140

gac ggc aaa agc gta aaa ggc aag acc gtc aac gaa gcg aca gcg atg     1743
Asp Gly Lys Ser Val Lys Gly Lys Thr Val Asn Glu Ala Thr Ala Met
            145                 150                 155

atc cgc ggc aaa aaa ggc acg aat gtc aaa ctt gtt ctc aac agg gaa     1791
Ile Arg Gly Lys Lys Gly Thr Asn Val Lys Leu Val Leu Asn Arg Glu
        160                 165                 170

ggc gtc ggc caa atc gat gtt acg att aag cgc gat acg att ccg atc     1839
Gly Val Gly Gln Ile Asp Val Thr Ile Lys Arg Asp Thr Ile Pro Ile
    175                 180                 185

gaa acg gtt tat tcc aaa atg atc gac ggc aat atc ggt gaa atc caa     1887
Glu Thr Val Tyr Ser Lys Met Ile Asp Gly Asn Ile Gly Glu Ile Gln
190                 195                 200                 205

att acg tct ttc tct gaa aat acc gca aaa gaa ctg aca aaa gcc att     1935
Ile Thr Ser Phe Ser Glu Asn Thr Ala Lys Glu Leu Thr Lys Ala Ile
                210                 215                 220

gat gac ttg tct gaa aaa ggt gcg gaa cga ttc gtg ctg gat ctc aga     1983
Asp Asp Leu Ser Glu Lys Gly Ala Glu Arg Phe Val Leu Asp Leu Arg
            225                 230                 235

gga aat cca ggc ggg ctg atg gac caa gcg att atg atg agc aac atg     2031
Gly Asn Pro Gly Gly Leu Met Asp Gln Ala Ile Met Met Ser Asn Met
        240                 245                 250

ttt gtc gat aaa ggc aaa acc atc atg cag gtt gaa tcc aaa aac gga     2079
Phe Val Asp Lys Gly Lys Thr Ile Met Gln Val Glu Ser Lys Asn Gly
    255                 260                 265

aaa aaa gaa gtg tac aaa gct gag aag gag cga aaa gtg aac aag ccg     2127
Lys Lys Glu Val Tyr Lys Ala Glu Lys Glu Arg Lys Val Asn Lys Pro
270                 275                 280                 285

acc gtt gtt ctc gtc aac gga gga acg gca agc gcc gct gag atc atg     2175
Thr Val Val Leu Val Asn Gly Gly Thr Ala Ser Ala Ala Glu Ile Met
                290                 295                 300

gcc gca gca ctt cac cag tct tca ggc atc ccg atc gtc ggt gaa aat 2223
Ala Ala Ala Leu His Gln Ser Ser Gly Ile Pro Ile Val Gly Glu Asn
            305                 310                 315

acg ttt ggg aaa ggg acg gtt caa aat gcg gag agc ttc tcc gac gga 2271
Thr Phe Gly Lys Gly Thr Val Gln Asn Ala Glu Ser Phe Ser Asp Gly
        320                 325                 330

tcg acc gtc aag ctg acg att gcc aag tgg ctg acg ccg aat ggg gac 2319
Ser Thr Val Lys Leu Thr Ile Ala Lys Trp Leu Thr Pro Asn Gly Asp
    335                 340                 345

tgg att cac gaa aaa gga atc aaa cct caa tat aaa gcg gag ctc cca 2367
Trp Ile His Glu Lys Gly Ile Lys Pro Gln Tyr Lys Ala Glu Leu Pro
350                 355                 360                 365

agc tat gcc aaa ctg ccg tac ct 2390
Ser Tyr Ala Lys Leu Pro Tyr
                370

<210> 20
<211> 372
<212> PRT
<213> Bacillus licheniformis

<400> 20

Leu Lys Gln Gln Leu Lys Leu Phe Leu Ala Val Leu Ile Thr Ala Val
1               5                   10                  15

Ala Ala Ser Ala Leu Thr Leu Tyr Leu Val Gly Lys Gly Ser Gly Gly
            20                  25                  30

Ser Val Tyr Ser Ala Ser Asp Ser Glu Lys Phe Glu Lys Leu Met Ala
        35                  40                  45

Ala Tyr Asp Lys Ile Lys Ser Asp Tyr Tyr Gln Asn Ala Asp Asp Glu
    50                  55                  60

Lys Leu Thr Asp Gly Ala Ile Lys Gly Met Leu Gly Ala Leu Asp Asp
65                  70                  75                  80

Pro Tyr Ser Thr Tyr Met Asp Lys Lys Glu Ala Lys Ser Phe Glu Glu
                85                  90                  95

Ser Ile Thr Ser Ser Phe Glu Gly Ile Gly Ala Gln Val Glu Glu Lys
            100                 105                 110

Asn Gly Gln Ile Leu Ile Val Ala Pro Ile Lys Gly Ser Pro Ala Glu
        115                 120                 125

Lys Ala Gly Leu Lys Pro His Asp Glu Ile Gln Lys Val Asp Gly Lys
    130                 135                 140

Ser Val Lys Gly Lys Thr Val Asn Glu Ala Thr Ala Met Ile Arg Gly
145                 150                 155                 160

Lys Lys Gly Thr Asn Val Lys Leu Val Leu Asn Arg Glu Gly Val Gly
                165                 170                 175

Gln Ile Asp Val Thr Ile Lys Arg Asp Thr Ile Pro Ile Glu Thr Val
            180                 185                 190

Tyr Ser Lys Met Ile Asp Gly Asn Ile Gly Glu Ile Gln Ile Thr Ser
        195                 200                 205

Phe Ser Glu Asn Thr Ala Lys Glu Leu Thr Lys Ala Ile Asp Asp Leu
    210                 215                 220

Ser Glu Lys Gly Ala Glu Arg Phe Val Leu Asp Leu Arg Gly Asn Pro
225                 230                 235                 240

Gly Gly Leu Met Asp Gln Ala Ile Met Met Ser Asn Met Phe Val Asp
                245                 250                 255

Lys Gly Lys Thr Ile Met Gln Val Glu Ser Lys Asn Gly Lys Lys Glu
            260                 265                 270

Val Tyr Lys Ala Glu Lys Glu Arg Lys Val Asn Lys Pro Thr Val Val
        275                 280                 285

Leu Val Asn Gly Gly Thr Ala Ser Ala Ala Glu Ile Met Ala Ala Ala
    290                 295                 300

Leu His Gln Ser Ser Gly Ile Pro Ile Val Gly Glu Asn Thr Phe Gly
305                 310                 315                 320

Lys Gly Thr Val Gln Asn Ala Glu Ser Phe Ser Asp Gly Ser Thr Val
                325                 330                 335

Lys Leu Thr Ile Ala Lys Trp Leu Thr Pro Asn Gly Asp Trp Ile His
            340                 345                 350

Glu Lys Gly Ile Lys Pro Gln Tyr Lys Ala Glu Leu Pro Ser Tyr Ala
        355                 360                 365

Lys Leu Pro Tyr
    370

<210>  21
<211>  2772
<212>  DNA
<213>  Bacillus licheniformis

<220>
<221>  CDS
<222>  (501)..(1406)

<400>  21
ttcttgaatc tgcggatata ttggcgtaag tattgtacga taaatccaaa tagcgaccca      60

ccctaaacac aatgcaataa ttatcttttt gtaataattg gggtcatgtt taattgtttt 120

ttccacggta tgagaacgct cccttctttt aatctatttt ttatttaatt aaattgtagg 180

ttgtaagcca gtcaataaca ttgttcactt tcaacaaaaa caaaagtaac attatacatt 240

ttagacaaaa ggcgtcgctt aaaaatgatt catcgataga agctggcctt atgactcttt 300

tttaaaacta acttggagta cccaatttaa cttcccgaat aaacaggaag cttcacaaat 360

gttgatataa cagtgttttt aacgttttaa aaactaaagg tgcatgacaa gcgaccttaa 420

gataagcttt ctttctcttt gaaaattcaa aaatcctccg atatatatag cgaacgccgc 480

taaaaaagga ggagagccgg atg aaa aag tta tgg aaa atc gcg gtt tcg gct 533
Met Lys Lys Leu Trp Lys Ile Ala Val Ser Ala
1               5                   10

gca atg ttc gtc ggt ttt ttc gca aat tca ccc cgc att caa gcg gaa 581
Ala Met Phe Val Gly Phe Phe Ala Asn Ser Pro Arg Ile Gln Ala Glu
            15                  20                  25

agc aat aag cag gaa aac gag gtc att gtt gta tat aaa aat acc agc 629
Ser Asn Lys Gln Glu Asn Glu Val Ile Val Val Tyr Lys Asn Thr Ser
        30                  35                  40

gga aaa gaa acc gtc atc gaa cag gca gac acg gta gaa cac gtt tac 677
Gly Lys Glu Thr Val Ile Glu Gln Ala Asp Thr Val Glu His Val Tyr
    45                  50                  55

cgg cac att ccc gca gcc gct gtc act gcg gac gac aaa aca gtg cgc 725
Arg His Ile Pro Ala Ala Ala Val Thr Ala Asp Asp Lys Thr Val Arg
60                  65                  70                  75

gaa ctt gaa cac gat ccc gac gtc ctg tat gtc gaa gac aac ctc ccg 773
Glu Leu Glu His Asp Pro Asp Val Leu Tyr Val Glu Asp Asn Leu Pro
                80                  85                  90

gta gct gct gcc gac agc acc gct cta aaa gct ttc tcc agc agc aca 821
Val Ala Ala Ala Asp Ser Thr Ala Leu Lys Ala Phe Ser Ser Ser Thr
            95                  100                 105

gcg caa aac gcc tcc gcg ttt tca cag tgg aac atc aag ctg att caa 869
Ala Gln Asn Ala Ser Ala Phe Ser Gln Trp Asn Ile Lys Leu Ile Gln
        110                 115                 120

gcc gca ctg gct tgg aat aaa ggt ttg acc gga aaa cag gtg aag atc 917
Ala Ala Leu Ala Trp Asn Lys Gly Leu Thr Gly Lys Gln Val Lys Ile
    125                 130                 135

gcg gtt att gac agc ggg att tcc ccc cat gag gag ctg tcg atc gcc 965
Ala Val Ile Asp Ser Gly Ile Ser Pro His Glu Glu Leu Ser Ile Ala
140                 145                 150                 155

ggc ggt gca tcg atg gtc ggc tat acc gct tca tac cgt gac gat aat 1013
Gly Gly Ala Ser Met Val Gly Tyr Thr Ala Ser Tyr Arg Asp Asp Asn
                160                 165                 170

ggc cac gga acc cat gtt gcc gga atc atc gga gcg aag cat aac gga 1061
Gly His Gly Thr His Val Ala Gly Ile Ile Gly Ala Lys His Asn Gly
            175                 180                 185

cgg ggg atc gac ggc atc gcg ccc ggc gcg cag ctg tat gcc gta aaa 1109
Arg Gly Ile Asp Gly Ile Ala Pro Gly Ala Gln Leu Tyr Ala Val Lys
        190                 195                 200

gcg ctg gac cgg aat ggt gcg ggg gat ctg aaa ggt atc tta caa ggc 1157

Ala Leu Asp Arg Asn Gly Ala Gly Asp Leu Lys Gly Ile Leu Gln Gly
    205                 210                 215

atc gat tgg tcg atc caa cac gga atc gat atc atc aat atg agc ctc      1205
Ile Asp Trp Ser Ile Gln His Gly Ile Asp Ile Ile Asn Met Ser Leu
220                 225                 230                 235

gtc gtt tca ggc gac agt caa gtt ctc cac gat gcg gta gat aaa gca      1253
Val Val Ser Gly Asp Ser Gln Val Leu His Asp Ala Val Asp Lys Ala
                240                 245                 250

tac aaa cgg gga atc att tta gtc gga gcg agc gga aat gcc gga aac      1301
Tyr Lys Arg Gly Ile Ile Leu Val Gly Ala Ser Gly Asn Ala Gly Asn
            255                 260                 265

gga aaa tcc gtt tat tac ccg gcc gcc tac agc agc gtc att gcc gtc      1349
Gly Lys Ser Val Tyr Tyr Pro Ala Ala Tyr Ser Ser Val Ile Ala Val
        270                 275                 280

tcg gca acc aat gaa aaa aat cag atc gcc tca ttt acc aac acc cgg      1397
Ser Ala Thr Asn Glu Lys Asn Gln Ile Ala Ser Phe Thr Asn Thr Arg
    285                 290                 295

agc gcc gta tagtattccg ctccgagcac atctattatc aatacatcgg              1446
Ser Ala Val
300

gcaatcgcgg atatgcaatc ggaagctgca ctgcacaagg gacacccgac gtcacctgcg    1506

tgatcgcact tatgaaacag ctgcacccaa ccgcttcaaa tgctgagctg cgcaagaaaa    1566

tgcagtttta tacaagcgat ttgggcgctc ccggtcgtga tcatttgttc ggctaccgat    1626

taatccgctt caaagaggtc acacagccat tggaaaaagc gcaaaaagcc gtcggacaag    1686

cggaaaagac aaagaaaaaa gcggacattc aaaccgcaca aaaagcgatc gaaccgctcc    1746

ctgcagacgc agataaaccc gctttgaaaa agagactcaa tactgttaaa gagcagctta    1806

aaaaaacagc cgaaagcaaa gtgaaactcg ctgagaagca aaaaaagaaa accaatgccg    1866

actccgccca aaaagcggtc aacgaactcg acagcggcac tttcaaaacg aatttgcaaa    1926

aacggatcaa cgcggtccgt tcaagcctct tgaagaccgc aaagcaggcc gttgccaaag    1986

ctgaaaaagc ggcaacagac tcaaacctcg gcaaagcgca aaaagcgatc aatgagcttc    2046

ccgccggaaa agacaaatca aatctgcaaa aaaggctgaa caccgcgaaa aagcaagcag    2106

ctgcagccta taataaaaaa gtttctgcag caaaggccaa agtcaaaacg gccgaacaaa    2166

agagaacgaa aaaaacaaaa tcagcagcgc aatcagcagt aggcaagctg aaagcatccg    2226

ccgaaaaaac gaagctgcaa aaacggatca atgccattaa gctgaagtag tacggaaaaa    2286

agcccggaat catccgggcc ttttaatttt catatttagc aaatgcgtca ttattgatgc    2346

tgttgtacta attgacattt cgggctcgtc atttgcgtaa tcgtattcct tggtcggccg    2406

cttactctcc cccgtactct ccaatcactt cttatcaatt ctttccgtca caatcccgac    2466

gatgtcccgt tctgaaacaa agccgaaatg gcggctgtca aagctgtgga tccggttgtc    2526

cccgaggacg aagtactcgt tttcaggcac cttctcctct ccggtcactt cctgcagtgt    2586

aaaatcccct gtcaagtccc ctccggcggt gacggccttt aagtcgtcca gataaggctc    2646

tttgatcttt tcttcgttga tatacagctg atcatcttca tacctgaggg tttcgcccgg    2706

aagcccgatc acccttttaa tgaatatatc tttgtcaggt cctttaaaca gcacgatgtc 2766

aaatcg 2772

<210> 22
<211> 302
<212> PRT
<213> Bacillus licheniformis

<400> 22

Met Lys Lys Leu Trp Lys Ile Ala Val Ser Ala Ala Met Phe Val Gly
1               5                   10                  15

Phe Phe Ala Asn Ser Pro Arg Ile Gln Ala Glu Ser Asn Lys Gln Glu
            20                  25                  30

Asn Glu Val Ile Val Val Tyr Lys Asn Thr Ser Gly Lys Glu Thr Val
        35                  40                  45

Ile Glu Gln Ala Asp Thr Val Glu His Val Tyr Arg His Ile Pro Ala
    50                  55                  60

Ala Ala Val Thr Ala Asp Asp Lys Thr Val Arg Glu Leu Glu His Asp
65                  70                  75                  80

Pro Asp Val Leu Tyr Val Glu Asp Asn Leu Pro Val Ala Ala Ala Asp
                85                  90                  95

Ser Thr Ala Leu Lys Ala Phe Ser Ser Ser Thr Ala Gln Asn Ala Ser
            100                 105                 110

Ala Phe Ser Gln Trp Asn Ile Lys Leu Ile Gln Ala Ala Leu Ala Trp
        115                 120                 125

Asn Lys Gly Leu Thr Gly Lys Gln Val Lys Ile Ala Val Ile Asp Ser
    130                 135                 140

Gly Ile Ser Pro His Glu Glu Leu Ser Ile Ala Gly Gly Ala Ser Met
145                 150                 155                 160

Val Gly Tyr Thr Ala Ser Tyr Arg Asp Asp Asn Gly His Gly Thr His
                165                 170                 175

Val Ala Gly Ile Ile Gly Ala Lys His Asn Gly Arg Gly Ile Asp Gly
            180                 185                 190

Ile Ala Pro Gly Ala Gln Leu Tyr Ala Val Lys Ala Leu Asp Arg Asn
        195                 200                 205

Gly Ala Gly Asp Leu Lys Gly Ile Leu Gln Gly Ile Asp Trp Ser Ile
    210                 215                 220

Gln His Gly Ile Asp Ile Ile Asn Met Ser Leu Val Val Ser Gly Asp
225                 230                 235                 240

Ser Gln Val Leu His Asp Ala Val Asp Lys Ala Tyr Lys Arg Gly Ile
                245                 250                 255

Ile Leu Val Gly Ala Ser Gly Asn Ala Gly Asn Gly Lys Ser Val Tyr
            260                 265                 270

Tyr Pro Ala Ala Tyr Ser Ser Val Ile Ala Val Ser Ala Thr Asn Glu
        275                 280                 285

Lys Asn Gln Ile Ala Ser Phe Thr Asn Thr Arg Ser Ala Val
    290                 295                 300

<210>  23
<211>  2185
<212>  DNA
<213>  Bacillus licheniformis

<220>
<221>  CDS
<222>  (214)..(1149)

<400>  23
atcagggcat gcagacggac cggtttattt tcaacctcgg acacgggatc tttccagaag      60

tgagtcccga cacactgaaa aaattgacgg catttgttca tgagtattcc gcctcaaaaa     120

aatcgagcct gatcacatga gcatttcagt tggtcttttc aatataaact gtcaaaatac     180

gtatagatca tgatctttaa agaggtgttc acg ttg ggt aaa aag aaa atg ggg      234
                                     Leu Gly Lys Lys Lys Met Gly
                                     1               5

ctt ctt gtc atg gca tat ggg acg ccg tac aaa gag gaa gac att gaa      282
Leu Leu Val Met Ala Tyr Gly Thr Pro Tyr Lys Glu Glu Asp Ile Glu
        10                  15                  20

cgt tat tat aca cat atc aga aga ggc aga aag cct gag cct gac atg      330
Arg Tyr Tyr Thr His Ile Arg Arg Gly Arg Lys Pro Glu Pro Asp Met
    25                  30                  35

ctt cag gac tta aaa gac cgc tat aag gcg atc gga gga att tct ccg      378
Leu Gln Asp Leu Lys Asp Arg Tyr Lys Ala Ile Gly Gly Ile Ser Pro
40                  45                  50                  55

ctt gca aag atc acg cag gag cag acg aag cag ctg gaa aaa cgc tta      426
Leu Ala Lys Ile Thr Gln Glu Gln Thr Lys Gln Leu Glu Lys Arg Leu
                60                  65                  70

aac gag ctt cag gat gat gtg gtg ttc aag gca tat atc ggt ctc aaa      474
Asn Glu Leu Gln Asp Asp Val Val Phe Lys Ala Tyr Ile Gly Leu Lys
            75                  80                  85

cat atc gaa cct ttc att gag gat gcc gtt cgg gag atg cat aaa gac      522
His Ile Glu Pro Phe Ile Glu Asp Ala Val Arg Glu Met His Lys Asp
        90                  95                  100

ggg att aca gaa gcc gtc agc atc gtg ctc gcg ccc cat ttc tcc acc      570
Gly Ile Thr Glu Ala Val Ser Ile Val Leu Ala Pro His Phe Ser Thr

105                 110                 115

ttt agc gta cag tcg tac aac aag cgg gcg aaa gac gag gct gaa aag      618
Phe Ser Val Gln Ser Tyr Asn Lys Arg Ala Lys Asp Glu Ala Glu Lys
120                 125                 130                 135

ctc ggc gaa ttg tcg atc aca tcg atc aac agc tgg tat gat gag ccg      666
Leu Gly Glu Leu Ser Ile Thr Ser Ile Asn Ser Trp Tyr Asp Glu Pro
                140                 145                 150

aaa ttt atc gct tat tgg gcg gat cag gtg aga aaa atc tat gac gaa      714
Lys Phe Ile Ala Tyr Trp Ala Asp Gln Val Arg Lys Ile Tyr Asp Glu
            155                 160                 165

atg cct tcg gaa gag agg gaa aac gcc gtc ttg att gta tcg gcg cac      762
Met Pro Ser Glu Glu Arg Glu Asn Ala Val Leu Ile Val Ser Ala His
        170                 175                 180

agc ctt ccc gaa aaa atc gtc gaa atg ggt gat ccg tat cct gaa cag      810
Ser Leu Pro Glu Lys Ile Val Glu Met Gly Asp Pro Tyr Pro Glu Gln
    185                 190                 195

ctt aaa gaa tca gcg aag ctg att gcc gaa gcg gcg gga gta aaa gat      858
Leu Lys Glu Ser Ala Lys Leu Ile Ala Glu Ala Ala Gly Val Lys Asp
200                 205                 210                 215

tac gcc gtc ggc tgg caa agc gaa gga aac acc cct gat ccg tgg ctg      906
Tyr Ala Val Gly Trp Gln Ser Glu Gly Asn Thr Pro Asp Pro Trp Leu
                220                 225                 230

gga ccg gat gtt cag gat ttg acg cgc gac ttg tca gag caa aaa ggg      954
Gly Pro Asp Val Gln Asp Leu Thr Arg Asp Leu Ser Glu Gln Lys Gly
            235                 240                 245

tac agc gct ttt gtt tat gct ccc gtc ggc ttt gta gcc gac cat ttg      1002
Tyr Ser Ala Phe Val Tyr Ala Pro Val Gly Phe Val Ala Asp His Leu
        250                 255                 260

gaa gtc ctt tat gac aat gac tat gag tgc aaa gtc gtc acg gac gat      1050
Glu Val Leu Tyr Asp Asn Asp Tyr Glu Cys Lys Val Val Thr Asp Asp
    265                 270                 275

atc gga gca agc tat tac aga cct gaa atg cct aac gct aaa cat gag      1098
Ile Gly Ala Ser Tyr Tyr Arg Pro Glu Met Pro Asn Ala Lys His Glu
280                 285                 290                 295

ttt atc gat gct tta gcg gat gtt gta tta aaa caa ttg gag aaa gaa      1146
Phe Ile Asp Ala Leu Ala Asp Val Val Leu Lys Gln Leu Glu Lys Glu
                300                 305                 310

cag taaagaaggc gattgtaaat gagtggagaa cgcaggcaga ttgtcatcat      1199
Gln

cggcggcggt attaccgggt tggccgccgc cttctacttg gagaaggaaa tcaaaaagaa      1259

tgatctcccg gccgaagtca cgcttgttga agcaagcccg agactgggcg gaaagatgca      1319

gacggtacac aaagacggct atgtgattga acggggtccg gattcatttt tggaacggaa      1379

acaaagcgca cctcagctcg tgaaggatct cggactggag catttgcttg tgaacaatgc      1439

gactggccag tcttatgtgc ttgtcagcga aacgctctat ccgattccgg aaggagccgt      1499

gatgggggtg ccgacaaaaa tcggtccttt tctgacaacc ggcttgtttt ccttgccggg      1559

gaagttgaga gccggaatgg atttggtgct tccagcaagc aagccgcagg aagaccaatc      1619

gctcggagaa tttttcagaa ggcgcgtcgg cggtgaagtg gtggagaacc tgatcgaacc 1679

gcttttgtca ggcatttatg ccggggacat cgaccgtctc agtctgatgt cgactttccc 1739

gcagttctat cagacagagc agaagtacag aagcttgatc atcgggatga aaaaatcgcg 1799

cggcgcccag agcgtcggaa agcctgcaaa caaaaagaaa ggccagttcc agacggtgaa 1859

aaccggcctg cagacgatag ttgaagaatt ggaaaagcag ctcggcatca caaaggttta 1919

taaaggcaca aaggtcgtca gcattgagcg tgcgggctcg gcttacagca tgaagcttga 1979

caacggcaag gtgcttcacg ccgattcggc cgtggtcacc gctcctcata aagcagctgc 2039

cgcgatgttt caaaaagagg attggctgaa gggtttagag gatatggtgt caacatctgt 2099

cgccaatgtg gcgctcggat ttccggaaga agccgtccaa atggagcatc aaggcacggg 2159

ctttgtcatt tcaagaaaca gcgatt 2185

<210> 24
<211> 312
<212> PRT
<213> Bacillus licheniformis

<400> 24

Leu Gly Lys Lys Lys Met Gly Leu Leu Val Met Ala Tyr Gly Thr Pro
1               5                   10                  15

Tyr Lys Glu Glu Asp Ile Glu Arg Tyr Tyr Thr His Ile Arg Arg Gly
            20                  25                  30

Arg Lys Pro Glu Pro Asp Met Leu Gln Asp Leu Lys Asp Arg Tyr Lys
        35                  40                  45

Ala Ile Gly Gly Ile Ser Pro Leu Ala Lys Ile Thr Gln Glu Gln Thr
    50                  55                  60

Lys Gln Leu Glu Lys Arg Leu Asn Glu Leu Gln Asp Asp Val Val Phe
65                  70                  75                  80

Lys Ala Tyr Ile Gly Leu Lys His Ile Glu Pro Phe Ile Glu Asp Ala
                85                  90                  95

Val Arg Glu Met His Lys Asp Gly Ile Thr Glu Ala Val Ser Ile Val
            100                 105                 110

Leu Ala Pro His Phe Ser Thr Phe Ser Val Gln Ser Tyr Asn Lys Arg
        115                 120                 125

Ala Lys Asp Glu Ala Glu Lys Leu Gly Glu Leu Ser Ile Thr Ser Ile
    130                 135                 140

Asn Ser Trp Tyr Asp Glu Pro Lys Phe Ile Ala Tyr Trp Ala Asp Gln
145                 150                 155                 160

Val Arg Lys Ile Tyr Asp Glu Met Pro Ser Glu Glu Arg Glu Asn Ala
                165                 170                 175

Val Leu Ile Val Ser Ala His Ser Leu Pro Glu Lys Ile Val Glu Met
            180                 185                 190

Gly Asp Pro Tyr Pro Glu Gln Leu Lys Glu Ser Ala Lys Leu Ile Ala
        195                 200                 205

Glu Ala Ala Gly Val Lys Asp Tyr Ala Val Gly Trp Gln Ser Glu Gly
    210                 215                 220

Asn Thr Pro Asp Pro Trp Leu Gly Pro Asp Val Gln Asp Leu Thr Arg
225                 230                 235                 240

Asp Leu Ser Glu Gln Lys Gly Tyr Ser Ala Phe Val Tyr Ala Pro Val
                245                 250                 255

Gly Phe Val Ala Asp His Leu Glu Val Leu Tyr Asp Asn Asp Tyr Glu
            260                 265                 270

Cys Lys Val Val Thr Asp Asp Ile Gly Ala Ser Tyr Tyr Arg Pro Glu
        275                 280                 285

Met Pro Asn Ala Lys His Glu Phe Ile Asp Ala Leu Ala Asp Val Val
    290                 295                 300

Leu Lys Gln Leu Glu Lys Glu Gln
305                 310

<210>  25
<211>  2107
<212>  DNA
<213>  Bacillus licheniformis

<220>
<221>  CDS
<222>  (501)..(1604)

<400>  25
   aaaaaagctt ggcatagcca agctttctat tatgcagaaa gtccgttcac ttttttcgct      60
   aggtttgatt tgtaacgagc ggcggtgttt ttgtggacaa gacctttttt agctgcttta     120
   tcgattttct ttgctgctgc agatagagct gctttcgctt tgtctgcttc gttgttgctt     180
   acgaaagttt caacttgttt catagcagtg cgcattgcag atttgattgt tgcgttgtgc     240
   gcgcggcgct cgttgtttgt ttttgtgcgt ttgatcgctg atttaatgtt tggcaatgtg     300
   tttcacctcc taagacataa cctctgttcc ttttctatag gttagatctc aacaatagaa     360
   caaattgtat tctatcaaac acacccttag attgcaatat aaatgtaaag tatttttatt     420
   tgaaagctcc gcttcgcggg aatgtttgca gaccttgtgg gcacactaac accagttatg     480
   tttttggagg gaatgcaaat atg gag aaa aag aag ctc gat tta agt caa tat     533
   Met Glu Lys Lys Lys Leu Asp Leu Ser Gln Tyr
   1               5                   10
   gca gtc cgg aca gac tta gcc gtg gaa gcg agg gat ctg gca gag gaa     581
   Ala Val Arg Thr Asp Leu Ala Val Glu Ala Arg Asp Leu Ala Glu Glu
               15                  20                  25
   aag gaa gct tct cca aag aaa gag ctg aaa ggg ttt aca gtc aaa gaa     629
   Lys Glu Ala Ser Pro Lys Lys Glu Leu Lys Gly Phe Thr Val Lys Glu
           30                  35                  40
   tac gaa aaa gac ggg atc aaa att cag acg atg gat att gat gaa gaa     677
   Tyr Glu Lys Asp Gly Ile Lys Ile Gln Thr Met Asp Ile Asp Glu Glu
       45                  50                  55
   gga gcg aag ctg tcg ggg aaa aaa gcc ggc cgc tat tta acc ttt gaa     725
   Gly Ala Lys Leu Ser Gly Lys Lys Ala Gly Arg Tyr Leu Thr Phe Glu
   60                  65                  70                  75
   aca caa ggg atc aga cag cag gat tcc gta ttg cag gaa aaa gtc gtc     773
   Thr Gln Gly Ile Arg Gln Gln Asp Ser Val Leu Gln Glu Lys Val Val
                   80                  85                  90
   gat gtc ttc gca aag gaa ttt tcc tcg ttt ctc gat tat ttg ggc att     821
   Asp Val Phe Ala Lys Glu Phe Ser Ser Phe Leu Asp Tyr Leu Gly Ile
               95                  100                 105
   ccg cgg gat gcc agc tgc tta ata gtc ggg ctc ggc aac tgg aat gtc     869
   Pro Arg Asp Ala Ser Cys Leu Ile Val Gly Leu Gly Asn Trp Asn Val
           110                 115                 120
   acg ccc gac tcg ctc ggc ccc cta gtg acg gag aac ctt ctc gtg acg     917
   Thr Pro Asp Ser Leu Gly Pro Leu Val Thr Glu Asn Leu Leu Val Thr
       125                 130                 135
   agg cac ctg ttt cag ctg cag ccc gaa aat gta gag gaa ggc tac cgt     965
   Arg His Leu Phe Gln Leu Gln Pro Glu Asn Val Glu Glu Gly Tyr Arg
   140                 145                 150                 155
   ccg gtg agt gcg ctt tcc ccg gga gtc atg ggc ttg acc gga att gaa     1013
   Pro Val Ser Ala Leu Ser Pro Gly Val Met Gly Leu Thr Gly Ile Glu
                   160                 165                 170
   acg agc gac att atc caa ggg gtg atc gat cgc tca aag ccc gat ttt     1061
   Thr Ser Asp Ile Ile Gln Gly Val Ile Asp Arg Ser Lys Pro Asp Phe
               175                 180                 185
   gtc att gcc att gat gcg ctg gca tca aga ggg atc gag cgt gtg aat     1109
   Val Ile Ala Ile Asp Ala Leu Ala Ser Arg Gly Ile Glu Arg Val Asn
           190                 195                 200
   tct acc att caa att tca gac agc ggg ata cac ccc ggg tct ggt gtg     1157
   Ser Thr Ile Gln Ile Ser Asp Ser Gly Ile His Pro Gly Ser Gly Val
       205                 210                 215
   ggg aat aaa aga aaa gag tta agc aag gat acg ctc ggc att ccg gtc     1205
   Gly Asn Lys Arg Lys Glu Leu Ser Lys Asp Thr Leu Gly Ile Pro Val
   220                 225                 230                 235
   atc gcc atc ggt gtt ccg aca gtg gtc gat gcc gtc acg atc acg agc     1253
   Ile Ala Ile Gly Val Pro Thr Val Val Asp Ala Val Thr Ile Thr Ser
                   240                 245                 250
   gac acg att gat tac atg ctg aag cat ttt gga aga gag atg aga gac     1301
   Asp Thr Ile Asp Tyr Met Leu Lys His Phe Gly Arg Glu Met Arg Asp
               255                 260                 265
   gac agt ccg tcc agg tcg ctc gtt ccc gcg ggg atg agc ttt ggg aaa     1349
   Asp Ser Pro Ser Arg Ser Leu Val Pro Ala Gly Met Ser Phe Gly Lys
   270 275 280
   aga aag gtc ctc acc gaa gaa gac ctt ccc gat gaa gag cac cgc aaa 1397
   Arg Lys Val Leu Thr Glu Glu Asp Leu Pro Asp Glu Glu His Arg Lys
   285 290 295
   tcg ttt ctc ggt ata gtc gga ggc ctc gca gag gat gaa aaa agg cag 1445
   Ser Phe Leu Gly Ile Val Gly Gly Leu Ala Glu Asp Glu Lys Arg Gln
   300 305 310 315
   ctg atc cat gaa gtc ctc gct ccg ctc ggc cat aac tta atg gtg acc 1493
   Leu Ile His Glu Val Leu Ala Pro Leu Gly His Asn Leu Met Val Thr
   320 325 330
   cct aaa gag gtc gat aca ttt atc gat gat atg gca aat gtg att gca 1541
   Pro Lys Glu Val Asp Thr Phe Ile Asp Asp Met Ala Asn Val Ile Ala
   335 340 345
   aac ggt ctg aac aca gcg ctc cat gaa aat gta tcg caa gac aat aaa 1589
   Asn Gly Leu Asn Thr Ala Leu His Glu Asn Val Ser Gln Asp Asn Lys
   350 355 360
   gga atg tat aac cac taagatcaag gcggctggcc cggccgcttt ttttcatgac 1644
   Gly Met Tyr Asn His
   365
   atttagcatt gccggaactt gttctacttc ctctatcttg tacatagtct atttactaga 1704
   caagctctgg agggatttac aatgagaaaa agaggcagga atcgtcagtt tgtgctggct 1764
   gtaaatggaa gaagcgcggt taagacagta ttcttattta tcgtcagcct gctgctcgtt 1824
   tttattttat caggcgtgct cacatcgctg cgtcctgagt taaggccgtc ttcatcgttg 1884
   taccgggtgg ctgatgaact gaagggcgag acctttgggc tcattttggg aatggaaaac 1944
   cactactttg catcagaatt gccggaaccg aataagcgct ttgagctttc ccccctcgtc 2004
   ctgaagctgg cgaccagcat taatttgaaa gatccgcgaa gttttctcgg acgggagctt 2064
   ccgggattct ctcattttga ctcggaaatc ctcattgccg ggc 2107

<210> 26
<211> 368
<212> PRT
<213> Bacillus licheniformis

<400> 26

Met Glu Lys Lys Lys Leu Asp Leu Ser Gln Tyr Ala Val Arg Thr Asp
1               5                   10                  15

Leu Ala Val Glu Ala Arg Asp Leu Ala Glu Glu Lys Glu Ala Ser Pro
            20                  25                  30

Lys Lys Glu Leu Lys Gly Phe Thr Val Lys Glu Tyr Glu Lys Asp Gly
        35                  40                  45

Ile Lys Ile Gln Thr Met Asp Ile Asp Glu Glu Gly Ala Lys Leu Ser
    50                  55                  60

Gly Lys Lys Ala Gly Arg Tyr Leu Thr Phe Glu Thr Gln Gly Ile Arg

65                  70                  75                  80

Gln Gln Asp Ser Val Leu Gln Glu Lys Val Val Asp Val Phe Ala Lys
                85                  90                  95

Glu Phe Ser Ser Phe Leu Asp Tyr Leu Gly Ile Pro Arg Asp Ala Ser
            100                 105                 110

Cys Leu Ile Val Gly Leu Gly Asn Trp Asn Val Thr Pro Asp Ser Leu
        115                 120                 125

Gly Pro Leu Val Thr Glu Asn Leu Leu Val Thr Arg His Leu Phe Gln
    130                 135                 140

Leu Gln Pro Glu Asn Val Glu Glu Gly Tyr Arg Pro Val Ser Ala Leu
145                 150                 155                 160

Ser Pro Gly Val Met Gly Leu Thr Gly Ile Glu Thr Ser Asp Ile Ile
                165                 170                 175

Gln Gly Val Ile Asp Arg Ser Lys Pro Asp Phe Val Ile Ala Ile Asp
            180                 185                 190

Ala Leu Ala Ser Arg Gly Ile Glu Arg Val Asn Ser Thr Ile Gln Ile
        195                 200                 205

Ser Asp Ser Gly Ile His Pro Gly Ser Gly Val Gly Asn Lys Arg Lys
    210                 215                 220

Glu Leu Ser Lys Asp Thr Leu Gly Ile Pro Val Ile Ala Ile Gly Val
225                 230                 235                 240

Pro Thr Val Val Asp Ala Val Thr Ile Thr Ser Asp Thr Ile Asp Tyr
                245                 250                 255

Met Leu Lys His Phe Gly Arg Glu Met Arg Asp Asp Ser Pro Ser Arg
            260                 265                 270

Ser Leu Val Pro Ala Gly Met Ser Phe Gly Lys Arg Lys Val Leu Thr
        275                 280                 285

Glu Glu Asp Leu Pro Asp Glu Glu His Arg Lys Ser Phe Leu Gly Ile
    290                 295                 300

Val Gly Gly Leu Ala Glu Asp Glu Lys Arg Gln Leu Ile His Glu Val
305                 310                 315                 320

Leu Ala Pro Leu Gly His Asn Leu Met Val Thr Pro Lys Glu Val Asp
                325                 330                 335

Thr Phe Ile Asp Asp Met Ala Asn Val Ile Ala Asn Gly Leu Asn Thr

340                 345                 350

Ala Leu His Glu Asn Val Ser Gln Asp Asn Lys Gly Met Tyr Asn His
        355                 360                 365

<210>  27
<211>  2358
<212>  DNA
<213>  Bacillus licheniformis

<220>
<221>  CDS
<222>  (501)..(1868)

<400>  27
tgtcaatgtc ggcatcgcgc gcagaaataa aaatgacggg gacttccgga tcttgatttt      60

ttatttcttt tattagcgta tacccgtcga tatctggtaa catgatatcg agaatccata     120

aatgaggtga ctggcctatt ttttgcctgg cgctttcacc gtttagaaaa gaagtgacgt     180

gccagccttc actctctaag tattttgtca gcaattcatt taaattctct tcatcttcaa     240

ctagataaat ggagtatgac aagggcggtt cacatccttt ctacgtcatt atactagttt     300

taacataagt ctggtcaatt ttcataattt cacatattct tttcattttt atcccacaat     360

gttttcgtaa catattcatc agggaagaaa gggaatgaaa acagagtgaa cgatggccgg     420

gtctcttttt ttgtggatgt taaagcttta atgccatttg cgatagttga agacaagaaa     480

aataaaggaa gtgtacgctt atg gat ttt aga cgc gat gac gaa caa aaa cat     533
                      Met Asp Phe Arg Arg Asp Asp Glu Gln Lys His
                      1               5                   10

gca aat gaa gag cag ttt cac gaa gaa gcg cag acg ccg caa acg gca       581
Ala Asn Glu Glu Gln Phe His Glu Glu Ala Gln Thr Pro Gln Thr Ala
            15                  20                  25

ggt tct gag gag cag cag tca gaa cag ccg gca aag gag ccc gag ctg       629
Gly Ser Glu Glu Gln Gln Ser Glu Gln Pro Ala Lys Glu Pro Glu Leu
        30                  35                  40

att tta aag aag gat aca gac gat cag tca gat ctt acg aat aaa gaa       677
Ile Leu Lys Lys Asp Thr Asp Asp Gln Ser Asp Leu Thr Asn Lys Glu
    45                  50                  55

acc gct gca gcc ctg gaa gat gcc agg gaa acg aga gcg gca aaa gaa       725
Thr Ala Ala Ala Leu Glu Asp Ala Arg Glu Thr Arg Ala Ala Lys Glu
60                  65                  70                  75

aag cgg cgc aag gct tcg tgg ctg agc ccg att ttg ggc ggg atc atc       773
Lys Arg Arg Lys Ala Ser Trp Leu Ser Pro Ile Leu Gly Gly Ile Ile
                80                  85                  90

gga ggc ggc ttg gtg ctt gga atc tcc ccg tat ctg ccg ggc gat cat       821
Gly Gly Gly Leu Val Leu Gly Ile Ser Pro Tyr Leu Pro Gly Asp His
            95                  100                 105

ccg agc gaa act gcc gga acc gga cag aca gag cca aag cag tcg cag       869
Pro Ser Glu Thr Ala Gly Thr Gly Gln Thr Glu Pro Lys Gln Ser Gln
        110                 115                 120

aat ttc acc aca aaa cct gtc acg aac gca gat aac gta cct gat atg       917
Asn Phe Thr Thr Lys Pro Val Thr Asn Ala Asp Asn Val Pro Asp Met

    125                 130                 135

gtg gaa gat ttg gag cct gcg atc gtc gga gtt tca aat att caa acg 965
Val Glu Asp Leu Glu Pro Ala Ile Val Gly Val Ser Asn Ile Gln Thr
140                 145                 150                 155

agc ttc ggc ttc tct gaa gac gac gtg gag gag agc ggc acg gga tca 1013
Ser Phe Gly Phe Ser Glu Asp Asp Val Glu Glu Ser Gly Thr Gly Ser
                160                 165                 170

ggc gtc atc ttt aag aaa gac ggc ggc aag gcc tac att atc aca aac 1061
Gly Val Ile Phe Lys Lys Asp Gly Gly Lys Ala Tyr Ile Ile Thr Asn
            175                 180                 185

aac cat gtc gtt gaa ggc gcc tcg aag gtg acc atc tcc tta tac aac 1109
Asn His Val Val Glu Gly Ala Ser Lys Val Thr Ile Ser Leu Tyr Asn
        190                 195                 200

gga aag acc gcg gat gcc aag att atc ggc agc gac gct ttg act gat 1157
Gly Lys Thr Ala Asp Ala Lys Ile Ile Gly Ser Asp Ala Leu Thr Asp
    205                 210                 215

ctg gct gtt tta gag atc agc agc aaa ggc gtt gat aag gtt gca agt 1205
Leu Ala Val Leu Glu Ile Ser Ser Lys Gly Val Asp Lys Val Ala Ser
220                 225                 230                 235

ttc gga gac tcc gca aaa ctg cgc gcc ggc gaa aaa gta att gcg atc 1253
Phe Gly Asp Ser Ala Lys Leu Arg Ala Gly Glu Lys Val Ile Ala Ile
                240                 245                 250

ggg aac cct ctc ggc ctt caa ttt tca aga acg gtt aca gaa ggc atc 1301
Gly Asn Pro Leu Gly Leu Gln Phe Ser Arg Thr Val Thr Glu Gly Ile
            255                 260                 265

atc agc ggt gtc aac cgg acg att gaa gtc tca aca tct gaa ggg aat 1349
Ile Ser Gly Val Asn Arg Thr Ile Glu Val Ser Thr Ser Glu Gly Asn
        270                 275                 280

tgg gat atg aac gtc ctt cag aca gat gcg gcg atc aac cct gga aac 1397
Trp Asp Met Asn Val Leu Gln Thr Asp Ala Ala Ile Asn Pro Gly Asn
    285                 290                 295

agc ggc ggg cct ctg att aac agc agc ggg cag gtc atc ggc atc aac 1445
Ser Gly Gly Pro Leu Ile Asn Ser Ser Gly Gln Val Ile Gly Ile Asn
300                 305                 310                 315

agc ctg aag atc agc caa agc ggt gtt gaa tcg ctt ggc ttc gcg att 1493
Ser Leu Lys Ile Ser Gln Ser Gly Val Glu Ser Leu Gly Phe Ala Ile
                320                 325                 330

ccg agc aac gat gtt cag ccg atc gtt gac gag ctt ttg gaa aaa ggg 1541
Pro Ser Asn Asp Val Gln Pro Ile Val Asp Glu Leu Leu Glu Lys Gly
            335                 340                 345

aaa gtg gaa cgg cct ttc ctc ggc gtg caa atg atc gat atg cag cag 1589
Lys Val Glu Arg Pro Phe Leu Gly Val Gln Met Ile Asp Met Gln Gln
        350                 355                 360

gtg cct gaa cag tac cag caa aat acg ctc ggc cta ttc gga gac cag 1637
Val Pro Glu Gln Tyr Gln Gln Asn Thr Leu Gly Leu Phe Gly Asp Gln
    365                 370                 375

ctt aat aaa ggc gtg tac atc gat aaa gta tcg ccg aaa tcg cct gcg 1685
Leu Asn Lys Gly Val Tyr Ile Asp Lys Val Ser Pro Lys Ser Pro Ala
380                 385                 390                 395

gca gac gcg ggt atg aaa gcc ggc gac gtc atc acc aaa atg aac ggc 1733
Ala Asp Ala Gly Met Lys Ala Gly Asp Val Ile Thr Lys Met Asn Gly

400                 405                 410

aaa aac gtc gaa acc acc tca gac ctc aga aaa att ctt tat aca gaa      1781
Lys Asn Val Glu Thr Thr Ser Asp Leu Arg Lys Ile Leu Tyr Thr Glu
            415                 420                 425

gca aaa gca gga gat act gtc aca ttt gag gtg ctg aga aac gga aag      1829
Ala Lys Ala Gly Asp Thr Val Thr Phe Glu Val Leu Arg Asn Gly Lys
        430                 435                 440

caa aca aca atg aaa gca aaa ctt gcg aaa agc aaa tca taaataaaag       1878
Gln Thr Thr Met Lys Ala Lys Leu Ala Lys Ser Lys Ser
    445                 450                 455

ccatggcaaa cgccatggct ttttcatatt acttgtccaa aaatgaagaa agcatccaaa     1938

tctgcttatc aatttcctct gtcagggcga tataaaggtc agcggtagaa tgatctgatt    1998

tatcttccgc aagttcgatg gtatgctgaa tttcatcgcg gatttgacgg taatcgctta    2058

ccagtgcgga aaccatttct tcggccgttt tttcggctcc gttttcctca attgtgccgt    2118

ggtcaatgta ttctttcatt gtggcgagcg gctgtccgcc gatggccagc aagcgttccg    2178

cgatcacatc tgctgtttct gacgcatggt tgtatagctc ttcaaatttt tcatgtaggg    2238

taaagaactg cggccctttt acgtaccagt gaaaacggtg aagcttcgtg taaaggatga    2298

accagtttga caaattcgta ttcattgagt tttcaagtac aggatttgct gttttaattt    2358

<210>  28
<211>  456
<212>  PRT
<213>  Bacillus licheniformis

<400>  28

Met Asp Phe Arg Arg Asp Asp Glu Gln Lys His Ala Asn Glu Glu Gln
1               5                   10                  15

Phe His Glu Glu Ala Gln Thr Pro Gln Thr Ala Gly Ser Glu Glu Gln
            20                  25                  30

Gln Ser Glu Gln Pro Ala Lys Glu Pro Glu Leu Ile Leu Lys Lys Asp
        35                  40                  45

Thr Asp Asp Gln Ser Asp Leu Thr Asn Lys Glu Thr Ala Ala Ala Leu
    50                  55                  60

Glu Asp Ala Arg Glu Thr Arg Ala Ala Lys Glu Lys Arg Arg Lys Ala
65                  70                  75                  80

Ser Trp Leu Ser Pro Ile Leu Gly Gly Ile Ile Gly Gly Gly Leu Val
                85                  90                  95

Leu Gly Ile Ser Pro Tyr Leu Pro Gly Asp His Pro Ser Glu Thr Ala
            100                 105                 110

Gly Thr Gly Gln Thr Glu Pro Lys Gln Ser Gln Asn Phe Thr Thr Lys
        115                 120                 125

Pro Val Thr Asn Ala Asp Asn Val Pro Asp Met Val Glu Asp Leu Glu
    130                 135                 140

Pro Ala Ile Val Gly Val Ser Asn Ile Gln Thr Ser Phe Gly Phe Ser
145                 150                 155                 160

Glu Asp Asp Val Glu Glu Ser Gly Thr Gly Ser Gly Val Ile Phe Lys
                165                 170                 175

Lys Asp Gly Gly Lys Ala Tyr Ile Ile Thr Asn Asn His Val Val Glu
            180                 185                 190

Gly Ala Ser Lys Val Thr Ile Ser Leu Tyr Asn Gly Lys Thr Ala Asp
        195                 200                 205

Ala Lys Ile Ile Gly Ser Asp Ala Leu Thr Asp Leu Ala Val Leu Glu
    210                 215                 220

Ile Ser Ser Lys Gly Val Asp Lys Val Ala Ser Phe Gly Asp Ser Ala
225                 230                 235                 240

Lys Leu Arg Ala Gly Glu Lys Val Ile Ala Ile Gly Asn Pro Leu Gly
                245                 250                 255

Leu Gln Phe Ser Arg Thr Val Thr Glu Gly Ile Ile Ser Gly Val Asn
            260                 265                 270

Arg Thr Ile Glu Val Ser Thr Ser Glu Gly Asn Trp Asp Met Asn Val
        275                 280                 285

Leu Gln Thr Asp Ala Ala Ile Asn Pro Gly Asn Ser Gly Gly Pro Leu
    290                 295                 300

Ile Asn Ser Ser Gly Gln Val Ile Gly Ile Asn Ser Leu Lys Ile Ser
305                 310                 315                 320

Gln Ser Gly Val Glu Ser Leu Gly Phe Ala Ile Pro Ser Asn Asp Val
                325                 330                 335

Gln Pro Ile Val Asp Glu Leu Leu Glu Lys Gly Lys Val Glu Arg Pro
            340                 345                 350

Phe Leu Gly Val Gln Met Ile Asp Met Gln Gln Val Pro Glu Gln Tyr
        355                 360                 365

Gln Gln Asn Thr Leu Gly Leu Phe Gly Asp Gln Leu Asn Lys Gly Val
    370                 375                 380

Tyr Ile Asp Lys Val Ser Pro Lys Ser Pro Ala Ala Asp Ala Gly Met
385                 390                 395                 400

Lys Ala Gly Asp Val Ile Thr Lys Met Asn Gly Lys Asn Val Glu Thr
                405                 410                 415

Thr Ser Asp Leu Arg Lys Ile Leu Tyr Thr Glu Ala Lys Ala Gly Asp
            420                 425                 430

Thr Val Thr Phe Glu Val Leu Arg Asn Gly Lys Gln Thr Thr Met Lys
        435                 440                 445

Ala Lys Leu Ala Lys Ser Lys Ser
    450                 455

<210> 29
<211> 1647
<212> DNA
<213> Bacillus licheniformis

<220>
<221> CDS
<222> (501)..(1559)

<400> 29
   cactgtaagt ggacattttt ttgcgtgatg agaaggggga ggcagagttg ccgcatattt 60
   taattttgaa cggaccgaat ttaaaccggc ttggaaaacg cgaacccgat gtatacggga 120
   cagacacatt aaccgatttg gaacaaagac tttttcaatt tgccgaaggc attcagacag 180
   aattgacttt ttttcagtca aaccacgaag gggacctgat tgatgctctt catgaggcag 240
   aggagcaata cgatggaatc gtcttgaatc cgggggcttt ctcgcattac agctatgccc 300
   tccgcgatgc ggtggctgca atcagcatac cggtgattga ggttcatctt tcaaatcctc 360
   atgcaaggga agagttccgg caccgctcgg tcatcgctcc ggttgcaaga ggccagatca 420
   ccggactcgg ctttgaaggt tataagctgg ccatctctta ttttatgaac acaaataaca 480
   aataggggga ctagtggaca atg aaa cta aac aaa ttg aga gag ctt ttc ggc     533
                         Met Lys Leu Asn Lys Leu Arg Glu Leu Phe Gly
                         1               5                   10
   ggt tta ggc att gac ggt ata ttg gtg acg agc ggc gtt aat ttg cgg 581
   Gly Leu Gly Ile Asp Gly Ile Leu Val Thr Ser Gly Val Asn Leu Arg
   15 20 25
   tac atc acc gga ttt acc gga tct tca gga ctg gcg gtc atc tca gac 629
   Tyr Ile Thr Gly Phe Thr Gly Ser Ser Gly Leu Ala Val Ile Ser Asp
   30 35 40
   gat caa gca gtc ttt att acg gat ttc cgc tat aca gaa cag gcg aag 677
   Asp Gln Ala Val Phe Ile Thr Asp Phe Arg Tyr Thr Glu Gln Ala Lys
   45 50 55
   gat caa atc aaa agc ttt gac atc gtt cag cat gcc ggc gga atc gtt 725
   Asp Gln Ile Lys Ser Phe Asp Ile Val Gln His Ala Gly Gly Ile Val
   60 65 70 75
   caa aag acc gcc gaa gtg atc aaa gag atg ggg ata aac aaa atc ggt 773
   Gln Lys Thr Ala Glu Val Ile Lys Glu Met Gly Ile Asn Lys Ile Gly
   80 85 90
   ttt gaa cag gat aaa atg aca tac gga acg tat gcc gcc tac aaa gag 821
   Phe Glu Gln Asp Lys Met Thr Tyr Gly Thr Tyr Ala Ala Tyr Lys Glu
   95 100 105
   cag ctt ggc gca gct gaa ctc gtt ccc gtt tct caa tct gtg gaa aag 869
   Gln Leu Gly Ala Ala Glu Leu Val Pro Val Ser Gln Ser Val Glu Lys
   110 115 120
   ttg cgc ttg att aag tca agt gaa gag att aag ata tta aag gaa gct 917
   Leu Arg Leu Ile Lys Ser Ser Glu Glu Ile Lys Ile Leu Lys Glu Ala
   125 130 135
   gca aag att gca gat gat gca ttc agc cat att ctg acg gtc atc aag 965
   Ala Lys Ile Ala Asp Asp Ala Phe Ser His Ile Leu Thr Val Ile Lys
   140 145 150 155
   ccc ggc atc acc gaa att gaa gtc gcc aat gag ctc gag ttt tac atg 1013
   Pro Gly Ile Thr Glu Ile Glu Val Ala Asn Glu Leu Glu Phe Tyr Met
   160 165 170
   cgg agc cag gga gcc gat cat tcg tcc ttc gat atg atc gtg gcg tca 1061
   Arg Ser Gln Gly Ala Asp His Ser Ser Phe Asp Met Ile Val Ala Ser
   175 180 185
   ggc gta cgc tca agt ctg ccg cac gga gtg gca agc ggc aaa gcg atc 1109
   Gly Val Arg Ser Ser Leu Pro His Gly Val Ala Ser Gly Lys Ala Ile
   190 195 200
   gag aaa ggc gac ctt gtg acg ctg gac ttt ggg gct tac tat aaa ggt 1157
   Glu Lys Gly Asp Leu Val Thr Leu Asp Phe Gly Ala Tyr Tyr Lys Gly
   205 210 215
   tac tgc tcg gac att acg cgt aca gtc gcg gta gga gag ccg gat gat 1205
   Tyr Cys Ser Asp Ile Thr Arg Thr Val Ala Val Gly Glu Pro Asp Asp
   220 225 230 235
   gag ctg aaa cgc att tat caa acc gtg ttt gag gcg cag gct atc ggc 1253
   Glu Leu Lys Arg Ile Tyr Gln Thr Val Phe Glu Ala Gln Ala Ile Gly
   240 245 250
   atg cgg agc atc aag ccg gga atc acc ggg aaa cag gct gac gca tac 1301
   Met Arg Ser Ile Lys Pro Gly Ile Thr Gly Lys Gln Ala Asp Ala Tyr
   255 260 265
   aca agg gac tac ata tct tca caa ggc tac ggc gat tat ttc ggc cat 1349
   Thr Arg Asp Tyr Ile Ser Ser Gln Gly Tyr Gly Asp Tyr Phe Gly His
   270 275 280
   tca aca ggc cat ggc ctc ggc atg gaa gtt cat gaa agt ccg gcg ctt 1397
   Ser Thr Gly His Gly Leu Gly Met Glu Val His Glu Ser Pro Ala Leu
   285 290 295
   tca gca cgt tct gac cag atg ctt gaa aaa ggc atg gtt gta acg gtt 1445
   Ser Ala Arg Ser Asp Gln Met Leu Glu Lys Gly Met Val Val Thr Val
   300 305 310 315
   gaa ccg gga att tac ata ccg ggc aaa ggc ggg gtc agg ata gaa gat 1493
   Glu Pro Gly Ile Tyr Ile Pro Gly Lys Gly Gly Val Arg Ile Glu Asp
   320 325 330
   gat att gtc ctg aca gag gaa gga aac gaa tcc ctt acc cat tcc gcg 1541
   Asp Ile Val Leu Thr Glu Glu Gly Asn Glu Ser Leu Thr His Ser Ala
   335 340 345
   aaa gat tta att att tta taatccgttt gattggagca ataggaggat 1589
   Lys Asp Leu Ile Ile Leu
   350
   aatcatgatt tcagtaaacg atttcgcaca gtttaagtat caataaggga gtttctaa 1647

<210> 30
<211> 353
<212> PRT
<213> Bacillus licheniformis

<400> 30

Met Lys Leu Asn Lys Leu Arg Glu Leu Phe Gly Gly Leu Gly Ile Asp
1               5                   10                  15

Gly Ile Leu Val Thr Ser Gly Val Asn Leu Arg Tyr Ile Thr Gly Phe
            20                  25                  30

Thr Gly Ser Ser Gly Leu Ala Val Ile Ser Asp Asp Gln Ala Val Phe
        35                  40                  45

Ile Thr Asp Phe Arg Tyr Thr Glu Gln Ala Lys Asp Gln Ile Lys Ser
    50                  55                  60

Phe Asp Ile Val Gln His Ala Gly Gly Ile Val Gln Lys Thr Ala Glu
65                  70                  75                  80

Val Ile Lys Glu Met Gly Ile Asn Lys Ile Gly Phe Glu Gln Asp Lys
                85                  90                  95

Met Thr Tyr Gly Thr Tyr Ala Ala Tyr Lys Glu Gln Leu Gly Ala Ala
            100                 105                 110

Glu Leu Val Pro Val Ser Gln Ser Val Glu Lys Leu Arg Leu Ile Lys
        115                 120                 125

Ser Ser Glu Glu Ile Lys Ile Leu Lys Glu Ala Ala Lys Ile Ala Asp
    130                 135                 140

Asp Ala Phe Ser His Ile Leu Thr Val Ile Lys Pro Gly Ile Thr Glu
145                 150                 155                 160

Ile Glu Val Ala Asn Glu Leu Glu Phe Tyr Met Arg Ser Gln Gly Ala
                165                 170                 175

Asp His Ser Ser Phe Asp Met Ile Val Ala Ser Gly Val Arg Ser Ser
            180                 185                 190

Leu Pro His Gly Val Ala Ser Gly Lys Ala Ile Glu Lys Gly Asp Leu
        195                 200                 205

Val Thr Leu Asp Phe Gly Ala Tyr Tyr Lys Gly Tyr Cys Ser Asp Ile
    210                 215                 220

Thr Arg Thr Val Ala Val Gly Glu Pro Asp Asp Glu Leu Lys Arg Ile

225                 230                 235                 240

Tyr Gln Thr Val Phe Glu Ala Gln Ala Ile Gly Met Arg Ser Ile Lys
                245                 250                 255

Pro Gly Ile Thr Gly Lys Gln Ala Asp Ala Tyr Thr Arg Asp Tyr Ile
            260                 265                 270

Ser Ser Gln Gly Tyr Gly Asp Tyr Phe Gly His Ser Thr Gly His Gly
        275                 280                 285

Leu Gly Met Glu Val His Glu Ser Pro Ala Leu Ser Ala Arg Ser Asp
    290                 295                 300

Gln Met Leu Glu Lys Gly Met Val Val Thr Val Glu Pro Gly Ile Tyr
305                 310                 315                 320

Ile Pro Gly Lys Gly Gly Val Arg Ile Glu Asp Asp Ile Val Leu Thr
                325                 330                 335

Glu Glu Gly Asn Glu Ser Leu Thr His Ser Ala Lys Asp Leu Ile Ile
            340                 345                 350

Leu

<210>  31
<211>  1941
<212>  DNA
<213>  Bacillus licheniformis

<220>
<221>  CDS
<222>  (501)..(1451)

<400>  31
gcaaaaaaat cgaaaagccg gaaattacaa agctcacaga catcatcgat acagatttga      60

tcgaacctca tttgtcagga gatacgagag acgacattat tgatgaaatg atccaaaaat     120

tgtcccggaa aggcgtactg cattcagaaa gcgaatttaa acaagctatc atcaatcgcg     180

aacttgaggg tacaacggcg atcggtatga atatcgcgat cccgcacggg aagtctgatg     240

ccgtcaagaa gccgagtgta gcattcggca ttaaacgttc gggcgtagac tggaggagtc     300

ttgacggaac tgacgccaaa ctgattttta tgatcgccgt cccgaaagaa agcggaggaa     360

acgagcatct gaagcttctg caaatgctct ccagaaaact gatggatgac acctaccggg     420

acaagcttct ctctgcccaa acaaaagaag aagcatacaa acttctggat gaaatcatct     480

aaaaaaggag gacgcccgtc atg act gaa ccg tta ttt ttc gag cct gtt ttt     533
                      Met Thr Glu Pro Leu Phe Phe Glu Pro Val Phe
                      1               5                   10

aaa gaa aga ata tgg ggc gga acg gct tta gcc tct ttc ggc tat gac     581
Lys Glu Arg Ile Trp Gly Gly Thr Ala Leu Ala Ser Phe Gly Tyr Asp

                15                  20                  25

att cca tcc gaa caa aca ggg gag tgc tgg gct ttc gcc gca cat caa      629
Ile Pro Ser Glu Gln Thr Gly Glu Cys Trp Ala Phe Ala Ala His Gln
        30                  35                  40

aat gga caa agc gtc gtg aaa aac ggc ttg tat aaa ggg ctc aca tta      677
Asn Gly Gln Ser Val Val Lys Asn Gly Leu Tyr Lys Gly Leu Thr Leu
    45                  50                  55

agc gaa tta tgg gat gaa cac cgt cat tta ttc gga cat ata aag gga      725
Ser Glu Leu Trp Asp Glu His Arg His Leu Phe Gly His Ile Lys Gly
60                  65                  70                  75

gac cgc ttt cct ctt ttg aca aaa att ttg gac gcc gac caa gac cta      773
Asp Arg Phe Pro Leu Leu Thr Lys Ile Leu Asp Ala Asp Gln Asp Leu
                80                  85                  90

tcc gta caa gtg cat cca aat gat gaa tat gca aag ctg cac gaa aac      821
Ser Val Gln Val His Pro Asn Asp Glu Tyr Ala Lys Leu His Glu Asn
            95                  100                 105

ggg gag ctt gga aag acg gaa tgc tgg tat atc att gat tgc aag gaa      869
Gly Glu Leu Gly Lys Thr Glu Cys Trp Tyr Ile Ile Asp Cys Lys Glu
        110                 115                 120

gat gcg gag atc att tac ggc cac cat gcc aag acg cgg gaa gaa ctg      917
Asp Ala Glu Ile Ile Tyr Gly His His Ala Lys Thr Arg Glu Glu Leu
    125                 130                 135

gtc tca atg atc gaa aac gga aaa tgg aat gag ctt ttg cgc cgc gtc      965
Val Ser Met Ile Glu Asn Gly Lys Trp Asn Glu Leu Leu Arg Arg Val
140                 145                 150                 155

aaa gtg aag ccg gga gat ttc ttc tat gtg ccg agc gga acc gtc cat     1013
Lys Val Lys Pro Gly Asp Phe Phe Tyr Val Pro Ser Gly Thr Val His
                160                 165                 170

gcg atc gga aaa ggc att ttg att ttg gag acg cag cag aat tct gac     1061
Ala Ile Gly Lys Gly Ile Leu Ile Leu Glu Thr Gln Gln Asn Ser Asp
            175                 180                 185

aca acc tac aga cta tat gat tat gac aga aaa gac gct gaa gga aac     1109
Thr Thr Tyr Arg Leu Tyr Asp Tyr Asp Arg Lys Asp Ala Glu Gly Asn
        190                 195                 200

ctg cgc gag ctc cat ttg caa aaa agc ata gaa gtg atc gac gtt ccg     1157
Leu Arg Glu Leu His Leu Gln Lys Ser Ile Glu Val Ile Asp Val Pro
    205                 210                 215

tct tcc cct gat cgc gaa acc gtt caa tac gaa aca tcg ggc gac gtc     1205
Ser Ser Pro Asp Arg Glu Thr Val Gln Tyr Glu Thr Ser Gly Asp Val
220                 225                 230                 235

aag act gcc gca ctg att gat tgt cct tat ttc tca gta gaa aaa tgg     1253
Lys Thr Ala Ala Leu Ile Asp Cys Pro Tyr Phe Ser Val Glu Lys Trp
                240                 245                 250

gat gta aaa gga tcg gcc tgt ttc aaa cag gat aaa ccg ttt ctt ctt     1301
Asp Val Lys Gly Ser Ala Cys Phe Lys Gln Asp Lys Pro Phe Leu Leu
            255                 260                 265

gcc agc gtc ata gaa ggg gaa ggg cgc atc gca tct ggc ggc cat aac     1349
Ala Ser Val Ile Glu Gly Glu Gly Arg Ile Ala Ser Gly Gly His Asn
        270                 275                 280

ttt tca ttt aaa aaa gga gat cac atg ctg ctg ccc tgg ggc ttt gga     1397
Phe Ser Phe Lys Lys Gly Asp His Met Leu Leu Pro Trp Gly Phe Gly

285                 290                 295

gaa ttt acg ctc gcc ggc cat gcg gaa tgt atc gtc tca agc tta tca      1445
Glu Phe Thr Leu Ala Gly His Ala Glu Cys Ile Val Ser Ser Leu Ser
300                 305                 310                 315

att gaa taaacgatca gcggcgccct ttctgaacag accataaaac tatggtctgt      1501
Ile Glu

tttattttg caaaaaaaat aatctcgact tgccaagacg tgttcacaga gggctgacaa      1561

tgcatattcc caagctttca caagtaaaat agagtaaacc aaaaaaacat ttggaaatct      1621

tccaattatt tgataagctg atattaaaag cttcatatct ttcatcagct gcactgcccg      1681

ggacttttcg cggaacagct gcttcatgaa acaaaaaggc ggtcctatgt tccggcgtct      1741

ggcaagaaaa gcctgctaaa tgaccgaaac aaccgtgccc ttcataattg gttggtcctt      1801

atctggatga gaaaaggaga cgatgattcg tatcagcaaa aatgtaagcg gattcatcgt      1861

ctttttatct cggtttatca ctctttttag cggaagcgag gaggtaagca atggggaaag      1921

caaaacttcg atggaatgaa                                                1941

<210>  32
<211>  317
<212>  PRT
<213>  Bacillus licheniformis

<400>  32

Met Thr Glu Pro Leu Phe Phe Glu Pro Val Phe Lys Glu Arg Ile Trp
1               5                   10                  15

Gly Gly Thr Ala Leu Ala Ser Phe Gly Tyr Asp Ile Pro Ser Glu Gln
            20                  25                  30

Thr Gly Glu Cys Trp Ala Phe Ala Ala His Gln Asn Gly Gln Ser Val
        35                  40                  45

Val Lys Asn Gly Leu Tyr Lys Gly Leu Thr Leu Ser Glu Leu Trp Asp
    50                  55                  60

Glu His Arg His Leu Phe Gly His Ile Lys Gly Asp Arg Phe Pro Leu
65                  70                  75                  80

Leu Thr Lys Ile Leu Asp Ala Asp Gln Asp Leu Ser Val Gln Val His
                85                  90                  95

Pro Asn Asp Glu Tyr Ala Lys Leu His Glu Asn Gly Glu Leu Gly Lys
            100                 105                 110

Thr Glu Cys Trp Tyr Ile Ile Asp Cys Lys Glu Asp Ala Glu Ile Ile
        115                 120                 125

Tyr Gly His His Ala Lys Thr Arg Glu Glu Leu Val Ser Met Ile Glu
    130                 135                 140

Asn Gly Lys Trp Asn Glu Leu Leu Arg Arg Val Lys Val Lys Pro Gly
145                 150                 155                 160

Asp Phe Phe Tyr Val Pro Ser Gly Thr Val His Ala Ile Gly Lys Gly
                165                 170                 175

Ile Leu Ile Leu Glu Thr Gln Gln Asn Ser Asp Thr Thr Tyr Arg Leu
            180                 185                 190

Tyr Asp Tyr Asp Arg Lys Asp Ala Glu Gly Asn Leu Arg Glu Leu His
        195                 200                 205

Leu Gln Lys Ser Ile Glu Val Ile Asp Val Pro Ser Ser Pro Asp Arg
    210                 215                 220

Glu Thr Val Gln Tyr Glu Thr Ser Gly Asp Val Lys Thr Ala Ala Leu
225                 230                 235                 240

Ile Asp Cys Pro Tyr Phe Ser Val Glu Lys Trp Asp Val Lys Gly Ser
                245                 250                 255

Ala Cys Phe Lys Gln Asp Lys Pro Phe Leu Leu Ala Ser Val Ile Glu
            260                 265                 270

Gly Glu Gly Arg Ile Ala Ser Gly Gly His Asn Phe Ser Phe Lys Lys
        275                 280                 285

Gly Asp His Met Leu Leu Pro Trp Gly Phe Gly Glu Phe Thr Leu Ala
    290                 295                 300

Gly His Ala Glu Cys Ile Val Ser Ser Leu Ser Ile Glu
305                 310                 315

<210>  33
<211>  1747
<212>  DNA
<213>  Bacillus licheniformis

<220>
<221>  CDS
<222>  (501)..(1244)

<400>  33
tcatacattg ataaaggtga gcttgttccg gatgaagtga caattggtat tgtcagggag      60

agacttggca agaatgattg tgacggaggt tttcttctgg acggatttcc gagaacagtc     120

gctcaagctg aagcacttga ggaaattctt aaaggacttg gcaagtcgat tgaccatgtc     180

atcaacattc aagtcgataa agatgcattg atggaacgtc tgacaggacg cagaatctgc     240

aaaaactgcg gtgcaactta tcatttagtc tttaatccgc ctgcaaaaga aaatgtatgc     300

gacaagtgtg gaggagagct ttatcagcgt gaagacgata atgaagctac cgtatccacg     360

cggttagagg tcaacatgaa gcaaacccag cctttactcg atttctatga agataaagga 420

tatctcgtaa acattgacgg gcagaagcac atcaatgaag tttacgctga tataaaggag 480

cttctcggag gccgggacaa atg att att tgt aaa act ccc cga gaa atc gaa 533
Met Ile Ile Cys Lys Thr Pro Arg Glu Ile Glu
1               5                   10

atc atg cgg gag gcc gga cga att gtc gct tta act cat cag gag cta 581
Ile Met Arg Glu Ala Gly Arg Ile Val Ala Leu Thr His Gln Glu Leu
            15                  20                  25

aaa aag cac att aaa ccc ggc atc tcg aca aaa gaa ttg gat caa att 629
Lys Lys His Ile Lys Pro Gly Ile Ser Thr Lys Glu Leu Asp Gln Ile
        30                  35                  40

gcc gaa cgt ttt att aca aag caa ggt gca atc cca tct ttt aaa ggg 677
Ala Glu Arg Phe Ile Thr Lys Gln Gly Ala Ile Pro Ser Phe Lys Gly
    45                  50                  55

tat aat ggt ttt cgc ggg agc att tgc gtt tcg gtc aac gaa gaa ctc 725
Tyr Asn Gly Phe Arg Gly Ser Ile Cys Val Ser Val Asn Glu Glu Leu
60                  65                  70                  75

gtg cac ggt att ccg gga aaa cga gtc ctt cgc gac ggg gac atc atc 773
Val His Gly Ile Pro Gly Lys Arg Val Leu Arg Asp Gly Asp Ile Ile
                80                  85                  90

agc atc gat atc ggt gcg aag cta aac ggt tat cat ggt gac tcc gct 821
Ser Ile Asp Ile Gly Ala Lys Leu Asn Gly Tyr His Gly Asp Ser Ala
            95                  100                 105

tgg act tat cca gtg ggt acg atc agc gat gat gat cag aag ctg ctg 869
Trp Thr Tyr Pro Val Gly Thr Ile Ser Asp Asp Asp Gln Lys Leu Leu
        110                 115                 120

gat gtg aca gag gag tct tta tat aga ggc ttg aag gaa gca aaa ccg 917
Asp Val Thr Glu Glu Ser Leu Tyr Arg Gly Leu Lys Glu Ala Lys Pro
    125                 130                 135

gga gaa cgt ttg tcg aat att tcc cac gca ata caa acg tat gtc gaa 965
Gly Glu Arg Leu Ser Asn Ile Ser His Ala Ile Gln Thr Tyr Val Glu
140                 145                 150                 155

agc gaa caa ttt tca gtc gtc agg gaa tat gta gga cac ggg gta ggg 1013
Ser Glu Gln Phe Ser Val Val Arg Glu Tyr Val Gly His Gly Val Gly
                160                 165                 170

cag gat ctg cat gag gat ccg caa att cct cac tac ggt ccg ccg aac 1061
Gln Asp Leu His Glu Asp Pro Gln Ile Pro His Tyr Gly Pro Pro Asn
            175                 180                 185

aaa ggc ccg cgt tta aag cct ggc atg gtc ttg gcc att gag ccg atg 1109
Lys Gly Pro Arg Leu Lys Pro Gly Met Val Leu Ala Ile Glu Pro Met
        190                 195                 200

gtc aat gca ggt aca cgc tat gtc aaa aca ttg gct gac aac tgg acg 1157
Val Asn Ala Gly Thr Arg Tyr Val Lys Thr Leu Ala Asp Asn Trp Thr
    205                 210                 215

gtt gta act gtg gac ggg aag aag tgt gct cac ttt gaa cac acg att 1205
Val Val Thr Val Asp Gly Lys Lys Cys Ala His Phe Glu His Thr Ile
220                 225                 230                 235

gcc att acg gat gcc ggt ttt gag ata ctg aca aaa gcc taggtgaaat 1254
Ala Ile Thr Asp Ala Gly Phe Glu Ile Leu Thr Lys Ala
                240                 245

gagatgtatc ggaaaacacc tgaaattgga cagttcgtac gcatgacaag aggccgcgaa 1314

atgaaccaat acgcagttgt tattgatata gtcgatcatc atcatgtgct ggtagcagac 1374

ggagaaaagc gtaaatttca ttctcctaag aaaaagaata tcaatcattt aacctttat 1434

gattgcgtat ctccggaagt tcagaacagt ataaacgaaa cagggcgtgt gacaaacgga 1494

aaattgagat tttctctttt gaaatttgtc agagagcaag ttactgattt gaagaaggga 1554

gaacacttga atggcgaaag acgatgtaat tgaagtggaa ggtactgtag tcgaaacgct 1614

gccaaacgca atgttcaaag ttgaacttga gaatggccac acggttttgg ctcacgtatc 1674

tggtaaaatc cgcatgcact tcattcgcat tttacctgga gacaaagtta cggtagaatt 1734

atctccatat gac 1747

<210> 34
<211> 248
<212> PRT
<213> Bacillus licheniformis

<400> 34

Met Ile Ile Cys Lys Thr Pro Arg Glu Ile Glu Ile Met Arg Glu Ala
1               5                   10                  15

Gly Arg Ile Val Ala Leu Thr His Gln Glu Leu Lys Lys His Ile Lys
            20                  25                  30

Pro Gly Ile Ser Thr Lys Glu Leu Asp Gln Ile Ala Glu Arg Phe Ile
        35                  40                  45

Thr Lys Gln Gly Ala Ile Pro Ser Phe Lys Gly Tyr Asn Gly Phe Arg
    50                  55                  60

Gly Ser Ile Cys Val Ser Val Asn Glu Glu Leu Val His Gly Ile Pro
65                  70                  75                  80

Gly Lys Arg Val Leu Arg Asp Gly Asp Ile Ile Ser Ile Asp Ile Gly
                85                  90                  95

Ala Lys Leu Asn Gly Tyr His Gly Asp Ser Ala Trp Thr Tyr Pro Val
            100                 105                 110

Gly Thr Ile Ser Asp Asp Asp Gln Lys Leu Leu Asp Val Thr Glu Glu
        115                 120                 125

Ser Leu Tyr Arg Gly Leu Lys Glu Ala Lys Pro Gly Glu Arg Leu Ser
    130                 135                 140

Asn Ile Ser His Ala Ile Gln Thr Tyr Val Glu Ser Glu Gln Phe Ser
145                 150                 155                 160

Val Val Arg Glu Tyr Val Gly His Gly Val Gly Gln Asp Leu His Glu

165                 170                 175

Asp Pro Gln Ile Pro His Tyr Gly Pro Pro Asn Lys Gly Pro Arg Leu
            180                 185                 190

Lys Pro Gly Met Val Leu Ala Ile Glu Pro Met Val Asn Ala Gly Thr
        195                 200                 205

Arg Tyr Val Lys Thr Leu Ala Asp Asn Trp Thr Val Val Thr Val Asp
    210                 215                 220

Gly Lys Lys Cys Ala His Phe Glu His Thr Ile Ala Ile Thr Asp Ala
225                 230                 235                 240

Gly Phe Glu Ile Leu Thr Lys Ala
                245

<210>  35
<211>  1928
<212>  DNA
<213>  Bacillus licheniformis

<220>
<221>  CDS
<222>  (501)..(1427)

<400>  35
   atcaccggct tgaaaccggc cgaagatctc gagctcaaaa cgggagaaac cgtgaaaatc      60
   gagtttgaaa gcgccgctga tttggatgct gtctttgtga tcagaatgcc gctgaccaat     120
   ttcaaaaccg ctgcccaaaa cgtaacggag ctgccgatca gagaagtctc aaaaggaaaa     180
   tatgaaggat attggaccgc tacttcaact gcaaaagcaa aaggagcgga aatcgaggtc     240
   atcgtcagag atgattacgg caatgaaacg agacaaacgg caaaaggcaa gctgtatatc     300
   aatgaaaagc tgaaataaag gtgaaaagac gctgtcttta atggcagcgt ttttttcgtt     360
   ttacgatcga caaattcagt acgaaaactt caaaaaatgt acgatttacg caacattaat     420
   tgacagactt tacctttggg cttgatttat acttaggaaa acaaacacta aggtcaccga     480
   gccgcagaaa ggggaaggat gtg gaa atc tat tta gat gcg ata tgg ctg tta     533
                         Val Glu Ile Tyr Leu Asp Ala Ile Trp Leu Leu
                         1               5                   10
   aac ttt tgt ttt gac ttg ctg ctt tta atg atg acc gca ttt att tta     581
   Asn Phe Cys Phe Asp Leu Leu Leu Leu Met Met Thr Ala Phe Ile Leu
               15                  20                  25
   aag cga agg gtt aaa aag cgg agg ctg atc cta ggg gca ttt gtc gcg     629
   Lys Arg Arg Val Lys Lys Arg Arg Leu Ile Leu Gly Ala Phe Val Ala
           30                  35                  40
   tca agc atc gtt ctg ttt atg ttt aca cct ttt tca ccg tac gtc ctt     677
   Ser Ser Ile Val Leu Phe Met Phe Thr Pro Phe Ser Pro Tyr Val Leu
       45                  50                  55
   cat cct gcc ggc aaa ctg tcg ttt tcg gtt gtg atc gtt ctt gtg gca     725
   His Pro Ala Gly Lys Leu Ser Phe Ser Val Val Ile Val Leu Val Ala
   60                  65                  70                  75
   ttt ggt ttt aag cgg ttc cgg ttt ttt ttg cag aat ttg ttt tct ttt      773
   Phe Gly Phe Lys Arg Phe Arg Phe Phe Leu Gln Asn Leu Phe Ser Phe
                   80                  85                  90
   tat ttt gcc act ttt tta atg gga gga ggg att atc gga gcg cat tct      821
   Tyr Phe Ala Thr Phe Leu Met Gly Gly Gly Ile Ile Gly Ala His Ser
               95                  100                 105
   ttg ctt gaa acg gat tcg atc atg gaa aac ggc gtc ttt atg acg aat      869
   Leu Leu Glu Thr Asp Ser Ile Met Glu Asn Gly Val Phe Met Thr Asn
           110                 115                 120
   tgg tcc ggt ttt gga gac ccc gtc agc tgg ctg ttt gtc tgt gtg ggt      917
   Trp Ser Gly Phe Gly Asp Pro Val Ser Trp Leu Phe Val Cys Val Gly
       125                 130                 135
   ttt gcg gct gta tgg ctg ttt tca aaa aag cgt ttt gaa gat gct gaa      965
   Phe Ala Ala Val Trp Leu Phe Ser Lys Lys Arg Phe Glu Asp Ala Glu
   140                 145                 150                 155
   gcg aag aaa att caa tac gaa gaa cgc gtc cgc cta gag gcc tgc att     1013
   Ala Lys Lys Ile Gln Tyr Glu Glu Arg Val Arg Leu Glu Ala Cys Ile
                   160                 165                 170
   ggt gaa cat acg ctt cat ttc acc gga ttg att gac tcc gga aac cag     1061
   Gly Glu His Thr Leu His Phe Thr Gly Leu Ile Asp Ser Gly Asn Gln
               175                 180                 185
   ctc tac gat cca atc aca aaa acg ccc gtc atg atc gtc aat att gaa     1109
   Leu Tyr Asp Pro Ile Thr Lys Thr Pro Val Met Ile Val Asn Ile Glu
           190                 195                 200
   aaa ttg aaa gtt gta ttg gga gaa gag gca agt gtg acc atc aag gaa     1157
   Lys Leu Lys Val Val Leu Gly Glu Glu Ala Ser Val Thr Ile Lys Glu
       205                 210                 215
   atg agc ccg ctt gat gcc gtc ggg aaa ctg gat gaa gca ctg ccg tat     1205
   Met Ser Pro Leu Asp Ala Val Gly Lys Leu Asp Glu Ala Leu Pro Tyr
   220                 225                 230                 235
   atc ggg cgg atc cgc ctg att ccg tac cgc ggg gtc ggc cat cag cat     1253
   Ile Gly Arg Ile Arg Leu Ile Pro Tyr Arg Gly Val Gly His Gln His
                   240                 245                 250
   cag ttt ctg ctc tgc tta aag ccg gat cat gtg ctc gtt tgt acg gaa     1301
   Gln Phe Leu Leu Cys Leu Lys Pro Asp His Val Leu Val Cys Thr Glu
               255                 260                 265
   aga gaa gtg att gaa gcg ccg aaa tgc ctg att ggc atc agc aca tca     1349
   Arg Glu Val Ile Glu Ala Pro Lys Cys Leu Ile Gly Ile Ser Thr Ser
           270                 275                 280
   ccg ctt tcc gct gac ggc gaa ttt gac gcc atc gtc cat ccg aaa atg     1397
   Pro Leu Ser Ala Asp Gly Glu Phe Asp Ala Ile Val His Pro Lys Met
       285                 290                 295
   ctg gcc gga aac ccg gtc aaa cac gtt tct taaacttgaa gtctgttaca       1447
   Leu Ala Gly Asn Pro Val Lys His Val Ser
   300                 305
   ttatcatact cctgaagacg tttatttaga agggggagga agatgaaaaa actaaaatta    1507
   aggttaacct atctatggta caaactttta atgaaactgg ggctgaaaag cgacgaaatt   1567
   tattatatcg gcggaagcga ggcgcttccc ccgccattgt caaaagatga agagcaggtg   1627
   cttctccata agctgcctga cggtgatcag gcggcacgag cgattttgat tgaacgaaat 1687
   ctcagactgg tcgtgtacat cgcgagaaaa tttgaaaata caggaatcaa tatcgaggat 1747
   ttaatctcca tcggcacgat cgggctcatc aaagcggtga atacgtttaa tcccgagaaa 1807
   aaaatcaaac tggctacata tgcttccaga tgcattgaaa atgaaatttt gatgtattta 1867
   agaagaaaca ataaaatccg ttcagaggta tcattcgacg aaccgctgaa catcgattgg 1927
   g 1928

<210> 36
<211> 309
<212> PRT
<213> Bacillus licheniformis

<400> 36

Val Glu Ile Tyr Leu Asp Ala Ile Trp Leu Leu Asn Phe Cys Phe Asp
1               5                   10                  15

Leu Leu Leu Leu Met Met Thr Ala Phe Ile Leu Lys Arg Arg Val Lys
            20                  25                  30

Lys Arg Arg Leu Ile Leu Gly Ala Phe Val Ala Ser Ser Ile Val Leu
        35                  40                  45

Phe Met Phe Thr Pro Phe Ser Pro Tyr Val Leu His Pro Ala Gly Lys
    50                  55                  60

Leu Ser Phe Ser Val Val Ile Val Leu Val Ala Phe Gly Phe Lys Arg
65                  70                  75                  80

Phe Arg Phe Phe Leu Gln Asn Leu Phe Ser Phe Tyr Phe Ala Thr Phe
                85                  90                  95

Leu Met Gly Gly Gly Ile Ile Gly Ala His Ser Leu Leu Glu Thr Asp
            100                 105                 110

Ser Ile Met Glu Asn Gly Val Phe Met Thr Asn Trp Ser Gly Phe Gly
        115                 120                 125

Asp Pro Val Ser Trp Leu Phe Val Cys Val Gly Phe Ala Ala Val Trp
    130                 135                 140

Leu Phe Ser Lys Lys Arg Phe Glu Asp Ala Glu Ala Lys Lys Ile Gln
145                 150                 155                 160

Tyr Glu Glu Arg Val Arg Leu Glu Ala Cys Ile Gly Glu His Thr Leu
                165                 170                 175

His Phe Thr Gly Leu Ile Asp Ser Gly Asn Gln Leu Tyr Asp Pro Ile
            180                 185                 190

Thr Lys Thr Pro Val Met Ile Val Asn Ile Glu Lys Leu Lys Val Val
        195                 200                 205

Leu Gly Glu Glu Ala Ser Val Thr Ile Lys Glu Met Ser Pro Leu Asp
    210                 215                 220

Ala Val Gly Lys Leu Asp Glu Ala Leu Pro Tyr Ile Gly Arg Ile Arg
225                 230                 235                 240

Leu Ile Pro Tyr Arg Gly Val Gly His Gln His Gln Phe Leu Leu Cys
                245                 250                 255

Leu Lys Pro Asp His Val Leu Val Cys Thr Glu Arg Glu Val Ile Glu
            260                 265                 270

Ala Pro Lys Cys Leu Ile Gly Ile Ser Thr Ser Pro Leu Ser Ala Asp
        275                 280                 285

Gly Glu Phe Asp Ala Ile Val His Pro Lys Met Leu Ala Gly Asn Pro
    290                 295                 300

Val Lys His Val Ser
305

<210>  37
<211>  1390
<212>  DNA
<213>  Bacillus licheniformis

<220>
<221>  CDS
<222>  (501)..(1310)

<400>  37
gaagaatttg ctcaagccgc cgtcagaaac agcagcctca cgaaagaaga agcgtttcaa      60

tttctgattc attcattgct gggaacaggc aagctgttaa tggaaaaaga catgacgttt     120

gctcaaacgc ttgatcgggt ggcgacaaaa ggcggcataa ccgcagaagg tgcggaagtc     180

attcaagatg ccatgccgga agtctttgac gagctgtttg ccagaacgat gaagaaatat     240

gaagtcatca aagacgctgt ccgccggcag tgaaccgcgg cattcatcga catatccgga     300

tatccgcaag ctgcctgacc aggcagcttt tttatttgca ttcgacaaca ttcatcaaaa     360

ttccaatttt atattaaatt tctaaaatta ccaattgtta ttgcatttct attaaaattc     420

taatataatt tgttagaata ttcccaaaat aataaaaaat ttgagacgtt tttaaggggt     480

ggtttttgt gaagctgtat atg tct gtt gat atg gag ggg att tcc ggg ctt      533
                     Met Ser Val Asp Met Glu Gly Ile Ser Gly Leu
                     1               5                   10

ccg gat gat acg ttc gtc gat tca agc aag ccg aac tat gag aga ggc      581
Pro Asp Asp Thr Phe Val Asp Ser Ser Lys Pro Asn Tyr Glu Arg Gly
            15                  20                  25

cgg aag atc atg acg gac gag gcc aat cat gtg att gac gaa gcg ttc      629

Arg Lys Ile Met Thr Asp Glu Ala Asn His Val Ile Asp Glu Ala Phe
        30                  35                  40

aaa aat gga tgc aga gag gtg att gtt aat gac agc cac tct aaa atg 677
Lys Asn Gly Cys Arg Glu Val Ile Val Asn Asp Ser His Ser Lys Met
    45                  50                  55

aac aac ctg tta att gag agg ctc cat cct gaa gcg cgg ctt att tcg 725
Asn Asn Leu Leu Ile Glu Arg Leu His Pro Glu Ala Arg Leu Ile Ser
60                  65                  70                  75

gga gat gtc aag ccg ttt tcg atg gtt cag ggg ctt gat gat aca tac 773
Gly Asp Val Lys Pro Phe Ser Met Val Gln Gly Leu Asp Asp Thr Tyr
                80                  85                  90

gac gga gcc gtg ttc gtc ggc tac cat gcg aga gcg tcg atg aga ggc 821
Asp Gly Ala Val Phe Val Gly Tyr His Ala Arg Ala Ser Met Arg Gly
            95                  100                 105

gtg atg tcg cac agc atg att ttc ggc gtc aga cat ttt tac atc aac 869
Val Met Ser His Ser Met Ile Phe Gly Val Arg His Phe Tyr Ile Asn
        110                 115                 120

gat cag gcg gtc ggc gag ctg ggg ttc aac gcc tat gtt gcc ggt tat 917
Asp Gln Ala Val Gly Glu Leu Gly Phe Asn Ala Tyr Val Ala Gly Tyr
    125                 130                 135

tac ggc gtc ccg gtc atc atg gct gcc ggt gac agc gag gcg gca gct 965
Tyr Gly Val Pro Val Ile Met Ala Ala Gly Asp Ser Glu Ala Ala Ala
140                 145                 150                 155

gag gcg gag gag ctg att ccg aac gta acg aca gca gcc gtc aaa gaa 1013
Glu Ala Glu Glu Leu Ile Pro Asn Val Thr Thr Ala Ala Val Lys Glu
                160                 165                 170

acg gtc tcc cgt tcg gcg gtc aaa tgt ctg acg ccg gag aaa gcg ggg 1061
Thr Val Ser Arg Ser Ala Val Lys Cys Leu Thr Pro Glu Lys Ala Gly
            175                 180                 185

cgg cta tta aga gaa cgg atg gca tat gcg att caa aac cgc ggt caa 1109
Arg Leu Leu Arg Glu Arg Met Ala Tyr Ala Ile Gln Asn Arg Gly Gln
        190                 195                 200

gtc aag ccg ctc aca cct ccg gac agg ccg gtg ctg cgg ata gaa ttt 1157
Val Lys Pro Leu Thr Pro Pro Asp Arg Pro Val Leu Arg Ile Glu Phe
    205                 210                 215

gcc aat tac ggc caa gcc gag tgg gcc agt ctg atg ccg ggt gct gaa 1205
Ala Asn Tyr Gly Gln Ala Glu Trp Ala Ser Leu Met Pro Gly Ala Glu
220                 225                 230                 235

atc gaa ccg caa acg acg act gtc cgc tat caa gcg aaa gac att ctt 1253
Ile Glu Pro Gln Thr Thr Thr Val Arg Tyr Gln Ala Lys Asp Ile Leu
                240                 245                 250

gaa gcc tat caa gcg atg ctt gtc atg acc gag ctt gcg atg aga acg 1301
Glu Ala Tyr Gln Ala Met Leu Val Met Thr Glu Leu Ala Met Arg Thr
            255                 260                 265

aca ttc tcc taggaaggtg ggatgaaaat ggcgggttac atcatgaagc 1350
Thr Phe Ser
        270

ggctgctgtg gatgctggca accattatgg tactccgagg 1390

<210> 38
<211> 270

<212>   PRT
<213>   Bacillus licheniformis

<400>   38

Met Ser Val Asp Met Glu Gly Ile Ser Gly Leu Pro Asp Asp Thr Phe
1               5                   10                  15

Val Asp Ser Ser Lys Pro Asn Tyr Glu Arg Gly Arg Lys Ile Met Thr
            20                  25                  30

Asp Glu Ala Asn His Val Ile Asp Glu Ala Phe Lys Asn Gly Cys Arg
        35                  40                  45

Glu Val Ile Val Asn Asp Ser His Ser Lys Met Asn Asn Leu Leu Ile
    50                  55                  60

Glu Arg Leu His Pro Glu Ala Arg Leu Ile Ser Gly Asp Val Lys Pro
65                  70                  75                  80

Phe Ser Met Val Gln Gly Leu Asp Asp Thr Tyr Asp Gly Ala Val Phe
                85                  90                  95

Val Gly Tyr His Ala Arg Ala Ser Met Arg Gly Val Met Ser His Ser
            100                 105                 110

Met Ile Phe Gly Val Arg His Phe Tyr Ile Asn Asp Gln Ala Val Gly
        115                 120                 125

Glu Leu Gly Phe Asn Ala Tyr Val Ala Gly Tyr Tyr Gly Val Pro Val
    130                 135                 140

Ile Met Ala Ala Gly Asp Ser Glu Ala Ala Ala Glu Ala Glu Glu Leu
145                 150                 155                 160

Ile Pro Asn Val Thr Thr Ala Ala Val Lys Glu Thr Val Ser Arg Ser
                165                 170                 175

Ala Val Lys Cys Leu Thr Pro Glu Lys Ala Gly Arg Leu Leu Arg Glu
            180                 185                 190

Arg Met Ala Tyr Ala Ile Gln Asn Arg Gly Gln Val Lys Pro Leu Thr
        195                 200                 205

Pro Pro Asp Arg Pro Val Leu Arg Ile Glu Phe Ala Asn Tyr Gly Gln
    210                 215                 220

Ala Glu Trp Ala Ser Leu Met Pro Gly Ala Glu Ile Glu Pro Gln Thr
225                 230                 235                 240

Thr Thr Val Arg Tyr Gln Ala Lys Asp Ile Leu Glu Ala Tyr Gln Ala
                245                 250                 255

Met Leu Val Met Thr Glu Leu Ala Met Arg Thr Thr Phe Ser
            260                 265                 270

<210> 39
<211> 2119
<212> DNA
<213> Bacillus licheniformis

<220>
<221> CDS
<222> (745)..(1974)

<400> 39
gcttctttta aagttaatcc cagtgctgct tttactaggc gttcttcttc gtcctcagtc 60

aagctaacga ttgcccgatt ttgcccttga ttcatttcga tcattttctt gagcgtttct 120

ttaatttctg caaagctggg caattgaaac tctacaattg tgatgtcctt ttccaattca 180

ggaggaagcg ttaatgttgg cgccacaagc acaacatttt tggaaacaag actcttttcc 240

aatacagaga caagatcttt cattttacga atcaaacggt attcaggggg gcgattattt 300

gcacccaaat aaacataaaa atccttcaat ataaagactg ctggctgatc atattttcca 360

atgaacgcca aagcctttaa cgggtctttt gtttcggctg atggaggcat tccattgctt 420

tcaaaccctt tggttaaact ccaaacaaat aaggttcttg gtgttctaat caattctgta 480

tccgaaacaa tatgactcag ttgatgtatg acacgttctt cttcccatgt agaaatatac 540

aggaacggaa agcgggccct gaaataattc gctacttctt ttctaaagtg atgatatcct 600

tccatttttc tccctttcta ttgacctatt ttttattatt atactaaatt tttccacaac 660

aggaataaaa gatttcaatc ctcccattcg ccttctctaa atgataagat taaaataaaa 720

ccaacaattg gagatgaagg aaac atg aag aac aaa ttg att gaa cgc ctc 771
Met Lys Asn Lys Leu Ile Glu Arg Leu
1               5

att tct tat gcg aaa gtg gat acg cag tca aat gaa aac agt cag acg 819
Ile Ser Tyr Ala Lys Val Asp Thr Gln Ser Asn Glu Asn Ser Gln Thr
10                  15                  20                  25

acg cct tcc act ccc ggg cag ctg gcg ctt gcc aat atg ctt gtc gaa 867
Thr Pro Ser Thr Pro Gly Gln Leu Ala Leu Ala Asn Met Leu Val Glu
                30                  35                  40

gag ctg aaa gag atc ggc atg aag gac gtc aca atc gat gaa aac ggc 915
Glu Leu Lys Glu Ile Gly Met Lys Asp Val Thr Ile Asp Glu Asn Gly
            45                  50                  55

tat gtg atg gcg acg ctt cct tcg aat aca gaa aaa gag gtg ccg acg 963
Tyr Val Met Ala Thr Leu Pro Ser Asn Thr Glu Lys Glu Val Pro Thr
        60                  65                  70

atc ggt ttc ttg gct cat gtg gat aca gca aca gat ttt acc gga aag 1011
Ile Gly Phe Leu Ala His Val Asp Thr Ala Thr Asp Phe Thr Gly Lys
    75                  80                  85

aac gtc aat ccg cag gtt atc gaa caa tac gac gga aag gat att gtg 1059
Asn Val Asn Pro Gln Val Ile Glu Gln Tyr Asp Gly Lys Asp Ile Val
90                  95                  100                 105

ctg aat gaa tcc ctc aat gtc gtc tta tcg ccc aag gaa ttc ccc gag 1107
Leu Asn Glu Ser Leu Asn Val Val Leu Ser Pro Lys Glu Phe Pro Glu
                110                 115                 120

cta gca gat tat gcg gga cat acg ttg att aca acc gac gga acg act 1155
Leu Ala Asp Tyr Ala Gly His Thr Leu Ile Thr Thr Asp Gly Thr Thr
            125                 130                 135

ttg ctc ggc gcc gac aat aaa gct ggg atc tct gaa atc atg acg gca 1203
Leu Leu Gly Ala Asp Asn Lys Ala Gly Ile Ser Glu Ile Met Thr Ala
        140                 145                 150

atg gaa tat tta att gcg cac ccc gaa atc aaa cac ggg aag atc aga 1251
Met Glu Tyr Leu Ile Ala His Pro Glu Ile Lys His Gly Lys Ile Arg
    155                 160                 165

gtc gct ttt aca cct gat gaa gag atc ggc aga ggg ccg cac aag ttt 1299
Val Ala Phe Thr Pro Asp Glu Glu Ile Gly Arg Gly Pro His Lys Phe
170                 175                 180                 185

gac gtc gag gcg ttc aat gcc aaa ttc gcg tat acg gtt gac ggc gga 1347
Asp Val Glu Ala Phe Asn Ala Lys Phe Ala Tyr Thr Val Asp Gly Gly
                190                 195                 200

ccg ctt ggc gaa ctg cag tac gaa agc ttc aat gcc gca gcc gcg aaa 1395
Pro Leu Gly Glu Leu Gln Tyr Glu Ser Phe Asn Ala Ala Ala Ala Lys
            205                 210                 215

atc act tgt aaa gga acg aac gtc cac ccc ggc aca gca aaa gga aaa 1443
Ile Thr Cys Lys Gly Thr Asn Val His Pro Gly Thr Ala Lys Gly Lys
        220                 225                 230

atg gtc aat gcc gcc aaa atc gcg atg cag ttc cac gcc gca ttg ccg 1491
Met Val Asn Ala Ala Lys Ile Ala Met Gln Phe His Ala Ala Leu Pro
    235                 240                 245

gaa aac gaa gct cct gaa ttt aca gaa ggt tat gaa ggc ttt tat cat 1539
Glu Asn Glu Ala Pro Glu Phe Thr Glu Gly Tyr Glu Gly Phe Tyr His
250                 255                 260                 265

ctg ctt tca atc aag ggc gat gtt tct gaa acg agc ctc tct tat atc 1587
Leu Leu Ser Ile Lys Gly Asp Val Ser Glu Thr Ser Leu Ser Tyr Ile
                270                 275                 280

att aga gat ttt gac aga gac cga ttt aac gag aga aaa gac acc gtc 1635
Ile Arg Asp Phe Asp Arg Asp Arg Phe Asn Glu Arg Lys Asp Thr Val
            285                 290                 295

caa aaa atc gca aac aac ctt aaa gcg aaa tac ggc gaa aac agc gtc 1683
Gln Lys Ile Ala Asn Asn Leu Lys Ala Lys Tyr Gly Glu Asn Ser Val
        300                 305                 310

aca gtg gac atg aat gat caa tat tac aac atg agg gaa aag atc gaa 1731
Thr Val Asp Met Asn Asp Gln Tyr Tyr Asn Met Arg Glu Lys Ile Glu
    315                 320                 325

ccg gtc aaa gag att gtc gac att gcc tat aaa gcg atg aaa aac ctt 1779
Pro Val Lys Glu Ile Val Asp Ile Ala Tyr Lys Ala Met Lys Asn Leu
330                 335                 340                 345

gat att gaa ccg gtc gtt aaa ccg atc cgc ggc ggt aca gac ggc tct 1827
Asp Ile Glu Pro Val Val Lys Pro Ile Arg Gly Gly Thr Asp Gly Ser
                350                 355                 360

cag ctc tca tat atg ggg ctt cct tgt ccg aac att ttc aca ggc ggg 1875
Gln Leu Ser Tyr Met Gly Leu Pro Cys Pro Asn Ile Phe Thr Gly Gly
            365                 370                 375

gaa aac ttt cac gga aaa tac gag tat att tcc gcc gac aat atg gta     1923
Glu Asn Phe His Gly Lys Tyr Glu Tyr Ile Ser Ala Asp Asn Met Val
        380                 385                 390

aaa gcc gcg aat gtc atc gtg gaa att gtg aag ctg ttt gaa gaa aga     1971
Lys Ala Ala Asn Val Ile Val Glu Ile Val Lys Leu Phe Glu Glu Arg
    395                 400                 405

gct taaacgaaaa aagcggaaag cggctgcaag acaaagcagc cgcttttttt          2024
Ala
410

tccagatcaa tctttatctg cgctttgctg aaattagaac atgttgcaag gcggtttctt   2084

tttggagcgg gataaagcca attaacatct caccg                              2119


<210>  40
<211>  410
<212>  PRT
<213>  Bacillus licheniformis

<400>  40

Met Lys Asn Lys Leu Ile Glu Arg Leu Ile Ser Tyr Ala Lys Val Asp
1               5                   10                  15

Thr Gln Ser Asn Glu Asn Ser Gln Thr Thr Pro Ser Thr Pro Gly Gln
            20                  25                  30

Leu Ala Leu Ala Asn Met Leu Val Glu Glu Leu Lys Glu Ile Gly Met
        35                  40                  45

Lys Asp Val Thr Ile Asp Glu Asn Gly Tyr Val Met Ala Thr Leu Pro
    50                  55                  60

Ser Asn Thr Glu Lys Glu Val Pro Thr Ile Gly Phe Leu Ala His Val
65                  70                  75                  80

Asp Thr Ala Thr Asp Phe Thr Gly Lys Asn Val Asn Pro Gln Val Ile
                85                  90                  95

Glu Gln Tyr Asp Gly Lys Asp Ile Val Leu Asn Glu Ser Leu Asn Val
            100                 105                 110

Val Leu Ser Pro Lys Glu Phe Pro Glu Leu Ala Asp Tyr Ala Gly His
        115                 120                 125

Thr Leu Ile Thr Thr Asp Gly Thr Thr Leu Leu Gly Ala Asp Asn Lys
    130                 135                 140

Ala Gly Ile Ser Glu Ile Met Thr Ala Met Glu Tyr Leu Ile Ala His
145                 150                 155                 160

Pro Glu Ile Lys His Gly Lys Ile Arg Val Ala Phe Thr Pro Asp Glu
                165                 170                 175

Glu Ile Gly Arg Gly Pro His Lys Phe Asp Val Glu Ala Phe Asn Ala
            180                 185                 190

Lys Phe Ala Tyr Thr Val Asp Gly Gly Pro Leu Gly Glu Leu Gln Tyr
        195                 200                 205

Glu Ser Phe Asn Ala Ala Ala Ala Lys Ile Thr Cys Lys Gly Thr Asn
    210                 215                 220

Val His Pro Gly Thr Ala Lys Gly Lys Met Val Asn Ala Ala Lys Ile
225                 230                 235                 240

Ala Met Gln Phe His Ala Ala Leu Pro Glu Asn Glu Ala Pro Glu Phe
                245                 250                 255

Thr Glu Gly Tyr Glu Gly Phe Tyr His Leu Leu Ser Ile Lys Gly Asp
            260                 265                 270

Val Ser Glu Thr Ser Leu Ser Tyr Ile Ile Arg Asp Phe Asp Arg Asp
        275                 280                 285

Arg Phe Asn Glu Arg Lys Asp Thr Val Gln Lys Ile Ala Asn Asn Leu
    290                 295                 300

Lys Ala Lys Tyr Gly Glu Asn Ser Val Thr Val Asp Met Asn Asp Gln
305                 310                 315                 320

Tyr Tyr Asn Met Arg Glu Lys Ile Glu Pro Val Lys Glu Ile Val Asp
                325                 330                 335

Ile Ala Tyr Lys Ala Met Lys Asn Leu Asp Ile Glu Pro Val Val Lys
            340                 345                 350

Pro Ile Arg Gly Gly Thr Asp Gly Ser Gln Leu Ser Tyr Met Gly Leu
        355                 360                 365

Pro Cys Pro Asn Ile Phe Thr Gly Gly Glu Asn Phe His Gly Lys Tyr
    370                 375                 380

Glu Tyr Ile Ser Ala Asp Asn Met Val Lys Ala Ala Asn Val Ile Val
385                 390                 395                 400

Glu Ile Val Lys Leu Phe Glu Glu Arg Ala
                405                 410

<210>  41
<211>  1782
<212>  DNA
<213>  Bacillus licheniformis

<220>
<221>  CDS

<222>  (1238)..(1780)

<400>  41
acaggcacca caacgcataa tcacactcag ccgagaccga aaataaacac ctcaccaaca      60

aacctaacac caacacaccc cacactcaaa ccacccaagg ataacaaaac cgaacgacca     120

cgacatcacg caaaaacggc ccccacaagc agacaaaata caaccgcaca tccaaaaaat     180

aaacacatcc caaaataaag acacacgaaa agcaccacct gacaacaaga ccacacacac     240

aacaccgaga acaacccacc aaaaccggcc ccacaaaaca catcacacac aaccgagacg     300

aaaaccgcag caacgtccca aaacaaccac accccgccac gatgacaaca gacgcacaac     360

cgcgcataaa gtagaaaaaa agacgatacg ccacagcaaa aaacagacac agcaccctta     420

acctcctacc cccgcatgcc atatacacac tactcatccc caccaaaaca ccccagggca     480

cacgcccaca ccgcaaccaa tcccatcacg gaagcgaaca aaaaaaaatc ggcgccatac     540

aacaccaacg gaccggaaac caagagaccc gctgcgcgca taatatatcg ctgatacata     600

tttggaaaac gttgaatgtt ataacgaagt acggggaatg tacgggttta ccggcactta     660

taaaggaaag cgcgtttccg ttcaggggac gggcatgggt gtgccgtcta tttcaattta     720

tgtcaatgaa ctgatcagaa gctatgatgt gaaaaacctg atccgcgtcg gatcgtgcgg     780

tgcgatcaga aaagacgtca atgtaagaga tgtcattctt gcgatgactt catcaactga     840

ttcacaaatc aacagagtgg cattcggctc gatcgacttt gcgccctgcg cggacttcgg     900

cctgctaaaa gctgcttatg acgctgcaag tgagcggaac atcccggtta cggcagggaa     960

tgtgtttacg gcggatcaat tctataacga cgacagccaa attgagaagc ttgccaaaca    1020

cggagtgctt gcggtcgaga tggaaacgac ggcgctttat acattggctg cgaaattcgg    1080

ccgaaaagcc ctctccattt taacggtcag cgatcacgtc attaccggcg aggaaacaac    1140

ggcagaagaa agacagacga cctttaatga tatgattctg ctggctctgg atactgcttt    1200

gtaaaagcat acatgcactg aaaaaggatg gattcga atg aac gga aag tac aaa     1255
                                         Met Asn Gly Lys Tyr Lys
                                         1               5

tac gtg acg att gct tca ttg ctg agt gca gcg gtc ctg ctt ggc aca      1303
Tyr Val Thr Ile Ala Ser Leu Leu Ser Ala Ala Val Leu Leu Gly Thr
            10                  15                  20

gga tgt acc atg acc gaa cag aag caa aat gac acg gct aaa aca cag      1351
Gly Cys Thr Met Thr Glu Gln Lys Gln Asn Asp Thr Ala Lys Thr Gln
        25                  30                  35

aat act gca aat gca agc aag gat gca aaa gac caa aag tca gac agc      1399
Asn Thr Ala Asn Ala Ser Lys Asp Ala Lys Asp Gln Lys Ser Asp Ser
    40                  45                  50

agc ctg gaa gat cag gac ttc ata ctc gag agc aag tac ttt aat caa      1447
Ser Leu Glu Asp Gln Asp Phe Ile Leu Glu Ser Lys Tyr Phe Asn Gln
55                  60                  65                  70

ata aaa gat gta gac ggg ctg ccg acg att caa aat ccg gaa aac ata      1495
Ile Lys Asp Val Asp Gly Leu Pro Thr Ile Gln Asn Pro Glu Asn Ile
                75                  80                  85

atg gcg ctt gta aat aaa gaa tac gcg ctt ccc ggc aac tat gca ccg      1543

Met Ala Leu Val Asn Lys Glu Tyr Ala Leu Pro Gly Asn Tyr Ala Pro
                90                  95                  100

agc gac ctg aca gtg cct gat gtc gcg ttt tct ttt acc gag gat atc        1591
Ser Asp Leu Thr Val Pro Asp Val Ala Phe Ser Phe Thr Glu Asp Ile
        105                 110                 115

gac aag cgt tat atc aga aaa gaa gcc gcc aaa gcg ctt gaa gag atg        1639
Asp Lys Arg Tyr Ile Arg Lys Glu Ala Ala Lys Ala Leu Glu Glu Met
    120                 125                 130

ttc agc gcc gct aag aaa gaa ggc tat gaa ctc gtt gca gtg tcc ggc        1687
Phe Ser Ala Ala Lys Lys Glu Gly Tyr Glu Leu Val Ala Val Ser Gly
135                 140                 145                 150

tac aga tcc tat gac aga caa aaa gcg atc tat aac aac gaa gtc agc        1735
Tyr Arg Ser Tyr Asp Arg Gln Lys Ala Ile Tyr Asn Asn Glu Val Ser
                155                 160                 165

caa aag gga gaa gaa aag gcg aaa gaa gct gtg gca tat ccg ggc ca         1782
Gln Lys Gly Glu Glu Lys Ala Lys Glu Ala Val Ala Tyr Pro Gly
            170                 175                 180

<210>   42
<211>   181
<212>   PRT
<213>   Bacillus licheniformis

<400>   42

Met Asn Gly Lys Tyr Lys Tyr Val Thr Ile Ala Ser Leu Leu Ser Ala
1               5                   10                  15

Ala Val Leu Leu Gly Thr Gly Cys Thr Met Thr Glu Gln Lys Gln Asn
            20                  25                  30

Asp Thr Ala Lys Thr Gln Asn Thr Ala Asn Ala Ser Lys Asp Ala Lys
        35                  40                  45

Asp Gln Lys Ser Asp Ser Ser Leu Glu Asp Gln Asp Phe Ile Leu Glu
    50                  55                  60

Ser Lys Tyr Phe Asn Gln Ile Lys Asp Val Asp Gly Leu Pro Thr Ile
65                  70                  75                  80

Gln Asn Pro Glu Asn Ile Met Ala Leu Val Asn Lys Glu Tyr Ala Leu
                85                  90                  95

Pro Gly Asn Tyr Ala Pro Ser Asp Leu Thr Val Pro Asp Val Ala Phe
            100                 105                 110

Ser Phe Thr Glu Asp Ile Asp Lys Arg Tyr Ile Arg Lys Glu Ala Ala
        115                 120                 125

Lys Ala Leu Glu Glu Met Phe Ser Ala Ala Lys Lys Glu Gly Tyr Glu
    130                 135                 140

Leu Val Ala Val Ser Gly Tyr Arg Ser Tyr Asp Arg Gln Lys Ala Ile

145                 150                 155                 160

Tyr Asn Asn Glu Val Ser Gln Lys Gly Glu Glu Lys Ala Lys Glu Ala
                165                 170                 175

Val Ala Tyr Pro Gly
            180

<210>  43
<211>  1663
<212>  DNA
<213>  Bacillus licheniformis

<220>
<221>  CDS
<222>  (3)..(1166)

<400>  43
tc cgg tct gtg gcc atc gga gtt tgg att gga acc gga tca aga cat      47
   Arg Ser Val Ala Ile Gly Val Trp Ile Gly Thr Gly Ser Arg His
   1               5                   10                  15

gaa aca ccc gaa att aac ggc att tcg cac ttt ctt gag cac atg ttt      95
Glu Thr Pro Glu Ile Asn Gly Ile Ser His Phe Leu Glu His Met Phe
                20                  25                  30

ttc aag ggg aca aaa acg cgc act gca agg gac atc gca gag tct ttc      143
Phe Lys Gly Thr Lys Thr Arg Thr Ala Arg Asp Ile Ala Glu Ser Phe
            35                  40                  45

gat aga atc ggc gga cag gtc aac gcg ttt acc tca aag gaa tat acg      191
Asp Arg Ile Gly Gly Gln Val Asn Ala Phe Thr Ser Lys Glu Tyr Thr
        50                  55                  60

tgc tat tac gcc aaa gtg ctc gat gag cat gca agc tat gct ctg gaa      239
Cys Tyr Tyr Ala Lys Val Leu Asp Glu His Ala Ser Tyr Ala Leu Glu
    65                  70                  75

gtg ctt tct gac atg ttc ttt cat tca tca ttt gac gaa gaa gaa tta      287
Val Leu Ser Asp Met Phe Phe His Ser Ser Phe Asp Glu Glu Glu Leu
80                  85                  90                  95

aaa aaa gaa aaa aac gtc gtc tat gaa gag att aaa atg tat gag gac      335
Lys Lys Glu Lys Asn Val Val Tyr Glu Glu Ile Lys Met Tyr Glu Asp
                100                 105                 110

aca cct gat gac atc gtc cac gat ctc ttg agc aag gcg tca tac ggc      383
Thr Pro Asp Asp Ile Val His Asp Leu Leu Ser Lys Ala Ser Tyr Gly
            115                 120                 125

agc cat tcc ctc ggc tat ccg atc ctc ggc aca gag gaa aca ctt gcc      431
Ser His Ser Leu Gly Tyr Pro Ile Leu Gly Thr Glu Glu Thr Leu Ala
        130                 135                 140

gaa ttt gac gga gat tcg ctc aga aaa tat atg aac gaa tat tac acg      479
Glu Phe Asp Gly Asp Ser Leu Arg Lys Tyr Met Asn Glu Tyr Tyr Thr
    145                 150                 155

cct gac cga gtc gtc att tcc ata gcc gga aat gtg ccg gaa act ttt      527
Pro Asp Arg Val Val Ile Ser Ile Ala Gly Asn Val Pro Glu Thr Phe
160                 165                 170                 175

ata aaa gaa gct gaa aag cat ttc ggt tca tat gaa gcc aaa ggc aaa      575
Ile Lys Glu Ala Glu Lys His Phe Gly Ser Tyr Glu Ala Lys Gly Lys

                180                 185                 190

aga acc ggc atg aca aaa ccg gat ttt cac cat gag aaa atg acg cgc 623
Arg Thr Gly Met Thr Lys Pro Asp Phe His His Glu Lys Met Thr Arg
            195                 200                 205

aaa aag gaa acc gaa cag gct cac ctc tgc ctc ggc ttc aac ggg ctg 671
Lys Lys Glu Thr Glu Gln Ala His Leu Cys Leu Gly Phe Asn Gly Leu
        210                 215                 220

gaa gca ggc cat ccg gaa atc tac gat ttg atc gtt ctc aat aat att 719
Glu Ala Gly His Pro Glu Ile Tyr Asp Leu Ile Val Leu Asn Asn Ile
    225                 230                 235

ttg gga gga agc atg agc agc cgc ctc ttt cag gat gtc cgc gaa gat 767
Leu Gly Gly Ser Met Ser Ser Arg Leu Phe Gln Asp Val Arg Glu Asp
240                 245                 250                 255

aaa ggt ctt gca tat tcc gtg ttc agc tac cac act tcc tat gaa gac 815
Lys Gly Leu Ala Tyr Ser Val Phe Ser Tyr His Thr Ser Tyr Glu Asp
                260                 265                 270

agc ggc atg atg acc atc tac gcg gga acc ggc gca aat cag ctt cag 863
Ser Gly Met Met Thr Ile Tyr Ala Gly Thr Gly Ala Asn Gln Leu Gln
            275                 280                 285

ctc ctg tca gaa acg att cat gaa aca ctg cgt gcg ctg aaa agc gac 911
Leu Leu Ser Glu Thr Ile His Glu Thr Leu Arg Ala Leu Lys Ser Asp
        290                 295                 300

ggt att aca ccg aaa gag ctt gaa aac agc aaa gag caa atg aaa ggc 959
Gly Ile Thr Pro Lys Glu Leu Glu Asn Ser Lys Glu Gln Met Lys Gly
    305                 310                 315

agc ctg atg ctc agt ctc gaa agc aca aac agc aaa atg agc cgc aac 1007
Ser Leu Met Leu Ser Leu Glu Ser Thr Asn Ser Lys Met Ser Arg Asn
320                 325                 330                 335

ggc aaa aat gag ctt ttg ctc ggt aaa cac cgg acg ctt gat gaa atc 1055
Gly Lys Asn Glu Leu Leu Leu Gly Lys His Arg Thr Leu Asp Glu Ile
                340                 345                 350

atc gaa aag ttg aat gcc gtc agc ctc gag cgt gtg aac aat ctt gcc 1103
Ile Glu Lys Leu Asn Ala Val Ser Leu Glu Arg Val Asn Asn Leu Ala
            355                 360                 365

aac cgg att ttc acc gat gat tac tca tct gca tta atc agc ccg tcg 1151
Asn Arg Ile Phe Thr Asp Asp Tyr Ser Ser Ala Leu Ile Ser Pro Ser
        370                 375                 380

ggt gag ctg ccg aaa taatgcctcc ttaaaacatg cttgtctttt taggcaggca 1206
Gly Glu Leu Pro Lys
    385

tgttttttgt tatcggttca tacatcataa caaaaggggg tttttttcat gaggctgagt 1266

gaactttcgg gaaaagaaat cgttgatgtt aagcgggccg aacggctcgg cgtactgggg 1326

cagacggacc ttgagatcaa tgagcaggac ggtcagatta ccgctcttat catcccttct 1386

gttaaatggt tcggactagg aagaaaacag gggaatgaca ttaaggtgcc ctggtcgcaa 1446

attcaaaaaa tcggctcgga tatgatcatt ttggatgttc cggaaagcag cgtgacaaaa 1506

gaggagtaag caaggtggaa acccgcccgt cccatggggg cgggcttttt gtttttctgg 1566

atttcaattc accgctattt cctttttgtc atatgatgaa attagcttat gaattagatc 1626

cttgtcaaaa aagaaggtga atgttagagc catgtta 1663

<210> 44
<211> 388
<212> PRT
<213> Bacillus licheniformis

<400> 44

Arg Ser Val Ala Ile Gly Val Trp Ile Gly Thr Gly Ser Arg His Glu
1               5                   10                  15

Thr Pro Glu Ile Asn Gly Ile Ser His Phe Leu Glu His Met Phe Phe
            20                  25                  30

Lys Gly Thr Lys Thr Arg Thr Ala Arg Asp Ile Ala Glu Ser Phe Asp
        35                  40                  45

Arg Ile Gly Gly Gln Val Asn Ala Phe Thr Ser Lys Glu Tyr Thr Cys
    50                  55                  60

Tyr Tyr Ala Lys Val Leu Asp Glu His Ala Ser Tyr Ala Leu Glu Val
65                  70                  75                  80

Leu Ser Asp Met Phe Phe His Ser Ser Phe Asp Glu Glu Glu Leu Lys
                85                  90                  95

Lys Glu Lys Asn Val Val Tyr Glu Glu Ile Lys Met Tyr Glu Asp Thr
            100                 105                 110

Pro Asp Asp Ile Val His Asp Leu Leu Ser Lys Ala Ser Tyr Gly Ser
        115                 120                 125

His Ser Leu Gly Tyr Pro Ile Leu Gly Thr Glu Glu Thr Leu Ala Glu
    130                 135                 140

Phe Asp Gly Asp Ser Leu Arg Lys Tyr Met Asn Glu Tyr Tyr Thr Pro
145                 150                 155                 160

Asp Arg Val Val Ile Ser Ile Ala Gly Asn Val Pro Glu Thr Phe Ile
                165                 170                 175

Lys Glu Ala Glu Lys His Phe Gly Ser Tyr Glu Ala Lys Gly Lys Arg
            180                 185                 190

Thr Gly Met Thr Lys Pro Asp Phe His His Glu Lys Met Thr Arg Lys
        195                 200                 205

Lys Glu Thr Glu Gln Ala His Leu Cys Leu Gly Phe Asn Gly Leu Glu
    210                 215                 220

Ala Gly His Pro Glu Ile Tyr Asp Leu Ile Val Leu Asn Asn Ile Leu
225                 230                 235                 240

Gly Gly Ser Met Ser Ser Arg Leu Phe Gln Asp Val Arg Glu Asp Lys
                245                 250                 255

Gly Leu Ala Tyr Ser Val Phe Ser Tyr His Thr Ser Tyr Glu Asp Ser
            260                 265                 270

Gly Met Met Thr Ile Tyr Ala Gly Thr Gly Ala Asn Gln Leu Gln Leu
        275                 280                 285

Leu Ser Glu Thr Ile His Glu Thr Leu Arg Ala Leu Lys Ser Asp Gly
    290                 295                 300

Ile Thr Pro Lys Glu Leu Glu Asn Ser Lys Glu Gln Met Lys Gly Ser
305                 310                 315                 320

Leu Met Leu Ser Leu Glu Ser Thr Asn Ser Lys Met Ser Arg Asn Gly
                325                 330                 335

Lys Asn Glu Leu Leu Leu Gly Lys His Arg Thr Leu Asp Glu Ile Ile
            340                 345                 350

Glu Lys Leu Asn Ala Val Ser Leu Glu Arg Val Asn Asn Leu Ala Asn
        355                 360                 365

Arg Ile Phe Thr Asp Asp Tyr Ser Ser Ala Leu Ile Ser Pro Ser Gly
    370                 375                 380

Glu Leu Pro Lys
385

<210>  45
<211>  1553
<212>  DNA
<213>  Bacillus licheniformis

<220>
<221>  CDS
<222>  (499)..(1296)

<400>  45
ctttcaaatc atcccagctg aaatgccccg aatcgttcct ggagacgagg aaggttccgt      60

ccgtctgggt aagctgtgca aaattaatca ccggatcgct tgcgccctgg gcatttacgt     120

atattgacgt ttccgagccg acaagcgcaa tatcggcgcc gtttgacaga agggcagtca     180

tcgttttatc tccgccccag gtcgttttca gttcaacatc aaggccttct tctttaaaaa     240

aaccttcaga taaggcgata tataaaggcg tatagaaaac ggaacgggtc acttccgcca     300

gacggatctt ttcggtcttt tgtccgccgc atgaagcgag gataaacagc atcatgatga     360

aggataatat ggtaaaaaag aaacgtttca aaccgatttc cctcctttga tttcggttat     420

tttcggcata aaatagttta tgttatatac taaacaggtg tgagggacag ctgcggatta     480

aaagaggtga cgtgacga atg tgg ata gaa aaa aga agg ttt cca tcg ccg 531
Met Trp Ile Glu Lys Arg Arg Phe Pro Ser Pro
1               5                   10

aac aaa gag gtg cgt ctg ttt tcc gtc aca tac ctt tca caa gga ctc 579
Asn Lys Glu Val Arg Leu Phe Ser Val Thr Tyr Leu Ser Gln Gly Leu
            15                  20                  25

aag gtt aag ggg ctt ttg gcc gaa ccg gcc gct ccc gga aat tat gac 627
Lys Val Lys Gly Leu Leu Ala Glu Pro Ala Ala Pro Gly Asn Tyr Asp
        30                  35                  40

ggg ttt ttg tat ttg cgc ggc gga att aaa aac gtc ggg atg gtc aga 675
Gly Phe Leu Tyr Leu Arg Gly Gly Ile Lys Asn Val Gly Met Val Arg
    45                  50                  55

ccg ggc cga atc gtc cag ttc gct tca cag ggc ttt gtc gtt ctt gct 723
Pro Gly Arg Ile Val Gln Phe Ala Ser Gln Gly Phe Val Val Leu Ala
60                  65                  70                  75

ccg ttt tac agg ggc aat caa gga gga gaa ggc gat gag gat ttt gca 771
Pro Phe Tyr Arg Gly Asn Gln Gly Gly Glu Gly Asp Glu Asp Phe Ala
                80                  85                  90

ggt aat gac cgg gaa gat gct ttt gcc gct ttt cgt ctt ctt cag aag 819
Gly Asn Asp Arg Glu Asp Ala Phe Ala Ala Phe Arg Leu Leu Gln Lys
            95                  100                 105

cac gaa ctg gta aag gac gga cgc gtt cac att ttt ggc ttt tcc cgc 867
His Glu Leu Val Lys Asp Gly Arg Val His Ile Phe Gly Phe Ser Arg
        110                 115                 120

ggc gga atc atg ggc atc tta acg gca atc gaa atg aaa gca gcg gcc 915
Gly Gly Ile Met Gly Ile Leu Thr Ala Ile Glu Met Lys Ala Ala Ala
    125                 130                 135

gct tcg ttt gtg tct tgg ggc ggt gtc agc gac atg gtc atg acc tat 963
Ala Ser Phe Val Ser Trp Gly Gly Val Ser Asp Met Val Met Thr Tyr
140                 145                 150                 155

cgc gaa cga aaa gat ttg cgg cgg atg atg aag cgg gtc atc ggc ggg 1011
Arg Glu Arg Lys Asp Leu Arg Arg Met Met Lys Arg Val Ile Gly Gly
                160                 165                 170

acg cct gaa aag gtt ccc gaa gcg tat gaa tgg agg acg cct ttt gac 1059
Thr Pro Glu Lys Val Pro Glu Ala Tyr Glu Trp Arg Thr Pro Phe Asp
            175                 180                 185

gag ctg gag aag att gaa gcg ccc ggc ttt ttg atc cac gga gca acc 1107
Glu Leu Glu Lys Ile Glu Ala Pro Gly Phe Leu Ile His Gly Ala Thr
        190                 195                 200

gtt gaa aat gtc tcc atc gag cat gcg cgg cgc ctt gaa gcg gga ctt 1155
Val Glu Asn Val Ser Ile Glu His Ala Arg Arg Leu Glu Ala Gly Leu
    205                 210                 215

ctg cga ggg aca agt ctg ttg gaa act ggg aat ttt agc cca tat acc 1203
Leu Arg Gly Thr Ser Leu Leu Glu Thr Gly Asn Phe Ser Pro Tyr Thr
220                 225                 230                 235

cac tat ttt cct ccg cgc gaa aac agg cgg att gtt aga gaa tta tcc 1251
His Tyr Phe Pro Pro Arg Glu Asn Arg Arg Ile Val Arg Glu Leu Ser
                240                 245                 250

caa tgg atg aaa agc cgt cct tgg aca agt tat ggt aaa cta aag 1296
Gln Trp Met Lys Ser Arg Pro Trp Thr Ser Tyr Gly Lys Leu Lys
            255                 260                 265

taaaagagaa aaggaggggg actttaggtg ggaatgccag ttgaatttaa cacgatgatt 1356

gtgacaaaag gaaaagaaac gcgggttgag gaaaatgtgt ttacactcga gaaagaaggt 1416

tatcgggttt accctcttca tgtgccgctt gaagtcagaa agaccaagcg gggcgacatt 1476

accggcaccg cgcaggcgga gcgccttgaa tggtcagacg gacggactca tttaacatac 1536

aggctcgtat cattaaa 1553

<210> 46
<211> 266
<212> PRT
<213> Bacillus licheniformis

<400> 46

Met Trp Ile Glu Lys Arg Arg Phe Pro Ser Pro Asn Lys Glu Val Arg
1               5                   10                  15

Leu Phe Ser Val Thr Tyr Leu Ser Gln Gly Leu Lys Val Lys Gly Leu
            20                  25                  30

Leu Ala Glu Pro Ala Ala Pro Gly Asn Tyr Asp Gly Phe Leu Tyr Leu
        35                  40                  45

Arg Gly Gly Ile Lys Asn Val Gly Met Val Arg Pro Gly Arg Ile Val
    50                  55                  60

Gln Phe Ala Ser Gln Gly Phe Val Val Leu Ala Pro Phe Tyr Arg Gly
65                  70                  75                  80

Asn Gln Gly Gly Glu Gly Asp Glu Asp Phe Ala Gly Asn Asp Arg Glu
                85                  90                  95

Asp Ala Phe Ala Ala Phe Arg Leu Leu Gln Lys His Glu Leu Val Lys
            100                 105                 110

Asp Gly Arg Val His Ile Phe Gly Phe Ser Arg Gly Gly Ile Met Gly
        115                 120                 125

Ile Leu Thr Ala Ile Glu Met Lys Ala Ala Ala Ala Ser Phe Val Ser
    130                 135                 140

Trp Gly Gly Val Ser Asp Met Val Met Thr Tyr Arg Glu Arg Lys Asp
145                 150                 155                 160

Leu Arg Arg Met Met Lys Arg Val Ile Gly Gly Thr Pro Glu Lys Val
                165                 170                 175

Pro Glu Ala Tyr Glu Trp Arg Thr Pro Phe Asp Glu Leu Glu Lys Ile
            180                 185                 190

Glu Ala Pro Gly Phe Leu Ile His Gly Ala Thr Val Glu Asn Val Ser

195                 200                 205

Ile Glu His Ala Arg Arg Leu Glu Ala Gly Leu Leu Arg Gly Thr Ser
    210                 215                 220

Leu Leu Glu Thr Gly Asn Phe Ser Pro Tyr Thr His Tyr Phe Pro Pro
225                 230                 235                 240

Arg Glu Asn Arg Arg Ile Val Arg Glu Leu Ser Gln Trp Met Lys Ser
                245                 250                 255

Arg Pro Trp Thr Ser Tyr Gly Lys Leu Lys
            260                 265

<210>  47
<211>  1576
<212>  DNA
<213>  Bacillus licheniformis

<220>
<221>  CDS
<222>  (501)..(1073)

<400>  47
acgaagacga aaggaacggt ctgtgcaccg ctgacattgt ttgcagaata taaaaaccgc      60

ggcagtcaaa tcgccgactc tgaatggaag tgggagcttc ataaactcgg tcattccaaa     120

aagccgctag aggacgggac tgtgatcgat aaaggcgtgt tcaaaaaaga agccgaaagc     180

agcatctacc ggatagagtc aaagcgggca gcaaaaggag ggctatacgc ttttaaattg     240

atcagacctg aaggccatcc agatgttaaa aagggcaaac ctttcatttg gtcaaatgta     300

atggagcttc aagactgcaa tgaagaaaca ccgaagcctc ctaaaaaagc ttcagcaaaa     360

ccaaagcaaa ctgaacagac tcaaacaacg caaaagtctg aacaggatat agaggaaaca     420

ccaaaggaca gtgaatccgt agataaagaa aatagaaaag aagataattc attagaaagc     480

ggggaagcgt catgaaacat gtg atg aaa tgg atc agc aat ttc tta tat gtg     533
                      Val Met Lys Trp Ile Ser Asn Phe Leu Tyr Val
                      1               5                   10

att att ttc aca atc atc atc gcc gcg gtc atc gtc gtg att tcg aca       581
Ile Ile Phe Thr Ile Ile Ile Ala Ala Val Ile Val Val Ile Ser Thr
            15                  20                  25

aag tcg tcc ggc ggg gag ccg cag ctc ttc ggc tat caa ttg aaa acg       629
Lys Ser Ser Gly Gly Glu Pro Gln Leu Phe Gly Tyr Gln Leu Lys Thr
        30                  35                  40

gtt tta tcc gga tcg atg gag ccg gag ttt aaa aca ggt tct gtg att       677
Val Leu Ser Gly Ser Met Glu Pro Glu Phe Lys Thr Gly Ser Val Ile
    45                  50                  55

gcc gtt caa aaa gtt gaa aat ccc ggg tct ttg aaa aag gga gat atc       725
Ala Val Gln Lys Val Glu Asn Pro Gly Ser Leu Lys Lys Gly Asp Ile
60                  65                  70                  75

att aca ttc atg caa gac gaa aac acc atg gtt acc cac cga att atc       773
Ile Thr Phe Met Gln Asp Glu Asn Thr Met Val Thr His Arg Ile Ile

                    80                  85                  90

ggt ata aca aaa aat aaa tca aat ctc atg ttc aag aca aag ggt gac        821
Gly Ile Thr Lys Asn Lys Ser Asn Leu Met Phe Lys Thr Lys Gly Asp
            95                  100                 105

aat aac caa aac cct gat tcc gat ccg gta ctg gcg gaa aat gtt gtc        869
Asn Asn Gln Asn Pro Asp Ser Asp Pro Val Leu Ala Glu Asn Val Val
        110                 115                 120

gct aag tat tcg ggc att acg gtt ccg tat gcc ggg tat ttg ctg gac        917
Ala Lys Tyr Ser Gly Ile Thr Val Pro Tyr Ala Gly Tyr Leu Leu Asp
    125                 130                 135

ttt gca agt aaa ccg atc ggc aca gcc att ttg ctg atc gtg ccg gga        965
Phe Ala Ser Lys Pro Ile Gly Thr Ala Ile Leu Leu Ile Val Pro Gly
140                 145                 150                 155

ctc ttg ttg att ctt tat gca gta att act gta tcc gcg gct tta aga       1013
Leu Leu Leu Ile Leu Tyr Ala Val Ile Thr Val Ser Ala Ala Leu Arg
                160                 165                 170

gag att gac caa aaa gct aaa gca att gaa gcc gct gga aaa gat caa       1061
Glu Ile Asp Gln Lys Ala Lys Ala Ile Glu Ala Ala Gly Lys Asp Gln
            175                 180                 185

tca gtt tcc atg taatcttcgt tggtcacctg aacattcagg tctccatata           1113
Ser Val Ser Met
        190

tacagatttt cttaattata aaaggaggag ttatcaatgg gtacaaagaa aaaactagga     1173

ttaggcgttg cgtctgctgc gcttggactg gcattagtag gaggaggaac ttgggctgcg     1233

tttaacgaca tcgaaacaac tcaagcaact tatgcagcag gtacgcttga cttaaatgcg     1293

aaagatacat ctgcaagagt gaacttgtcc aacttaaaac caggcgacaa attcactaaa     1353

gatttcgagt tcaaaaatga cggatcactt gcgattaaag aagtgctgat gcaggttggc     1413

tacagcaatt tcgttgacgg aaacgcgaaa aacggcggaa aaagcacagc ggaagacttc     1473

ctgaaacaat ttaaagtcag cgttctgact gtcggagttg aaggcggtaa cggctatcct     1533

aaaaacatca ttttagatga agccaacctt tatgatctgt aca                       1576

<210>  48
<211>  191
<212>  PRT
<213>  Bacillus licheniformis

<400>  48

Val Met Lys Trp Ile Ser Asn Phe Leu Tyr Val Ile Ile Phe Thr Ile
1               5                   10                  15

Ile Ile Ala Ala Val Ile Val Val Ile Ser Thr Lys Ser Ser Gly Gly
            20                  25                  30

Glu Pro Gln Leu Phe Gly Tyr Gln Leu Lys Thr Val Leu Ser Gly Ser
        35                  40                  45

Met Glu Pro Glu Phe Lys Thr Gly Ser Val Ile Ala Val Gln Lys Val
    50                  55                  60

Glu Asn Pro Gly Ser Leu Lys Lys Gly Asp Ile Ile Thr Phe Met Gln
65                  70                  75                  80

Asp Glu Asn Thr Met Val Thr His Arg Ile Ile Gly Ile Thr Lys Asn
                85                  90                  95

Lys Ser Asn Leu Met Phe Lys Thr Lys Gly Asp Asn Asn Gln Asn Pro
            100                 105                 110

Asp Ser Asp Pro Val Leu Ala Glu Asn Val Val Ala Lys Tyr Ser Gly
        115                 120                 125

Ile Thr Val Pro Tyr Ala Gly Tyr Leu Leu Asp Phe Ala Ser Lys Pro
    130                 135                 140

Ile Gly Thr Ala Ile Leu Leu Ile Val Pro Gly Leu Leu Leu Ile Leu
145                 150                 155                 160

Tyr Ala Val Ile Thr Val Ser Ala Ala Leu Arg Glu Ile Asp Gln Lys
                165                 170                 175

Ala Lys Ala Ile Glu Ala Ala Gly Lys Asp Gln Ser Val Ser Met
            180                 185                 190

<210>  49
<211>  1514
<212>  DNA
<213>  Bacillus licheniformis

<220>
<221>  CDS
<222>  (501)..(1016)

<400>  49
   tgcccctgct cgtgtttgtt gttgccaatg taaattcgtt cggtttatgg caggggtttt      60
   tgttgacgtg ggtcggggca tctttaggtt ctatagcggt ttttcttttt atccgccatt      120
   tcggccagca aaaagcactc ggttttatcc gcaggcatcc atccgtaaaa aaactgatga      180
   tctgggtgga aaaacggggc ttcggtccgc tgtttctcct gctttgcttt ccattcacgc      240
   cttcggcggc ggtaaatgtt gtggcaggac tttcgcgagt cagcctgctg cctttttcat      300
   tggcggtgct ttgcggcaag ttcgtgatga tttttatggt gagctttgtc ggccatgatc      360
   tgaaggcgct ttttacccag cctttgcgga cagccgccgt gatcatcgtc atgctgatct      420
   tatggtacgc cggaaaacgg gttgaacatc gtctgaacgt gagattcagc cagagagaga      480
   aagactaggg ggacacagaa ttg aaa aag cgt ttt ttg gtc ata gcc ggg gta      533
                         Leu Lys Lys Arg Phe Leu Val Ile Ala Gly Val
                         1               5                   10
   ctg ctt gtc atc gtc gtc ggg att cag gtc aaa aat gcc gtt ttc att      581
   Leu Leu Val Ile Val Val Gly Ile Gln Val Lys Asn Ala Val Phe Ile
               15                  20                  25
   gaa tat aaa gta gaa ggc gtc agc atg aat ccg acc ttt cag gag ggc     629
   Glu Tyr Lys Val Glu Gly Val Ser Met Asn Pro Thr Phe Gln Glu Gly
           30                  35                  40
   aac gag ctt ttg atc aac agg ttc gcc cac cgc ttt aaa acg atc agc     677
   Asn Glu Leu Leu Ile Asn Arg Phe Ala His Arg Phe Lys Thr Ile Ser
       45                  50                  55
   cga ttt gac atc gtg ctg ttt aaa gga cct gac aaa gat ata ttc att     725
   Arg Phe Asp Ile Val Leu Phe Lys Gly Pro Asp Lys Asp Ile Phe Ile
   60                  65                  70                  75
   aaa agg gtg atc ggg ctt ccg ggc gaa acc ctc agg tat gaa gat gat     773
   Lys Arg Val Ile Gly Leu Pro Gly Glu Thr Leu Arg Tyr Glu Asp Asp
                   80                  85                  90
   cag ctg tat atc aac gaa gaa aag atc aaa gag cct tat ctg gac gac     821
   Gln Leu Tyr Ile Asn Glu Glu Lys Ile Lys Glu Pro Tyr Leu Asp Asp
               95                  100                 105
   tta aag gcc gtc acc gcc gga ggg gac ttg aca ggg gat ttt aca ctg     869
   Leu Lys Ala Val Thr Ala Gly Gly Asp Leu Thr Gly Asp Phe Thr Leu
           110                 115                 120
   cag gaa gtg acc gga gag gag aag gtg cct gaa aac gag tac ttc gtc     917
   Gln Glu Val Thr Gly Glu Glu Lys Val Pro Glu Asn Glu Tyr Phe Val
       125                 130                 135
   ctc ggg gac aac cgg atc cac agc ttt gac agc cgc cat ttc ggc ttt     965
   Leu Gly Asp Asn Arg Ile His Ser Phe Asp Ser Arg His Phe Gly Phe
   140                 145                 150                 155
   gtt tca gaa cgg gac atc gtc ggg att gtg acg gaa aga att gat aag     1013
   Val Ser Glu Arg Asp Ile Val Gly Ile Val Thr Glu Arg Ile Asp Lys
                   160                 165                 170
   aag tgattggaga gtacggggga gagtaagcgg ccgaccaagg aatacgatta     1066
   Lys
   cgcaaatgac gagcccgaaa tgtcaattag tacaacagca tcaataatga cgcatttgct     1126
   aaatatgaaa attaaaaggc ccggatgatt ccgggctttt ttccgtacta cttcagctta     1186
   atggcattga tccgtttttg cagcttcgtt ttttcggcgg atgctttcag cttgcctact     1246
   gctgattgcg ctgctgattt tgtttttttc gttctctttt gttcggccgt tttgactttg     1306
   gcctttgctg cagaaacttt tttattatag gctgcagctg cttgcttttt cgcggtgttc     1366
   agcctttttt gcagatttga tttgtctttt ccggcgggaa gctcattgat cgctttttgc     1426
   gctttgccga ggtttgagtc tgttgccgct ttttcagctt tggcaacggc ctgctttgcg     1486
   gtcttcaaga ggcttgaacg gaccgcgt     1514

<210>  50
<211>  172
<212>  PRT
<213>  Bacillus licheniformis

<400>  50

Leu Lys Lys Arg Phe Leu Val Ile Ala Gly Val Leu Leu Val Ile Val
1               5                   10                  15

Val Gly Ile Gln Val Lys Asn Ala Val Phe Ile Glu Tyr Lys Val Glu
            20                  25                  30

Gly Val Ser Met Asn Pro Thr Phe Gln Glu Gly Asn Glu Leu Leu Ile
        35                  40                  45

Asn Arg Phe Ala His Arg Phe Lys Thr Ile Ser Arg Phe Asp Ile Val
    50                  55                  60

Leu Phe Lys Gly Pro Asp Lys Asp Ile Phe Ile Lys Arg Val Ile Gly
65                  70                  75                  80

Leu Pro Gly Glu Thr Leu Arg Tyr Glu Asp Asp Gln Leu Tyr Ile Asn
                85                  90                  95

Glu Glu Lys Ile Lys Glu Pro Tyr Leu Asp Asp Leu Lys Ala Val Thr
            100                 105                 110

Ala Gly Gly Asp Leu Thr Gly Asp Phe Thr Leu Gln Glu Val Thr Gly
        115                 120                 125

Glu Glu Lys Val Pro Glu Asn Glu Tyr Phe Val Leu Gly Asp Asn Arg
    130                 135                 140

Ile His Ser Phe Asp Ser Arg His Phe Gly Phe Val Ser Glu Arg Asp
145                 150                 155                 160

Ile Val Gly Ile Val Thr Glu Arg Ile Asp Lys Lys
                165                 170

<210>  51
<211>  1081
<212>  DNA
<213>  Bacillus licheniformis

<220>
<221>  CDS
<222>  (1)..(585)

<400>  51
aaa cag ctg tgg tgc atc ttt tta acc ctc gga gtc aga agt ttc ggc      48
Lys Gln Leu Trp Cys Ile Phe Leu Thr Leu Gly Val Arg Ser Phe Gly
1               5                   10                  15

ctg aca tgg aac tgg gcc aat acg ttc gca gac ggc gct tta gaa gaa      96
Leu Thr Trp Asn Trp Ala Asn Thr Phe Ala Asp Gly Ala Leu Glu Glu
            20                  25                  30

aga aac gcc ggc ttg act tct tac ggg gaa aga ctg att cgc aag gcg      144
Arg Asn Ala Gly Leu Thr Ser Tyr Gly Glu Arg Leu Ile Arg Lys Ala
        35                  40                  45

aac aaa aac cgg gca tgg aca gat ctc tcc cat tta tcc gaa aaa agc      192
Asn Lys Asn Arg Ala Trp Thr Asp Leu Ser His Leu Ser Glu Lys Ser
    50                  55                  60

ttt tgg ggt gct att gaa acg gcg gcg tat ccg atc gcc tct cat tcg     240
Phe Trp Gly Ala Ile Glu Thr Ala Ala Tyr Pro Ile Ala Ser His Ser
65                  70                  75                  80

aat gcc cgc agc ctt tgc cct cat ccg aga aat tta aaa gac gat caa     288
Asn Ala Arg Ser Leu Cys Pro His Pro Arg Asn Leu Lys Asp Asp Gln
                85                  90                  95

atc aaa gcg ctt att gat aag aac ggc gtc atc ggc ttg aca ttt gtc     336
Ile Lys Ala Leu Ile Asp Lys Asn Gly Val Ile Gly Leu Thr Phe Val
            100                 105                 110

cct gaa ttc gtg aga aac gga aaa acg cct gtg atc aaa gat atc ctc     384
Pro Glu Phe Val Arg Asn Gly Lys Thr Pro Val Ile Lys Asp Ile Leu
        115                 120                 125

tcg cat att gac cat gtc tgc tct tta cga ggc gag cgg cat atc gga     432
Ser His Ile Asp His Val Cys Ser Leu Arg Gly Glu Arg His Ile Gly
    130                 135                 140

ttc ggg tct gat ttt gac ggc atc gac cgt gtc att ccg aat ctt gaa     480
Phe Gly Ser Asp Phe Asp Gly Ile Asp Arg Val Ile Pro Asn Leu Glu
145                 150                 155                 160

gcc cac aaa gat tac ggg aat ctg atc gaa gcg ctg cag cgc tca tat     528
Ala His Lys Asp Tyr Gly Asn Leu Ile Glu Ala Leu Gln Arg Ser Tyr
                165                 170                 175

aca cca agt caa gtc gac gga ttt ctc ttt caa aat ttc atc agc cgc     576
Thr Pro Ser Gln Val Asp Gly Phe Leu Phe Gln Asn Phe Ile Ser Arg
            180                 185                 190

atc cca ttt taaaaaaata tcatacattt ttcagagggt gttcatctgt             625
Ile Pro Phe
        195

tttctccaga aggacactgg tgtttaccaa gactgattat cgtgctacaa tccttttgta     685

aagcgatttt acaaaggggg tctgttcatc ttggacggaa atatgaaagc gcttatcaag     745

aagccggggg agccgggcgc cagcttcgag ctcgtcccga ttccgaagat cgacaaacac     805

gatgtcctga tccaagtcac agccgcatcg atttgcggaa acgatgtcca tatttataat     865

tgggcatgaa tgcgccacac acctccggta aacccgacct aatgtctccg gacaccgaac     925

tttctcggcc tacaatcctt ccaatgcacc ccacatccgt ccaacaactc cccccagaac     985

ccacctagca ttccccacac cccccccact accctttcg ccccatcacc acctcccccc     1045

cctcccccta atattatccc tcctccatag aaattt                              1081

<210>  52
<211>  195
<212>  PRT
<213>  Bacillus licheniformis

<400>  52

Lys Gln Leu Trp Cys Ile Phe Leu Thr Leu Gly Val Arg Ser Phe Gly
1               5                   10                  15

Leu Thr Trp Asn Trp Ala Asn Thr Phe Ala Asp Gly Ala Leu Glu Glu
            20                  25                  30

Arg Asn Ala Gly Leu Thr Ser Tyr Gly Glu Arg Leu Ile Arg Lys Ala
        35                  40                  45

Asn Lys Asn Arg Ala Trp Thr Asp Leu Ser His Leu Ser Glu Lys Ser
    50                  55                  60

Phe Trp Gly Ala Ile Glu Thr Ala Ala Tyr Pro Ile Ala Ser His Ser
65                  70                  75                  80

Asn Ala Arg Ser Leu Cys Pro His Pro Arg Asn Leu Lys Asp Asp Gln
                85                  90                  95

Ile Lys Ala Leu Ile Asp Lys Asn Gly Val Ile Gly Leu Thr Phe Val
            100                 105                 110

Pro Glu Phe Val Arg Asn Gly Lys Thr Pro Val Ile Lys Asp Ile Leu
        115                 120                 125

Ser His Ile Asp His Val Cys Ser Leu Arg Gly Glu Arg His Ile Gly
    130                 135                 140

Phe Gly Ser Asp Phe Asp Gly Ile Asp Arg Val Ile Pro Asn Leu Glu
145                 150                 155                 160

Ala His Lys Asp Tyr Gly Asn Leu Ile Glu Ala Leu Gln Arg Ser Tyr
                165                 170                 175

Thr Pro Ser Gln Val Asp Gly Phe Leu Phe Gln Asn Phe Ile Ser Arg
            180                 185                 190

Ile Pro Phe
        195

<210> 53
<211> 3560
<212> DNA
<213> Bacillus licheniformis

<220>
<221> CDS
<222> (501)..(3122)

<400> 53
aagaaggaaa agccgaatta aagcttggcc atgccgaaca aattccttat ccggatcgtt 60

cttttcataa agtattttca gtccacacca tttatttttg gacggatttc aaacaggcat 120

taaaagaaat ttaccgtgtt ttacaggttg acggcacgct gtttctcgct gtgcatctgg 180

aagggcaaat gaaaaaatcg aaaaaaacaa aaggcttttc cttatactca gaagagcaaa 240

tcaagcaact gcttgaggaa agccatttca gagacatcac ggtacatatg aacaaaaatt 300

actgctgcat ttctgcggta aaatcatgaa ctttgtatga tcatcccttt caatacggaa 360

gggatttttt atgtttgata gagttgaaac tggatcttaa atatcatatt tttgattttt 420

aaagaaaagt attccattaa catagcaaac atggtttaat atcaaagtga acgtttttac 480

tatattttcc ggaggtattt atg aac aaa agg atc gtg aaa agt tca att gtt 533
Met Asn Lys Arg Ile Val Lys Ser Ser Ile Val
1               5                   10

ttc ttt ctc ctt gcc gca tta att ttt ggc cag ctg cct tta ccg aag 581
Phe Phe Leu Leu Ala Ala Leu Ile Phe Gly Gln Leu Pro Leu Pro Lys
            15                  20                  25

aca atg gca gcg gaa gac agc gtt ccg aat aac gaa acc aca ttg acc 629
Thr Met Ala Ala Glu Asp Ser Val Pro Asn Asn Glu Thr Thr Leu Thr
        30                  35                  40

agc gcg tcc cct gtt gaa gct tcg ttt caa agc gat gac gag gtg cat 677
Ser Ala Ser Pro Val Glu Ala Ser Phe Gln Ser Asp Asp Glu Val His
    45                  50                  55

tgg tat aaa gtc aat cct tca aat cag gaa atc gca aac tat acg cac 725
Trp Tyr Lys Val Asn Pro Ser Asn Gln Glu Ile Ala Asn Tyr Thr His
60                  65                  70                  75

ttc cgc gtc aaa ttg aaa tca gat gca gag ctg aac att tcc gtc tac 773
Phe Arg Val Lys Leu Lys Ser Asp Ala Glu Leu Asn Ile Ser Val Tyr
                80                  85                  90

tcc agc ctg gaa aat gca act ggt cat caa acg ttt gac cga tac aac 821
Ser Ser Leu Glu Asn Ala Thr Gly His Gln Thr Phe Asp Arg Tyr Asn
            95                  100                 105

ggc tac tcc tat gaa aat aat cct gct tta atc gat ttt ccg att gcc 869
Gly Tyr Ser Tyr Glu Asn Asn Pro Ala Leu Ile Asp Phe Pro Ile Ala
        110                 115                 120

tgg aaa ggt cct tac tac ata aaa gta gaa aat cac cat gat gag gaa 917
Trp Lys Gly Pro Tyr Tyr Ile Lys Val Glu Asn His His Asp Glu Glu
    125                 130                 135

aac gaa acc act tca att aca gat att tct tac acc atc agc tat gaa 965
Asn Glu Thr Thr Ser Ile Thr Asp Ile Ser Tyr Thr Ile Ser Tyr Glu
140                 145                 150                 155

ggc gtc acc ctg cct ccg tca atc caa gag gca gaa gaa gag tgt ccg 1013
Gly Val Thr Leu Pro Pro Ser Ile Gln Glu Ala Glu Glu Glu Cys Pro
                160                 165                 170

gca gaa tta agc gtt tcc gaa agg gaa aca ggc aag ggc ata tta aaa 1061
Ala Glu Leu Ser Val Ser Glu Arg Glu Thr Gly Lys Gly Ile Leu Lys
            175                 180                 185

cag tta aga acg atc agg gat gaa gtt ctt tca aaa act gaa aaa gga 1109
Gln Leu Arg Thr Ile Arg Asp Glu Val Leu Ser Lys Thr Glu Lys Gly
        190                 195                 200

aaa gag ctg tct tcc ctt tac tat aaa gca gct cca ttc atc agc gca 1157
Lys Glu Leu Ser Ser Leu Tyr Tyr Lys Ala Ala Pro Phe Ile Ser Ala
    205                 210                 215

aaa atg ctc ttt aac aaa tcg atg aga gac agt gtc tac aaa gac ctg 1205
Lys Met Leu Phe Asn Lys Ser Met Arg Asp Ser Val Tyr Lys Asp Leu
220                 225                 230                 235

gtg cag ctg aag ccg ctg ttt gca gat gtc gct aaa aac gga caa gtg 1253
Val Gln Leu Lys Pro Leu Phe Ala Asp Val Ala Lys Asn Gly Gln Val
                240                 245                 250

agc gca tac tcg att acg aat gat gat caa aaa gcg atc agt cgc ctg 1301

Ser Ala Tyr Ser Ile Thr Asn Asp Asp Gln Lys Ala Ile Ser Arg Leu
            255                 260                 265

tac gaa aca gct cgc gcg tcc gtt ccc gag ccg ttg aaa aaa cag ctg      1349
Tyr Glu Thr Ala Arg Ala Ser Val Pro Glu Pro Leu Lys Lys Gln Leu
        270                 275                 280

gat caa gtc gcg aaa gac atc ggc atc gaa caa tta aca ggc agc aaa      1397
Asp Gln Val Ala Lys Asp Ile Gly Ile Glu Gln Leu Thr Gly Ser Lys
    285                 290                 295

gta tcg gct gtg ctt gaa aaa gcg ggg atg gcg aca gct tca tca agc      1445
Val Ser Ala Val Leu Glu Lys Ala Gly Met Ala Thr Ala Ser Ser Ser
300                 305                 310                 315

gcg ccc gaa aac cgt tac atc gta aaa ttg aaa gaa ggc aaa aaa ccg      1493
Ala Pro Glu Asn Arg Tyr Ile Val Lys Leu Lys Glu Gly Lys Lys Pro
                320                 325                 330

gga tct ttc aaa tct aaa gcc caa tca tcc ggc gtc cag gca tta gag      1541
Gly Ser Phe Lys Ser Lys Ala Gln Ser Ser Gly Val Gln Ala Leu Glu
            335                 340                 345

ccc ctc ggt aaa agc aaa acg gca ttt aaa gat atg tac gtt gtg gaa      1589
Pro Leu Gly Lys Ser Lys Thr Ala Phe Lys Asp Met Tyr Val Val Glu
        350                 355                 360

atg aag gaa agc cgt tct tcc gga ttc aaa gcg gcg gca aag caa tat      1637
Met Lys Glu Ser Arg Ser Ser Gly Phe Lys Ala Ala Ala Lys Gln Tyr
    365                 370                 375

cag gcg gca gcc tcc aag atc gcc aag atg cct gaa gtg gaa ttc gtc      1685
Gln Ala Ala Ala Ser Lys Ile Ala Lys Met Pro Glu Val Glu Phe Val
380                 385                 390                 395

gaa cag gtt cag caa tat gaa gca ctg tca aga gac acc caa tat cca      1733
Glu Gln Val Gln Gln Tyr Glu Ala Leu Ser Arg Asp Thr Gln Tyr Pro
                400                 405                 410

tat caa tgg tcg ctc aaa aat aac ggc aaa aac cgt gct gcg aat gct      1781
Tyr Gln Trp Ser Leu Lys Asn Asn Gly Lys Asn Arg Ala Ala Asn Ala
            415                 420                 425

gac ata caa ttt gaa cag ctt cag aag ctg atg aaa ggc aaa aag ctg      1829
Asp Ile Gln Phe Glu Gln Leu Gln Lys Leu Met Lys Gly Lys Lys Leu
        430                 435                 440

aaa gat aca gta atc gcc gtc gtt gac aca ggc gtt gat cat acc ctt      1877
Lys Asp Thr Val Ile Ala Val Val Asp Thr Gly Val Asp His Thr Leu
    445                 450                 455

gcg gat tta agc ggc agc gtc aaa aaa gac gaa ggc tat aac tat gtc      1925
Ala Asp Leu Ser Gly Ser Val Lys Lys Asp Glu Gly Tyr Asn Tyr Val
460                 465                 470                 475

ggc cgc acg gcg gat gcg atg gat gac aat ggc cac ggc aca cac gtg      1973
Gly Arg Thr Ala Asp Ala Met Asp Asp Asn Gly His Gly Thr His Val
                480                 485                 490

tca ggc atc att gca gcc gcg caa gac aac cat ttt tcg atg gcg gga      2021
Ser Gly Ile Ile Ala Ala Ala Gln Asp Asn His Phe Ser Met Ala Gly
            495                 500                 505

atc aat gct tat gcc aaa att ctg cct gtc aaa gtg ctg gat tct tca      2069
Ile Asn Ala Tyr Ala Lys Ile Leu Pro Val Lys Val Leu Asp Ser Ser
        510                 515                 520

ggc agt gga gat acg gaa cag att gca aac ggc atc atc tat gcc gcc      2117

Gly Ser Gly Asp Thr Glu Gln Ile Ala Asn Gly Ile Ile Tyr Ala Ala
    525                 530                 535

gac cac ggt gca aaa gtc atc aat tta agt ctt ggc ggg cca tac agc      2165
Asp His Gly Ala Lys Val Ile Asn Leu Ser Leu Gly Gly Pro Tyr Ser
540                 545                 550                 555

cgg gtg atg gaa tat gcg ctt aaa tat gcg gct tct aaa aat gtg acg      2213
Arg Val Met Glu Tyr Ala Leu Lys Tyr Ala Ala Ser Lys Asn Val Thr
                560                 565                 570

atc gtt gcc gcc acc gga aat gac gga gta tcg gag att tcc tac cct      2261
Ile Val Ala Ala Thr Gly Asn Asp Gly Val Ser Glu Ile Ser Tyr Pro
            575                 580                 585

gca tct tcg aaa tat acg ctt tca gtc ggg gcg acc aat aat ctc gat      2309
Ala Ser Ser Lys Tyr Thr Leu Ser Val Gly Ala Thr Asn Asn Leu Asp
        590                 595                 600

ctt gtc tcg gac tac tcc aat tat gga aaa ggt ctc gat atg gtg gcg      2357
Leu Val Ser Asp Tyr Ser Asn Tyr Gly Lys Gly Leu Asp Met Val Ala
    605                 610                 615

ccg gga acc gat att cca agc ctc gtt ccg gac ggg aat gtc act tat      2405
Pro Gly Thr Asp Ile Pro Ser Leu Val Pro Asp Gly Asn Val Thr Tyr
620                 625                 630                 635

atg agc gga aca tcg atg gcg gcg ccg cac gtg gca gct gca gca gga      2453
Met Ser Gly Thr Ser Met Ala Ala Pro His Val Ala Ala Ala Ala Gly
                640                 645                 650

ctt ctt ttg tca cag aat ccg tcc ttg aaa cca aag caa atc gca agc      2501
Leu Leu Leu Ser Gln Asn Pro Ser Leu Lys Pro Lys Gln Ile Ala Ser
            655                 660                 665

cta ttg acc gag acg aca gca gat gtg gca ttt gaa gag cag gat aat      2549
Leu Leu Thr Glu Thr Thr Ala Asp Val Ala Phe Glu Glu Gln Asp Asn
        670                 675                 680

cca aac ccg gat tat gac ctg gat ata gaa ccg gct gca caa att ccc      2597
Pro Asn Pro Asp Tyr Asp Leu Asp Ile Glu Pro Ala Ala Gln Ile Pro
    685                 690                 695

gga tat gac ttc gtc tcc ggg tgg gga agg ctg aat gtt ttt cat gca      2645
Gly Tyr Asp Phe Val Ser Gly Trp Gly Arg Leu Asn Val Phe His Ala
700                 705                 710                 715

gcc agc gtt ttt gag ctg aac atg aag gtt cat ccc gtt tta aac cgc      2693
Ala Ser Val Phe Glu Leu Asn Met Lys Val His Pro Val Leu Asn Arg
                720                 725                 730

cat acg gca gtg aca ggc aca gcc aaa agc ggt gtg acg gtc aaa atc      2741
His Thr Ala Val Thr Gly Thr Ala Lys Ser Gly Val Thr Val Lys Ile
            735                 740                 745

ttg cga ggg aag caa gta ttg ggg acg ggc acg gcc gga aaa tca ggc      2789
Leu Arg Gly Lys Gln Val Leu Gly Thr Gly Thr Ala Gly Lys Ser Gly
        750                 755                 760

gcg ttt tca gtg aaa att ccg gcc cag aag gcg ggg caa gtt ctt cat      2837
Ala Phe Ser Val Lys Ile Pro Ala Gln Lys Ala Gly Gln Val Leu His
    765                 770                 775

gtc gcg gca tcg ggc cat cag gcg gaa acc tcg ctc aga acc gtc gtg      2885
Val Ala Ala Ser Gly His Gln Ala Glu Thr Ser Leu Arg Thr Val Val
780                 785                 790                 795

gaa aaa gcg ccg aaa aac ccg tcc gtc aaa cgc atc acg aac aaa gat      2933

Glu Lys Ala Pro Lys Asn Pro Ser Val Lys Arg Ile Thr Asn Lys Asp
                800                 805                 810

act gcc gta acg ggt aga acg gca gcc ggc tac acg atc aaa gtg aaa      2981
Thr Ala Val Thr Gly Arg Thr Ala Ala Gly Tyr Thr Ile Lys Val Lys
            815                 820                 825

aac gcg tgc aaa aaa gtg atc gcg caa ggc aga gcg gat gca tcc gtg      3029
Asn Ala Cys Lys Lys Val Ile Ala Gln Gly Arg Ala Asp Ala Ser Val
        830                 835                 840

agc gtt aaa gtg aaa atc aac aag caa aaa gat atg ccg ttt tgt atg      3077
Ser Val Lys Val Lys Ile Asn Lys Gln Lys Asp Met Pro Phe Cys Met
    845                 850                 855

tct ccg cat ctg ctg atg acc aca gag aaa gcg gcg atg tca aaa          3122
Ser Pro His Leu Leu Met Thr Thr Glu Lys Ala Ala Met Ser Lys
860                 865                 870

tgacggtggc tgacgtcatc ccgccaggcg ccccgaaagt ttatcaggtt tccgataaaa    3182

gtacggtgat tcagggaaaa acagaagcaa acgcgcaagt cagcgcaaaa gctaaaggaa    3242

agacgatcgc ttccggcaaa gcgaacggaa aaggtgaata caagctgaaa atcagcaggc    3302

aaaaagccgg aaccgtcatc ggtgtaacag caaaggataa agccggaaat gtcagcaaag    3362

cgacagccgt cactgtgctt gacaaaaccc cgccgtctgc gccgaaggtg aatccggtga    3422

caaacaagag cacggccgtt aaggggaaag cagaagcgaa cgccgccatc atcgtcaaat    3482

caggaaagaa aacgatcgga accggcaagg ccgataaaaa aggcgcgttt tttgtcaaaa    3542

taaaaaaaca aaaggcaa                                                  3560

<210>  54
<211>  874
<212>  PRT
<213>  Bacillus licheniformis

<400>  54

Met Asn Lys Arg Ile Val Lys Ser Ser Ile Val Phe Phe Leu Leu Ala
1               5                   10                  15

Ala Leu Ile Phe Gly Gln Leu Pro Leu Pro Lys Thr Met Ala Ala Glu
            20                  25                  30

Asp Ser Val Pro Asn Asn Glu Thr Thr Leu Thr Ser Ala Ser Pro Val
        35                  40                  45

Glu Ala Ser Phe Gln Ser Asp Asp Glu Val His Trp Tyr Lys Val Asn
    50                  55                  60

Pro Ser Asn Gln Glu Ile Ala Asn Tyr Thr His Phe Arg Val Lys Leu
65                  70                  75                  80

Lys Ser Asp Ala Glu Leu Asn Ile Ser Val Tyr Ser Ser Leu Glu Asn
                85                  90                  95

Ala Thr Gly His Gln Thr Phe Asp Arg Tyr Asn Gly Tyr Ser Tyr Glu

            100                 105                 110

Asn Asn Pro Ala Leu Ile Asp Phe Pro Ile Ala Trp Lys Gly Pro Tyr
        115                 120                 125

Tyr Ile Lys Val Glu Asn His His Asp Glu Glu Asn Glu Thr Thr Ser
    130                 135                 140

Ile Thr Asp Ile Ser Tyr Thr Ile Ser Tyr Glu Gly Val Thr Leu Pro
145                 150                 155                 160

Pro Ser Ile Gln Glu Ala Glu Glu Glu Cys Pro Ala Glu Leu Ser Val
                165                 170                 175

Ser Glu Arg Glu Thr Gly Lys Gly Ile Leu Lys Gln Leu Arg Thr Ile
            180                 185                 190

Arg Asp Glu Val Leu Ser Lys Thr Glu Lys Gly Lys Glu Leu Ser Ser
        195                 200                 205

Leu Tyr Tyr Lys Ala Ala Pro Phe Ile Ser Ala Lys Met Leu Phe Asn
    210                 215                 220

Lys Ser Met Arg Asp Ser Val Tyr Lys Asp Leu Val Gln Leu Lys Pro
225                 230                 235                 240

Leu Phe Ala Asp Val Ala Lys Asn Gly Gln Val Ser Ala Tyr Ser Ile
                245                 250                 255

Thr Asn Asp Asp Gln Lys Ala Ile Ser Arg Leu Tyr Glu Thr Ala Arg
            260                 265                 270

Ala Ser Val Pro Glu Pro Leu Lys Lys Gln Leu Asp Gln Val Ala Lys
        275                 280                 285

Asp Ile Gly Ile Glu Gln Leu Thr Gly Ser Lys Val Ser Ala Val Leu
    290                 295                 300

Glu Lys Ala Gly Met Ala Thr Ala Ser Ser Ser Ala Pro Glu Asn Arg
305                 310                 315                 320

Tyr Ile Val Lys Leu Lys Glu Gly Lys Lys Pro Gly Ser Phe Lys Ser
                325                 330                 335

Lys Ala Gln Ser Ser Gly Val Gln Ala Leu Glu Pro Leu Gly Lys Ser
            340                 345                 350

Lys Thr Ala Phe Lys Asp Met Tyr Val Val Glu Met Lys Glu Ser Arg
        355                 360                 365

Ser Ser Gly Phe Lys Ala Ala Ala Lys Gln Tyr Gln Ala Ala Ala Ser

370                 375                 380

Lys Ile Ala Lys Met Pro Glu Val Glu Phe Val Glu Gln Val Gln Gln
385                 390                 395                 400

Tyr Glu Ala Leu Ser Arg Asp Thr Gln Tyr Pro Tyr Gln Trp Ser Leu
                405                 410                 415

Lys Asn Asn Gly Lys Asn Arg Ala Ala Asn Ala Asp Ile Gln Phe Glu
            420                 425                 430

Gln Leu Gln Lys Leu Met Lys Gly Lys Lys Leu Lys Asp Thr Val Ile
        435                 440                 445

Ala Val Val Asp Thr Gly Val Asp His Thr Leu Ala Asp Leu Ser Gly
    450                 455                 460

Ser Val Lys Lys Asp Glu Gly Tyr Asn Tyr Val Gly Arg Thr Ala Asp
465                 470                 475                 480

Ala Met Asp Asp Asn Gly His Gly Thr His Val Ser Gly Ile Ile Ala
                485                 490                 495

Ala Ala Gln Asp Asn His Phe Ser Met Ala Gly Ile Asn Ala Tyr Ala
            500                 505                 510

Lys Ile Leu Pro Val Lys Val Leu Asp Ser Ser Gly Ser Gly Asp Thr
        515                 520                 525

Glu Gln Ile Ala Asn Gly Ile Ile Tyr Ala Ala Asp His Gly Ala Lys
    530                 535                 540

Val Ile Asn Leu Ser Leu Gly Gly Pro Tyr Ser Arg Val Met Glu Tyr
545                 550                 555                 560

Ala Leu Lys Tyr Ala Ala Ser Lys Asn Val Thr Ile Val Ala Ala Thr
                565                 570                 575

Gly Asn Asp Gly Val Ser Glu Ile Ser Tyr Pro Ala Ser Ser Lys Tyr
            580                 585                 590

Thr Leu Ser Val Gly Ala Thr Asn Asn Leu Asp Leu Val Ser Asp Tyr
        595                 600                 605

Ser Asn Tyr Gly Lys Gly Leu Asp Met Val Ala Pro Gly Thr Asp Ile
    610                 615                 620

Pro Ser Leu Val Pro Asp Gly Asn Val Thr Tyr Met Ser Gly Thr Ser
625                 630                 635                 640

Met Ala Ala Pro His Val Ala Ala Ala Ala Gly Leu Leu Leu Ser Gln

                645                 650                 655

Asn Pro Ser Leu Lys Pro Lys Gln Ile Ala Ser Leu Leu Thr Glu Thr
            660                 665                 670

Thr Ala Asp Val Ala Phe Glu Glu Gln Asp Asn Pro Asn Pro Asp Tyr
        675                 680                 685

Asp Leu Asp Ile Glu Pro Ala Ala Gln Ile Pro Gly Tyr Asp Phe Val
    690                 695                 700

Ser Gly Trp Gly Arg Leu Asn Val Phe His Ala Ala Ser Val Phe Glu
705                 710                 715                 720

Leu Asn Met Lys Val His Pro Val Leu Asn Arg His Thr Ala Val Thr
                725                 730                 735

Gly Thr Ala Lys Ser Gly Val Thr Val Lys Ile Leu Arg Gly Lys Gln
            740                 745                 750

Val Leu Gly Thr Gly Thr Ala Gly Lys Ser Gly Ala Phe Ser Val Lys
        755                 760                 765

Ile Pro Ala Gln Lys Ala Gly Gln Val Leu His Val Ala Ala Ser Gly
    770                 775                 780

His Gln Ala Glu Thr Ser Leu Arg Thr Val Val Glu Lys Ala Pro Lys
785                 790                 795                 800

Asn Pro Ser Val Lys Arg Ile Thr Asn Lys Asp Thr Ala Val Thr Gly
                805                 810                 815

Arg Thr Ala Ala Gly Tyr Thr Ile Lys Val Lys Asn Ala Cys Lys Lys
            820                 825                 830

Val Ile Ala Gln Gly Arg Ala Asp Ala Ser Val Ser Val Lys Val Lys
        835                 840                 845

Ile Asn Lys Gln Lys Asp Met Pro Phe Cys Met Ser Pro His Leu Leu
    850                 855                 860

Met Thr Thr Glu Lys Ala Ala Met Ser Lys
865                 870

<210> 55
<211> 1540
<212> DNA
<213> Bacillus licheniformis

<220>
<221> CDS
<222> (505)..(1050)

<400>  55
catcagaaaa gcactttctt tttctgcatt tgaaccggtc atcgtcagag ctgcgggttt      60

tatccgtttt atccgcttta gctgttttat ctgttttatt cgttttatct gttttattac     120

tatccgccga acgccgcgct ttcattgact gtctttcttt ttcccgctca gggctgtatg     180

gatcgtaatt gtcatagtac tcttcgtagc tgctcatttt gaggcaatca ccgcctttgg     240

gtcatgatat gataatgtat tataaagagg ggcgttttgc ctgtgcatat ataacggttt     300

ttcgtgctgt aaataagctg atcaactgac aaaattcaac attgaataaa ggatttttcg     360

tttcaaccgc taatattata tgtatcaacc atttttttac ggctgcgcct gccggtgctt     420

gcggaaagca ttcagacgga cggcctgata tgatagaagg agcggaatgc cgatgaaacg     480

atccggaagt gaaggagagt actt atg aaa aat agt aga aaa aaa gag atc       531
                           Met Lys Asn Ser Arg Lys Lys Glu Ile
                           1               5

tta tcg tgg gtg aaa aca ctt gtc atc gcc gca gcg ctt gtg atg gtt      579
Leu Ser Trp Val Lys Thr Leu Val Ile Ala Ala Ala Leu Val Met Val
10                  15                  20                  25

tgc cgc tat ttt ctg ttt acg ccg tca acc gtt ttg gga gat tca atg      627
Cys Arg Tyr Phe Leu Phe Thr Pro Ser Thr Val Leu Gly Asp Ser Met
                30                  35                  40

tat ccg act tta gag gac ggc aac atg gtc atg gtt agt aag atc agc      675
Tyr Pro Thr Leu Glu Asp Gly Asn Met Val Met Val Ser Lys Ile Ser
            45                  50                  55

gat att cag cgc ttt gat aaa att att ttt cac gcg cct gat tcg gat      723
Asp Ile Gln Arg Phe Asp Lys Ile Ile Phe His Ala Pro Asp Ser Asp
        60                  65                  70

gaa aac tat gtg aaa agg gtg atc ggg ctc ccg gga gat acg atc gaa      771
Glu Asn Tyr Val Lys Arg Val Ile Gly Leu Pro Gly Asp Thr Ile Glu
    75                  80                  85

atg aag gat gat gtc ctt tat ata aac gga aaa gca tac gat gaa cct      819
Met Lys Asp Asp Val Leu Tyr Ile Asn Gly Lys Ala Tyr Asp Glu Pro
90                  95                  100                 105

tat tta aag aaa aac aag caa aag ctg acg ctt cat gaa cat tta acc      867
Tyr Leu Lys Lys Asn Lys Gln Lys Leu Thr Leu His Glu His Leu Thr
                110                 115                 120

gat aat ttc aca ttg gag aaa tta aca ggt gaa caa aag gtg ccg gaa      915
Asp Asn Phe Thr Leu Glu Lys Leu Thr Gly Glu Gln Lys Val Pro Glu
            125                 130                 135

gat cac ttg ttc gtg atg ggg gat aac cgt caa aac agc aaa gac agc      963
Asp His Leu Phe Val Met Gly Asp Asn Arg Gln Asn Ser Lys Asp Ser
        140                 145                 150

cgc ttt ttc gga ttt atc aca atg gac tcc gtt gtc ggc aaa gtt gag     1011
Arg Phe Phe Gly Phe Ile Thr Met Asp Ser Val Val Gly Lys Val Glu
    155                 160                 165

ttc cgt tat ttc cct ttc aat gag atc ggc gga ata gaa tagaaaacaa      1060
Phe Arg Tyr Phe Pro Phe Asn Glu Ile Gly Gly Ile Glu
170                 175                 180

aaaggcttcc catgatacct aatgggaagt ctttttgttt gtcgcaaagt caccaaaagt   1120

ggtttaaacc aagccgctat gcttgagcac ggcggcaatt ctactattca cttaaattta 1180

aattagagcc agtttttgat atcgcagttg cagaattagg tctctctgaa ttataaggat 1240

tgcctttaat ttggtataaa agcctgttta atcgctgatt acccaaacaa aaataactaa 1300

aaaatcctcg atttaacaat ttagtgtatg gtactcttta aacatgtaat caaaaaacta 1360

tctttttcgc tccaaagcaa cataccgtat ttcttggttt gtttttagt tgttctaaat 1420

atttttttatt gttatcgcaa ttcttacgtt gtttgctgca atgattgaag tactatatta 1480

cataaattct gataggaggt cattatgttt aatagacaac agatagatca tctgcttgaa 1540

<210> 56
<211> 182
<212> PRT
<213> Bacillus licheniformis

<400> 56

Met Lys Asn Ser Arg Lys Lys Glu Ile Leu Ser Trp Val Lys Thr Leu
1               5                   10                  15

Val Ile Ala Ala Ala Leu Val Met Val Cys Arg Tyr Phe Leu Phe Thr
            20                  25                  30

Pro Ser Thr Val Leu Gly Asp Ser Met Tyr Pro Thr Leu Glu Asp Gly
        35                  40                  45

Asn Met Val Met Val Ser Lys Ile Ser Asp Ile Gln Arg Phe Asp Lys
    50                  55                  60

Ile Ile Phe His Ala Pro Asp Ser Asp Glu Asn Tyr Val Lys Arg Val
65                  70                  75                  80

Ile Gly Leu Pro Gly Asp Thr Ile Glu Met Lys Asp Asp Val Leu Tyr
                85                  90                  95

Ile Asn Gly Lys Ala Tyr Asp Glu Pro Tyr Leu Lys Lys Asn Lys Gln
            100                 105                 110

Lys Leu Thr Leu His Glu His Leu Thr Asp Asn Phe Thr Leu Glu Lys
        115                 120                 125

Leu Thr Gly Glu Gln Lys Val Pro Glu Asp His Leu Phe Val Met Gly
    130                 135                 140

Asp Asn Arg Gln Asn Ser Lys Asp Ser Arg Phe Phe Gly Phe Ile Thr
145                 150                 155                 160

Met Asp Ser Val Val Gly Lys Val Glu Phe Arg Tyr Phe Pro Phe Asn
                165                 170                 175

Glu Ile Gly Gly Ile Glu
            180

<210> 57
<211> 2212
<212> DNA
<213> Bacillus licheniformis

<220>
<221> CDS
<222> (2)..(604)

<400> 57
g ttt tca ggc tct gtt aca caa gga atc atc tca gga acc gaa cgc gct 49
Phe Ser Gly Ser Val Thr Gln Gly Ile Ile Ser Gly Thr Glu Arg Ala
1               5                   10                  15

gtg ccg gtc gat tcc aac gga gac gga cag ccg gac tgg aac gcc gaa 97
Val Pro Val Asp Ser Asn Gly Asp Gly Gln Pro Asp Trp Asn Ala Glu
            20                  25                  30

gta ctg cag acg gat gct gcc att aac ccg ggt aac agc gga ggc ggg 145
Val Leu Gln Thr Asp Ala Ala Ile Asn Pro Gly Asn Ser Gly Gly Gly
        35                  40                  45

ctg ttt aat att gat gga aaa gtc atc ggc att aat tcg atg aaa atc 193
Leu Phe Asn Ile Asp Gly Lys Val Ile Gly Ile Asn Ser Met Lys Ile
    50                  55                  60

gct gaa tca gcc gtc gaa ggc atc ggt ctg agc att ccg gcc aac ctt 241
Ala Glu Ser Ala Val Glu Gly Ile Gly Leu Ser Ile Pro Ala Asn Leu
65                  70                  75                  80

gcc att ccg gtt atc gaa gat ttg gaa acg tac ggt gaa gtg aga cgg 289
Ala Ile Pro Val Ile Glu Asp Leu Glu Thr Tyr Gly Glu Val Arg Arg
                85                  90                  95

ccg tat ctc gga att gag atg aaa tca ctt ggc gat atc gca agc tac 337
Pro Tyr Leu Gly Ile Glu Met Lys Ser Leu Gly Asp Ile Ala Ser Tyr
            100                 105                 110

cac tgg cag gaa acg ctt aaa ctg ccg aaa aac gta aca tca ggc gta 385
His Trp Gln Glu Thr Leu Lys Leu Pro Lys Asn Val Thr Ser Gly Val
        115                 120                 125

gtt gtg atg ggt gtt cag ccg gta tct cct gca ggc aga gcc ggt ctg 433
Val Val Met Gly Val Gln Pro Val Ser Pro Ala Gly Arg Ala Gly Leu
    130                 135                 140

aaa gag ctc gat gtc atc gtc gaa ttt aac ggt gac cgt gtt tac gat 481
Lys Glu Leu Asp Val Ile Val Glu Phe Asn Gly Asp Arg Val Tyr Asp
145                 150                 155                 160

atc gtt gat ctg cgt aaa aag ctt tac acc aaa aac gtc ggc gac aag 529
Ile Val Asp Leu Arg Lys Lys Leu Tyr Thr Lys Asn Val Gly Asp Lys
                165                 170                 175

gta aaa atc aaa tac ctg cgg ggc gga aaa gaa aag aca aca gaa gta 577
Val Lys Ile Lys Tyr Leu Arg Gly Gly Lys Glu Lys Thr Thr Glu Val
            180                 185                 190

aag ctg acc cga tcc caa tta gga agc tgataaagac agtctggccg 624
Lys Leu Thr Arg Ser Gln Leu Gly Ser
        195                 200

gtacggccgg gctgtttttt tattatgaag cgcccaacga atgatcgtca aaaggaaaaa 684

atgtgatttt tggagagtga aatgagattt tttctgagtg aataatcgaa tttttgggtg 744

tgaattccaa gtttttgccc tgcaaaaatt atagtttgta aaaaatggac aaagcatcat 804
attgcgtttc aattttgcgt acgatatgat gactttaata ttctgacaaa tggccgattt 864
aatgcaggat ttaaagatca gggcggaaag ggagatagga tgagtcagca tgcacttgta 924
ttacaatctg attttggcct tgatgacggg gcggtaaatg cgatgtacgg tgtggcttat 984
tcggttgaca gttcaattcg gatttttgat ttgacgcaca acattcccgt ttttcacatt 1044
tgtgaagctt cctatcggct gctgcagtcc gtttcttatt ggcctgaagg cactgtcttc 1104
gtgtcggtcg tcgatccagg ggtcggctca gaacgccgca gcgtcgcagt cagaacctct 1164
tccaatcaat atatcatcac accagacaac ggaacactca cccacatcag ccggcaaaat 1224
ggaattgccg aggcgcgcta tcttgatgaa gcgcaaaaca ggcttccaaa atcaggcgaa 1284
tcacatacat tccatggacg cgacatttac gcctacaccg gggcaagact ggcagccggc 1344
gctctcagct tcgacaggat cggtccggaa gtttctatag attccatcgt caaactgccg 1404
gtcatggaag cgtacatcga aaatgattgg ataaccggaa cgatcgatat tttagatgtc 1464
cggtttggaa acctttggac gaatatcagc cggacgctct tcaagtcact tgatgttcaa 1524
tacggagatc cagtagaagt cgtcatcaag catgaagaca caaaagtcta tcagcaaacg 1584
ttggcatttg gccggtcgtt tgccgatatg cctgtaggca agccggttgt ttatatcaat 1644
tcattagacc gcttgggcat tgccctcaac caaggatcat ttgctaaaac ataccatatt 1704
ggcacaggca tcaactggcg gatttctctt cgaaaaagta tatagcgtta gacaaaagag 1764
cctgattttc cagtcaggct cttgcttttg tgtcattctt atgacataaa aacggttgtc 1824
acacattttt cagctaatcg tgaagacagc aacctcttca aaaaatggtt cttttgtcac 1884
attggtacct aagtcatatt gtctaaaaca agagaaaaga atatattgag ggggtgtgcc 1944
gctgctatga aaaaattcat tcaattcatt tgtttggaaa acgagtgtcc ggaggaatga 2004
tatgaacgac aatgtgcagg ctgcgttgaa aacgctgtct aatgtaaaga gcagaagtat 2064
cttgtccgtt tttgtgctat gtctgatcac actgggggtt tacataccat actggtttct 2124
atccaggaaa aaaagcttcg atttgctgcc ttatcatgat attccctaca ccacgttaaa 2184
agctatcata ttttatcatt tattttct 2212

<210> 58
<211> 201
<212> PRT
<213> Bacillus licheniformis

<400> 58

Phe Ser Gly Ser Val Thr Gln Gly Ile Ile Ser Gly Thr Glu Arg Ala
1               5                   10                  15

Val Pro Val Asp Ser Asn Gly Asp Gly Gln Pro Asp Trp Asn Ala Glu
            20                  25                  30

Val Leu Gln Thr Asp Ala Ala Ile Asn Pro Gly Asn Ser Gly Gly Gly

35                  40                  45

Leu Phe Asn Ile Asp Gly Lys Val Ile Gly Ile Asn Ser Met Lys Ile
    50                  55                  60

Ala Glu Ser Ala Val Glu Gly Ile Gly Leu Ser Ile Pro Ala Asn Leu
65                  70                  75                  80

Ala Ile Pro Val Ile Glu Asp Leu Glu Thr Tyr Gly Glu Val Arg Arg
                85                  90                  95

Pro Tyr Leu Gly Ile Glu Met Lys Ser Leu Gly Asp Ile Ala Ser Tyr
            100                 105                 110

His Trp Gln Glu Thr Leu Lys Leu Pro Lys Asn Val Thr Ser Gly Val
        115                 120                 125

Val Val Met Gly Val Gln Pro Val Ser Pro Ala Gly Arg Ala Gly Leu
    130                 135                 140

Lys Glu Leu Asp Val Ile Val Glu Phe Asn Gly Asp Arg Val Tyr Asp
145                 150                 155                 160

Ile Val Asp Leu Arg Lys Lys Leu Tyr Thr Lys Asn Val Gly Asp Lys
                165                 170                 175

Val Lys Ile Lys Tyr Leu Arg Gly Gly Lys Glu Lys Thr Thr Glu Val
            180                 185                 190

Lys Leu Thr Arg Ser Gln Leu Gly Ser
        195                 200

<210>  59
<211>  1519
<212>  DNA
<213>  Bacillus licheniformis

<220>
<221>  CDS
<222>  (501)..(1016)

<400>  59
aaaaagctcg aaaggggttt atgatgaagg atattctgcg tgaaattgga atgattgccc      60

gagcattgga ttctataagc aatatcgaat ttaaagaaca tgacctgaca aaagggcaat     120

atttgtacct tgtccgaata tgcgaaaacc cgggaatcat tcaggagaag ctggctgaga     180

tgattaaagt agacaggacc accgcagccc gtgcaataaa aaaacttgaa atgaagggct     240

ttattgaacg gagagatgat gaacataaca aaaaaataaa aaactttttt ccgacggaaa     300

agggaaatga ggtatttcct tttataaaaa gagaaaatga ccattcgaat cgtgtggcat     360

tagagggatt ttccgagggg gaagcggaga tcatcttcaa tcttcttcaa agagtaagaa     420

aaaatgtaga aaaggactgg gaattcgtga aaaaggggaa caagagaaat tattgacacg 480

taaaggagcg gcagattgaa atg aca gta aat ata aaa agg tgt acc ctt gaa 533
Met Thr Val Asn Ile Lys Arg Cys Thr Leu Glu
1               5                   10

gac tta cac aag ctt cag gaa att ggt tat gaa aca ttt aat gag aca 581
Asp Leu His Lys Leu Gln Glu Ile Gly Tyr Glu Thr Phe Asn Glu Thr
            15                  20                  25

ttt aag cat cag aat tcg cca gaa aat atg aaa gcc tac ttg gac aag 629
Phe Lys His Gln Asn Ser Pro Glu Asn Met Lys Ala Tyr Leu Asp Lys
        30                  35                  40

gca ttt aac tta aaa caa tta gaa aaa gaa tta tcc aat agc tct tcg 677
Ala Phe Asn Leu Lys Gln Leu Glu Lys Glu Leu Ser Asn Ser Ser Ser
    45                  50                  55

caa ttc ttt ttt gtt tat ttt aac aac gaa atc gct gga tat tta aag 725
Gln Phe Phe Phe Val Tyr Phe Asn Asn Glu Ile Ala Gly Tyr Leu Lys
60                  65                  70                  75

gtc aac acc gat gag gcc cag tct gaa aaa atg ggt gat gaa tca ctt 773
Val Asn Thr Asp Glu Ala Gln Ser Glu Lys Met Gly Asp Glu Ser Leu
                80                  85                  90

gaa atc gag aga att tat atc aag aac aac ttt caa aaa cac ggc ctg 821
Glu Ile Glu Arg Ile Tyr Ile Lys Asn Asn Phe Gln Lys His Gly Leu
            95                  100                 105

ggc aaa tat ctg ttc aat aaa gct gtg gaa att gca aag gaa ctg aat 869
Gly Lys Tyr Leu Phe Asn Lys Ala Val Glu Ile Ala Lys Glu Leu Asn
        110                 115                 120

aaa aag aaa atc tgg ctg ggc gta tgg gaa aaa aac gaa aat gcg att 917
Lys Lys Lys Ile Trp Leu Gly Val Trp Glu Lys Asn Glu Asn Ala Ile
    125                 130                 135

gct ttt tat aag aaa atg ggg ttt gta caa acc ggc gcc cac tct ttt 965
Ala Phe Tyr Lys Lys Met Gly Phe Val Gln Thr Gly Ala His Ser Phe
140                 145                 150                 155

tat atg gga gat gaa gag caa acg gac ttt atc atg gcc aaa aca atc 1013
Tyr Met Gly Asp Glu Glu Gln Thr Asp Phe Ile Met Ala Lys Thr Ile
                160                 165                 170

cta taacttttta aaaggaggat gacgatgtat attccaaaat attttaaagt 1066
Leu

cgaaaatgct gatgaaattt tggattttgt ccaaaaaaac tcttttggca cgattgttac 1126

gacagaacaa gggaagccaa tcgcgactca tttgccttta gggctcaata aaaaaggcga 1186

tgattactat atcactggcc atatcgctta tggaaacccg caggtgggaa ctttggaaac 1246

ctgtaaagat gtgcttgtca tgtttcaggg gccgcacgca tatatttctt cttcctggta 1306

tgggcatgag gatgttccaa catggaatta tcaagccgtc catatatacg gtaaagcaag 1366

cattctagag agagatgaat taatagaaga attaacaatc atgatggaaa aatacgagaa 1426

gcatcgggaa aatccaattt tatgggataa cctttctcct caacttttgg agagacaact 1486

gaaagcgatt gtcgggttta agattaaggt gga 1519

<210> 60

<211>  172
<212>  PRT
<213>  Bacillus licheniformis

<400>  60

Met Thr Val Asn Ile Lys Arg Cys Thr Leu Glu Asp Leu His Lys Leu
1               5                   10                  15

Gln Glu Ile Gly Tyr Glu Thr Phe Asn Glu Thr Phe Lys His Gln Asn
            20                  25                  30

Ser Pro Glu Asn Met Lys Ala Tyr Leu Asp Lys Ala Phe Asn Leu Lys
        35                  40                  45

Gln Leu Glu Lys Glu Leu Ser Asn Ser Ser Ser Gln Phe Phe Phe Val
    50                  55                  60

Tyr Phe Asn Asn Glu Ile Ala Gly Tyr Leu Lys Val Asn Thr Asp Glu
65                  70                  75                  80

Ala Gln Ser Glu Lys Met Gly Asp Glu Ser Leu Glu Ile Glu Arg Ile
                85                  90                  95

Tyr Ile Lys Asn Asn Phe Gln Lys His Gly Leu Gly Lys Tyr Leu Phe
            100                 105                 110

Asn Lys Ala Val Glu Ile Ala Lys Glu Leu Asn Lys Lys Lys Ile Trp
        115                 120                 125

Leu Gly Val Trp Glu Lys Asn Glu Asn Ala Ile Ala Phe Tyr Lys Lys
    130                 135                 140

Met Gly Phe Val Gln Thr Gly Ala His Ser Phe Tyr Met Gly Asp Glu
145                 150                 155                 160

Glu Gln Thr Asp Phe Ile Met Ala Lys Thr Ile Leu
                165                 170

<210>  61
<211>  2072
<212>  DNA
<213>  Bacillus licheniformis

<220>
<221>  CDS
<222>  (501)..(2009)

<400>  61
tcgttgacaa agcggttaaa atcgcggaca agccggttga tcactgttgc ttgttctccg      60

gcatgtcttc tcactccggt tgcatcaaac atcgtaataa cggcaaatat tgcggaaacg     120

gcgaacaaag aagaatccat cccgtgatca agagctacac cggtcgagag cgccgtgacg     180

gccgctgagt gtgaactcgg cattccgccc gtgcttgtga caagccgcca atcccatttt     240

tttgagacga caaagtaaat gggaaccttg ataaactgag cgaaaaagat cgcggcaaag 300

ctcgccagca aaggaaagtt tgtgaggatt tccattttgc agaacatcct ttctataaag 360

aaaatagaga tgaattcaga aaagcacgca gttatttgct aaaatatatg atatgaaacc 420

ccatcgggaa aaaatgtttt tcctattata acattgttgt tttaatttta ctcactttgc 480

tgataaggag ctgtgaacgc atg ttt tat gcc ttt aaa gat ttc gaa aaa aaa 533
Met Phe Tyr Ala Phe Lys Asp Phe Glu Lys Lys
1               5                   10

gaa acg ctt tta atc gga ctg ttt aaa aaa agc cgg ctg tac ggt aag 581
Glu Thr Leu Leu Ile Gly Leu Phe Lys Lys Ser Arg Leu Tyr Gly Lys
            15                  20                  25

gct gaa gaa atc gac cgc ctt ttg aac ggc cag ctt tct cag ctg ctg 629
Ala Glu Glu Ile Asp Arg Leu Leu Asn Gly Gln Leu Ser Gln Leu Leu
        30                  35                  40

aaa gac ggg gat gta tct tcc aaa aaa gcg aaa gtg tct aaa ata ttt 677
Lys Asp Gly Asp Val Ser Ser Lys Lys Ala Lys Val Ser Lys Ile Phe
    45                  50                  55

acc cct tcg ctt cag gga gtc aaa cgc att tat atc gtc gga ttg ggc 725
Thr Pro Ser Leu Gln Gly Val Lys Arg Ile Tyr Ile Val Gly Leu Gly
60                  65                  70                  75

cgt gaa gcg gaa ttt acc ttc gag gat gcg aag cag tgc ttt gcc gaa 773
Arg Glu Ala Glu Phe Thr Phe Glu Asp Ala Lys Gln Cys Phe Ala Glu
                80                  85                  90

gct gtt cag ctg att cac aaa gat cga aag cag gag tta acc gtc atg 821
Ala Val Gln Leu Ile His Lys Asp Arg Lys Gln Glu Leu Thr Val Met
            95                  100                 105

ctc gac agc ttc gta tcc gaa gaa gtc cca gct gct gac gcc gca cat 869
Leu Asp Ser Phe Val Ser Glu Glu Val Pro Ala Ala Asp Ala Ala His
        110                 115                 120

gca ttg gct gaa tcg tgc atg ctg tca tgc tat gaa gtg cag gat tac 917
Ala Leu Ala Glu Ser Cys Met Leu Ser Cys Tyr Glu Val Gln Asp Tyr
    125                 130                 135

aag cac aga tca aat gtg cct gat caa tgt ctg caa agc gtc tat gtt 965
Lys His Arg Ser Asn Val Pro Asp Gln Cys Leu Gln Ser Val Tyr Val
140                 145                 150                 155

ttg aca gac cat gac ctg aag gag att cag gcg agt ctg cat gtc gga 1013
Leu Thr Asp His Asp Leu Lys Glu Ile Gln Ala Ser Leu His Val Gly
                160                 165                 170

cag gtg tac gga aat gcg acg aat tcg gcg aga acc ctc gtg aac atg 1061
Gln Val Tyr Gly Asn Ala Thr Asn Ser Ala Arg Thr Leu Val Asn Met
            175                 180                 185

ccg ggg aac atg ctg aca gcg gcg gat ctc gcg tca tat gcc gct gaa 1109
Pro Gly Asn Met Leu Thr Ala Ala Asp Leu Ala Ser Tyr Ala Ala Glu
        190                 195                 200

ttg gcg gcg aaa tac gaa ttt gaa tgt gaa att ctt gaa aag gct gaa 1157
Leu Ala Ala Lys Tyr Glu Phe Glu Cys Glu Ile Leu Glu Lys Ala Glu
    205                 210                 215

atg gaa gag ctc ggg atg ggc ggt ctc ctc gcg gtc aac caa gga tca 1205
Met Glu Glu Leu Gly Met Gly Gly Leu Leu Ala Val Asn Gln Gly Ser
220                 225                 230                 235

gaa gag ccg ccg aaa atg atc gtc ttg aaa tat caa gga aaa gaa aca 1253
Glu Glu Pro Pro Lys Met Ile Val Leu Lys Tyr Gln Gly Lys Glu Thr
                240                 245                 250

tgg gat gac gtc atc ggt tta gtc ggc aaa ggg att acg ttt gat acc 1301
Trp Asp Asp Val Ile Gly Leu Val Gly Lys Gly Ile Thr Phe Asp Thr
            255                 260                 265

gga ggc tat tcg att aag aca aag agc ggg att gtc ggc atg aag tcc 1349
Gly Gly Tyr Ser Ile Lys Thr Lys Ser Gly Ile Val Gly Met Lys Ser
        270                 275                 280

gat atg ggc gga gcc gcc agc gtt ttg gga gcg atg gaa gcg atc ggc 1397
Asp Met Gly Gly Ala Ala Ser Val Leu Gly Ala Met Glu Ala Ile Gly
    285                 290                 295

gaa ttg agg ccg gaa caa aac gtg ctt gcc gtg att ccg tcg act gat 1445
Glu Leu Arg Pro Glu Gln Asn Val Leu Ala Val Ile Pro Ser Thr Asp
300                 305                 310                 315

aac atg att tcg gga agc gca atg aag ccg gac gac gtc atc gtt tcc 1493
Asn Met Ile Ser Gly Ser Ala Met Lys Pro Asp Asp Val Ile Val Ser
                320                 325                 330

ttg agc ggc aag acg att gag atc ttg aat acg gac gcc gaa gga agg 1541
Leu Ser Gly Lys Thr Ile Glu Ile Leu Asn Thr Asp Ala Glu Gly Arg
            335                 340                 345

ctg gcg ctc gct gac gga ctg aca tat gcg aag cac cac gga gct tcc 1589
Leu Ala Leu Ala Asp Gly Leu Thr Tyr Ala Lys His His Gly Ala Ser
        350                 355                 360

gtg ctg atc gat gtc gcg aca ctg acc gga ggc gtc gtc gta gct ctg 1637
Val Leu Ile Asp Val Ala Thr Leu Thr Gly Gly Val Val Val Ala Leu
    365                 370                 375

gga acg gaa acg acg gga gcg atg aca aat cat gat ccg ctt tat cag 1685
Gly Thr Glu Thr Thr Gly Ala Met Thr Asn His Asp Pro Leu Tyr Gln
380                 385                 390                 395

cag gtg aga cag gcg gct gaa gaa gcg gga gaa gcg att tgg cag ctt 1733
Gln Val Arg Gln Ala Ala Glu Glu Ala Gly Glu Ala Ile Trp Gln Leu
                400                 405                 410

ccg att act gaa aaa gac aaa aaa aga gta aaa aac agc caa atg gcc 1781
Pro Ile Thr Glu Lys Asp Lys Lys Arg Val Lys Asn Ser Gln Met Ala
            415                 420                 425

gat ctt aac aat tca ccg ggc agg gaa ggc cat gcg att atg gcc gga 1829
Asp Leu Asn Asn Ser Pro Gly Arg Glu Gly His Ala Ile Met Ala Gly
        430                 435                 440

acg ttc ctc ggc gaa ttc gcc gag cag acg cca tgg gtt cac ctt gac 1877
Thr Phe Leu Gly Glu Phe Ala Glu Gln Thr Pro Trp Val His Leu Asp
    445                 450                 455

atc gcc gga acg gca acg aca gct caa aac tca tgc ttt gga ccg aaa 1925
Ile Ala Gly Thr Ala Thr Thr Ala Gln Asn Ser Cys Phe Gly Pro Lys
460                 465                 470                 475

ggc gga acg ggc gtg atg gtg aga acg ctt gtc acg ttt gtc gag cgg 1973
Gly Gly Thr Gly Val Met Val Arg Thr Leu Val Thr Phe Val Glu Arg
                480                 485                 490

gtt tcg gga aat ttt gta gtc ttt cat tcc ctt tgc tagaggggga 2019
Val Ser Gly Asn Phe Val Val Phe His Ser Leu Cys
            495                 500

tttttttatg gaaaaacgca gatgtctcat tgacgcatta taaacatggt gta          2072

<210>  62
<211>  503
<212>  PRT
<213>  Bacillus licheniformis

<400>  62

Met Phe Tyr Ala Phe Lys Asp Phe Glu Lys Lys Glu Thr Leu Leu Ile
1               5                   10                  15

Gly Leu Phe Lys Lys Ser Arg Leu Tyr Gly Lys Ala Glu Glu Ile Asp
            20                  25                  30

Arg Leu Leu Asn Gly Gln Leu Ser Gln Leu Leu Lys Asp Gly Asp Val
        35                  40                  45

Ser Ser Lys Lys Ala Lys Val Ser Lys Ile Phe Thr Pro Ser Leu Gln
    50                  55                  60

Gly Val Lys Arg Ile Tyr Ile Val Gly Leu Gly Arg Glu Ala Glu Phe
65                  70                  75                  80

Thr Phe Glu Asp Ala Lys Gln Cys Phe Ala Glu Ala Val Gln Leu Ile
                85                  90                  95

His Lys Asp Arg Lys Gln Glu Leu Thr Val Met Leu Asp Ser Phe Val
            100                 105                 110

Ser Glu Glu Val Pro Ala Ala Asp Ala Ala His Ala Leu Ala Glu Ser
        115                 120                 125

Cys Met Leu Ser Cys Tyr Glu Val Gln Asp Tyr Lys His Arg Ser Asn
    130                 135                 140

Val Pro Asp Gln Cys Leu Gln Ser Val Tyr Val Leu Thr Asp His Asp
145                 150                 155                 160

Leu Lys Glu Ile Gln Ala Ser Leu His Val Gly Gln Val Tyr Gly Asn
                165                 170                 175

Ala Thr Asn Ser Ala Arg Thr Leu Val Asn Met Pro Gly Asn Met Leu
            180                 185                 190

Thr Ala Ala Asp Leu Ala Ser Tyr Ala Ala Glu Leu Ala Ala Lys Tyr
        195                 200                 205

Glu Phe Glu Cys Glu Ile Leu Glu Lys Ala Glu Met Glu Glu Leu Gly
    210                 215                 220

Met Gly Gly Leu Leu Ala Val Asn Gln Gly Ser Glu Glu Pro Pro Lys

225                 230                 235                 240

Met Ile Val Leu Lys Tyr Gln Gly Lys Glu Thr Trp Asp Asp Val Ile
                245                 250                 255

Gly Leu Val Gly Lys Gly Ile Thr Phe Asp Thr Gly Gly Tyr Ser Ile
            260                 265                 270

Lys Thr Lys Ser Gly Ile Val Gly Met Lys Ser Asp Met Gly Gly Ala
        275                 280                 285

Ala Ser Val Leu Gly Ala Met Glu Ala Ile Gly Glu Leu Arg Pro Glu
    290                 295                 300

Gln Asn Val Leu Ala Val Ile Pro Ser Thr Asp Asn Met Ile Ser Gly
305                 310                 315                 320

Ser Ala Met Lys Pro Asp Asp Val Ile Val Ser Leu Ser Gly Lys Thr
                325                 330                 335

Ile Glu Ile Leu Asn Thr Asp Ala Glu Gly Arg Leu Ala Leu Ala Asp
            340                 345                 350

Gly Leu Thr Tyr Ala Lys His His Gly Ala Ser Val Leu Ile Asp Val
        355                 360                 365

Ala Thr Leu Thr Gly Gly Val Val Val Ala Leu Gly Thr Glu Thr Thr
    370                 375                 380

Gly Ala Met Thr Asn His Asp Pro Leu Tyr Gln Gln Val Arg Gln Ala
385                 390                 395                 400

Ala Glu Glu Ala Gly Glu Ala Ile Trp Gln Leu Pro Ile Thr Glu Lys
                405                 410                 415

Asp Lys Lys Arg Val Lys Asn Ser Gln Met Ala Asp Leu Asn Asn Ser
            420                 425                 430

Pro Gly Arg Glu Gly His Ala Ile Met Ala Gly Thr Phe Leu Gly Glu
        435                 440                 445

Phe Ala Glu Gln Thr Pro Trp Val His Leu Asp Ile Ala Gly Thr Ala
    450                 455                 460

Thr Thr Ala Gln Asn Ser Cys Phe Gly Pro Lys Gly Gly Thr Gly Val
465                 470                 475                 480

Met Val Arg Thr Leu Val Thr Phe Val Glu Arg Val Ser Gly Asn Phe
                485                 490                 495

Val Val Phe His Ser Leu Cys

            500

<210>  63
<211>  1544
<212>  DNA
<213>  Bacillus licheniformis

<220>
<221>  CDS
<222>  (501)..(1043)

<400>  63
ctacgcgaca ggcatgcggg taagcgaact ttgcagcctg aaggaatctg atttggatct      60

gtttttagat acggttttgg tccacgggaa aggcaggaag caaaggtata ttccatttgg     120

ttcattcgcc cgggaggctt tggatctgta tttgcaaaat ggaagacgca tccttctgtt     180

aaaagcgaaa gagccctgcc cttttatttt tttgaatcaa aggggcgggc ccttaacccc     240

gaggggggtt cgttacattt tgggagaact ggtcaagaag acttcgggta cattacatat     300

acatccgcat atgctccgcc atacatttgc cacccatttg ttaaatgagg gtgcggatct     360

gaggagcgtt caggaactgc ttgggcattc aaatctttct tcaacccagg tgtatacgca     420

tgtatctaaa gatatgctca gaaagacata tatgtctcat caccctcggg cacataaagg     480

gaaataaagg aggttctttt atg tca tct ttt cac gca acg acg ata ttt gcc     533
                      Met Ser Ser Phe His Ala Thr Thr Ile Phe Ala
                      1               5                   10

gtc cag cac aac gga aaa agc gcg atg gcg gga gac ggc cag gtt acg     581
Val Gln His Asn Gly Lys Ser Ala Met Ala Gly Asp Gly Gln Val Thr
            15                  20                  25

ttc ggt cag gct gtc gtc atg aag cat acc gca aga aag gta aga aaa     629
Phe Gly Gln Ala Val Val Met Lys His Thr Ala Arg Lys Val Arg Lys
        30                  35                  40

ctc ttc aac gga aag gtc att gcc ggt ttt gcc ggt tct gtt gca gac     677
Leu Phe Asn Gly Lys Val Ile Ala Gly Phe Ala Gly Ser Val Ala Asp
    45                  50                  55

gct ttc acg ctc ttt gaa atg ttt gaa gcg aag ctt gaa gaa tac aac     725
Ala Phe Thr Leu Phe Glu Met Phe Glu Ala Lys Leu Glu Glu Tyr Asn
60                  65                  70                  75

ggc aat ttg cag cgg gcg gca gtc gag ctt gca aaa gag tgg cga agc     773
Gly Asn Leu Gln Arg Ala Ala Val Glu Leu Ala Lys Glu Trp Arg Ser
                80                  85                  90

gat aaa gtc ctc aga aag ctt gag gcg atg ctg atc gtc atg aat gcc     821
Asp Lys Val Leu Arg Lys Leu Glu Ala Met Leu Ile Val Met Asn Ala
            95                  100                 105

gac agc atg ctg ctc gtt tcc gga aca ggc gaa gtg att gaa ccg gac     869
Asp Ser Met Leu Leu Val Ser Gly Thr Gly Glu Val Ile Glu Pro Asp
        110                 115                 120

gac ggc att ttg gcg atc ggc tca ggt gga aac tac gcg ctt gca gcc     917
Asp Gly Ile Leu Ala Ile Gly Ser Gly Gly Asn Tyr Ala Leu Ala Ala
    125                 130                 135

gga agg gcg ctg aaa cgt cat gcc ggc agc cag ctt gac gcg aaa gcg     965
Gly Arg Ala Leu Lys Arg His Ala Gly Ser Gln Leu Asp Ala Lys Ala

140                 145                 150                 155

att gcg agg gct tca ctt gaa acc gcg ggc gaa atc tgt gtg tat acg      1013
Ile Ala Arg Ala Ser Leu Glu Thr Ala Gly Glu Ile Cys Val Tyr Thr
            160                 165                 170

aac gat cag atc att gta gaa gaa ctt gaa tagaaaggat atgaggcata        1063
Asn Asp Gln Ile Ile Val Glu Glu Leu Glu
        175                 180

tggaaaaaaa gccgctgact cctaggcaga tcgtcgaaag gcttgatcaa tacatcgtcg    1123

gccagctcga tgcgaaaaaa gcggtcgccg tggcattgag gaaccgctac agaagaagtc    1183

ttcttgatga aaaactgcgc gaagaaatcg ttccgaaaaa catattgatg atgggcccga    1243

caggtgtggg aaaaaccgag attgcaagac gaatcgcgaa actcgtcggc gctccgttcg    1303

tcaaaatcga agcgacgaag ttcacggaag tcggttatgt gggcagagat gtggagtcga    1363

tggtcaggga tcttgtggaa acgtcagtcc gcctcgtcaa agaggaaaaa atgaacgaag    1423

tgaaaggcat cgctgaagaa aacgcaaata aacgcctcgt ccgcctgctt gtaccgggaa    1483

gaaaaaaaca gacaggtgcg aaaaatcctt ttgaaatgct gtttggcgga aatcaggatc    1543

a                                                                    1544

<210>  64
<211>  181
<212>  PRT
<213>  Bacillus licheniformis

<400>  64

Met Ser Ser Phe His Ala Thr Thr Ile Phe Ala Val Gln His Asn Gly
1               5                   10                  15

Lys Ser Ala Met Ala Gly Asp Gly Gln Val Thr Phe Gly Gln Ala Val
            20                  25                  30

Val Met Lys His Thr Ala Arg Lys Val Arg Lys Leu Phe Asn Gly Lys
        35                  40                  45

Val Ile Ala Gly Phe Ala Gly Ser Val Ala Asp Ala Phe Thr Leu Phe
    50                  55                  60

Glu Met Phe Glu Ala Lys Leu Glu Glu Tyr Asn Gly Asn Leu Gln Arg
65                  70                  75                  80

Ala Ala Val Glu Leu Ala Lys Glu Trp Arg Ser Asp Lys Val Leu Arg
                85                  90                  95

Lys Leu Glu Ala Met Leu Ile Val Met Asn Ala Asp Ser Met Leu Leu
            100                 105                 110

Val Ser Gly Thr Gly Glu Val Ile Glu Pro Asp Asp Gly Ile Leu Ala
        115                 120                 125

Ile Gly Ser Gly Gly Asn Tyr Ala Leu Ala Ala Gly Arg Ala Leu Lys
    130                 135                 140

Arg His Ala Gly Ser Gln Leu Asp Ala Lys Ala Ile Ala Arg Ala Ser
145                 150                 155                 160

Leu Glu Thr Ala Gly Glu Ile Cys Val Tyr Thr Asn Asp Gln Ile Ile
                165                 170                 175

Val Glu Glu Leu Glu
            180

<210>  65
<211>  2263
<212>  DNA
<213>  Bacillus licheniformis

<220>
<221>  CDS
<222>  (501)..(1763)

<400>  65
aatgaaagag gatgctgaaa aacgcgtgaa atccaacctg acactggaag cgatcgccaa      60

agcggaaaac cttcaagtca ctgacgaaga agtcgaagaa gagctttcta aaatggctga     120

agcatacaac atgcctgttg aaaacatcaa acaagcaatc ggttctacag acggaatgaa     180

agaggactta aaagttcgca aagcaattga ttttcttgta gaaaaccgtt aatatgtttc     240

ataatcatac tttatataaa acagggcgcg aatgtttcgt gccttgtttt atacaatttt     300

gacttatcat cttttctttg ggaaaggata gataatgtcg ttttgatcaa catacatatt     360

gaatatgccg gacggcaaac acaacgttat cgcctttttg gaaagtgtgg taaaatgcaa     420

tacataatgg aacgaatgtt ttcaagcgat gtttctttta acgaagaaac aggaatgaca     480

aggatataag gggtgaaaga atg ttt aaa ttc aac gag gaa aaa gga caa ttg     533
                     Met Phe Lys Phe Asn Glu Glu Lys Gly Gln Leu
                     1               5                   10

aaa tgc tcg ttt tgc ggt aaa aca caa gat cag gta cgc aag ctt gtc       581
Lys Cys Ser Phe Cys Gly Lys Thr Gln Asp Gln Val Arg Lys Leu Val
            15                  20                  25

gcc gga cca ggc gta tat ata tgt gac gag tgc atc gaa ctt tgc act       629
Ala Gly Pro Gly Val Tyr Ile Cys Asp Glu Cys Ile Glu Leu Cys Thr
        30                  35                  40

gaa att gta gaa gag gaa ctc ggc tct gaa gaa gaa gtg gag ttc aaa       677
Glu Ile Val Glu Glu Glu Leu Gly Ser Glu Glu Glu Val Glu Phe Lys
    45                  50                  55

gat gtt ccg aag cca aag gaa atc cgc gag att ctt gat gaa tac gtc       725
Asp Val Pro Lys Pro Lys Glu Ile Arg Glu Ile Leu Asp Glu Tyr Val
60                  65                  70                  75

atc ggc cag gac cag gca aaa aaa tcc ctt gcc gtg gcc gtc tac aat       773
Ile Gly Gln Asp Gln Ala Lys Lys Ser Leu Ala Val Ala Val Tyr Asn
                80                  85                  90

cat tac aag cgg atc aat tcg aac agc aaa gtc gac gac gtc gag ctg       821

His Tyr Lys Arg Ile Asn Ser Asn Ser Lys Val Asp Asp Val Glu Leu
            95                  100                 105

tca aaa agt aac att tcc atg atc gga ccg act gga agc gga aaa acg      869
Ser Lys Ser Asn Ile Ser Met Ile Gly Pro Thr Gly Ser Gly Lys Thr
        110                 115                 120

ctt ctt gct caa acg ctt gcc cgc atc ctg aat gtg ccg ttc gcc att      917
Leu Leu Ala Gln Thr Leu Ala Arg Ile Leu Asn Val Pro Phe Ala Ile
    125                 130                 135

gcg gat gcc acg tcg ctt act gaa gca ggc tat gtc ggt gaa gat gtg      965
Ala Asp Ala Thr Ser Leu Thr Glu Ala Gly Tyr Val Gly Glu Asp Val
140                 145                 150                 155

gag aac att ttg ctt aag ctg atc cag gca gct gac tac gat gtt gaa     1013
Glu Asn Ile Leu Leu Lys Leu Ile Gln Ala Ala Asp Tyr Asp Val Glu
                160                 165                 170

aaa gcg gaa aaa ggc att atc tac atc gat gaa atc gat aaa gtg gcc     1061
Lys Ala Glu Lys Gly Ile Ile Tyr Ile Asp Glu Ile Asp Lys Val Ala
            175                 180                 185

aga aag tcc gag aac cct tcg att aca cgc gat gta tca ggc gaa ggc     1109
Arg Lys Ser Glu Asn Pro Ser Ile Thr Arg Asp Val Ser Gly Glu Gly
        190                 195                 200

gtt cag cag gca ttg ctt aaa att ctt gaa ggt acg gtt gcc agc gtt     1157
Val Gln Gln Ala Leu Leu Lys Ile Leu Glu Gly Thr Val Ala Ser Val
    205                 210                 215

cct cca caa ggc gga aga aag cat cct cat caa gag ttc atc caa att     1205
Pro Pro Gln Gly Gly Arg Lys His Pro His Gln Glu Phe Ile Gln Ile
220                 225                 230                 235

gac aca acc aac atc ctg ttc att tgc ggc gga gct ttt gac gga atc     1253
Asp Thr Thr Asn Ile Leu Phe Ile Cys Gly Gly Ala Phe Asp Gly Ile
                240                 245                 250

gag cag atc atc aag cgc cgt ctc ggc cag aag gtc atc ggc ttc ggc     1301
Glu Gln Ile Ile Lys Arg Arg Leu Gly Gln Lys Val Ile Gly Phe Gly
            255                 260                 265

tct gac aac aag cat gaa gac ctt gaa aaa gaa gcg ctt ctt tcc aaa     1349
Ser Asp Asn Lys His Glu Asp Leu Glu Lys Glu Ala Leu Leu Ser Lys
        270                 275                 280

gtg ctt ccg gaa gac ttg ctt cgc ttc ggg ctg atc cct gaa ttc atc     1397
Val Leu Pro Glu Asp Leu Leu Arg Phe Gly Leu Ile Pro Glu Phe Ile
    285                 290                 295

ggc cgt ctc ccg atc atc gca agc ctt gag ccg ctg gat gaa aaa gcg     1445
Gly Arg Leu Pro Ile Ile Ala Ser Leu Glu Pro Leu Asp Glu Lys Ala
300                 305                 310                 315

ctg gtt gaa att tta aca aaa ccg aaa aac gcg ctt gtt aaa caa tat     1493
Leu Val Glu Ile Leu Thr Lys Pro Lys Asn Ala Leu Val Lys Gln Tyr
                320                 325                 330

aga aaa atg ctt gaa ttg gat gat gtg gag ctt gtg ttt gaa gac gaa     1541
Arg Lys Met Leu Glu Leu Asp Asp Val Glu Leu Val Phe Glu Asp Glu
            335                 340                 345

gcg ctc aca gaa atc gct aaa aaa gca att gaa cgt aaa aca gga gca     1589
Ala Leu Thr Glu Ile Ala Lys Lys Ala Ile Glu Arg Lys Thr Gly Ala
        350                 355                 360

cgc gga ctt cgt tcc att atc gaa gga atc atg ctc gac gtg atg ttt     1637

Arg Gly Leu Arg Ser Ile Ile Glu Gly Ile Met Leu Asp Val Met Phe
    365                 370                 375

gat ctg ccg tct cgc gag gat att gaa aaa tgc gtg atc aca ggc aaa      1685
Asp Leu Pro Ser Arg Glu Asp Ile Glu Lys Cys Val Ile Thr Gly Lys
380                 385                 390                 395

acc gtc act gac ggc gaa ccg ccg cgt ctc att atg aaa gac ggc acg      1733
Thr Val Thr Asp Gly Glu Pro Pro Arg Leu Ile Met Lys Asp Gly Thr
                400                 405                 410

gtc gtc aat aaa gac aaa aaa act tca gca taatgcagag aacctcctgg        1783
Val Val Asn Lys Asp Lys Lys Thr Ser Ala
            415                 420

cctaaaggag gttctttttt tgcatccgga aaccgtcggc atcccggagc agagtgacgg    1843

cttcaaaagc aaaataatcc ctttctgtac gtttattccc tccctgatca ggaaatacta    1903

tctgacatac taaagatacg aaaccatttc aggagggacc atcagttgag ctggacgagc    1963

attgcacttt ttgtacaact atttttcggg atcatcatcg gattatattt ttggaatttg    2023

ctcaaaaatc aaagaaccca aaaggttacg atagacagag agtccaaaaa agagatggaa    2083

cagctccgga aaatgagatc aatccattta tcagaacctt tatcggaaaa ggtgaggccg    2143

acggctttta aagacatcgt cggccaggag gacggcatta aagcgctgaa agccgccctt    2203

tgcggaccga atccgcagca tgtcattatt tacggccctc caggtgtggg aaaaacggca    2263

<210>  66
<211>  421
<212>  PRT
<213>  Bacillus licheniformis

<400>  66

Met Phe Lys Phe Asn Glu Glu Lys Gly Gln Leu Lys Cys Ser Phe Cys
1               5                   10                  15

Gly Lys Thr Gln Asp Gln Val Arg Lys Leu Val Ala Gly Pro Gly Val
            20                  25                  30

Tyr Ile Cys Asp Glu Cys Ile Glu Leu Cys Thr Glu Ile Val Glu Glu
        35                  40                  45

Glu Leu Gly Ser Glu Glu Glu Val Glu Phe Lys Asp Val Pro Lys Pro
    50                  55                  60

Lys Glu Ile Arg Glu Ile Leu Asp Glu Tyr Val Ile Gly Gln Asp Gln
65                  70                  75                  80

Ala Lys Lys Ser Leu Ala Val Ala Val Tyr Asn His Tyr Lys Arg Ile
                85                  90                  95

Asn Ser Asn Ser Lys Val Asp Asp Val Glu Leu Ser Lys Ser Asn Ile
            100                 105                 110

Ser Met Ile Gly Pro Thr Gly Ser Gly Lys Thr Leu Leu Ala Gln Thr

115                 120                 125

Leu Ala Arg Ile Leu Asn Val Pro Phe Ala Ile Ala Asp Ala Thr Ser
    130                 135                 140

Leu Thr Glu Ala Gly Tyr Val Gly Glu Asp Val Glu Asn Ile Leu Leu
145                 150                 155                 160

Lys Leu Ile Gln Ala Ala Asp Tyr Asp Val Glu Lys Ala Glu Lys Gly
                165                 170                 175

Ile Ile Tyr Ile Asp Glu Ile Asp Lys Val Ala Arg Lys Ser Glu Asn
            180                 185                 190

Pro Ser Ile Thr Arg Asp Val Ser Gly Glu Gly Val Gln Gln Ala Leu
        195                 200                 205

Leu Lys Ile Leu Glu Gly Thr Val Ala Ser Val Pro Pro Gln Gly Gly
    210                 215                 220

Arg Lys His Pro His Gln Glu Phe Ile Gln Ile Asp Thr Thr Asn Ile
225                 230                 235                 240

Leu Phe Ile Cys Gly Gly Ala Phe Asp Gly Ile Glu Gln Ile Ile Lys
                245                 250                 255

Arg Arg Leu Gly Gln Lys Val Ile Gly Phe Gly Ser Asp Asn Lys His
            260                 265                 270

Glu Asp Leu Glu Lys Glu Ala Leu Leu Ser Lys Val Leu Pro Glu Asp
        275                 280                 285

Leu Leu Arg Phe Gly Leu Ile Pro Glu Phe Ile Gly Arg Leu Pro Ile
    290                 295                 300

Ile Ala Ser Leu Glu Pro Leu Asp Glu Lys Ala Leu Val Glu Ile Leu
305                 310                 315                 320

Thr Lys Pro Lys Asn Ala Leu Val Lys Gln Tyr Arg Lys Met Leu Glu
                325                 330                 335

Leu Asp Asp Val Glu Leu Val Phe Glu Asp Glu Ala Leu Thr Glu Ile
            340                 345                 350

Ala Lys Lys Ala Ile Glu Arg Lys Thr Gly Ala Arg Gly Leu Arg Ser
        355                 360                 365

Ile Ile Glu Gly Ile Met Leu Asp Val Met Phe Asp Leu Pro Ser Arg
    370                 375                 380

Glu Asp Ile Glu Lys Cys Val Ile Thr Gly Lys Thr Val Thr Asp Gly

385                 390                 395                 400

Glu Pro Pro Arg Leu Ile Met Lys Asp Gly Thr Val Val Asn Lys Asp
            405                 410                 415

Lys Lys Thr Ser Ala
            420

<210>  67
<211>  2653
<212>  DNA
<213>  Bacillus licheniformis

<220>
<221>  CDS
<222>  (501)..(2153)

<400>  67
gttgaaattt taacaaaacc gaaaaacgcg cttgttaaac aatatagaaa aatgcttgaa      60

ttggatgatg tggagcttgt gtttgaagac gaagcgctca cagaaatcgc taaaaaagca     120

attgaacgta aaacaggagc acgcggactt cgttccatta tcgaaggaat catgctcgac     180

gtgatgtttg atctgccgtc tcgcgaggat attgaaaaat gcgtgatcac aggcaaaacc     240

gtcactgacg gcgaaccgcc gcgtctcatt atgaaagacg gcacggtcgt caataaagac     300

aaaaaaactt cagcataatg cagagaacct cctggcctaa aggaggttct tttttgcat      360

ccggaaaccg tcggcatccc ggagcagagt gacggcttca aaagcaaaat aatccctttc     420

tgtacgttta ttccctccct gatcaggaaa tactatctga catactaaag atacgaaacc     480

atttcaggag ggaccatcag ttg agc tgg acg agc att gca ctt ttt gta caa     533
                      Leu Ser Trp Thr Ser Ile Ala Leu Phe Val Gln
                      1               5                   10

cta ttt ttc ggg atc atc atc gga tta tat ttt tgg aat ttg ctc aaa       581
Leu Phe Phe Gly Ile Ile Ile Gly Leu Tyr Phe Trp Asn Leu Leu Lys
            15                  20                  25

aat caa aga acc caa aag gtt acg ata gac aga gag tcc aaa aaa gag       629
Asn Gln Arg Thr Gln Lys Val Thr Ile Asp Arg Glu Ser Lys Lys Glu
        30                  35                  40

atg gaa cag ctc cgg aaa atg aga tca atc cat tta tca gaa cct tta       677
Met Glu Gln Leu Arg Lys Met Arg Ser Ile His Leu Ser Glu Pro Leu
    45                  50                  55

tcg gaa aag gtg agg ccg acg gct ttt aaa gac atc gtc ggc cag gag       725
Ser Glu Lys Val Arg Pro Thr Ala Phe Lys Asp Ile Val Gly Gln Glu
60                  65                  70                  75

gac ggc att aaa gcg ctg aaa gcc gcc ctt tgc gga ccg aat ccg cag       773
Asp Gly Ile Lys Ala Leu Lys Ala Ala Leu Cys Gly Pro Asn Pro Gln
                80                  85                  90

cat gtc att att tac ggc cct cca ggt gtg gga aaa acg gca gct gca       821
His Val Ile Ile Tyr Gly Pro Pro Gly Val Gly Lys Thr Ala Ala Ala
            95                  100                 105

agg ctt gtc ctt gaa gaa gcg aaa aag aac aag cgc tca ccg ttc cag       869
Arg Leu Val Leu Glu Glu Ala Lys Lys Asn Lys Arg Ser Pro Phe Gln

110                 115                 120

gaa cat tcc gcc ttt gtc gag ctt gac gcg aca acg gcc cgt ttc gac     917
Glu His Ser Ala Phe Val Glu Leu Asp Ala Thr Thr Ala Arg Phe Asp
    125                 130                 135

gag cgg gga atc gcc gac ccg ttg atc ggt tcg gtc cac gat ccg att     965
Glu Arg Gly Ile Ala Asp Pro Leu Ile Gly Ser Val His Asp Pro Ile
140                 145                 150                 155

tat cag ggc gcc gga gcg atg ggg cag gcc gga att ccc cag cca aag     1013
Tyr Gln Gly Ala Gly Ala Met Gly Gln Ala Gly Ile Pro Gln Pro Lys
                160                 165                 170

cag gga gcc gtc acg cat gca cac ggt gga gta tta ttc atc gat gaa     1061
Gln Gly Ala Val Thr His Ala His Gly Gly Val Leu Phe Ile Asp Glu
            175                 180                 185

atc gga gag ctt cat ccg att caa atg aat aaa atg ctg aag gtg ctc     1109
Ile Gly Glu Leu His Pro Ile Gln Met Asn Lys Met Leu Lys Val Leu
        190                 195                 200

gaa gat cgg aaa gtt ttt ttg gaa agc gcc tat tac agt gaa gaa aac     1157
Glu Asp Arg Lys Val Phe Leu Glu Ser Ala Tyr Tyr Ser Glu Glu Asn
    205                 210                 215

acg caa atc ccc aag cat att cac gac att ttt caa aac ggg ctg ccg     1205
Thr Gln Ile Pro Lys His Ile His Asp Ile Phe Gln Asn Gly Leu Pro
220                 225                 230                 235

gcg gat ttc aga ctg atc ggc gcg acg aca agg atg cct gat gaa atc     1253
Ala Asp Phe Arg Leu Ile Gly Ala Thr Thr Arg Met Pro Asp Glu Ile
                240                 245                 250

ccg cct gcc att cgg tcg aga tgt ttg gaa gtg ttc ttc cgc gac ctt     1301
Pro Pro Ala Ile Arg Ser Arg Cys Leu Glu Val Phe Phe Arg Asp Leu
            255                 260                 265

gaa aag cac gaa tta aaa acg gtc gcc aaa aaa gcg gca gat aag att     1349
Glu Lys His Glu Leu Lys Thr Val Ala Lys Lys Ala Ala Asp Lys Ile
        270                 275                 280

caa aag aaa gtc gca gag gaa gga ctc gac ctg ttg acg cgg tat gcg     1397
Gln Lys Lys Val Ala Glu Glu Gly Leu Asp Leu Leu Thr Arg Tyr Ala
    285                 290                 295

aga aac ggc cgc gaa gtc gtc aat atg atg caa atc gcc gcc gga atg     1445
Arg Asn Gly Arg Glu Val Val Asn Met Met Gln Ile Ala Ala Gly Met
300                 305                 310                 315

gct ttg act gaa gag cgc gac gag gtg acg gtt gaa gat atc gaa tgg     1493
Ala Leu Thr Glu Glu Arg Asp Glu Val Thr Val Glu Asp Ile Glu Trp
                320                 325                 330

gtg atc cat tcg agc cag ctg aca ccg aaa tac gaa caa aaa atc gcc     1541
Val Ile His Ser Ser Gln Leu Thr Pro Lys Tyr Glu Gln Lys Ile Ala
            335                 340                 345

tct aaa ccg cag gtc ggc atc gtc aac gga ctt gcc gtc cac ggg cct     1589
Ser Lys Pro Gln Val Gly Ile Val Asn Gly Leu Ala Val His Gly Pro
        350                 355                 360

aac agc ggt tcg ctg ctc gaa att gag gtc aca gtc aat cag gcg gct     1637
Asn Ser Gly Ser Leu Leu Glu Ile Glu Val Thr Val Asn Gln Ala Ala
    365                 370                 375

gat aaa gga tcg att aat atc aca ggc att gca gaa gaa gag aat atc     1685
Asp Lys Gly Ser Ile Asn Ile Thr Gly Ile Ala Glu Glu Glu Asn Ile

380                 385                 390                 395

ggc aac cag tca aaa tcg atc cgc cgg aaa agc atg gcg aaa gga tcg     1733
Gly Asn Gln Ser Lys Ser Ile Arg Arg Lys Ser Met Ala Lys Gly Ser
                400                 405                 410

gtc gaa aac gtc atg act gta ttg cgg aca atg gga gtc aaa gct tcg     1781
Val Glu Asn Val Met Thr Val Leu Arg Thr Met Gly Val Lys Ala Ser
            415                 420                 425

gat tat gat att cac gtg aat ttc ccc ggc ggt atc ccg gtt gac gga     1829
Asp Tyr Asp Ile His Val Asn Phe Pro Gly Gly Ile Pro Val Asp Gly
        430                 435                 440

cca tct gca ggg att gca atg gcg gcg ggc ata ttc tcc gct att cat     1877
Pro Ser Ala Gly Ile Ala Met Ala Ala Gly Ile Phe Ser Ala Ile His
    445                 450                 455

aag att ccg ata gac cat act gtc gcc atg acg ggt gaa atc agc ctg     1925
Lys Ile Pro Ile Asp His Thr Val Ala Met Thr Gly Glu Ile Ser Leu
460                 465                 470                 475

aac ggc cgg gta aag ccg atc ggc ggc gtg att ccg aaa atc aaa gcg     1973
Asn Gly Arg Val Lys Pro Ile Gly Gly Val Ile Pro Lys Ile Lys Ala
                480                 485                 490

gcc aag cat gcg ggg gca aaa aca gtc att att ccg cac gaa aat gtt     2021
Ala Lys His Ala Gly Ala Lys Thr Val Ile Ile Pro His Glu Asn Val
            495                 500                 505

cag tcg att tta aag cgg ata gaa ggc ata cat atc gtt ccc gtc aaa     2069
Gln Ser Ile Leu Lys Arg Ile Glu Gly Ile His Ile Val Pro Val Lys
        510                 515                 520

acc ttg cag gaa gta ctc gac ctc gta ttg gtc aac cct cct tca gaa     2117
Thr Leu Gln Glu Val Leu Asp Leu Val Leu Val Asn Pro Pro Ser Glu
    525                 530                 535

atg ccc gac ggc gtt caa atg aaa aga gaa tcc gtt taaatcccca          2163
Met Pro Asp Gly Val Gln Met Lys Arg Glu Ser Val
540                 545                 550

tcgtttcaga tggggatttt tttcggggcg gtttctccga agccgggagc cggcatgttt   2223

tcttagcgga ggacatcttt tcctttttca gaactgaaag aaagggtata ctacaggaga   2283

ccctttttat ttcgccgggc ttacaattgt acaggaataa aaaaagtata ttataatggt   2343

tcatactaaa gttacggagg tgtcagtcaa aatggcagat gaaacgaaac gaaacatccc   2403

gctcctccct ttaagaggtt tactcgtcta tccgacgatg gttttgcacc ttgacgtcgg   2463

gcgtgaaaag tcggttcagg cacttgaaca ggcgatgatg aatgaccata tgatcttttt   2523

ggcaacgcaa aaggacatat ccatagacga accggatgaa gacgagattt ttacattcgg   2583

aacctatacg aaaatcaagc agatgctcaa gctgccgaac ggaacaatcc gcgttttggt   2643

ggaggggttg                                                          2653

<210>  68
<211>  551
<212>  PRT
<213>  Bacillus licheniformis

<400>  68

Leu Ser Trp Thr Ser Ile Ala Leu Phe Val Gln Leu Phe Phe Gly Ile
1               5                   10                  15

Ile Ile Gly Leu Tyr Phe Trp Asn Leu Leu Lys Asn Gln Arg Thr Gln
            20                  25                  30

Lys Val Thr Ile Asp Arg Glu Ser Lys Lys Glu Met Glu Gln Leu Arg
        35                  40                  45

Lys Met Arg Ser Ile His Leu Ser Glu Pro Leu Ser Glu Lys Val Arg
    50                  55                  60

Pro Thr Ala Phe Lys Asp Ile Val Gly Gln Glu Asp Gly Ile Lys Ala
65                  70                  75                  80

Leu Lys Ala Ala Leu Cys Gly Pro Asn Pro Gln His Val Ile Ile Tyr
                85                  90                  95

Gly Pro Pro Gly Val Gly Lys Thr Ala Ala Ala Arg Leu Val Leu Glu
            100                 105                 110

Glu Ala Lys Lys Asn Lys Arg Ser Pro Phe Gln Glu His Ser Ala Phe
        115                 120                 125

Val Glu Leu Asp Ala Thr Thr Ala Arg Phe Asp Glu Arg Gly Ile Ala
    130                 135                 140

Asp Pro Leu Ile Gly Ser Val His Asp Pro Ile Tyr Gln Gly Ala Gly
145                 150                 155                 160

Ala Met Gly Gln Ala Gly Ile Pro Gln Pro Lys Gln Gly Ala Val Thr
                165                 170                 175

His Ala His Gly Gly Val Leu Phe Ile Asp Glu Ile Gly Glu Leu His
            180                 185                 190

Pro Ile Gln Met Asn Lys Met Leu Lys Val Leu Glu Asp Arg Lys Val
        195                 200                 205

Phe Leu Glu Ser Ala Tyr Tyr Ser Glu Glu Asn Thr Gln Ile Pro Lys
    210                 215                 220

His Ile His Asp Ile Phe Gln Asn Gly Leu Pro Ala Asp Phe Arg Leu
225                 230                 235                 240

Ile Gly Ala Thr Thr Arg Met Pro Asp Glu Ile Pro Pro Ala Ile Arg
                245                 250                 255

Ser Arg Cys Leu Glu Val Phe Phe Arg Asp Leu Glu Lys His Glu Leu
            260                 265                 270

Lys Thr Val Ala Lys Lys Ala Ala Asp Lys Ile Gln Lys Lys Val Ala
        275                 280                 285

Glu Glu Gly Leu Asp Leu Leu Thr Arg Tyr Ala Arg Asn Gly Arg Glu
    290                 295                 300

Val Val Asn Met Met Gln Ile Ala Ala Gly Met Ala Leu Thr Glu Glu
305                 310                 315                 320

Arg Asp Glu Val Thr Val Glu Asp Ile Glu Trp Val Ile His Ser Ser
                325                 330                 335

Gln Leu Thr Pro Lys Tyr Glu Gln Lys Ile Ala Ser Lys Pro Gln Val
            340                 345                 350

Gly Ile Val Asn Gly Leu Ala Val His Gly Pro Asn Ser Gly Ser Leu
        355                 360                 365

Leu Glu Ile Glu Val Thr Val Asn Gln Ala Ala Asp Lys Gly Ser Ile
    370                 375                 380

Asn Ile Thr Gly Ile Ala Glu Glu Glu Asn Ile Gly Asn Gln Ser Lys
385                 390                 395                 400

Ser Ile Arg Arg Lys Ser Met Ala Lys Gly Ser Val Glu Asn Val Met
                405                 410                 415

Thr Val Leu Arg Thr Met Gly Val Lys Ala Ser Asp Tyr Asp Ile His
            420                 425                 430

Val Asn Phe Pro Gly Gly Ile Pro Val Asp Gly Pro Ser Ala Gly Ile
        435                 440                 445

Ala Met Ala Ala Gly Ile Phe Ser Ala Ile His Lys Ile Pro Ile Asp
    450                 455                 460

His Thr Val Ala Met Thr Gly Glu Ile Ser Leu Asn Gly Arg Val Lys
465                 470                 475                 480

Pro Ile Gly Gly Val Ile Pro Lys Ile Lys Ala Ala Lys His Ala Gly
                485                 490                 495

Ala Lys Thr Val Ile Ile Pro His Glu Asn Val Gln Ser Ile Leu Lys
            500                 505                 510

Arg Ile Glu Gly Ile His Ile Val Pro Val Lys Thr Leu Gln Glu Val
        515                 520                 525

Leu Asp Leu Val Leu Val Asn Pro Pro Ser Glu Met Pro Asp Gly Val
    530                 535                 540

Gln Met Lys Arg Glu Ser Val
545                 550

<210>  69
<211>  2230
<212>  DNA
<213>  Bacillus licheniformis

<220>
<221>  CDS
<222>  (501)..(1730)

<400>  69
cagagacaag atctttcatt ttacgaatca aacggtattc aggggggcga ttatttgcac      60

ccaaataaac ataaaaatcc ttcaatataa agactgctgg ctgatcatat ttttcaatga     120

acgccaaagc ctttaacggg tcttttgttt cggctgatgg aggcattcca ttgctttcaa     180

accctttggt taaactccaa acaaataagg ttcttggtgt tctaatcaat tctgtatccg     240

aaacaatatg actcagttga tctatgacac gttcttcttc ccatgtagaa atatacagga     300

acggaaagcg ggccctgaaa taattcgcta cttcttttct aaagtgatga tatccttcca     360

tttttctccc tttctattga cctatttttt attattatac taaatttttc cacaacagga     420

ataaaagatt tcaatcctcc cattcgcctt ctctaaatga taagattaaa ataaaaccaa     480

caattggaga tgaaggaaac atg aag aac aaa ttg att gaa cgc ctc att tct     533
                     Met Lys Asn Lys Leu Ile Glu Arg Leu Ile Ser
                     1               5                   10

tat gcg aaa gtg gat acg cag tca aat gaa aac agt cag acg acg cct     581
Tyr Ala Lys Val Asp Thr Gln Ser Asn Glu Asn Ser Gln Thr Thr Pro
            15                  20                  25

tcc act ccc ggg cag ctg gcg ctt gcc aat atg ctt gtc gaa gag ctg     629
Ser Thr Pro Gly Gln Leu Ala Leu Ala Asn Met Leu Val Glu Glu Leu
        30                  35                  40

aaa gag atc ggc atg aag gac gtc aca atc gat gaa aac ggc tat gtg     677
Lys Glu Ile Gly Met Lys Asp Val Thr Ile Asp Glu Asn Gly Tyr Val
    45                  50                  55

atg gcg acg ctt cct tcg aat aca gaa aaa gag gtg ccg acg atc ggt     725
Met Ala Thr Leu Pro Ser Asn Thr Glu Lys Glu Val Pro Thr Ile Gly
60                  65                  70                  75

ttc ttg gct cat gtg gat aca gca aca gat ttt acc gga aag aac gtc     773
Phe Leu Ala His Val Asp Thr Ala Thr Asp Phe Thr Gly Lys Asn Val
                80                  85                  90

aat ccg cag gtt atc gaa caa tac gac gga aag gat att gtg ctg aat     821
Asn Pro Gln Val Ile Glu Gln Tyr Asp Gly Lys Asp Ile Val Leu Asn
            95                  100                 105

gaa tcc ctc aat gtc gtc tta tcg ccc aag gaa ttc ccc gag cta gca     869
Glu Ser Leu Asn Val Val Leu Ser Pro Lys Glu Phe Pro Glu Leu Ala
        110                 115                 120

gat tat gcg gga cat acg ttg att aca acc gac gga acg act ttg ctc     917
Asp Tyr Ala Gly His Thr Leu Ile Thr Thr Asp Gly Thr Thr Leu Leu
    125                 130                 135

ggc gcc gac aat aaa gct ggg atc tct gaa atc atg acg gca atg gaa     965

Gly Ala Asp Asn Lys Ala Gly Ile Ser Glu Ile Met Thr Ala Met Glu
140                 145                 150                 155

tat tta att gcg cac ccc gaa atc aaa cac ggg aag atc aga gtc gct      1013
Tyr Leu Ile Ala His Pro Glu Ile Lys His Gly Lys Ile Arg Val Ala
                160                 165                 170

ttt aca cct gat gaa gag atc ggc aga ggg ccg cac aag ttt gac gtc      1061
Phe Thr Pro Asp Glu Glu Ile Gly Arg Gly Pro His Lys Phe Asp Val
            175                 180                 185

gag gcg ttc aat gcc aaa ttc gcg tat acg gtt gac ggc gga ccg ctt      1109
Glu Ala Phe Asn Ala Lys Phe Ala Tyr Thr Val Asp Gly Gly Pro Leu
        190                 195                 200

ggc gaa ctg cag tac gaa agc ttc aat gcc gca gcc gcg aaa atc act      1157
Gly Glu Leu Gln Tyr Glu Ser Phe Asn Ala Ala Ala Ala Lys Ile Thr
    205                 210                 215

tgt aaa gga acg aac gtc cac ccc ggc aca gca aaa gga aaa atg gtc      1205
Cys Lys Gly Thr Asn Val His Pro Gly Thr Ala Lys Gly Lys Met Val
220                 225                 230                 235

aat gcc gcc aaa atc gcg atg cag ttc cac gcc gca ttg ccg gaa aac      1253
Asn Ala Ala Lys Ile Ala Met Gln Phe His Ala Ala Leu Pro Glu Asn
                240                 245                 250

gaa gct cct gaa ttt aca gaa ggt tat gaa ggc ttt tat cat ctg ctt      1301
Glu Ala Pro Glu Phe Thr Glu Gly Tyr Glu Gly Phe Tyr His Leu Leu
            255                 260                 265

tca atc aag ggc gat gtt tct gaa acg agc ctc tct tat atc att aga      1349
Ser Ile Lys Gly Asp Val Ser Glu Thr Ser Leu Ser Tyr Ile Ile Arg
        270                 275                 280

gat ttt gac aga gac cga ttt aac gag aga aaa gac acc gtc caa aaa      1397
Asp Phe Asp Arg Asp Arg Phe Asn Glu Arg Lys Asp Thr Val Gln Lys
    285                 290                 295

atc gca aac aac ctt aaa gcg aaa tac ggc gaa aac agc gtc aca gtg      1445
Ile Ala Asn Asn Leu Lys Ala Lys Tyr Gly Glu Asn Ser Val Thr Val
300                 305                 310                 315

gac atg aat gat caa tat tac aac atg agg gaa aag atc gaa ccg gtc      1493
Asp Met Asn Asp Gln Tyr Tyr Asn Met Arg Glu Lys Ile Glu Pro Val
                320                 325                 330

aaa gag att gtc gac att gcc tat aaa gcg atg aaa aac ctt gat att      1541
Lys Glu Ile Val Asp Ile Ala Tyr Lys Ala Met Lys Asn Leu Asp Ile
            335                 340                 345

gaa ccg gtc gtt aaa ccg atc cgc ggc ggt aca gac ggc tct cag ctc      1589
Glu Pro Val Val Lys Pro Ile Arg Gly Gly Thr Asp Gly Ser Gln Leu
        350                 355                 360

tca tat atg ggg ctt cct tgt ccg aac att ttc aca ggc ggg gaa aac      1637
Ser Tyr Met Gly Leu Pro Cys Pro Asn Ile Phe Thr Gly Gly Glu Asn
    365                 370                 375

ttt cac gga aaa tac gag tat att tcc gcc gac aat atg gta aaa gcc      1685
Phe His Gly Lys Tyr Glu Tyr Ile Ser Ala Asp Asn Met Val Lys Ala
380                 385                 390                 395

gcg aat gtc atc gtg gaa att gtg aag ctg ttt gaa gaa aga gct          1730
Ala Asn Val Ile Val Glu Ile Val Lys Leu Phe Glu Glu Arg Ala
                400                 405                 410

taaacgaaaa aagcggaaag cggctgcaag acaaagcagc cgcttttttt tccagatcaa    1790

tctttatctg cgctttgctg aaattagaac atgttgcaag gcggtttctt tttggagcgg 1850

gataaagcca attaacatct caccgatgca aaaaattctg ttcatttggc ttcatcacct 1910

ttccaaatat ctttcaacgt ccagtaagtg aattcttcaa cccgtacgtt gcctttctca 1970

gaaaacagct caatccccgc tcttccttcc ttcggatata tgcggcttgt cattgtcgct 2030

tcaccatgat ttgcaaatac ttcaatagac gatctgtcga gaaacagatg caaagtgagc 2090

ttctcatccg cttcaagccg tacgtttctc acaccgtccc tcgctttgcc ggactttgaa 2150

cagtcgagcg tcagcttttg atcaatcaag ctgtacttga tggttgtttc ttcctgttca 2210

attccccgga tctttaagcc 2230

<210> 70
<211> 410
<212> PRT
<213> Bacillus licheniformis

<400> 70

Met Lys Asn Lys Leu Ile Glu Arg Leu Ile Ser Tyr Ala Lys Val Asp
1               5                   10                  15

Thr Gln Ser Asn Glu Asn Ser Gln Thr Thr Pro Ser Thr Pro Gly Gln
            20                  25                  30

Leu Ala Leu Ala Asn Met Leu Val Glu Glu Leu Lys Glu Ile Gly Met
        35                  40                  45

Lys Asp Val Thr Ile Asp Glu Asn Gly Tyr Val Met Ala Thr Leu Pro
    50                  55                  60

Ser Asn Thr Glu Lys Glu Val Pro Thr Ile Gly Phe Leu Ala His Val
65                  70                  75                  80

Asp Thr Ala Thr Asp Phe Thr Gly Lys Asn Val Asn Pro Gln Val Ile
                85                  90                  95

Glu Gln Tyr Asp Gly Lys Asp Ile Val Leu Asn Glu Ser Leu Asn Val
            100                 105                 110

Val Leu Ser Pro Lys Glu Phe Pro Glu Leu Ala Asp Tyr Ala Gly His
        115                 120                 125

Thr Leu Ile Thr Thr Asp Gly Thr Thr Leu Leu Gly Ala Asp Asn Lys
    130                 135                 140

Ala Gly Ile Ser Glu Ile Met Thr Ala Met Glu Tyr Leu Ile Ala His
145                 150                 155                 160

Pro Glu Ile Lys His Gly Lys Ile Arg Val Ala Phe Thr Pro Asp Glu
                165                 170                 175

Glu Ile Gly Arg Gly Pro His Lys Phe Asp Val Glu Ala Phe Asn Ala
            180                 185                 190

Lys Phe Ala Tyr Thr Val Asp Gly Gly Pro Leu Gly Glu Leu Gln Tyr
        195                 200                 205

Glu Ser Phe Asn Ala Ala Ala Ala Lys Ile Thr Cys Lys Gly Thr Asn
    210                 215                 220

Val His Pro Gly Thr Ala Lys Gly Lys Met Val Asn Ala Ala Lys Ile
225                 230                 235                 240

Ala Met Gln Phe His Ala Ala Leu Pro Glu Asn Glu Ala Pro Glu Phe
                245                 250                 255

Thr Glu Gly Tyr Glu Gly Phe Tyr His Leu Leu Ser Ile Lys Gly Asp
            260                 265                 270

Val Ser Glu Thr Ser Leu Ser Tyr Ile Ile Arg Asp Phe Asp Arg Asp
        275                 280                 285

Arg Phe Asn Glu Arg Lys Asp Thr Val Gln Lys Ile Ala Asn Asn Leu
    290                 295                 300

Lys Ala Lys Tyr Gly Glu Asn Ser Val Thr Val Asp Met Asn Asp Gln
305                 310                 315                 320

Tyr Tyr Asn Met Arg Glu Lys Ile Glu Pro Val Lys Glu Ile Val Asp
                325                 330                 335

Ile Ala Tyr Lys Ala Met Lys Asn Leu Asp Ile Glu Pro Val Val Lys
            340                 345                 350

Pro Ile Arg Gly Gly Thr Asp Gly Ser Gln Leu Ser Tyr Met Gly Leu
        355                 360                 365

Pro Cys Pro Asn Ile Phe Thr Gly Gly Glu Asn Phe His Gly Lys Tyr
    370                 375                 380

Glu Tyr Ile Ser Ala Asp Asn Met Val Lys Ala Ala Asn Val Ile Val
385                 390                 395                 400

Glu Ile Val Lys Leu Phe Glu Glu Arg Ala
                405                 410

<210>  71
<211>  2002
<212>  DNA
<213>  Bacillus licheniformis

<220>

<221>  CDS
<222>  (501)..(1502)

<400>  71
ttttattatg gggcattttt atcacagttg gtaaatagcg gatttgcccc agctattttt      60

gaaaatagcg aaaaaagacg tatttataaa ttgtcaacta attatggtga ctataaagtt     120

tatacgaagt atgtatcgaa gcaaagtaat aaaggtcaaa acagaatgtg gcattttcca     180

ttcaatttgg atgaagtaaa caacattcaa caagaccaat ctatcaatat gtttgctttt     240

atttgcgggg ttgaagattt agttaatagt gagatagtga ttttaagtag agaggaattt     300

aatcagtgta tcggtgtagg ttttaaaaca gaaaatcgac gtgtgtcggt taaatctgaa     360

aaaggtgcat ggaaccattt agtttatgga actggtatag aactaaaaga aactccactt     420

aaggttatta aaaacttgaa taagagatta agtgaatttt caatggtctc tacttgatga     480

taattaaaag aggtgattag gtg aat aat gaa tct aat ttt cta atg tac caa     533
                      Val Asn Asn Glu Ser Asn Phe Leu Met Tyr Gln
                      1               5                   10

acc gaa aat ggc gat acg aaa att caa gtc cgt tta gaa ggt gaa acc       581
Thr Glu Asn Gly Asp Thr Lys Ile Gln Val Arg Leu Glu Gly Glu Thr
            15                  20                  25

gtc tgg atg acg caa aag gca atg gca gag ctg ttt caa aag ggt gtt       629
Val Trp Met Thr Gln Lys Ala Met Ala Glu Leu Phe Gln Lys Gly Val
        30                  35                  40

cca aca ata aat gag cat att aaa aat ata tat gct gaa ggt gag tta       677
Pro Thr Ile Asn Glu His Ile Lys Asn Ile Tyr Ala Glu Gly Glu Leu
    45                  50                  55

aca gag gag gca act att cgg aaa aac cga ata gtt caa gtt gag ggt       725
Thr Glu Glu Ala Thr Ile Arg Lys Asn Arg Ile Val Gln Val Glu Gly
60                  65                  70                  75

tct cgt gaa gtt gaa cgt gaa gtg acc ttt tac aac ctt gaa gtt atc       773
Ser Arg Glu Val Glu Arg Glu Val Thr Phe Tyr Asn Leu Glu Val Ile
                80                  85                  90

att gca gtc ggc tat cgt gtt cga tct cat cgt ggt aca caa ttc cgt       821
Ile Ala Val Gly Tyr Arg Val Arg Ser His Arg Gly Thr Gln Phe Arg
            95                  100                 105

caa tgg gca aca gag cgt tta aat gaa tac atg gta aaa ggt ttt acg       869
Gln Trp Ala Thr Glu Arg Leu Asn Glu Tyr Met Val Lys Gly Phe Thr
        110                 115                 120

atg gat gat gaa cgc tta aaa gaa atg cgg aat att ggg gca gat tat       917
Met Asp Asp Glu Arg Leu Lys Glu Met Arg Asn Ile Gly Ala Asp Tyr
    125                 130                 135

ttt gat gaa tta ttg gaa cgt att cgt gat att cgt gct tcc gaa aga       965
Phe Asp Glu Leu Leu Glu Arg Ile Arg Asp Ile Arg Ala Ser Glu Arg
140                 145                 150                 155

cgt ttt tat tac aaa ata aca gat atc tat gct aca tct att gat tac      1013
Arg Phe Tyr Tyr Lys Ile Thr Asp Ile Tyr Ala Thr Ser Ile Asp Tyr
                160                 165                 170

gat ccg aat aca cca att gca aga gaa ttc ttt gca acc gtt caa aat      1061
Asp Pro Asn Thr Pro Ile Ala Arg Glu Phe Phe Ala Thr Val Gln Asn
            175                 180                 185

aaa ctc cat ttt gca atc cat gga cat act gct tcg gaa ctg ata atg     1109
Lys Leu His Phe Ala Ile His Gly His Thr Ala Ser Glu Leu Ile Met
        190                 195                 200

aaa cga gcg gat gca aca aag cca aac atg ggc tta acg agt tgg aaa     1157
Lys Arg Ala Asp Ala Thr Lys Pro Asn Met Gly Leu Thr Ser Trp Lys
    205                 210                 215

ggt gac aaa gtg cgt aaa cat gat gtc act gtg gcg aaa aat tat tta     1205
Gly Asp Lys Val Arg Lys His Asp Val Thr Val Ala Lys Asn Tyr Leu
220                 225                 230                 235

aca caa gaa gaa ctc agc gat tta aac cgt atc gtg aca atg tat tta     1253
Thr Gln Glu Glu Leu Ser Asp Leu Asn Arg Ile Val Thr Met Tyr Leu
                240                 245                 250

gac tat gca gaa aca caa gcg aaa aag aaa aag ccg atg tac atg aag     1301
Asp Tyr Ala Glu Thr Gln Ala Lys Lys Lys Lys Pro Met Tyr Met Lys
            255                 260                 265

gat tgg gca gaa aaa tta gat gca ttt tta gag ttt aat gaa cat gaa     1349
Asp Trp Ala Glu Lys Leu Asp Ala Phe Leu Glu Phe Asn Glu His Glu
        270                 275                 280

ata tta act aac gct ggg aaa att aaa gcc aaa gta gcg gaa caa ttt     1397
Ile Leu Thr Asn Ala Gly Lys Ile Lys Ala Lys Val Ala Glu Gln Phe
    285                 290                 295

gca aac gaa caa tat gaa gtg ttt cat caa caa cga tta gca gaa ccg     1445
Ala Asn Glu Gln Tyr Glu Val Phe His Gln Gln Arg Leu Ala Glu Pro
300                 305                 310                 315

aag aaa gat gat ttt gat caa ttt ttg gaa caa agg aag cag tta gac     1493
Lys Lys Asp Asp Phe Asp Gln Phe Leu Glu Gln Arg Lys Gln Leu Asp
                320                 325                 330

agt gaa aaa tagaaaggat tttaggtgaa tttcatggaa gcatttaagc             1542
Ser Glu Lys

aatacaaatg ggaaaatgga aatcttagtg acattgctga cattactatg ggccagtcgc   1602

caccaggtaa ttcctataat gatatcaagg atggaatcgg tttaatcaat ggaccaacag   1662

aatttacgaa taaataccca gttgtaaaac aatggacatc taaacctaca aaactttgta   1722

aagctgggga tatattattg tgtgtaagag gaagttcaac aggacggatg aatatagctg   1782

atgatgaata ttgcattggt cgaggagtag cttctattag agctaaaaaa gataaagctg   1842

aaacaagttt tatttattac acattaaatt ataaagttaa tcagttatta caaaagactg   1902

cgggttctac tttcccaaat cttagtagta atgaaataaa agatatgatt gtcggtattc   1962

cattatttgc agaacaacaa aaaatcgcct ccatcctctc                         2002

<210>  72
<211>  334
<212>  PRT
<213>  Bacillus licheniformis

<400>  72

Val Asn Asn Glu Ser Asn Phe Leu Met Tyr Gln Thr Glu Asn Gly Asp
1               5                   10                  15

Thr Lys Ile Gln Val Arg Leu Glu Gly Glu Thr Val Trp Met Thr Gln
            20                  25                  30

Lys Ala Met Ala Glu Leu Phe Gln Lys Gly Val Pro Thr Ile Asn Glu
        35                  40                  45

His Ile Lys Asn Ile Tyr Ala Glu Gly Glu Leu Thr Glu Glu Ala Thr
    50                  55                  60

Ile Arg Lys Asn Arg Ile Val Gln Val Glu Gly Ser Arg Glu Val Glu
65                  70                  75                  80

Arg Glu Val Thr Phe Tyr Asn Leu Glu Val Ile Ile Ala Val Gly Tyr
                85                  90                  95

Arg Val Arg Ser His Arg Gly Thr Gln Phe Arg Gln Trp Ala Thr Glu
            100                 105                 110

Arg Leu Asn Glu Tyr Met Val Lys Gly Phe Thr Met Asp Asp Glu Arg
        115                 120                 125

Leu Lys Glu Met Arg Asn Ile Gly Ala Asp Tyr Phe Asp Glu Leu Leu
    130                 135                 140

Glu Arg Ile Arg Asp Ile Arg Ala Ser Glu Arg Arg Phe Tyr Tyr Lys
145                 150                 155                 160

Ile Thr Asp Ile Tyr Ala Thr Ser Ile Asp Tyr Asp Pro Asn Thr Pro
                165                 170                 175

Ile Ala Arg Glu Phe Phe Ala Thr Val Gln Asn Lys Leu His Phe Ala
            180                 185                 190

Ile His Gly His Thr Ala Ser Glu Leu Ile Met Lys Arg Ala Asp Ala
        195                 200                 205

Thr Lys Pro Asn Met Gly Leu Thr Ser Trp Lys Gly Asp Lys Val Arg
    210                 215                 220

Lys His Asp Val Thr Val Ala Lys Asn Tyr Leu Thr Gln Glu Glu Leu
225                 230                 235                 240

Ser Asp Leu Asn Arg Ile Val Thr Met Tyr Leu Asp Tyr Ala Glu Thr
                245                 250                 255

Gln Ala Lys Lys Lys Lys Pro Met Tyr Met Lys Asp Trp Ala Glu Lys
            260                 265                 270

Leu Asp Ala Phe Leu Glu Phe Asn Glu His Glu Ile Leu Thr Asn Ala
        275                 280                 285

Gly Lys Ile Lys Ala Lys Val Ala Glu Gln Phe Ala Asn Glu Gln Tyr
    290                 295                 300

Glu Val Phe His Gln Gln Arg Leu Ala Glu Pro Lys Lys Asp Asp Phe
305                 310                 315                 320

Asp Gln Phe Leu Glu Gln Arg Lys Gln Leu Asp Ser Glu Lys
                325                 330

<210>  73
<211>  2170
<212>  DNA
<213>  Bacillus licheniformis

<220>
<221>  CDS
<222>  (501)..(1670)

<400>  73
cgaatacacc aattgcaaga gaattctttg caaccgttca aaataaactc cattttgcaa      60

tccatggaca tactgcttcg gaactgataa tgaaacgagc ggatgcaaca aagccaaaca     120

tgggcttaac gagttggaaa ggtgacaaag tgcgtaaaca tgatgtcact gtggcgaaaa     180

attatttaac acaagaagaa ctcagcgatt taaaccgtat cgtgacaatg tatttagact     240

atgcagaaac acaagcgaaa aagaaaaagc cgatgtacat gaaggattgg gcagaaaaat     300

tagatgcatt tttagagttt aatgaacatg aaatattaac taacgctggg aaaattaaag     360

ccaaagtagc ggaacaattt gcaaacgaac aatatgaagt gtttcatcaa caacgattag     420

cagaaccgaa gaaagatgat tttgatcaat ttttggaaca aaggaagcag ttagacagtg     480

aaaaatagaa aggattttag gtg aat ttc atg gaa gca ttt aag caa tac aaa     533
                      Val Asn Phe Met Glu Ala Phe Lys Gln Tyr Lys
                      1               5                   10

tgg gaa aat gga aat ctt agt gac att gct gac att act atg ggc cag       581
Trp Glu Asn Gly Asn Leu Ser Asp Ile Ala Asp Ile Thr Met Gly Gln
            15                  20                  25

tcg cca cca ggt aat tcc tat aat gat atc aag gat gga atc ggt tta       629
Ser Pro Pro Gly Asn Ser Tyr Asn Asp Ile Lys Asp Gly Ile Gly Leu
        30                  35                  40

atc aat gga cca aca gaa ttt acg aat aaa tac cca gtt gta aaa caa       677
Ile Asn Gly Pro Thr Glu Phe Thr Asn Lys Tyr Pro Val Val Lys Gln
    45                  50                  55

tgg aca tct aaa cct aca aaa ctt tgt aaa gct ggg gat ata tta ttg       725
Trp Thr Ser Lys Pro Thr Lys Leu Cys Lys Ala Gly Asp Ile Leu Leu
60                  65                  70                  75

tgt gta aga gga agt tca aca gga cgg atg aat ata gct gat gat gaa       773
Cys Val Arg Gly Ser Ser Thr Gly Arg Met Asn Ile Ala Asp Asp Glu
                80                  85                  90

tat tgc att ggt cga gga gta gct tct att aga gct aaa aaa gat aaa       821
Tyr Cys Ile Gly Arg Gly Val Ala Ser Ile Arg Ala Lys Lys Asp Lys
            95                  100                 105

gct gaa aca agt ttt att tat tac aca tta aat tat aaa gtt aat cag       869

Ala Glu Thr Ser Phe Ile Tyr Tyr Thr Leu Asn Tyr Lys Val Asn Gln
        110                 115                 120

tta tta caa aag act gcg ggt tct act ttc cca aat ctt agt agt aat      917
Leu Leu Gln Lys Thr Ala Gly Ser Thr Phe Pro Asn Leu Ser Ser Asn
    125                 130                 135

gaa ata aaa gat atg att gtc ggt att cca tta ttt gca gaa caa caa      965
Glu Ile Lys Asp Met Ile Val Gly Ile Pro Leu Phe Ala Glu Gln Gln
140                 145                 150                 155

aaa atc gcc tcc atc ctc tct acc tgg gat aag gca att gag cta aaa      1013
Lys Ile Ala Ser Ile Leu Ser Thr Trp Asp Lys Ala Ile Glu Leu Lys
                160                 165                 170

gaa aag cta atc gag cag aag aaa gag cag aaa aaa ggg tta atg cag      1061
Glu Lys Leu Ile Glu Gln Lys Lys Glu Gln Lys Lys Gly Leu Met Gln
            175                 180                 185

aag ttg ctg act ggt aaa gtg aga ttg cct ggg ttt agt gac aaa tgg      1109
Lys Leu Leu Thr Gly Lys Val Arg Leu Pro Gly Phe Ser Asp Lys Trp
        190                 195                 200

gaa aag aaa aaa atc ggt gaa cta tta gaa gaa agc aag gta att gca      1157
Glu Lys Lys Lys Ile Gly Glu Leu Leu Glu Glu Ser Lys Val Ile Ala
    205                 210                 215

aaa aat ccg caa tta gat aaa agg att act gtg cga tta aat tta aaa      1205
Lys Asn Pro Gln Leu Asp Lys Arg Ile Thr Val Arg Leu Asn Leu Lys
220                 225                 230                 235

ggt gtt tgt aaa aga gaa ata tct acg gtg gaa aaa gag gga gca act      1253
Gly Val Cys Lys Arg Glu Ile Ser Thr Val Glu Lys Glu Gly Ala Thr
                240                 245                 250

aca caa tat atc aga aaa gaa ggc caa ttt att tat ggg aaa cag aat      1301
Thr Gln Tyr Ile Arg Lys Glu Gly Gln Phe Ile Tyr Gly Lys Gln Asn
            255                 260                 265

ctt cat aag ggg gca ttt gga ttg att cca aaa gag tta gat gga ttc      1349
Leu His Lys Gly Ala Phe Gly Leu Ile Pro Lys Glu Leu Asp Gly Phe
        270                 275                 280

cag tct tca tct gac ata cct tgt ttt gat ttt aaa gaa ggt gta gat      1397
Gln Ser Ser Ser Asp Ile Pro Cys Phe Asp Phe Lys Glu Gly Val Asp
    285                 290                 295

ggt ctc tgg ttt tat tat tat ttt tca aga gaa agc ttc tat act aat      1445
Gly Leu Trp Phe Tyr Tyr Tyr Phe Ser Arg Glu Ser Phe Tyr Thr Asn
300                 305                 310                 315

tta gaa aat atc tca agt ggt aca ggt tct aaa cgg atc caa cct aag      1493
Leu Glu Asn Ile Ser Ser Gly Thr Gly Ser Lys Arg Ile Gln Pro Lys
                320                 325                 330

gaa tta tac aag tta aca att aaa ctc ccg tca ctt aga gaa caa caa      1541
Glu Leu Tyr Lys Leu Thr Ile Lys Leu Pro Ser Leu Arg Glu Gln Gln
            335                 340                 345

aga cag tca aaa att tta gaa tgt agt gat aaa gaa att tat ttg tta      1589
Arg Gln Ser Lys Ile Leu Glu Cys Ser Asp Lys Glu Ile Tyr Leu Leu
        350                 355                 360

gag aaa gaa tta gag act tat aga aaa caa aaa caa ggt ctt atg caa      1637
Glu Lys Glu Leu Glu Thr Tyr Arg Lys Gln Lys Gln Gly Leu Met Gln
    365                 370                 375

ctt ctt cta aca ggg aaa gtc cga gtg aag gta taattccttt actccttttc      1690

Leu Leu Leu Thr Gly Lys Val Arg Val Lys Val
380                 385                 390

ctttattctc aacaatggag gtggagaaat gtcgattccg caaagttatg atgaacgtta    1750

cattagtcaa cagcctgcga tcgaggtgtt gcaaggctta ggatatgagt acatagaggc    1810

tgaacgagct gaggcgatgc gtgagaatct ctatagcgtt ttgctgaaaa cggtattgga    1870

aaataaattg aaggagttaa attcctatca gtataaaggt gttacgtaca agttcagtga    1930

agcgaacatc caacaagcga tgcgagattt ggatgagcca ctcacaaacg gacttgtaaa    1990

ggcaaatgaa agcatttatg aaacgttgat gttaggaaga acttatacgg aattacttcc    2050

agacggctcg aaaaagtcat ttaccattca atatattgat tgggagaatg ttgaaaataa    2110

tgtttttcac attgtagagg aattttctgt tgaacgtgta gacggcaggg gtacagttcg    2170

<210>  74
<211>  390
<212>  PRT
<213>  Bacillus licheniformis

<400>  74

Val Asn Phe Met Glu Ala Phe Lys Gln Tyr Lys Trp Glu Asn Gly Asn
1               5                   10                  15

Leu Ser Asp Ile Ala Asp Ile Thr Met Gly Gln Ser Pro Pro Gly Asn
            20                  25                  30

Ser Tyr Asn Asp Ile Lys Asp Gly Ile Gly Leu Ile Asn Gly Pro Thr
        35                  40                  45

Glu Phe Thr Asn Lys Tyr Pro Val Val Lys Gln Trp Thr Ser Lys Pro
    50                  55                  60

Thr Lys Leu Cys Lys Ala Gly Asp Ile Leu Leu Cys Val Arg Gly Ser
65                  70                  75                  80

Ser Thr Gly Arg Met Asn Ile Ala Asp Asp Glu Tyr Cys Ile Gly Arg
                85                  90                  95

Gly Val Ala Ser Ile Arg Ala Lys Lys Asp Lys Ala Glu Thr Ser Phe
            100                 105                 110

Ile Tyr Tyr Thr Leu Asn Tyr Lys Val Asn Gln Leu Leu Gln Lys Thr
        115                 120                 125

Ala Gly Ser Thr Phe Pro Asn Leu Ser Ser Asn Glu Ile Lys Asp Met
    130                 135                 140

Ile Val Gly Ile Pro Leu Phe Ala Glu Gln Gln Lys Ile Ala Ser Ile
145                 150                 155                 160

Leu Ser Thr Trp Asp Lys Ala Ile Glu Leu Lys Glu Lys Leu Ile Glu

165                 170                 175

Gln Lys Lys Glu Gln Lys Lys Gly Leu Met Gln Lys Leu Leu Thr Gly
            180                 185                 190

Lys Val Arg Leu Pro Gly Phe Ser Asp Lys Trp Glu Lys Lys Lys Ile
        195                 200                 205

Gly Glu Leu Leu Glu Glu Ser Lys Val Ile Ala Lys Asn Pro Gln Leu
    210                 215                 220

Asp Lys Arg Ile Thr Val Arg Leu Asn Leu Lys Gly Val Cys Lys Arg
225                 230                 235                 240

Glu Ile Ser Thr Val Glu Lys Glu Gly Ala Thr Thr Gln Tyr Ile Arg
                245                 250                 255

Lys Glu Gly Gln Phe Ile Tyr Gly Lys Gln Asn Leu His Lys Gly Ala
            260                 265                 270

Phe Gly Leu Ile Pro Lys Glu Leu Asp Gly Phe Gln Ser Ser Ser Asp
        275                 280                 285

Ile Pro Cys Phe Asp Phe Lys Glu Gly Val Asp Gly Leu Trp Phe Tyr
    290                 295                 300

Tyr Tyr Phe Ser Arg Glu Ser Phe Tyr Thr Asn Leu Glu Asn Ile Ser
305                 310                 315                 320

Ser Gly Thr Gly Ser Lys Arg Ile Gln Pro Lys Glu Leu Tyr Lys Leu
                325                 330                 335

Thr Ile Lys Leu Pro Ser Leu Arg Glu Gln Gln Arg Gln Ser Lys Ile
            340                 345                 350

Leu Glu Cys Ser Asp Lys Glu Ile Tyr Leu Leu Glu Lys Glu Leu Glu
        355                 360                 365

Thr Tyr Arg Lys Gln Lys Gln Gly Leu Met Gln Leu Leu Leu Thr Gly
    370                 375                 380

Lys Val Arg Val Lys Val
385                 390

<210>  75
<211>  1528
<212>  DNA
<213>  Bacillus licheniformis

<220>
<221>  CDS
<222>  (501)..(1118)

<220>
<221>  misc_feature
<222>  (1518)..(1518)
<223>  n is a, c, g, or t

<400>  75
cggcggtgtt gcagcattgc tgaccgcgcc gacttcagga aaaaacttga gaggacagct      60

taaagccaac tgcggtaaat gggaagacac cgtcaaaagg ctgaaaaacg acggaatcgc     120

attaaaagag caattcgtca aaaccgccat agaaggcgcg gaaatcatta aagacgtcgg     180

cagcgaactg caaacgtcca tccaggaatg gcgcgaggaa atcaaacctc atcagcagga     240

cttaaaaaag gaaatcgctg agatcgaaga aaagataaaa cagctggaga aaaccttaca     300

aaactaatat actcagcgaa aaaaataaac cttaaaaaag gggtgtgaat ggaaaatttt     360

cacgctcctt ttttaggtaa aatcctgatt tcacaccgta aaactggatt ttttaaaaaa     420

ttcgtaaatt tacatcttta ttaatcttta ttttattggc ataatagaaa ttataagata     480

aagagaagca ggtgaagtga atg aat cga gcg gaa gag ccc tat acg gtg aaa     533
                      Met Asn Arg Ala Glu Glu Pro Tyr Thr Val Lys
                      1               5                   10

gaa gcc ctt tta ttc agt caa aga atg gca cag ctt agc aag gcg ctt       581
Glu Ala Leu Leu Phe Ser Gln Arg Met Ala Gln Leu Ser Lys Ala Leu
            15                  20                  25

tgg aag tcg ata gaa aag gat tgg cag cag tgg att aaa cct tat gat       629
Trp Lys Ser Ile Glu Lys Asp Trp Gln Gln Trp Ile Lys Pro Tyr Asp
        30                  35                  40

ctg aac att aac gag cac cat att ttg tgg atc gcc tac cag ctg aac       677
Leu Asn Ile Asn Glu His His Ile Leu Trp Ile Ala Tyr Gln Leu Asn
    45                  50                  55

gga gca tcc att tca gaa atc gcc aag ttc ggc gtc atg cac gta tcc       725
Gly Ala Ser Ile Ser Glu Ile Ala Lys Phe Gly Val Met His Val Ser
60                  65                  70                  75

aca gcg ttt aat ttt tca aaa aag ctt gag gaa aga ggt tac ctg gag       773
Thr Ala Phe Asn Phe Ser Lys Lys Leu Glu Glu Arg Gly Tyr Leu Glu
                80                  85                  90

ttt tcc aaa aaa ttg aat gac aaa cgc aac act tac att cag ctg act       821
Phe Ser Lys Lys Leu Asn Asp Lys Arg Asn Thr Tyr Ile Gln Leu Thr
            95                  100                 105

ccg aaa ggt gaa gaa gtt ttc ctt aaa atc ctc gag tct tac gat ccg       869
Pro Lys Gly Glu Glu Val Phe Leu Lys Ile Leu Glu Ser Tyr Asp Pro
        110                 115                 120

acc cgg aac gcc gtc tta aaa ggc gct cag ccc ctt cat cag ctg tac       917
Thr Arg Asn Ala Val Leu Lys Gly Ala Gln Pro Leu His Gln Leu Tyr
    125                 130                 135

ggg aag ttt cca gaa atc gtc gag atg atg tcg atc atc cgc cat att       965
Gly Lys Phe Pro Glu Ile Val Glu Met Met Ser Ile Ile Arg His Ile
140                 145                 150                 155

tac ggc gat gat ttc atg gaa att ttc gaa aaa tcg ttt tca aac atc      1013
Tyr Gly Asp Asp Phe Met Glu Ile Phe Glu Lys Ser Phe Ser Asn Ile
                160                 165                 170

gaa aat gaa ttt acg agc gaa gag ggc aaa atg aag aaa aaa caa gaa      1061

Glu Asn Glu Phe Thr Ser Glu Glu Gly Lys Met Lys Lys Lys Gln Glu
            175                 180                 185

gca aaa gaa gcc ggg gaa tcg ata gag gtg gac aaa ccg ctt gag cct      1109
Ala Lys Glu Ala Gly Glu Ser Ile Glu Val Asp Lys Pro Leu Glu Pro
        190                 195                 200

ctt aaa aac taagccagat cgtactgcct gataatgctg atgaattcgc              1158
Leu Lys Asn
    205

tcatcagcgg tttgtaaaga ccttgtctgt gaagggcgaa aatcgtttcg tgcacttcaa    1218

gtttttcatt taactgtccg gcaaaaagcg tgaggagatg atcgaacatc acaaatcctt    1278

gcgctttttg tgcttcaagc tcctttgaga gctcttcgca cagcttttcc gtttcctttg     1338

cttcttcctc tgataagccc ttttcgatca atattttata gtacggatac tcttccatat    1398

ccgcattgtt caataacagt ttgatgtaat attccagccg ctcgaggcgt tgctccattg    1458

agttcatatt tgtctcttct ctccctttct cttattttag ccgatattcc cttctgatan    1518

aagttcaaca                                                           1528

<210>  76
<211>  206
<212>  PRT
<213>  Bacillus licheniformis

<400>  76

Met Asn Arg Ala Glu Glu Pro Tyr Thr Val Lys Glu Ala Leu Leu Phe
1               5                   10                  15

Ser Gln Arg Met Ala Gln Leu Ser Lys Ala Leu Trp Lys Ser Ile Glu
            20                  25                  30

Lys Asp Trp Gln Gln Trp Ile Lys Pro Tyr Asp Leu Asn Ile Asn Glu
        35                  40                  45

His His Ile Leu Trp Ile Ala Tyr Gln Leu Asn Gly Ala Ser Ile Ser
    50                  55                  60

Glu Ile Ala Lys Phe Gly Val Met His Val Ser Thr Ala Phe Asn Phe
65                  70                  75                  80

Ser Lys Lys Leu Glu Glu Arg Gly Tyr Leu Glu Phe Ser Lys Lys Leu
                85                  90                  95

Asn Asp Lys Arg Asn Thr Tyr Ile Gln Leu Thr Pro Lys Gly Glu Glu
            100                 105                 110

Val Phe Leu Lys Ile Leu Glu Ser Tyr Asp Pro Thr Arg Asn Ala Val
        115                 120                 125

Leu Lys Gly Ala Gln Pro Leu His Gln Leu Tyr Gly Lys Phe Pro Glu
    130                 135                 140

Ile Val Glu Met Met Ser Ile Ile Arg His Ile Tyr Gly Asp Asp Phe
145                 150                 155                 160

Met Glu Ile Phe Glu Lys Ser Phe Ser Asn Ile Glu Asn Glu Phe Thr
                165                 170                 175

Ser Glu Glu Gly Lys Met Lys Lys Lys Gln Glu Ala Lys Glu Ala Gly
            180                 185                 190

Glu Ser Ile Glu Val Asp Lys Pro Leu Glu Pro Leu Lys Asn
        195                 200                 205

<210>  77
<211>  2104
<212>  DNA
<213>  Bacillus licheniformis

<220>
<221>  CDS
<222>  (501)..(1604)

<400>  77
ggaaacgtca gtccctgtcg gttctgtcca ggatacttta tagacgcctt cagtcagctt      60

gacgatatcg gctttttgat cgcgaaccca gcgtccccca accattccgc tgtgaatgcg     120

gtaatcgatg gtatggtcat ttttaatgta gatttcatat tcccatccgt tttcatacgt     180

atagatcata tggcttccta caaactcttt tacatcttga ttcatatgaa ccgctccttt     240

atttgtttat tgtaatcgaa acatgttgat atttacatat ataattataa aatgccgtca     300

aaaaagatgt caaacgaaaa tacttcctga gagatttgca caaataaaga agattgttac     360

gattaatgtc agaattttga gttatcttag gaaattatgc caatatttag aaaagtgatt     420

gtcaaaaaat aagcgattct gtaaaatgaa aaacaaccca taaaaaggaa atgacatggg     480

aagaaaggaa ggataaacga ttg aag aca aaa att gcg tat gag gaa gtt gcg     533
                     Leu Lys Thr Lys Ile Ala Tyr Glu Glu Val Ala
                     1               5                   10

gga atg ctt aat caa tgg tat gtc atg atc aag cgt cac gaa gta tca     581
Gly Met Leu Asn Gln Trp Tyr Val Met Ile Lys Arg His Glu Val Ser
            15                  20                  25

caa gcg gtc tcg att aaa tgc gac att gag cac cag ctg ccg aat atg     629
Gln Ala Val Ser Ile Lys Cys Asp Ile Glu His Gln Leu Pro Asn Met
        30                  35                  40

gaa gaa aat caa gat ctg ctt ctt tat ttt aat ctt tta gac tat cgg     677
Glu Glu Asn Gln Asp Leu Leu Leu Tyr Phe Asn Leu Leu Asp Tyr Arg
    45                  50                  55

cac aag ctg ctg aca gaa gag ttt gcc gct tcc aac aaa ctg ttc gag     725
His Lys Leu Leu Thr Glu Glu Phe Ala Ala Ser Asn Lys Leu Phe Glu
60                  65                  70                  75

gat att cag gag caa aaa gcc gat atg caa agc aca gat gac atg att     773
Asp Ile Gln Glu Gln Lys Ala Asp Met Gln Ser Thr Asp Asp Met Ile
                80                  85                  90

gaa tat tat tat ttc ttt ttc gct ggc atg tac gaa ttt cat aag aag     821
Glu Tyr Tyr Tyr Phe Phe Phe Ala Gly Met Tyr Glu Phe His Lys Lys
            95                  100                 105

gat tat aca aat gca atc aat tat tat aaa tta gcc gag gaa aag ctc     869
Asp Tyr Thr Asn Ala Ile Asn Tyr Tyr Lys Leu Ala Glu Glu Lys Leu
        110                 115                 120

agg aca atc ccc gat caa atc gaa atc gcc gaa ttc cat tac aaa ctg     917
Arg Thr Ile Pro Asp Gln Ile Glu Ile Ala Glu Phe His Tyr Lys Leu
    125                 130                 135

gct atc gcc tac tat caa atc aaa caa aat ttc ctt tcc tta aac cat     965
Ala Ile Ala Tyr Tyr Gln Ile Lys Gln Asn Phe Leu Ser Leu Asn His
140                 145                 150                 155

gcg aaa aca gct cta aaa acc ttc aaa gca cat gat gat tac att caa     1013
Ala Lys Thr Ala Leu Lys Thr Phe Lys Ala His Asp Asp Tyr Ile Gln
                160                 165                 170

aaa gcg atc agc aac gat atg ctg atc ggg gca aat aaa ctc gat tta     1061
Lys Ala Ile Ser Asn Asp Met Leu Ile Gly Ala Asn Lys Leu Asp Leu
            175                 180                 185

ttt cgt ttt gat gaa gcc gaa cag cat tac aag caa gcc ctt aaa gac     1109
Phe Arg Phe Asp Glu Ala Glu Gln His Tyr Lys Gln Ala Leu Lys Asp
        190                 195                 200

gcg gca ctg atc aaa cat cat gtc ctc ctc ggc atg gct cac cac aac     1157
Ala Ala Leu Ile Lys His His Val Leu Leu Gly Met Ala His His Asn
    205                 210                 215

tta ggg ttg agc tat gtc aat cgc aac ctc ctc aca ttg gct gaa cat     1205
Leu Gly Leu Ser Tyr Val Asn Arg Asn Leu Leu Thr Leu Ala Glu His
220                 225                 230                 235

cat ttc aaa gaa gcg ctg ctt atc aaa gag cat gaa gaa tcg gtt tac     1253
His Phe Lys Glu Ala Leu Leu Ile Lys Glu His Glu Glu Ser Val Tyr
                240                 245                 250

ggc atc cat tcc atg ttt gaa ctg aca cat gtg ctg tac aaa tca aat     1301
Gly Ile His Ser Met Phe Glu Leu Thr His Val Leu Tyr Lys Ser Asn
            255                 260                 265

gtt gtc aaa gaa gca cgc aaa ttg tat gaa aaa gga ttt ttc cgt gcg     1349
Val Val Lys Glu Ala Arg Lys Leu Tyr Glu Lys Gly Phe Phe Arg Ala
        270                 275                 280

gaa aaa gca gga gaa agg gaa tat ttg tcg aaa ttt aaa ctt att cat     1397
Glu Lys Ala Gly Glu Arg Glu Tyr Leu Ser Lys Phe Lys Leu Ile His
    285                 290                 295

gct ctg tat gat gaa cag gat cca ctt acg gtt gaa cat gct tta gaa     1445
Ala Leu Tyr Asp Glu Gln Asp Pro Leu Thr Val Glu His Ala Leu Glu
300                 305                 310                 315

tat ctt aaa acg atc aat ctc tgg acg gat gta gcg gaa tta aca ttt     1493
Tyr Leu Lys Thr Ile Asn Leu Trp Thr Asp Val Ala Glu Leu Thr Phe
                320                 325                 330

gat atc gca ctt tac tat aaa gaa aat gga gat gca gac aaa gct gcc     1541
Asp Ile Ala Leu Tyr Tyr Lys Glu Asn Gly Asp Ala Asp Lys Ala Ala
            335                 340                 345

gaa tat ttt gaa gaa tct cat cat gca aga gac caa att ctt aaa aga     1589
Glu Tyr Phe Glu Glu Ser His His Ala Arg Asp Gln Ile Leu Lys Arg
        350                 355                 360

acg gag gag tta aag tgaaaaagat gattgccgtt gcgttgactg ccgtctttgc 1644
Thr Glu Glu Leu Lys
    365

aagccttgtc gttttaagtt tttcgtctca gccgaaggga gatgccgagt ttgccggccg 1704

agccattttt cttgacgata agccggctca gctgatggcg ggcagagcga tctttctcga 1764

cagctttgac ggaaattcgc cagccgcctg acaaaacacc gcataaccct gcttgtcacc 1824

ggacaagctc tcttcgaaaa gcgccgccag cggcgctttt tttatgaaag caaatataac 1884

cggcgtccct gcaaatcgct tgcgaagatt aaaaatatga aacacatgac aggaatggcg 1944

ctcaccggct tttggtcgaa tcctattgaa aaatctgccc gatttatagt gcttgtcatt 2004

ctataaatcc ttataatggt cttaacgcca atgtgaggca tgccaataga cagattgcag 2064

gggaaattct atactgaaca gcgcaattta gcatgtaatc 2104

<210> 78
<211> 368
<212> PRT
<213> Bacillus licheniformis

<400> 78

Leu Lys Thr Lys Ile Ala Tyr Glu Glu Val Ala Gly Met Leu Asn Gln
1               5                   10                  15

Trp Tyr Val Met Ile Lys Arg His Glu Val Ser Gln Ala Val Ser Ile
            20                  25                  30

Lys Cys Asp Ile Glu His Gln Leu Pro Asn Met Glu Glu Asn Gln Asp
        35                  40                  45

Leu Leu Leu Tyr Phe Asn Leu Leu Asp Tyr Arg His Lys Leu Leu Thr
    50                  55                  60

Glu Glu Phe Ala Ala Ser Asn Lys Leu Phe Glu Asp Ile Gln Glu Gln
65                  70                  75                  80

Lys Ala Asp Met Gln Ser Thr Asp Asp Met Ile Glu Tyr Tyr Tyr Phe
                85                  90                  95

Phe Phe Ala Gly Met Tyr Glu Phe His Lys Lys Asp Tyr Thr Asn Ala
            100                 105                 110

Ile Asn Tyr Tyr Lys Leu Ala Glu Glu Lys Leu Arg Thr Ile Pro Asp
        115                 120                 125

Gln Ile Glu Ile Ala Glu Phe His Tyr Lys Leu Ala Ile Ala Tyr Tyr
    130                 135                 140

Gln Ile Lys Gln Asn Phe Leu Ser Leu Asn His Ala Lys Thr Ala Leu
145                 150                 155                 160

Lys Thr Phe Lys Ala His Asp Asp Tyr Ile Gln Lys Ala Ile Ser Asn
                165                 170                 175

Asp Met Leu Ile Gly Ala Asn Lys Leu Asp Leu Phe Arg Phe Asp Glu
            180                 185                 190

Ala Glu Gln His Tyr Lys Gln Ala Leu Lys Asp Ala Ala Leu Ile Lys
        195                 200                 205

His His Val Leu Leu Gly Met Ala His His Asn Leu Gly Leu Ser Tyr
    210                 215                 220

Val Asn Arg Asn Leu Leu Thr Leu Ala Glu His His Phe Lys Glu Ala
225                 230                 235                 240

Leu Leu Ile Lys Glu His Glu Glu Ser Val Tyr Gly Ile His Ser Met
                245                 250                 255

Phe Glu Leu Thr His Val Leu Tyr Lys Ser Asn Val Val Lys Glu Ala
            260                 265                 270

Arg Lys Leu Tyr Glu Lys Gly Phe Phe Arg Ala Glu Lys Ala Gly Glu
        275                 280                 285

Arg Glu Tyr Leu Ser Lys Phe Lys Leu Ile His Ala Leu Tyr Asp Glu
    290                 295                 300

Gln Asp Pro Leu Thr Val Glu His Ala Leu Glu Tyr Leu Lys Thr Ile
305                 310                 315                 320

Asn Leu Trp Thr Asp Val Ala Glu Leu Thr Phe Asp Ile Ala Leu Tyr
                325                 330                 335

Tyr Lys Glu Asn Gly Asp Ala Asp Lys Ala Ala Glu Tyr Phe Glu Glu
            340                 345                 350

Ser His His Ala Arg Asp Gln Ile Leu Lys Arg Thr Glu Glu Leu Lys
        355                 360                 365

<210>  79
<211>  2005
<212>  DNA
<213>  Bacillus licheniformis

<220>
<221>  CDS
<222>  (501)..(1505)

<400>  79
acctcgattt gctccgagtt tgtcgcttca atcattttat cggtatcata tatattaaaa      60

tccgcataca aatgagcttt cccctttttg gccacgccta cgtacaggca gtcatccctg      120

aagttcgtct ttcgaacagc ctgcaaaaat gtcccgtcgc cgccgatgct tgcgataata      180

ttggcttcat tgtgatggtg aacgacgcga aatccgtgtt cttcggcaag ctgtttcagt 240

tttttgcact gctcgtcggt ttcttcgctt tttttgtaaa agaaataaat gtttcggcgg 300

tctgtcacaa tcattctccc ctttgtctgt taagtttaga tttttctgcc atctaacata 360

tatgttaaaa tgacctgtga cccttagttt accatgtcca aaaacttctt gtatatgaag 420

cggcatcata atgaactttt tctcttcata tacgtactaa gacatgcagc tttttattc 480

aaaggaggag aacagacaaa atg aat gcg aaa aga tgg atc gca ctt gtc atc 533
Met Asn Ala Lys Arg Trp Ile Ala Leu Val Ile
1               5                   10

gcg ctg ggc gtt ttt gcg tta tct gcg gtg atg agc ctt ttg ctt gca 581
Ala Leu Gly Val Phe Ala Leu Ser Ala Val Met Ser Leu Leu Leu Ala
            15                  20                  25

gta ttt gac acg atg ggg aat gac gga aag atg cag ttt ggc ctc aat 629
Val Phe Asp Thr Met Gly Asn Asp Gly Lys Met Gln Phe Gly Leu Asn
        30                  35                  40

gac acc cag gaa gaa acg gtg ctt gaa caa gga aac agc tca aga aaa 677
Asp Thr Gln Glu Glu Thr Val Leu Glu Gln Gly Asn Ser Ser Arg Lys
    45                  50                  55

atc gcc gtt ctt gaa gtg aac gga acg att tcc gac aat ggc ggc gct 725
Ile Ala Val Leu Glu Val Asn Gly Thr Ile Ser Asp Asn Gly Gly Ala
60                  65                  70                  75

tca ggc ctg ttc agc tct gaa ggc tac aac cac aga tcg ttt ttg caa 773
Ser Gly Leu Phe Ser Ser Glu Gly Tyr Asn His Arg Ser Phe Leu Gln
                80                  85                  90

atg ctt gaa aaa gca aaa gac gat tct gcc gta aag ggc att gtt ctg 821
Met Leu Glu Lys Ala Lys Asp Asp Ser Ala Val Lys Gly Ile Val Leu
            95                  100                 105

cgc gtc aat tct ccg ggt ggc gga gta tac gag agc gct gag ata cat 869
Arg Val Asn Ser Pro Gly Gly Gly Val Tyr Glu Ser Ala Glu Ile His
        110                 115                 120

aag aag ctt gaa gag atc aaa aaa gat acg aaa aaa ccg att tac gta 917
Lys Lys Leu Glu Glu Ile Lys Lys Asp Thr Lys Lys Pro Ile Tyr Val
    125                 130                 135

tca atg gga tcg atg gct gcg tcc ggc ggc tat tac att tca acg cct 965
Ser Met Gly Ser Met Ala Ala Ser Gly Gly Tyr Tyr Ile Ser Thr Pro
140                 145                 150                 155

gcc gac aaa atc ttt gca gcg cct gac acg ctg acg ggt tca ctc ggg 1013
Ala Asp Lys Ile Phe Ala Ala Pro Asp Thr Leu Thr Gly Ser Leu Gly
                160                 165                 170

gtc atc atg gaa agc ctt aac tac agc aag ctt gca gag aag ctt gga 1061
Val Ile Met Glu Ser Leu Asn Tyr Ser Lys Leu Ala Glu Lys Leu Gly
            175                 180                 185

tta aaa acg gag acg att aaa agc ggt gag ttt aaa gat atc atg tca 1109
Leu Lys Thr Glu Thr Ile Lys Ser Gly Glu Phe Lys Asp Ile Met Ser
        190                 195                 200

ccg acg cgc gat atg acg aaa aaa gaa aga gaa atc atg cag tca atg 1157
Pro Thr Arg Asp Met Thr Lys Lys Glu Arg Glu Ile Met Gln Ser Met
    205                 210                 215

gtc gat gac gcg tac gaa ggg ttt gta gat gtt att gcc gaa ggg cgc 1205

Val Asp Asp Ala Tyr Glu Gly Phe Val Asp Val Ile Ala Glu Gly Arg
220                 225                 230                 235

ggc atg tcc gaa aat gac gtg aaa aaa att gcc gac gga cgt gtc tat      1253
Gly Met Ser Glu Asn Asp Val Lys Lys Ile Ala Asp Gly Arg Val Tyr
                240                 245                 250

gac ggc cgc cag gcg aaa cag aac cat ctc att gat gag ctc ggc tat      1301
Asp Gly Arg Gln Ala Lys Gln Asn His Leu Ile Asp Glu Leu Gly Tyr
            255                 260                 265

tat gaa gat gcg gtc aaa gcg atg aaa aag gac cac aaa aac ctt gcc      1349
Tyr Glu Asp Ala Val Lys Ala Met Lys Lys Asp His Lys Asn Leu Ala
        270                 275                 280

ggc gca tct gtt gtc agc tat gaa gaa tca gcc ggc ctt gcc tcg ctg      1397
Gly Ala Ser Val Val Ser Tyr Glu Glu Ser Ala Gly Leu Ala Ser Leu
    285                 290                 295

ttt tca atg act gca aat aag atg ttt aaa agc gaa gct gat ttc tta      1445
Phe Ser Met Thr Ala Asn Lys Met Phe Lys Ser Glu Ala Asp Phe Leu
300                 305                 310                 315

aac att aaa gaa gcg att tct caa tcc ggt gca ccg aga ctc atg tat      1493
Asn Ile Lys Glu Ala Ile Ser Gln Ser Gly Ala Pro Arg Leu Met Tyr
                320                 325                 330

tta tat gcc aaa taggagggga ccgaaaaaat ggatgtgact tatgatggaa          1545
Leu Tyr Ala Lys
            335

aggatcataa tgaattatcg ccgaatgtcg gcgtttccag ggaagaatcg gccccgcatg    1605

tagagcatgc ctatgccggt ttttggatca ggctgtttgc ttttctgctt gacggggttg    1665

ttatcggcag catcaacaac ttagcggttt cacccgtttt cagcctgctg aacctcccta    1725

aagaatcagg gtttttcacc ttttcgcttt attcttttgt gacggcggca gtattttttg    1785

cttactttgt cctgatgacg aagtatttcg gacagacgtt gggcaagatg gtattcgggc    1845

tgaaggtcgt gtcgcttgtc ccggaaaaag ggctgacatg ggatgtggtc ctgtttaggg    1905

agtttatcgg ccgctatatc aacagtttat atattacgta tctcgtagca gcgttttcgc    1965

ctaagaaaca agggattcac gatttctttg cggacccctg                          2005

<210>  80
<211>  335
<212>  PRT
<213>  Bacillus licheniformis

<400>  80

Met Asn Ala Lys Arg Trp Ile Ala Leu Val Ile Ala Leu Gly Val Phe
1               5                   10                  15

Ala Leu Ser Ala Val Met Ser Leu Leu Leu Ala Val Phe Asp Thr Met
            20                  25                  30

Gly Asn Asp Gly Lys Met Gln Phe Gly Leu Asn Asp Thr Gln Glu Glu
        35                  40                  45

Thr Val Leu Glu Gln Gly Asn Ser Ser Arg Lys Ile Ala Val Leu Glu

50                  55                  60

Val Asn Gly Thr Ile Ser Asp Asn Gly Gly Ala Ser Gly Leu Phe Ser
65                  70                  75                  80

Ser Glu Gly Tyr Asn His Arg Ser Phe Leu Gln Met Leu Glu Lys Ala
                85                  90                  95

Lys Asp Asp Ser Ala Val Lys Gly Ile Val Leu Arg Val Asn Ser Pro
            100                 105                 110

Gly Gly Gly Val Tyr Glu Ser Ala Glu Ile His Lys Lys Leu Glu Glu
        115                 120                 125

Ile Lys Lys Asp Thr Lys Lys Pro Ile Tyr Val Ser Met Gly Ser Met
    130                 135                 140

Ala Ala Ser Gly Gly Tyr Tyr Ile Ser Thr Pro Ala Asp Lys Ile Phe
145                 150                 155                 160

Ala Ala Pro Asp Thr Leu Thr Gly Ser Leu Gly Val Ile Met Glu Ser
                165                 170                 175

Leu Asn Tyr Ser Lys Leu Ala Glu Lys Leu Gly Leu Lys Thr Glu Thr
            180                 185                 190

Ile Lys Ser Gly Glu Phe Lys Asp Ile Met Ser Pro Thr Arg Asp Met
        195                 200                 205

Thr Lys Lys Glu Arg Glu Ile Met Gln Ser Met Val Asp Asp Ala Tyr
    210                 215                 220

Glu Gly Phe Val Asp Val Ile Ala Glu Gly Arg Gly Met Ser Glu Asn
225                 230                 235                 240

Asp Val Lys Lys Ile Ala Asp Gly Arg Val Tyr Asp Gly Arg Gln Ala
                245                 250                 255

Lys Gln Asn His Leu Ile Asp Glu Leu Gly Tyr Tyr Glu Asp Ala Val
            260                 265                 270

Lys Ala Met Lys Lys Asp His Lys Asn Leu Ala Gly Ala Ser Val Val
        275                 280                 285

Ser Tyr Glu Glu Ser Ala Gly Leu Ala Ser Leu Phe Ser Met Thr Ala
    290                 295                 300

Asn Lys Met Phe Lys Ser Glu Ala Asp Phe Leu Asn Ile Lys Glu Ala
305                 310                 315                 320

Ile Ser Gln Ser Gly Ala Pro Arg Leu Met Tyr Leu Tyr Ala Lys

325                 330                 335

<210>  81
<211>  1326
<212>  DNA
<213>  Bacillus licheniformis

<220>
<221>  CDS
<222>  (501)..(869)

<400>  81
tgcgttgact gccgtctttg caagccttgt cgttttaagt ttttcgtctc agccgaaggg      60

agatgccgag tttgccggcc gagccatttt tcttgacgat aagccggctc agctgatggc     120

gggcagagcg atctttctcg acagctttga cggaaattcg ccagccgcct gacaaaacac     180

cgcataaccc tgcttgtcac cggacaagct ctcttcgaaa agcgccgcca gcggcgcttt     240

ttttatgaaa gcaaatataa ccggcgtccc tgcaaatcgc ttgcgaagat taaaaatatg     300

aaacacatga caggaatggc gctcaccggc ttttggtcga atcctattga aaaatctgcc     360

cgatttatag tgcttgtcat tctataaatc cttataatgg tcttaacgcc aatgtgaggc     420

atgccaatag acagattgca ggggaaattc tatactgaac agcgcaattt agcatgtaat     480

catttttagg agggagcagc atg gaa gtc ttt gaa gaa tat tta gcg gga att     533
                      Met Glu Val Phe Glu Glu Tyr Leu Ala Gly Ile
                      1               5                   10

gat cat ccg gaa cat cgt gcc cgg atg gaa gaa gta ttg ggt tgg gta       581
Asp His Pro Glu His Arg Ala Arg Met Glu Glu Val Leu Gly Trp Val
            15                  20                  25

gcg gag aca ttt cca aat tta acg ccg aaa atc gct tgg aat cag ccg       629
Ala Glu Thr Phe Pro Asn Leu Thr Pro Lys Ile Ala Trp Asn Gln Pro
        30                  35                  40

atg ttt act gat cac ggc aca ttt att atc ggc ttt agc gtc gcg aaa       677
Met Phe Thr Asp His Gly Thr Phe Ile Ile Gly Phe Ser Val Ala Lys
    45                  50                  55

aat cat ttg gct gtc gcc cct gaa agc gct ggg atc gat cgt ttt tct       725
Asn His Leu Ala Val Ala Pro Glu Ser Ala Gly Ile Asp Arg Phe Ser
60                  65                  70                  75

gag gaa att gtg cag gcc ggc tat gat cat acg aag cag ctt gtc cgt       773
Glu Glu Ile Val Gln Ala Gly Tyr Asp His Thr Lys Gln Leu Val Arg
                80                  85                  90

ttc cgc tgg gat cgt ccg gtg gat ttt tca ttg ctt gag aaa atg atc       821
Phe Arg Trp Asp Arg Pro Val Asp Phe Ser Leu Leu Glu Lys Met Ile
            95                  100                 105

gag ttt aat att gcg gat aag gcg gac tgc tcg act ttt tgg cgg aaa       869
Glu Phe Asn Ile Ala Asp Lys Ala Asp Cys Ser Thr Phe Trp Arg Lys
        110                 115                 120

taaaaaagcg caaaaataag gggcatcctc tcatgaaggc gagagagcgc cccgaaagaa     929

aaacgctgtt tatctaaaac tccaaggttt cgcgcatgta cgcggcaaac ctacccgtta     989

tgatgcttat acatccaaag caagccggat ttcagcaggc gaggcactct gccgatcaac    1049

ggccgttcag caactaaacc gaaaccggct tttttgccga gcgacccgag gacgcctttc 1109

agcttgaatt tcggcattgt ttcaggaaga ggttcgcctt tccagcgcag ttcaagcact 1169

tggacgattt gttccgcctg ggcttcggcc agctgtgcgc tcggcgcgtg aggcaggctt 1229

gcacagtcgc cgacaacata aacgtgttcg tctcccggca ggttatggtg cggcgtcagg 1289

acgacgcgcc cttgcggatc tttttccaca tcgagat 1326

<210> 82
<211> 123
<212> PRT
<213> Bacillus licheniformis

<400> 82

Met Glu Val Phe Glu Glu Tyr Leu Ala Gly Ile Asp His Pro Glu His
1               5                   10                  15

Arg Ala Arg Met Glu Glu Val Leu Gly Trp Val Ala Glu Thr Phe Pro
            20                  25                  30

Asn Leu Thr Pro Lys Ile Ala Trp Asn Gln Pro Met Phe Thr Asp His
        35                  40                  45

Gly Thr Phe Ile Ile Gly Phe Ser Val Ala Lys Asn His Leu Ala Val
    50                  55                  60

Ala Pro Glu Ser Ala Gly Ile Asp Arg Phe Ser Glu Glu Ile Val Gln
65                  70                  75                  80

Ala Gly Tyr Asp His Thr Lys Gln Leu Val Arg Phe Arg Trp Asp Arg
                85                  90                  95

Pro Val Asp Phe Ser Leu Leu Glu Lys Met Ile Glu Phe Asn Ile Ala
            100                 105                 110

Asp Lys Ala Asp Cys Ser Thr Phe Trp Arg Lys
        115                 120

<210> 83
<211> 1891
<212> DNA
<213> Bacillus licheniformis

<220>
<221> CDS
<222> (501)..(1391)

<400> 83
gtacaatgag aaggatgacg gcttggtcga tggaatattt tttgccgagt tccggatgcc 60

ccatcaaatc ttttatatcg cgtttgatcc agttttggat ggctgtcgat tttaaggatg 120

tcagttcgca caaatttcca agttcaacaa tttcatttgt ggaaagtccg tgttccgttt 180

tttgtttttt ccgttcgtac cgggcaatga attcggggat gtccaaattg tcttccggct 240

catcgggcgc caaatcggcc gatctcaaaa gaatcgctag cggactgaca tccagctgcc 300

cttttaaagc gtacatcagt ttggccattt ccaggcgagt cagtttaaaa ggtttcatca 360

ttccacctca ttatgtatta aaatgaaata gcaaacaata taaacattat cttacgtctt 420

atcacttgca ttttcaattc tatgccgtat aatttttatt atacgagttc atttgatctt 480

attataacat ggaggtaatc atg gga aaa aga atc ttt ctc ttt ctt tta tcg 533
Met Gly Lys Arg Ile Phe Leu Phe Leu Leu Ser
1               5                   10

aac atc tta gtc att acg acg atc ggt atc gtg ctt tcg att atc ggt 581
Asn Ile Leu Val Ile Thr Thr Ile Gly Ile Val Leu Ser Ile Ile Gly
            15                  20                  25

tcg ctg aca ggg gta ggc acg tat ttc acg gct aac ggc ggc atc gat 629
Ser Leu Thr Gly Val Gly Thr Tyr Phe Thr Ala Asn Gly Gly Ile Asp
        30                  35                  40

att gtt gcg ctt ctc gta ttt agt gca gtt gtc ggt ttt gtc ggc tcc 677
Ile Val Ala Leu Leu Val Phe Ser Ala Val Val Gly Phe Val Gly Ser
    45                  50                  55

ttt atg tcg ctc ttg atg tca aga tgg atg gcg aaa atg gcg atg ggc 725
Phe Met Ser Leu Leu Met Ser Arg Trp Met Ala Lys Met Ala Met Gly
60                  65                  70                  75

gtt cag gtg ctg aat cct gat aag caa acg ctc agc tat gaa gaa cag 773
Val Gln Val Leu Asn Pro Asp Lys Gln Thr Leu Ser Tyr Glu Glu Gln
                80                  85                  90

cag ctc gtt gac cgg gta tat aag ctg tcc cgc gct gcc gga ctg aca 821
Gln Leu Val Asp Arg Val Tyr Lys Leu Ser Arg Ala Ala Gly Leu Thr
            95                  100                 105

aag atg cct gaa gtc ggc atc tac aat tca cgc gaa gtg aat gcc ttt 869
Lys Met Pro Glu Val Gly Ile Tyr Asn Ser Arg Glu Val Asn Ala Phe
        110                 115                 120

gcg acc ggg ccg tct aaa aac cgc tcg ctt gtc gct gta tcg acg ggc 917
Ala Thr Gly Pro Ser Lys Asn Arg Ser Leu Val Ala Val Ser Thr Gly
    125                 130                 135

ctt ctt cag gaa atg gat gac gac gca gtg gaa ggc gta ctt gcc cac 965
Leu Leu Gln Glu Met Asp Asp Asp Ala Val Glu Gly Val Leu Ala His
140                 145                 150                 155

gaa gtg gcg cat att gca aac ggc gat atg gtg acg atg acg ctc ctt 1013
Glu Val Ala His Ile Ala Asn Gly Asp Met Val Thr Met Thr Leu Leu
                160                 165                 170

caa gga atc gtc aat acg ttc gtt gtt ttc ttc gca agg att gcg gca 1061
Gln Gly Ile Val Asn Thr Phe Val Val Phe Phe Ala Arg Ile Ala Ala
            175                 180                 185

tgg gct gtt tca cgc gtt gtc cgt gaa gag ctg gcg ccg atc gtt cac 1109
Trp Ala Val Ser Arg Val Val Arg Glu Glu Leu Ala Pro Ile Val His
        190                 195                 200

ttt atc gcg gtc atc gta ttc caa att gtg ttc tct att ctc gga agc 1157
Phe Ile Ala Val Ile Val Phe Gln Ile Val Phe Ser Ile Leu Gly Ser
    205                 210                 215

ctt gtc gtg ttt gcg tac tcc cgc cac cgc gaa tat cat gcg gat aga 1205
Leu Val Val Phe Ala Tyr Ser Arg His Arg Glu Tyr His Ala Asp Arg
220                 225                 230                 235

gga gga gca gac ctt gca ggc aag gat aag atg att cac gcg ctc cgc 1253
Gly Gly Ala Asp Leu Ala Gly Lys Asp Lys Met Ile His Ala Leu Arg
                240                 245                 250

tcg ctt gag cag tac act tca aga gtc aaa gaa gaa cag gca tct gtt 1301
Ser Leu Glu Gln Tyr Thr Ser Arg Val Lys Glu Glu Gln Ala Ser Val
            255                 260                 265

gca act tta aaa atc aac ggg aaa aag cat aca tcg ctt ttc tca aca 1349
Ala Thr Leu Lys Ile Asn Gly Lys Lys His Thr Ser Leu Phe Ser Thr
        270                 275                 280

cac cct gat tta agc gac aga atc cgc cgt ctg gaa gca aaa 1391
His Pro Asp Leu Ser Asp Arg Ile Arg Arg Leu Glu Ala Lys
    285                 290                 295

taataaagat gcaaaccccc tttttcaagg gggtttgctt tttaaattta aaaatcgaaa 1451

agcggctgta cataatagca tgtttccttt aaaatggaac acgttataca caagaagcaa 1511

cttttgaata ttgaaaagaa ggaacgatta tgaaggtttt attgagaaaa ctactgcgcg 1571

tgctgtctcc tgtacagctg atcgcgctct attatttttt agcggtgaca gtttcggttg 1631

ttttattgag tctgcccgtt gcacacaaaa ataatgttga atggtctttt atcgacgccc 1691

tttttaccgc ggtgagcgca gtgagtgtaa ccggtttgac ggttgttgat acagccgata 1751

cattcagcac ggccggaatt tggattttgg cgtttgttct gcaatttggc gggatcggcg 1811

tgatggcttt gggtacattc gtctggctga ttttcggcaa gcggatcggt ttgaaagagc 1871

gccgtctgat tatgaccgat 1891

<210> 84
<211> 297
<212> PRT
<213> Bacillus licheniformis

<400> 84

Met Gly Lys Arg Ile Phe Leu Phe Leu Leu Ser Asn Ile Leu Val Ile
1               5                   10                  15

Thr Thr Ile Gly Ile Val Leu Ser Ile Ile Gly Ser Leu Thr Gly Val
            20                  25                  30

Gly Thr Tyr Phe Thr Ala Asn Gly Gly Ile Asp Ile Val Ala Leu Leu
        35                  40                  45

Val Phe Ser Ala Val Val Gly Phe Val Gly Ser Phe Met Ser Leu Leu
    50                  55                  60

Met Ser Arg Trp Met Ala Lys Met Ala Met Gly Val Gln Val Leu Asn
65                  70                  75                  80

Pro Asp Lys Gln Thr Leu Ser Tyr Glu Glu Gln Gln Leu Val Asp Arg
                85                  90                  95

Val Tyr Lys Leu Ser Arg Ala Ala Gly Leu Thr Lys Met Pro Glu Val

            100                 105                 110

Gly Ile Tyr Asn Ser Arg Glu Val Asn Ala Phe Ala Thr Gly Pro Ser
        115                 120                 125

Lys Asn Arg Ser Leu Val Ala Val Ser Thr Gly Leu Leu Gln Glu Met
    130                 135                 140

Asp Asp Asp Ala Val Glu Gly Val Leu Ala His Glu Val Ala His Ile
145                 150                 155                 160

Ala Asn Gly Asp Met Val Thr Met Thr Leu Leu Gln Gly Ile Val Asn
                165                 170                 175

Thr Phe Val Val Phe Phe Ala Arg Ile Ala Ala Trp Ala Val Ser Arg
            180                 185                 190

Val Val Arg Glu Glu Leu Ala Pro Ile Val His Phe Ile Ala Val Ile
        195                 200                 205

Val Phe Gln Ile Val Phe Ser Ile Leu Gly Ser Leu Val Val Phe Ala
    210                 215                 220

Tyr Ser Arg His Arg Glu Tyr His Ala Asp Arg Gly Gly Ala Asp Leu
225                 230                 235                 240

Ala Gly Lys Asp Lys Met Ile His Ala Leu Arg Ser Leu Glu Gln Tyr
                245                 250                 255

Thr Ser Arg Val Lys Glu Glu Gln Ala Ser Val Ala Thr Leu Lys Ile
            260                 265                 270

Asn Gly Lys Lys His Thr Ser Leu Phe Ser Thr His Pro Asp Leu Ser
        275                 280                 285

Asp Arg Ile Arg Arg Leu Glu Ala Lys
    290                 295